# EUROPÄISCHE PATENTSCHRIFT

(11) **EP 1 797 110 B1**
(45) Veröffentlichungstag und Bekanntmachung des Hinweises auf die Patenterteilung: **12.01.2011**
(21) Anmeldenummer: 05782906.1
(22) Anmeldetag: 15.09.2005
(51) Int. Cl.: C07K 5/08, A61K 38/06

(54) **ANTIBAKTERIELLE AMID-MAKROZYKLEN IV**
ANTIBACTERIAL AMIDE MACROCYCLES IV
MACROCYCLES IV D'AMIDE ANTIBACTERIENS

(30) Priorität: 24.09.2004 DE 102004046307; 30.03.2005 DE 102005014240
(43) Veröffentlichungstag der Anmeldung: 20.06.2007
(73) Patentinhaber: AiCuris GmbH & Co. KG, 42117 Wuppertal (DE)
(72) Erfinder: ENDERMANN, Rainer, 42113 Wuppertal (DE); EHLERT, Kerstin, 42553 Velbert (DE); FREIBERG, Christoph, 42115 Wuppertal (DE); RADDATZ, Siegfried, 51065 Köln (DE); MICHELS, Martin, West Haven, 06516-4140 (US); CANCHO-GRANDE, Yolanda, 51373 Leverkusen (DE); SCHUHMACHER, Joachim, 42113 Wuppertal (DE); WEIGAND, Stefan, 82377 Penzberg (DE)
(74) Vertreter: Weller, Wolfgang
(86) Internationale Anmeldenummer: PCT/EP2005/009912
(87) Internationale Veröffentlichungsnummer: WO 2006/034786

(56) Entgegenhaltungen:
- WO-A-03/106480
- WO-A-2005/033129

## Beschreibung

Die Erfindung betrifft antibakterielle Amid-Makrozyklen und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von bakteriellen Infektionen.

In WO 03/106480 und WO 04/012816 werden antibakteriell wirkende Makrozyklen vom Biphenomycin B Typ mit Amid- bzw. Estersubstituenten beschrieben.

In US 3,452,136, Dissertation R. U. Meyer, Universität Stuttgart, Deutschland 1991, Dissertation V. Leitenberger, Universität Stuttgart, Deutschland 1991, Synthesis (1992), (10), 1025-30, J. Chem. Soc., Perkin Trans. 1 (1992), (1), 123-30, J. Chem. Soc., Chem. Commun. (1991); (10), 744, Synthesis (1991), (5), 409-13, J. Chem. Soc., Chem. Commun. (1991), (5), 275-7, J. Antibiot. (1985), 38(11), 1462-8, J. Antibiot. (1985), 38(11), 1453-61, wird der NaturstoffBiphenomycin B als antibakteriell wirksam beschrieben. Teilschritte der Synthese von Biphenomycin B werden in Synlett (2003), 4, 522-526 beschrieben.

Chirality (1995), 7(4), 181-92, J. Antibiot. (1991), 44(6), 674-7, J. Am. Chem. Soc. (1989), 111(19), 7323-7, J. Am. Chem. Soc. (1989), 111(19), 7328-33, J. Org. Chem. (1987), 52(24), 5435-7, Anal. Biochem. (1987), 165(1), 108-13, J. Org. Chem. (1985), 50(8), 1341-2, J. Antibiot. (1993), 46(3), C-2, J. Antibiot. (1993), 46(1), 135-40, Synthesis (1992), (12), 1248-54, Appl. Environ. Microbiol. (1992), 58(12), 3879-8, J. Chem. Soc., Chem. Commun. (1992), (13), 951-3 beschreiben einen strukturell verwandten Naturstoff, Biphenomycin A, der am Makrozyklus eine weitere Substitution mit einer Hydroxygruppe aufweist.

Die Naturstoffe entsprechen hinsichtlich ihrer Eigenschaften nicht den Anforderungen, die an antibakterielle Arzneimittel gestellt werden. Auf dem Markt sind zwar strukturell andersartige antibakteriell wirkende Mittel vorhanden, es kann aber regelmäßig zu einer Resistenzentwicklung kommen. Neue Mittel für eine gute und wirksamere Therapie sind daher wünschenswert.

Eine Aufgabe der vorliegenden Erfindung ist es daher, neue und alternative Verbindungen mit gleicher oder verbesserter antibakterieller Wirkung zur Behandlung von bakteriellen Erkrankungen bei Menschen und Tieren zur Verfügung zu stellen.

Überraschenderweise wurde gefunden, dass bestimmte Derivate dieser Naturstoffe, worin die Carboxylgruppe des Naturstoffs gegen eine Amidgruppe ausgetauscht wird, die eine basische Gruppe enthält, gegen Biphenomycin resistente S. *aureus* Stämme (RN4220BiR und T17) antibakteriell wirksam sind.

weiterhin zeigen die Derivate gegen S. *aureus* Wildtyp-Stämme und Biphenomycin resistente S. *aureus* Stämme eine verbesserte Spontanresistenz-Frequenz.

Gegenstand der Erfindung sind Verbindungen der Formel 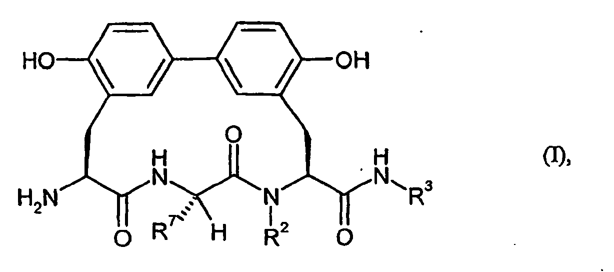 bei denen
- R⁷: gleich eine Gruppe der Formel 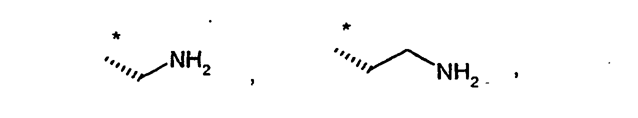 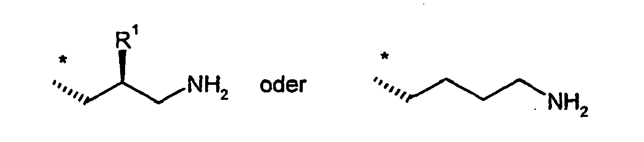
ist,
wobei
- R¹: gleich Wasserstoff oder Hydroxy ist,
- *: die Anknüpfstelle an das Kohlenstoffatom ist,
- R²: gleich Wasserstoff, Methyl oder Ethyl ist,
- R³: gleich eine Gruppe der Formel 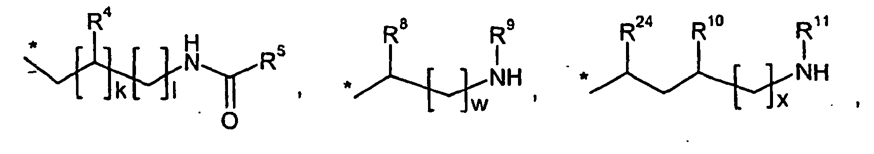 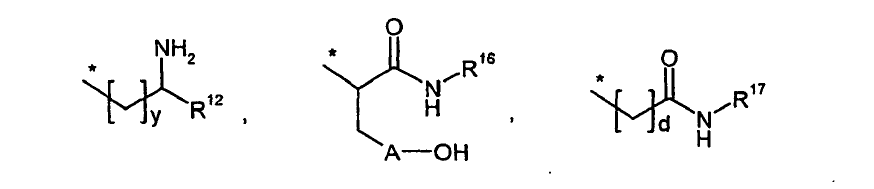
oder 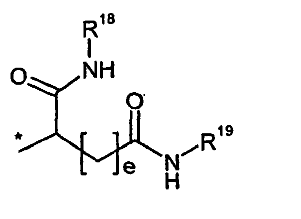 ist,
wobei
- *: die Anknüpfstelle an das Stickstoffatom ist,
- A: gleich eine Bindung oder Phenyl ist,
- R⁴: gleich Wasserstoff, Amino oder Hydroxy ist,
- R⁵: eine Gruppe der Formel 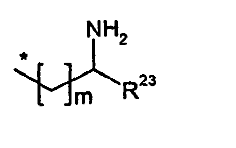
ist,
worin
- *: die Anknüpfstelle an das Kohlenstoffatom ist,
- R²³: Wasserstoff oder eine Gruppe der Formel *-(CH₂)ₙ-OH oder *-(CH₂)ₒ- NH₂ ist,
worin
* die Anknüpfstelle an das Kohlenstoffatom ist,
n und o unabhängig voneinander eine Zahl 1, 2, 3 oder 4 sind,
- m: eine Zahl 0 oder 1 ist,
- R⁸ und R¹²: unabhängig voneinander eine Gruppe der Formel *-CONHR ¹⁴ oder *-CH₂CONHR¹⁵ sind,
worin
- *: die Anknüpfstelle an das Kohlenstoffatom ist,
- R¹⁴ und R¹⁵: unabhängig voneinander eine Gruppe der Formel 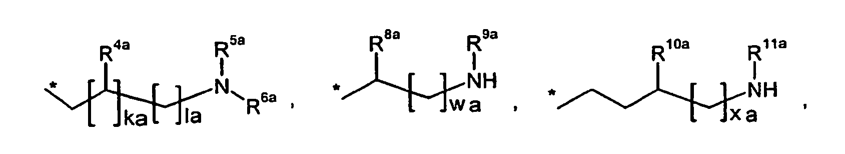 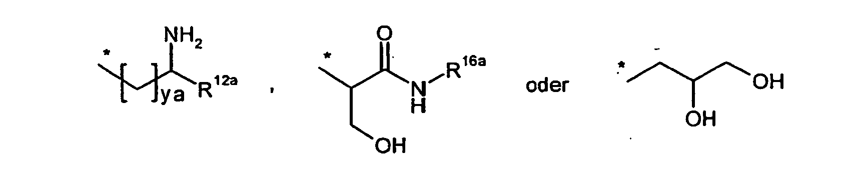
sind,
worin
- *: die Anknüpfstelle an das Stickstoffatom ist,
- R^{4a}: gleich Wasserstoff, Amino oder Hydroxy ist,
- R^{5a}: gleich Wasserstoff, Methyl oder Aminoethyl ist,
- R^{6a}: gleich Wasserstoff oder Aminoethyl ist,
oder
- R^{5a} und R^{6a}: bilden zusammen mit dem Stickstoffatom an das sie gebunden sind einen Piperazin-Ring,
- R^{8a} und R^{12a}: unabhängig voneinander *-(CH₂)_{Z1a}.-OH, *-(CH₂)_{Z2a}- NHR^{13a}, *-CONHR^{14a} oder *-CH₂CONHR^{15a} sind,
worin
- *: die Anknüpfstelle an das Kohlenstoffatom ist, Z1a und Z2a unabhängig voneinander eine Zahl 1, 2 oder 3 sind, R^{13a} gleich Wasserstoff oder Methyl ist
und
R^{14a} und R^{15a} unabhängig voneinander eine Gruppe der Formel 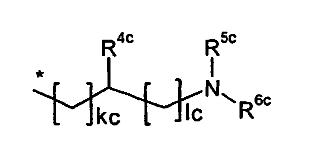
sind,
worin
- *: die Anknüpfstelle an das Stickstoffatom ist,
- R^{4c}: gleich Wasserstoff, Amino oder Hydroxy ist,
- R^{5c}: gleich Wasserstoff, Methyl oder Aminoethyl ist,
- R^{6c}: gleich Wasserstoff oder Aminoethyl ist,
- kc: eine Zahl 0 oder 1 ist
und
- 1c: eine Zahl 1, 2, 3 oder 4 ist,
- R^{9a} und R^{11a}: unabhängig voneinander Wasserstoff oder Methyl sind,
- R^{10a}: gleich Amino oder Hydroxy ist,
- R^{16a}: eine Gruppe der Formel 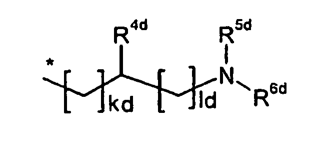
sind,
worin
- *: die Anknüpfstelle an das Stickstoffatom ist,
- R^{4d}: gleich Wasserstoff, Amino oder Hydroxy ist,
- R^{5d}: gleich Wasserstoff, Methyl oder Aminoethyl ist,
- R^{6d}: gleich Wasserstoff oder Aminoethyl ist,
- kd: eine Zahl 0 oder 1 ist
und
- ld: eine Zahl 1, 2, 3 oder 4 ist,
- ka: eine Zahl 0 oder 1 ist und
- la, wa, xa und ya: unabhängig voneinander eine Zahl 1, 2, 3 oder 4 sind,
- R⁹ und R¹¹: unabhängig voneinander Wasserstoff, Methyl, *-C(NH₂)=NH oder eine Gruppe der Formel 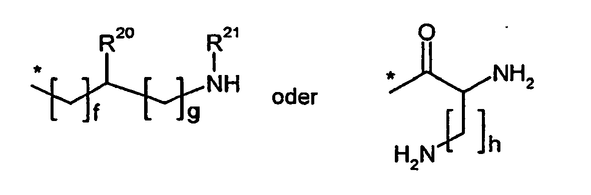
sind,
worin
- *: die Anknüpfstelle an das Stickstoffatom ist,
- R²⁰: gleich Wasserstoff oder *-(CH2)ᵢ-NHR²² ist,
worin
- R²²: gleich Wasserstoff oder Methyl ist
und
- i: eine Zahl 1, 2 oder 3 ist,
- R²¹: gleich Wasserstoff oder Methyl ist,
- f: eine Zahl 0, 1, 2 oder 3 ist,
- g: eine Zahl 1, 2 oder 3 ist und
- h: eine Zahl 1, 2, 3 oder 4 ist,
oder
- R⁸: gleich *-(CH₂)_{z1}-OH ist,
worin
- *: die Anknüpfstelle an das Kohlenstoffatom ist,
- ZI: eine Zahl 1, 2 oder 3 ist,
und
- R⁹: eine Gruppe der Formel 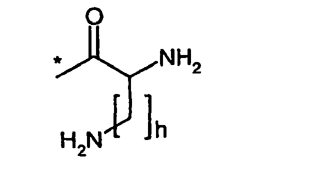
ist,
worin
- *: die Anknüpfstelle an das Stickstoffatom ist, und
- h: eine Zahl 1, 2, 3 oder 4 ist,
- R¹⁰: gleich Amino oder Hydroxy ist,
- R¹⁶ und R¹⁷: unabhängig voneinander eine Gruppe der Formel 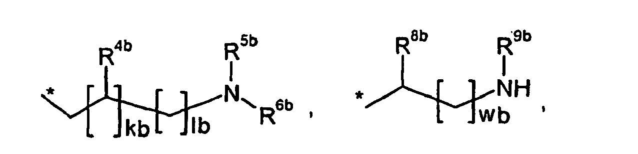 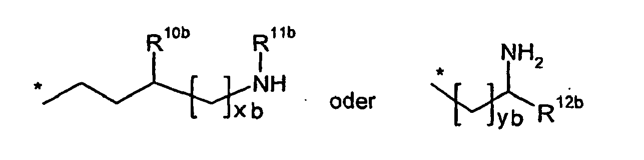
sind,
worin
- *: die Anknüpfstelle an das Stickstoffatom ist,
- R^{4b}: gleich Wasserstoff, Amino oder Hydroxy ist,
- R^{5b}: gleich Wasserstoff, Methyl oder Aminoethyl ist,
- R^{6b}: gleich Wasserstoff oder Aminoethyl ist,
oder
- R^{5b} und R^{6b}: bilden zusammen mit dem Stickstoffatom an das sie gebunden sind einen Piperazin-Ring,
- R^{8b} und R^{12b}: unabhängig voneinander *-(CH₂)_{Z1b}-OH, *-(CH₂)_{Z2b}-NHR^{13b}, *-CONHR^{14b} oder *-CH₂CONHR^{15b} sind, worin
- *: die Anknüpfstelle an das Kohlenstoffatom ist,
- R^{13b}: gleich Wasserstoff oder Methyl ist und
- Z1b und Z2b: unabhängig voneinander eine Zahl 1, 2 oder 3 sind, und
- R^{14b} und R^{15b}: unabhängig voneinander eine Gruppe der Formel 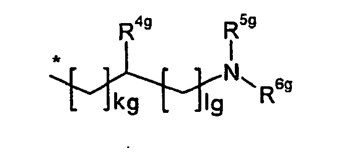
sind,
worin
- *: die Anknüpfstelle an das Stickstoffatom ist,
- R^{4g}: gleich Wasserstoff, Amino oder Hydroxy ist,
- R^{5g}: gleich Wasserstoff, Methyl oder Aminoethyl ist,
- R^{6g}: gleich Wasserstoff oder Aminoethyl ist,
- kg: eine Zahl 0. oder 1 ist
und
- 1g: eine Zahl 1, 2, 3 oder 4 ist,
- R^{9b} und R^{11b}: unabhängig voneinander Wasserstoff oder Methyl sind,
- R^{10b}: gleich Amino oder Hydroxy ist,
- kb: eine Zahl 0 oder 1 ist,
- 1b, wb, xb und yb: unabhängig voneinander eine Zahl 1, 2, 3 oder 4 sind,
- R¹⁸ und R¹⁹: unabhängig voneinander Wasserstoff oder eine Gruppe der Formel 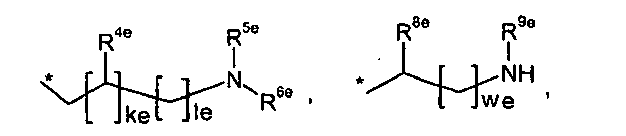 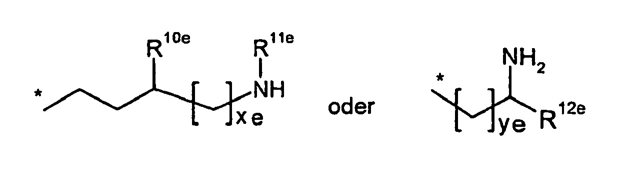
sind,
worin
- *: die Anknüpfstelle an das Stickstoffatom ist,
- R^{4e}: gleich Wasserstoff, Amino oder Hydroxy ist,
- R^{5e}: gleich Wasserstoff, Methyl oder Aminoethyl ist,
- R^{6e}: gleich Wasserstoff oder Aminoethyl ist, oder
- R^{5e} und R^{6e}: bilden zusammen mit dem Stickstoffatom an das sie gebunden sind einen Piperazin-Ring,
- R^{8e} und R^{12e}: unabhängig voneinander *-(CH₂)_{Z1e}-OH oder *-(CH₂)_{Z2e}-NHR^{13e} sind,
worin
- *: die Anknüpfstelle an das Kohlenstoffatom ist,
- R^{13e}: gleich Wasserstoff oder Methyl ist und
- Z1e und Z2e: unabhängig voneinander eine Zahl 1, 2 oder 3 sind,
- R^{9e} und R^{11e}: unabhängig voneinander Wasserstoff oder Methyl sind,
- R^{10e}: gleich Amino oder Hydroxy ist,
- ke: eine Zahl 0 oder 1 ist
und
- le, we, xe und ye: unabhängig voneinander eine Zahl 1, 2, 3 oder 4 sind,
- wobei R¹⁸ und R¹⁹: nicht gleichzeitig Wasserstoff sind,
- R²⁴: eine Gruppe der Formel *-CONHR²⁵ ist,
worin
- *: die Anknüpfstelle an das Kohlenstoffatom ist,
- R²⁵: eine Gruppe der Formel 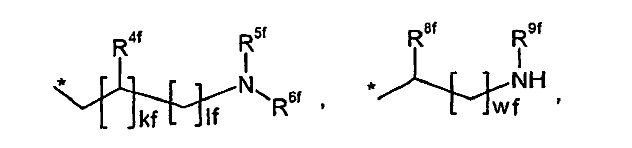 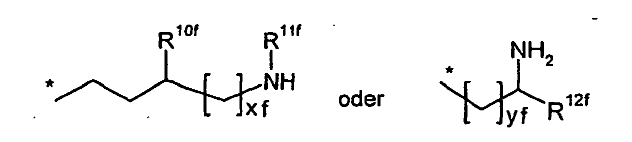
ist,
worin
- *: die Anknüpfstelle an das Stickstoffatom ist,
- R^{4f}: gleich Wasserstoff, Amino oder Hydroxy ist,
- R^{5f}: gleich Wasserstoff, Methyl oder Aminoethyl ist,
- R^{6f}: gleich Wasserstoff oder Aminoethyl ist, oder
- R^{5f} und R^{6f}: bilden zusammen mit dem Stickstoffatom an das sie gebunden sind einen Piperazin-Ring,
- R^{8f} und R^{12f}: unabhängig voneinander *-(CH₂)_{Z1f}-OH oder *-(CH₂)_{Z2f}- NHR^{13f} sind,
worin
- *: die Anknüpfstelle an das Kohlenstoffatom ist,
- R^{13f}: gleich Wasserstoff oder Methyl ist
und
- Z1f und Z2f: unabhängig voneinander eine Zahl 1, 2 oder 3 sind,
- R^{9f} und R^{11f}: unabhängig voneinander Wasserstoff oder Methyl sind,
- R^{10f}: gleich Amino oder Hydroxy ist,
- kf: eine Zahl 0 oder 1 ist
und
- lf, wf, xf und yf: unabhängig voneinander eine Zahl 1, 2, 3 oder 4 sind, d und e unabhängig voneinander eine Zahl 1, 2 oder 3 sind,
- k: eine Zahl 0 oder 1 ist,
- l, w, x und y: unabhängig voneinander eine Zahl 1, 2, 3 oder 4 sind, 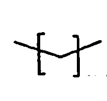
w, x oder y unabhängig voneinander bei w, x oder y gleich 3 eine Hydroxy- Gruppe tragen kann,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich durch bekannte Verfahren wie Chromatographie an chiraler Phase oder Kristallisation mit chiralen Aminen oder chiralen Säuren die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure, Trifluoressigsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dihydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Ein Symbol # an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung ein Enantiomerenüberschuss (enantiomeric excess) von mehr als 90% verstanden wird (> 90% ee).

In den Formeln der Gruppen, für die R³ stehen kann, steht der Endpunkt der Linie, neben der jeweils ein * steht, nicht für ein Kohlenstoffatom beziehungsweise eine CH₂-Gruppe sondern ist Bₑ-standteil der Bindung zu dem Stickstoffatom, an das R³ gebunden ist.

In den Formeln der Gruppen, für die R⁷ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Kohlenstoffatom, an das R⁷ gebunden ist.

Bevorzugt im Rahmen der vorliegenden Erfindung sind Verbindungen der Formel (I), bei denen,
- R⁷: gleich eine Gruppe der Formel 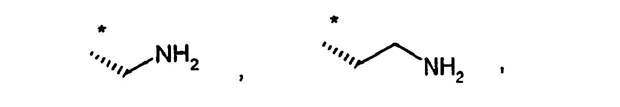 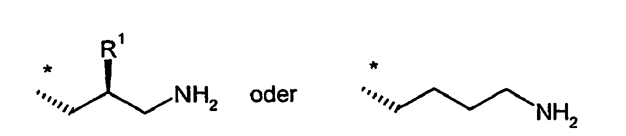
ist,
wobei
- R¹: gleich Wasserstoff oder Hydroxy ist,
- *: die Anknüpfstelle an das Kohlenstoffatom ist,

- R²: gleich Wasserstoff, Methyl oder Ethyl ist,
- R³: gleich eine Gruppe der Formel 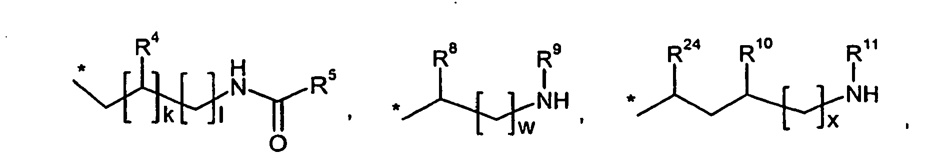 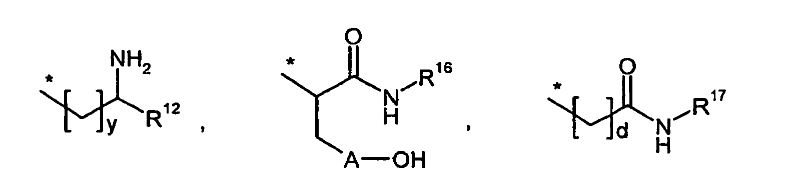
oder 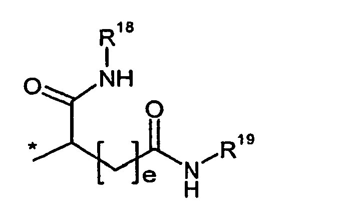 ist,
wobei
- *: die Anknüpfstelle an das Stickstoffatom ist,
- A: gleich eine Bindung oder Phenyl ist,
- R⁴: gleich Wasserstoff, Amino oder Hydroxy ist,
- R⁵: eine Gruppe der Formel 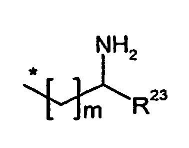
ist,
worin
- *: die Anknüpfstelle an das Kohlenstoffatom ist,
- R²³: Wasserstoff oder eine Gruppe der Formel *-(CH₂)ₙ-OH oder *-(CH₂)ₒ- NH₂ ist,
worin
- *: die Anknüpfstelle an das Kohlenstoffatom ist,
- n und o: unabhängig voneinander eine Zahl 1, 2, 3 oder 4 sind,
- m: eine Zahl 0 oder 1 ist,
- R⁸ und R¹²: unabhängig voneinander eine Gruppe der Formel *-CONHR¹⁴ oder *-CH₂CONHR¹⁵ sind, worin
- *: die Anknüpfstelle an das Kohlenstoffatom ist,
- R¹⁴ und R¹⁵: unabhängig voneinander eine Gruppe der Formel 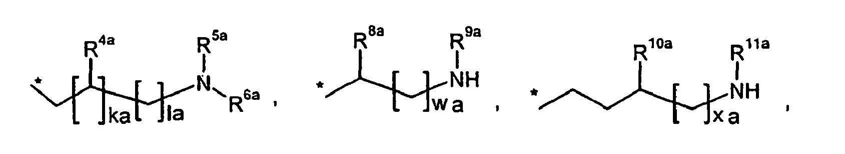 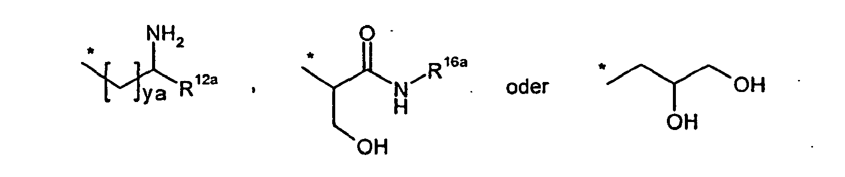
sind,
worin
- *: die Anknüpfstelle an das Stickstoffatom ist,
- R^{4a}: gleich Wasserstoff, Amino oder Hydroxy ist,
- R^{5a}: gleich Wasserstoff, Methyl oder Aminoethyl ist,
- R^{6a}: gleich Wasserstoff oder Aminoethyl ist,
oder
- R⁵⁸ und R^{6a}: bilden zusammen mit dem Stickstoffatom an das sie gebunden sind einen Piperazin-Ring,
- R^{8a} und R^{12a}: unabhängig voneinander *-(CH₂)_{Z1a}-OH, *-(CH₂)_{Z2a}- NHR^{13a}, *-CONHR^{14a} oder *-CH₂CONHR^{15a} sind, worin
- *: die Anknüpfstelle an das Kohlenstoffatom ist,
- Z1a und Z2a: unabhängig voneinander eine Zahl 1, 2 oder 3 sind,
- R^{38a}: gleich Wasserstoff oder Methyl ist und
- R^{14a} und R^{15a}: unabhängig voneinander eine Gruppe der Formel 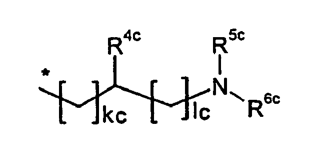
sind,
worin
- *: die Anknüpfstelle an das Stickstoffatom ist,
- R^{4c}: gleich Wasserstoff, Amino oder Hydroxy ist,
- R^{5c}: gleich Wasserstoff, Methyl oder Aminoethyl ist,
- R^{6c}: gleich Wasserstoff oder Aminoethyl ist,
- kc: eine Zahl 0 oder 1 ist und
- lc: eine Zahl 1, 2, 3 oder 4 ist,
- R^{9a} und R^{11a}: unabhängig voneinander Wasserstoff oder Methyl sind,
- R^{10a}: gleich Amino oder Hydroxy ist,
- R^{16a}: eine Gruppe der Formel 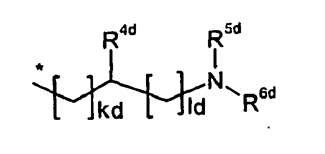
sind,
worin
- *: die Anknüpfstelle an das Stickstoffatom ist,
- R^{4d}: gleich Wasserstoff, Amino oder Hydroxy ist,
- R^{5d}: gleich Wasserstoff, Methyl oder Aminoethyl ist,
- R^{6d}: gleich Wasserstoff oder Aminoethyl ist,
- kd: eine Zahl 0 oder 1 ist und
- ld: eine Zahl 1, 2, 3 oder 4 ist,
- ka: eine Zahl 0 oder 1 ist
und
- la, wa, xa und ya: unabhängig voneinander eine Zahl 1, 2, 3 oder 4 sind,
- R⁹ und R¹¹: unabhängig voneinander Wasserstoff, Methyl, *-C(NH₂)=NH oder eine Gruppe der Formel 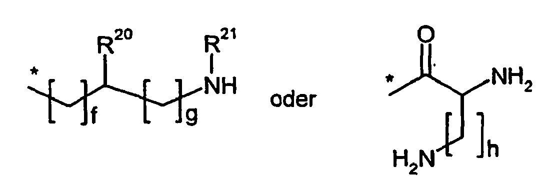
sind,
worin
- *: die Anknüpfstelle an das Stickstoffatom ist,
- R²⁰: gleich Wasserstoff oder *-(CH₂)ᵢ-NHR²² ist, worin
- R²²: gleich Wasserstoff oder Methyl ist
und
- i: eine Zahl 1, 2 oder 3 ist,
- R²¹: gleich Wasserstoff oder Methyl ist,
- f: eine Zahl 0, 1, 2 oder 3 ist,
- g: eine Zahl 1, 2 oder 3 ist
und
- h: eine Zahl 1, 2, 3 oder 4 ist,
oder
- R⁸: gleich *-(CH₂)_{z1}-OH ist,
worin
- *: die Anknüpfstelle an das Kohlenstoffatom ist,
- Z1: eine Zahl 1, 2 oder 3 ist,
und
- R⁹: eine Gruppe der Formel 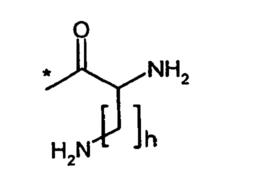
ist,
worin
- *: die Anknüpfstelle an das Stickstoffatom ist, und
- h: eine Zahl 1, 2, 3 oder 4 ist,
- R¹⁰: gleich Amino oder Hydroxy ist,
- R¹⁶ und R¹⁷: unabhängig voneinander eine Gruppe der Formel 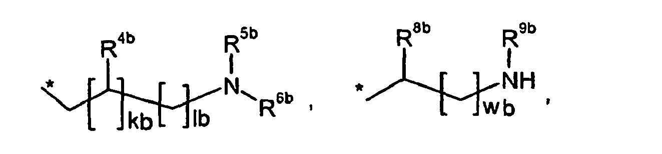 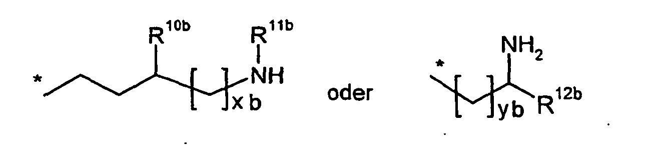
sind,
worin
- *: die Anknüpfstelle an das Stickstoffatom ist,
- R^{4b}: gleich Wasserstoff, Amino oder Hydroxy ist,
- R^{5b}: gleich Wasserstoff, Methyl oder Aminoethyl ist,
- R^{6b}: gleich Wasserstoff oder Aminoethyl ist,
oder
- R^{5b} und R^{6b}: bilden zusammen mit dem Stickstoffatom an das sie gebunden sind einen Piperazin-Ring,
- R^{8b} und R^{12b}: unabhängig voneinander *-(CH₂)_{Z1b}-OH oder *-CH2)_{Z2b}-NHR^{13b} sind,
worin
- *: die Anknüpfstelle an das Kohlenstoffatom ist,
- R^{13b}: gleich Wasserstoff oder Methyl ist
und
- Z1b und Z2b: unabhängig voneinander eine Zahl 1, 2 oder 3 sind,
- R^{9b} und R^{11b}: unabhängig voneinander Wasserstoff oder Methyl sind,
- R^{10b}: gleich Amino oder Hydroxy ist,
- kb: eine Zahl 0 oder 1 ist,
- lb, wb, xb und yb: unabhängig voneinander eine Zahl 1, 2, 3 oder 4 sind,
- R¹⁸ und R¹⁹: unabhängig voneinander Wasserstoff oder eine Gruppe der Formel 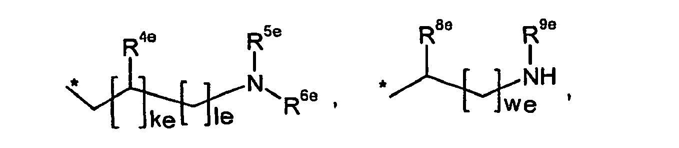 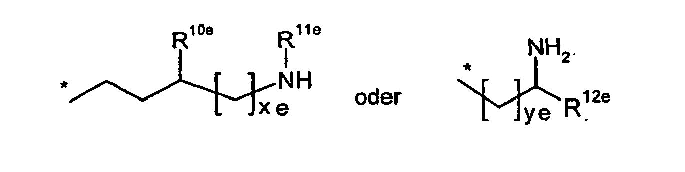
sind,
worin
- *: die Anknüpfstelle an das Stickstoffatom ist,
- R^{4c}: gleich Wasserstoff, Amino oder Hydroxy ist,
- R^{5c}: gleich Wasserstoff, Methyl oder Aminoethyl ist,
- R^{6e}: gleich Wasserstoff oder Aminoethyl ist,
oder
- R^{5e} und R^{6e}: bilden zusammen mit dem Stickstoffatom an das sie gebunden sind einen Piperazin-Ring,
- R^{8e} und R^{12e}: unabhängig voneinander *-(CH₂)_{Z1e}-OH oder *-(CH2)_{Z2e}-NHR^{13e} sind,
worin
- *: die Anknüpfstelle an das Kohlenstoffatom ist,
- R13^{e}: gleich Wasserstoff oder Methyl ist
und
- Z1e und Z2e: unabhängig voneinander eine Zahl 1, 2 oder 3 sind,
- R^{9e} und R^{11e}: unabhängig voneinander Wasserstoff oder Methyl sind,
- R^{10e}: gleich Amino oder Hydroxy ist,
- ke: eine Zahl 0 oder 1 ist
und
- le, we, xe und ye: unabhängig voneinander eine Zahl 1, 2, 3 oder 4 sind,
- wobei R¹⁸ und R¹⁹: nicht gleichzeitig Wasserstoff sind,
- R²⁴: eine Gruppe der Formel *-CONHR²⁵ ist,
worin
- *: die Anknüpfstelle an das Kohlenstoffatom ist,
- R²⁵: eine Gruppe der Formel 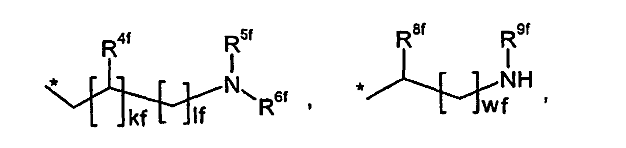 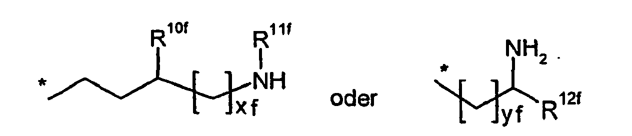
ist,
worin
- *: die Anknüpfstelle an das Stickstoffatom ist,
- R^{4f}: gleich Wasserstoff, Amino oder Hydroxy ist,
- R^{5f}: gleich Wasserstoff, Methyl oder Aminoethyl ist,
- R^{6f}: gleich Wasserstoff oder Aminoethyl ist,
oder
- R^{5f} und R^{6f}: bilden zusammen mit dem Stickstoffatom an das sie gebunden sind einen Piperazin-Ring,
- R^{8f} und R^{12f}: unabhängig voneinander *-(CH₂)_{Z1f}-OH oder *-(CH₂)_{Z2f}- NHR^{13f} sind,
worin
- *: die Anknüpfstelle an das Kohlenstoffatom ist,
- R^{13f}: gleich Wasserstoff oder Methyl ist
und
- Z1f und Z2f: unabhängig voneinander eine Zahl 1, 2 oder 3 sind,
- R^{9f} und R^{11f}: unabhängig voneinander Wasserstoff oder Methyl sind,
- R^{10f}: gleich Amino oder Hydroxy ist,
- kf: eine Zahl 0 oder 1 ist
und
- lf, wf, xf und yf: unabhängig voneinander eine Zahl 1, 2, 3 oder 4 sind,
- d und e: unabhängig voneinander eine Zahl 1, 2 oder 3 sind,
- k: eine Zahl 0 oder 1 ist,
- 1, w, x und y: unabhängig voneinander eine Zahl 1, 2, 3 oder 4 sind, 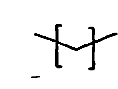
w, x oder y unabhängig voneinander bei w, x oder y gleich 3 eine Hydroxy-Gruppe tragen kann, und ihre Salze, ihre Solvate und die Solvate ihrer Salze.

Bevorzugt im Rahmen der vorliegenden Erfindung sind auch Verbindungen der Formel 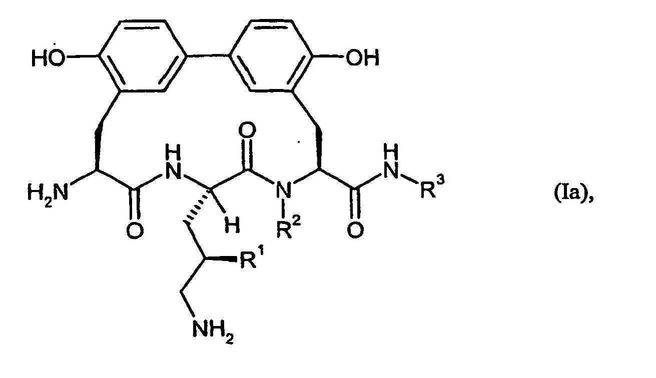 bei denen
- R¹: gleich Wasserstoff oder Hydroxy ist,
- R²: gleich Wasserstoff, Methyl oder Ethyl ist,
- R³: wie oben definiert ist, und ihre Salze, ihre Solvate und die Solvate ihrer Salze. Bevorzugt im Rahmen der vorliegenden Erfindung sind auch Verbindungen der Formel (I) oder (Ia), bei denen
- R³: gleich eine Gruppe der Formel 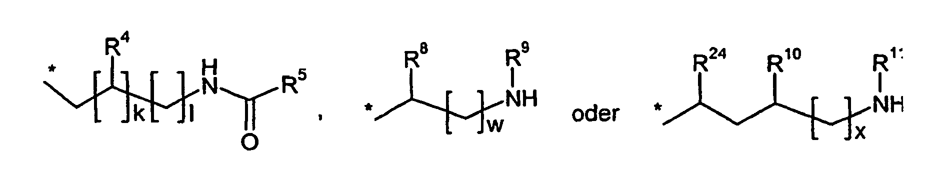
ist,
wobei
- *: die Anknüpfstelle an das Stickstoffatom ist,
- R⁴: gleich Wasserstoff, Amino oder Hydroxy ist,
- R⁵: eine Gruppe der Formel 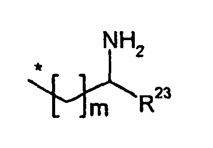
ist,
worin
- *: die Anknüpfstelle an das Kohlenstoffatom ist,
- R²³: Wasserstoff oder eine Gruppe der Formel *-(CH₂)ₙ-OH oder *-(CH₂)ₒ- NH₂ ist, worin
- *: die Anknüpfstelle an das Kohlenstoffatom ist,
- n und o: unabhängig voneinander eine Zahl 1, 2, 3 oder 4 sind,
- m: eine Zahl 0 oder 1 ist,
- R⁸: eine Gruppe der Formel *-CONHR¹⁴ oder *-CH₂CONHR¹⁵ ist, worin
- *: die Anknüpfstelle an das Kohlenstoffatom ist,
- R¹⁴ und R¹⁵: unabhängig voneinander eine Gruppe der Formel 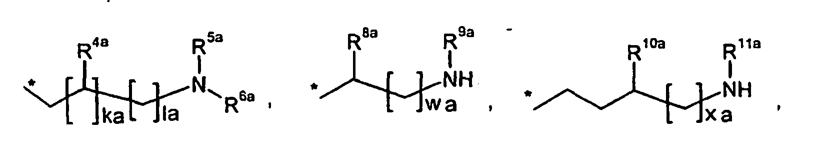 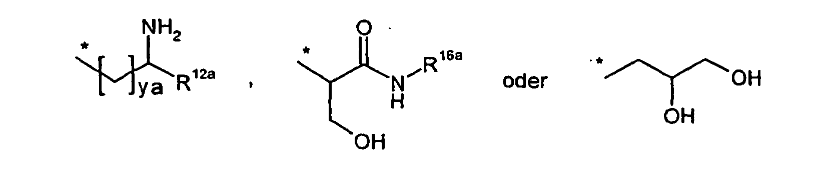
sind,
worin
- *: die Anknüpfstelle an das Stickstoffatom ist,
- R^{4a}: gleich Wasserstoff, Amino oder Hydroxy ist,
- R^{5a}: gleich Wasserstoff, Methyl oder Aminoethyl ist,
- R^{6a}: gleich Wasserstoff oder Aminoethyl ist,
oder
- R^{5a} und R^{6a}: bilden zusammen mit dem Stickstoffatom an das sie gebunden sind einen Piperazin-Ring,
- R^{8a} und R^{12a}: unabhängig voneinander *-(CH₂)_{Z1a}-OH, *-(CH₂)_{Z2a}- NHR^{13a}, *-CONHR^{14a} oder *-CH₂CONHR^{15a} sind, worin
- *: die Anknüpfstelle an das Kohlenstoffatom ist,
- Z1a und Z2a: unabhängig voneinander eine Zahl 1, 2 oder 3 sind,
- R^{13a}: gleich Wasserstoff oder Methyl ist
und
- R^{14a} und R^{15a}: unabhängig voneinander eine Gruppe der Formel 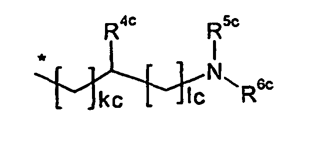
sind,
worin
- *: die Anknüpfstelle an das Stickstoffatom ist,
- R^{4c}: gleich Wasserstoff, Amino oder Hydroxy ist,
- R^{5c}: gleich Wasserstoff, Methyl oder Aminoethyl ist,
- R^{6c}: gleich Wasserstoff oder Aminoethyl ist,
- kc: eine Zahl 0 oder 1 ist
und
- lc: eine Zahl 1, 2, 3 oder 4 ist,
- R^{9a} und R^{11a}: unabhängig voneinander Wasserstoff oder Methyl sind,
- R^{10a}: gleich Amino oder Hydroxy ist,
- R^{16a}: eine Gruppe der Formel 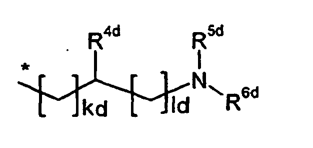
sind,
worin
- *: die Anknüpfstelle an das Stickstoffatom ist,
- R^{4d}: gleich Wasserstoff, Amino oder Hydroxy ist,
- R^{5d}: gleich Wasserstoff, Methyl oder Aminoethyl ist,
- R^{6d}: gleich Wasserstoff oder Aminoethyl ist,
- kd: eine Zahl 0 oder 1 ist
und
- ld: eine Zahl 1, 2, 3 oder 4 ist,
- ka: eine Zahl 0 oder 1 ist und
- la, wa, xa und ya: unabhängig voneinander eine Zahl 1, 2, 3 oder 4 sind,
- R⁹ und R¹¹: unabhängig voneinander Wasserstoff, Methyl, *-C(NH₂₎=NH oder eine Gruppe der Formel 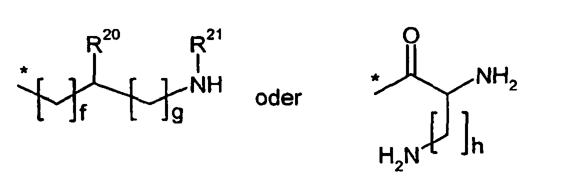
sind,
worin
- *: die Anknüpfstelle an das Stickstoffatom ist,
- R²⁰: gleich Wasserstoff oder *-(CH₂)ᵢ-NHR
²² ist, worin
- R²²: gleich Wasserstoff oder Methyl ist
und
- i: eine Zahl 1, 2 oder 3 ist,
- R²¹: gleich Wasserstoff oder Methyl ist,
- f: eine Zahl 0, 1, 2 oder 3 ist,
- g: eine Zahl 1, 2 oder 3 ist
und
- h: eine Zahl 1, 2, 3 oder 4 ist,
oder
- R⁸: gleich *-(CH₂)_{z1}-OH ist,
worin
- *: die Anknüpfstelle an das Kohlenstoffatom ist,
- Z1: eine Zahl 1, 2 oder 3 ist,
und
- R⁹: eine Gruppe der Formel 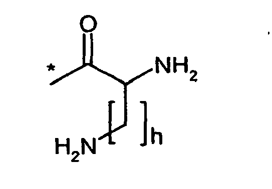
ist,
worin
- *: die Anknüpfstelle an das Stickstoffatom ist,
und
- h: eine Zahl 1, 2, 3 oder 4 ist,
- R¹⁰: gleich Amino oder Hydroxy ist,
- R²⁴: eine Gruppe der Formel *-CONHR²⁵ ist,
worin
- *: die Anknüpfstelle an das Kohlenstoffatom ist,
- R²⁵: eine Gruppe der Formel 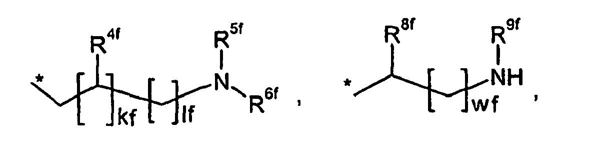 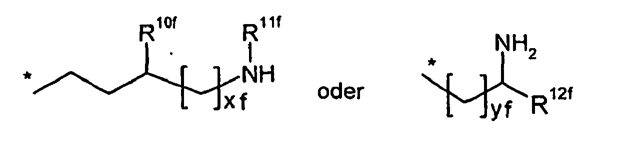
ist,
worin
- *: die Anknüpfstelle an das Stickstoffatom ist,
- R^{4f}: gleich Wasserstoff, Amino oder Hydroxy ist,
- R^{5f}: gleich Wasserstoff, Methyl oder Aminoethyl ist,
- R^{6f}: gleich Wasserstoff oder Aminoethyl ist,

oder
- R^{5f} und R^{6f}: bilden zusammen mit dem Stickstoffatom an das sie gebunden sind einen Piperazin-Ring,
- R^{8f} und R^{12f}: unabhängig voneinander *-(CH₂)_{Z1f}-OH oder *-(CH₂)_{Z2f}- NHR^{13f} sind,
worin
- *: die Anknüpfstelle an das Kohlenstoffatom ist,
- R^{13f}: gleich Wasserstoff oder Methyl ist
und
- Z1f und Z2f: unabhängig voneinander eine Zahl 1, 2 oder 3 sind,
- R^{9f} und R^{11f}: unabhängig voneinander Wasserstoff oder Methyl sind,
- R^{10f}: gleich Amino oder Hydroxy ist,
- kf: eine Zahl 0 oder 1 ist
und
- lf, wf, xf und yf: unabhängig voneinander eine Zahl 1, 2, 3 oder 4 sind,
- k: eine Zahl 0 oder 1 ist,
- l, w und x: unabhängig voneinander eine Zahl 1, 2, 3 oder 4 sind, 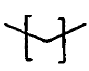
w oder x unabhängig voneinander bei w oder x gleich 3 eine Hydroxy-Gruppe tragen kann,
und ihre Salze, ihre Solvate und die Solvate ihrer Salze.

Besonders bevorzugt im Rahmen der vorliegenden Erfindung sind Verbindungen der Formel (I) oder (Ia), bei denen
- R³: gleich eine Gruppe der Formel 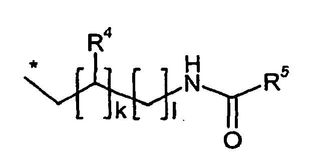
ist,
wobei
- *: die Anknüpfstelle an das Stickstoffatom ist,
- R⁴: gleich Wasserstoff, Amino oder Hydroxy ist;
- R⁵: eine Gruppe der Formel 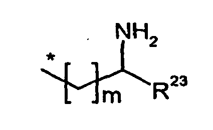
ist,
worin
- *: die Anknüpfstelle an das Kohlenstoffatom ist,
- R²³: Wasserstoff oder eine Gruppe der Formel *-(CH₂)ₙ-OH oder *-(CH₂)ₒ- NH₂ ist,
worin
- *: die Anknüpfstelle an das Kohlenstoffatom ist, n und o unabhängig voneinander eine Zahl 1, 2, 3 oder 4 sind,
- m: eine Zahl 0 oder 1 ist,
- k: eine Zahl 0 oder 1 ist,
- 1: eine Zahl 1, 2, 3 oder 4 ist, und ihre Salze, ihre Solvate und die Solvate ihrer Salze. Besonders bevorzugt im Rahmen der vorliegenden Erfindung sind auch Verbindungen der Formel (I) oder (Ia), bei denen
- R³: gleich eine Gruppe der Formel

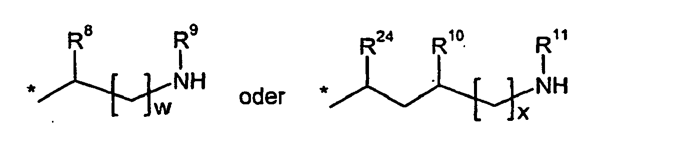

ist,
wobei
- *: die Anknüpfstelle an das Stickstoffatom ist,
- R⁸: eine Gruppe der Formel *-CONHR¹⁴ oder *-CH₂CONHR¹⁵ ist,
worin
- *: die Anknüpfstelle an das Kohlenstoffatom ist,
- R¹⁴ und R¹⁵: unabhängig voneinander eine Gruppe der Formel 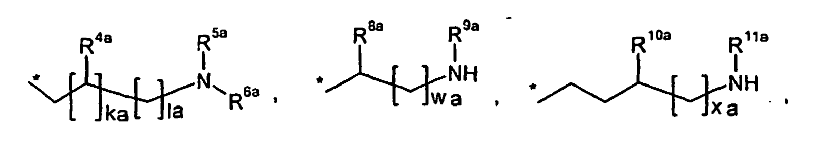 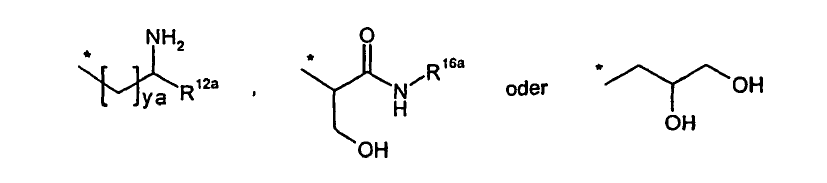
sind,
worin
- *: die Anknüpfstelle an das Stickstoffatom ist,
- R^{4a}: gleich Wasserstoff, Amino oder Hydroxy ist,
- R^{5a}: gleich Wasserstoff, Methyl oder Aminoethyl ist,
- R^{6a}: gleich Wasserstoff oder Aminoethyl ist,
oder
- R^{5a} und R^{6a}: bilden zusammen mit dem Stickstoffatom an das sie gebunden sind einen Piperazin-Ring,
- R^{8a} und R^{12a}: unabhängig voneinander *-(CH₂)_{Z1a}-OH, *-(CH₂)_{Z2a}- NHR^{13a}, *-CONHR^{14a} oder *-CH₂CONHR^{15a} sind,
worin
- *: die Anknüpfstelle an das Kohlenstoffatom ist,
- Z1a und Z2a: unabhängig voneinander eine Zahl 1, 2 oder 3 sind,
- R^{13a}: gleich Wasserstoff oder Methyl ist
und
- R^{14a} und R^{15a}: unabhängig voneinander eine Gruppe der Formel 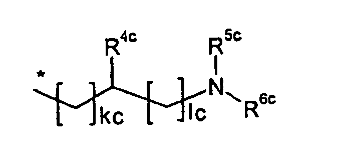
sind,
worin
- *: die Anknüpfstelle an das Stickstoffatom ist,
- R^{4c}: gleich Wasserstoff, Amino oder Hydroxy ist,
- R^{5c}: gleich Wasserstoff, Methyl oder Aminoethyl ist,
- R^{6c}: gleich Wasserstoff oder Aminoethyl ist,
- kc: eine Zahl 0 oder 1 ist
und
- lc: eine Zahl 1, 2, 3 oder 4 ist,
- R^{9a} und R^{11a}: unabhängig voneinander Wasserstoff oder Methyl sind,
- R^{10a}: gleich Amino oder Hydroxy ist,
- R^{16a}: eine Gruppe der Formel 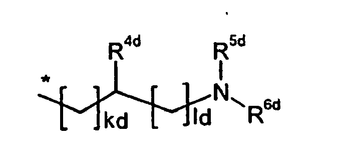
sind,
worin
- *: die Anknüpfstelle an das Stickstoffatom ist,
- R^{4d}: gleich Wasserstoff, Amino oder Hydroxy ist,
- R^{5d}: gleich Wasserstoff, Methyl oder Aminoethyl ist,
- R^{6d}: gleich Wasserstoff oder Aminoethyl ist,
- kd: eine Zahl 0 oder 1 ist
und
- ld: eine Zahl 1, 2, 3 oder 4 ist,
- ka: eine Zahl 0 oder 1 ist
und
- la, wa, xa und ya: unabhängig voneinander eine Zahl 1, 2, 3 oder 4 sind,
- R⁹ und R¹¹: unabhängig voneinander Wasserstoff, Methyl, *-C(NH₂)=NH oder eine Gruppe der Formel 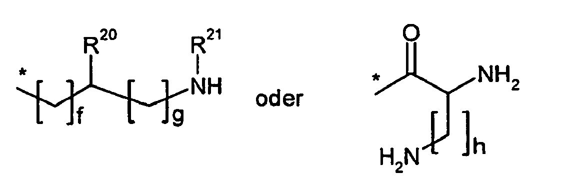
sind,
worin
- *: die Anknüpfstelle an das Stickstoffatom ist,
- R²⁰: gleich Wasserstoff oder *-(CH₂)ᵢ-NHR
²² ist, worin
- R²²: gleich Wasserstoff oder Methyl ist
und
- i: eine Zahl 1, 2 oder 3 ist,
- R²¹: gleich Wasserstoff oder Methyl ist,
- f: eine Zahl 0, 1, 2 oder 3 ist,
- g: eine Zahl 1, 2 oder 3 ist
und
- h: eine Zahl 1, 2, 3 oder 4 ist,
oder
- R⁸: gleich *-(CH₂)_{Z1}-OH ist,
worin
- *: die Anknüpfstelle an das Kohlenstoffatom ist,
- Z1: eine Zahl 1, 2 oder 3 ist,
und
- R⁹: eine Gruppe der Formel 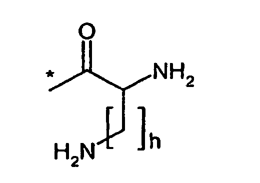
ist,
worin
- *: die Anknüpfstelle an das Stickstoffatom ist,
und
- h: eine Zahl 1, 2, 3 oder 4 ist,
- R¹⁰: gleich Amino oder Hydroxy ist,
- R²⁴: eine Gruppe der Formel *-CONHR²⁵ ist,
worin
- *: die Anknüpfstelle an das Kohlenstoffatom ist,
- R²⁵: eine Gruppe der Formel 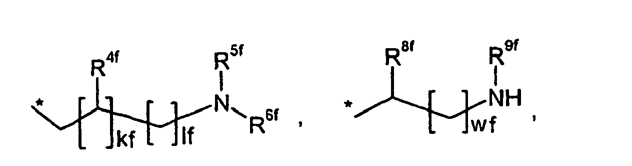 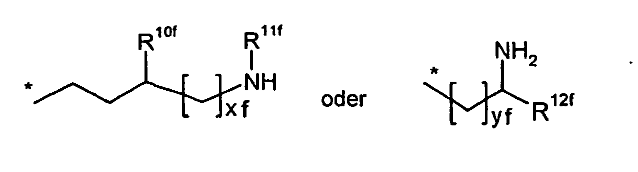
ist,
worin
- *: die Anknüpfstelle an das Stickstoffatom ist,
- R^{4f}: gleich Wasserstoff, Amino oder Hydroxy ist,
- R^{5f}: gleich Wasserstoff, Methyl oder Aminoethyl ist,
- R^{6f}: gleich Wasserstoff oder Aminoethyl ist,
oder
- R^{5f}und R^{6f}: bilden zusammen mit dem Stickstoffatom an das sie gebunden sind einen Piperazin-Ring,
- R^{8f} und R^{12f}: unabhängig voneinander *-(CH₂)_{Z1f}-OH oder *-(CH₂)_{Z2f}- NHR^{13f} sind,
worin
- *: die Anknüpfstelle an das Kohlenstoffatom ist,
- R^{13f} und: gleich Wasserstoff oder Methyl ist
- Z1f und Z2f: unabhängig voneinander eine Zahl 1, 2 oder 3 sind,
- R^{9f} und R^{11f}: unabhängig voneinander Wasserstoff oder Methyl sind,
- R^{10f}: gleich Amino oder Hydroxy ist,
- kf und: eine Zahl 0 oder 1 ist
- lf, wf, xf und yf: unabhängig voneinander eine Zahl 1, 2, 3 oder 4 sind,
- w und x: unabhängig voneinander eine Zahl 1, 2, 3 oder 4 sind, 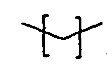
w oder x unabhängig voneinander bei w oder x gleich 3 eine Hydroxy-Gruppe tragen kann,
und ihre Salze, ihre Solvate und die Solvate ihrer Salze.

Bevorzugt im Rahmen der vorliegenden Erfindung sind auch Verbindungen der Formel (I) oder (Ia), bei denen
- R³: gleich eine Gruppe der Formel 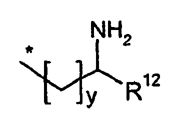
ist,
wobei
- *: die Anknüpfstelle an das Stickstoffatom ist,
- R¹²: eine Gruppe der Formel *-CONHR¹⁴ oder *-CH₂CONHR¹⁵ ist,
worin
- *: die Anknüpfstelle an das Kohlenstoffatom ist,
- R¹⁴ und R¹⁵: unabhängig voneinander eine Gruppe der Formel 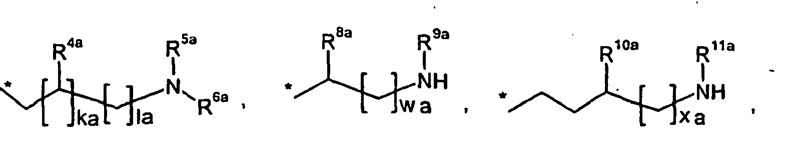 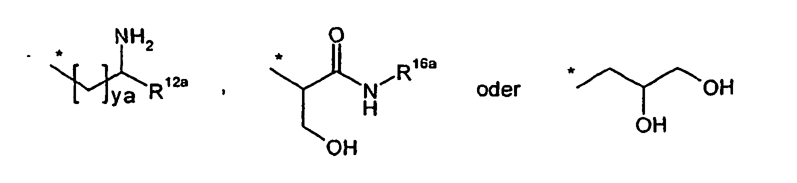
sind,
worin
- *: die Anknüpfstelle an das Stickstoffatom ist,
- R^{4a}: gleich Wasserstoff, Amino oder Hydroxy ist,
- R^{5a}: gleich Wasserstoff, Methyl oder Aminoethyl ist,
- R^{6a}: gleich Wasserstoff oder Aminoethyl ist,
oder
- R^{5a} und R^{6a}: bilden zusammen mit dem Stickstoffatom an das sie gebunden sind einen Piperazin-Ring,
- R^{8a} und R^{12a}: unabhängig voneinander *-(CH₂)_{Z1a}-OH, *-(CH₂)_{Z2a}- NHR^{13a}, *-CONHR^{14a} oder *-CH₂CONHR^{15a}sind,
worin
- *: die Anknüpfstelle an das Kohlenstoffatom ist,
- Z1a und Z2a: unabhängig voneinander eine Zahl 1, 2 oder 3 sind,
- R^{13a}: gleich Wasserstoff oder Methyl ist und
- R^{14a} und R^{15a}: unabhängig voneinander eine Gruppe der Formel 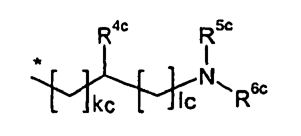
sind,
worin
- *: die Anknüpfstelle an das Stickstoffatom ist,
- R^{4c}: gleich Wasserstoff, Amino oder Hydroxy ist,
- R^{5c}: gleich Wasserstoff, Methyl oder Aminoethyl ist,
- R^{6c}: gleich Wasserstoff oder Aminoethyl ist,
- kc: eine Zahl 0 oder 1 ist
und
- lc: eine Zahl 1, 2, 3 oder 4 ist,
- R^{9a} und R^{11a}: unabhängig voneinander Wasserstoff oder Methyl sind,
- R^{10a}: gleich Amino oder Hydroxy ist,
- R^{16a}: eine Gruppe der Formel 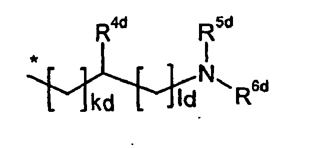
sind,
worin
- *: die Anknüpfstelle an das Stickstoffatom ist,
- R^{4d}: gleich Wasserstoff, Amino oder Hydroxy ist,
- R^{5d}: gleich Wasserstoff, Methyl oder Aminoethyl ist,
- R^{6d}: gleich Wasserstoff oder Aminoethyl ist,
- kd: eine Zahl 0 oder 1 ist
und
- 1d: eine Zahl 1, 2, 3 oder 4 ist,
- ka: eine Zahl 0 oder 1 ist
und
- la, wa, xa und ya: unabhängig voneinander eine Zahl 1, 2, 3 oder 4 sind,
- y: eine Zahl 1, 2, 3 oder 4 ist, 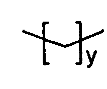
bei y gleich 3 eine Hydroxy-Gruppe tragen kann,
- und: ihre Salze, ihre Solvate und die Solvate ihrer Salze. Bevorzugt im Rahmen der vorliegenden Erfindung sind auch Verbindungen der Formel (I) oder (Ia), bei denen
- R³: gleich eine Gruppe der Formel 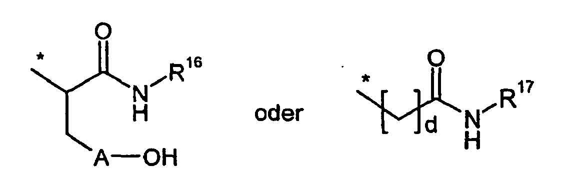
ist, wobei
- *: die Anknüpfstelle an das Stickstoffatom ist,
- A: gleich eine Bindung oder Phenyl ist,
- R¹⁶ und R¹⁷: unabhängig voneinander eine Gruppe der Formel 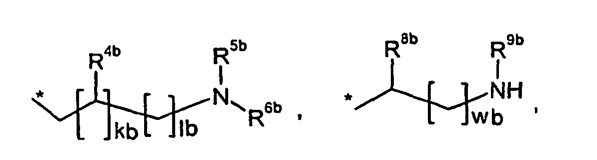 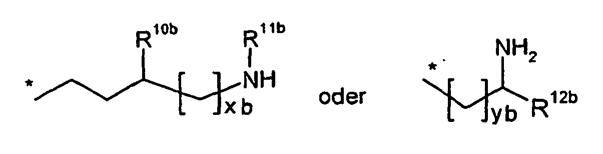
sind,
worin
- *: die Anknüpfstelle an das Stickstoffatom ist,
- R^{4b}: gleich Wasserstoff, Amino oder Hydroxy ist,
- R^{5b}: gleich Wasserstoff, Methyl oder Aminoethyl ist,
- R^{6b}: gleich Wasserstoff oder Aminoethyl ist,
oder
- R^{5b} und R^{6b}: bilden zusammen mit dem Stickstoffatom an das sie gebunden sind einen Piperazin-Ring,
- R^{8b} und R^{12b}: unabhängig voneinander *-(CH₂)_{Z1b}-OH oder *-(CH₂)_{Z2b}-NHR^{13b} sind,
worin
- *: die Anknüpfstelle an das Kohlenstoffatom ist,
- R^{13b}: gleich Wasserstoff oder Methyl istund
- Z1b und Z2b: unabhängig voneinander eine Zahl 1, 2 oder 3 sind,
- R^{9b} und R^{11b}: unabhängig voneinander Wasserstoff oder Methyl sind,
- R^{10b}: gleich Amino oder Hydroxy ist,
- kb: eine Zahl 0 oder 1 ist,
- lb, wb, xb und yb: unabhängig voneinander eine Zahl 1, 2, 3 oder 4 sind,
- d: eine Zahl 1, 2 oder 3 ist, und ihre Salze, ihre Solvate und die Solvate ihrer Salze.

Besonders bevorzugt sind darunter Verbindungen, bei denen R³ eine Gruppe der Formel 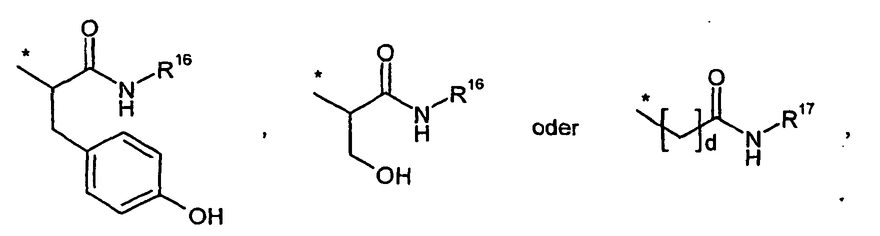 insbesondere eine Gruppe der Formel 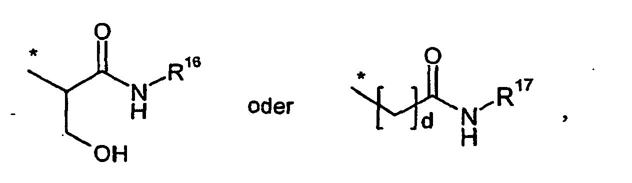 ist.

Bevorzugt im Rahmen der vorliegenden Erfindung sind auch Verbindungen der Formel (I) oder (Ia), bei denen
- R³: gleich eine Gruppe der Formel 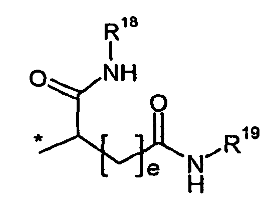
ist,
wobei
- *: die Anknüpfstelle an das Stickstoffatom ist,
- R¹⁸ und R¹⁹: unabhängig voneinander Wasserstoff oder eine Gruppe der Formel 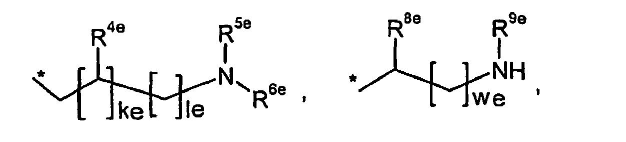 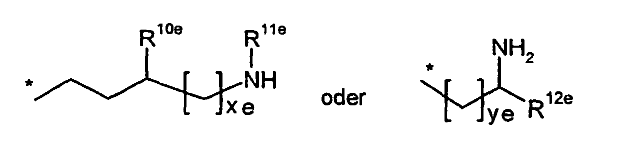
sind,
worin
- *: die Anknüpfstelle an das Stickstoffatom ist,
- R^{4e}: gleich Wasserstoff, Amino oder Hydroxy ist,
- R^{5e}: gleich Wasserstoff, Methyl oder Aminoethyl ist,
- R^{6e}: gleich Wasserstoff oder Aminoethyl ist,
oder
- R^{5e} und R^{6e}: bilden zusammen mit dem Stickstoffatom an das sie gebunden sind einen Piperazin-Ring,
- R^{8e} und R^{12e}: unabhängig voneinander *-CH₂)_{Z1e}-OH oder *-(CH₂)_{Z2e}-NHR^{13e} sind,
worin
- *: die Anknüpfstelle an das Kohlenstoffatom ist,
- R^{13e}: gleich Wasserstoff oder Methyl ist und
- Z1e und Z2e: unabhängig voneinander eine Zahl 1, 2 oder 3 sind,
- R^{9c} und R^{11e}: unabhängig voneinander Wasserstoff oder Methyl sind,
- R^{10c}: gleich Amino oder Hydroxy ist,
- ke: eine Zahl 0 oder 1 ist
und
- le, we,: xe und ye unabhängig voneinander eine Zahl 1, 2, 3 oder 4 sind, wobei R¹⁸ und R¹⁹ nicht gleichzeitig Wasserstoff sind,
- e: eine Zahl 1, 2 oder 3 ist, und ihre Salze, ihre Solvate und die Solvate ihrer Salze.

Gegenstand der Erfindung ist weiterhin ein Verfahren zur Herstellung der Verbindungen der Formel (I) oder ihrer Salze, ihrer Solvate oder der Solvate ihrer Salze, wobei nach Verfahren
[A] Verbindungen der Formel 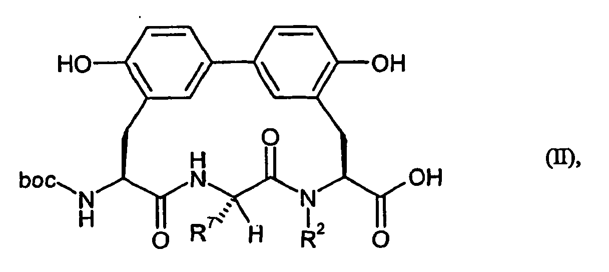 worin R² und R⁷ die oben angegebene Bedeutung haben und boc gleich tert-Butoxycarbonyl ist, in einem zweistufigen Verfahren zunächst in Gegenwart von einem oder mehreren Dehydratisierungsreagenzien mit Verbindungen der Formel

   H₂NR³ (III),

   worin R³ die oben angegebene Bedeutung hat,
   und anschließend mit einer Säure und/oder durch Hydrogenolyse umgesetzt werden,
   oder
[B] Verbindungen der Formel 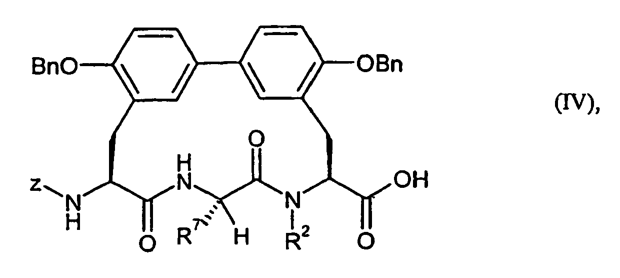 worin R² und R⁷ die oben angegebene Bedeutung haben und Z gleich Benzyloxycarbonyl ist,
   in einem zweistufigen Verfahren zunächst in Gegenwart von einem oder mehreren Dehydratisierungsreagenzien mit Verbindungen der Formel

   H₂NR³ (III),

   worin R³ die oben angegebene Bedeutung hat,
   und anschließend mit einer Säure oder durch Hydrogenolyse umgesetzt werden.

Die freie Base der Salze kann zum Beispiel durch Chromatographie an einer Reversed Phase Säule mit einem Acetonitril-Wasser-Gradienten unter Zusatz einer Base erhalten werden, insbesondere durch Verwendung einer RP18 Phenomenex Luna C18(2) Säule und Diethylamin als Base.

Weiterer Gegenstand der Erfindung ist ein Verfahren zur Herstellung der Verbindungen der Formel (I) oder ihrer Solvate nach Anspruch 1, bei dem Salze der Verbindungen oder Solvate der Salze der Verbindungen durch Chromatographie unter Zusatz einer Base in die Verbindungen überführt werden.

Die Hydroxygruppe an R¹ ist gegebenenfalls während der Umsetzung mit Verbindungen der Formel (III) mit einer *tert-*Butyldimethylsilyl-Gruppe geschützt, die im zweiten Reaktionsschritt abgespalten wird.

Reaktive Funktionalitäten in dem Rest R³ von Verbindungen der Formel (III) werden bereits geschützt mit in die Synthese eingebracht, bevorzugt sind säurelabile Schutzgruppen (z.B. boc). Nach erfolgter Umsetzung zu Verbindungen der Formel (I) können die Schutzgruppen durch Entschützungsreaktion abgespalten werden. Dies geschieht nach Standardverfahren der Schutzgruppenchemie. Bevorzugt sind Entschützungsreaktionen unter sauren Bedingungen oder durch Hydrogenolyse.

Die Umsetzung der ersten Stufe der Verfahren [A] und [B] erfolgt im Allgemeinen in inerten Lösungsmitteln, gegebenenfalls in Gegenwart einer Base, bevorzugt in einem Temperaturbereich von 0°C bis 40°C bei Normaldruck.

Als Dehydratisierungsreagenzien eignen sich hierbei beispielsweise Carbodiimide wie z.B. *N,N'-*Diethyl-, *N,N'*-Dipropyl-, *N,N'*-Diisopropyl-, *N,N'*-Dicyclohexylcarbodiimid, *N-*(3-Dimethylamino-isopropyl)-*N'*-ethylcarbodiimid-Hydrochlorid (EDC), *N-*Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexa-fluorophosphat, oder O-(Benzotriazol-1-yl)-*N,N*,*N'*,*N'*-tetra-methyluroniumhexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-*N,N*,*N'*,*N'*-tetramethyluroniumhexafluorophosphat (HATU), oder 1-Hydroxybenztriazol (HOBt), oder Benzotriazol-1-yloxytris(dimethylamino)-phosphonium-hexafluoro-phosphat (BOP), oder Mischungen aus diesen, oder Mischung aus diesen zusammen mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N-*Methylmorpholin, *N-*Methylpiperidin, 4-Dimethylaminopyridin oder Diisopropylethylamin.

Vorzugsweise wird die Kondensation mit HATU in Gegenwart einer Base, insbesondere Düsopropylethylamin, oder mit EDC und HOBt in Gegenwart einer Base, insbesondere Triethylamin, durchgeführt.

Inerte Lösungsmittel sind beispielsweise Halogenkohlenwasserstoffe wie Dichlormethan oder Trichlormethan, Kohlenwasserstoff wie Benzol, oder Nitromethan, Dioxan, Dimethylformamid oder Acetonitril. Ebenso ist es möglich, Gemische der Lösemittel einzusetzen. Besonders bevorzugt ist Dimethylformamid.

Die Umsetzung mit einer Säure in der zweiten Stufe der Verfahren [A] und [B] erfolgt bevorzugt in einem Temperaturbereich von 0°C bis 40°C bei Normaldruck.

Als Säuren eignen sich hierbei Chlorwasserstoff in Dioxan, Bromwasserstoff in Essigsäure oder Trifluoressigsäure in Methylenchlorid.

Die Hydrogenolyse in der zweiten Stufe des Verfahrens [B] erfolgt im Allgemeinen in einem Lösungsmittel in Gegenwart von Wasserstoff und Palladium auf Aktivkohle, bevorzugt in einem Temperaturbereich von 0°C bis 40°C bei Normaldruck.

Lösungsmittel sind beispielsweise Alkohole wie Methanol, Ethanol, n-Propanol oder iso-Propanol, in einem Gemisch mit Wasser und Eisessig, bevorzugt ist ein Gemisch aus Ethanol, Wasser und Eisessig.

Die Verbindungen der Formel (III) sind bekannt oder können analog bekannten Verfahren hergestellt werden.

Die Verbindungen der Formel (II) sind bekannt oder können hergestellt werden, indem Verbindungen der Formel 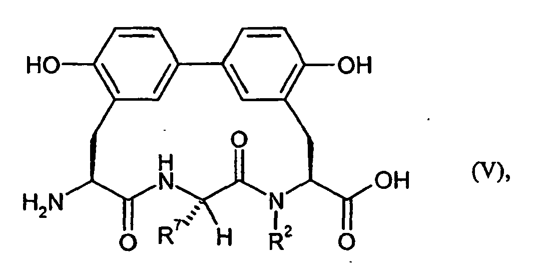 worin R² und R⁷ die oben angegebene Bedeutung haben,
mit Di-(*tert-*butyl)-dicarbonat in Gegenwart einer Base umgesetzt werden.

Die Umsetzung erfolgt im Allgemeinen in einem Lösungsmitteln, bevorzugt in einem Temperaturbereich von 0°C bis 40°C bei Normaldruck.

Basen sind beispielsweise Alkalihydroxide wie Natrium- oder Kaliumhydroxid, oder Alkalicarbonate wie Cäsiumcarbonat, Natrium- oder Kaliumcarbonat, oder andere Basen wie DBU, Triethylamin oder Diisopropylethylamin, bevorzugt ist Natriumhydroxid oder Natriumcarbonat.

Lösungsmittel sind beispielsweise Halogenkohlenwasserstoffe wie Methylenchlorid oder 1,2-Dichlorethan, Alkohole wie Methanol, Ethanol oder iso-Propanol, oder Wasser.

Vorzugsweise wird die Umsetzung mit Natriumhydroxid in Wasser oder Natriumcarbonat in Methanol durchgeführt.

Die Verbindungen der Formel (V) sind bekannt oder können hergestellt werden, indem Verbindungen der Formel 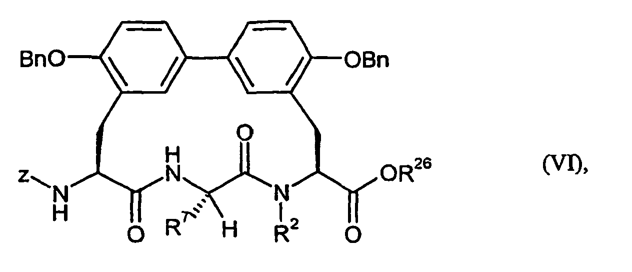 worin R² und R⁷ die oben angegebene Bedeutung haben, und
R²⁶ gleich Benzyl, Methyl oder Ethyl ist,
mit einer Säure oder durch Hydrogenolyse, wie für die zweite Stufe des Verfahrens [B] beschrieben, gegebenenfalls durch anschließende Umsetzung mit einer Base zur Verseifung des Methyl- oder Ethylesters, umgesetzt werden.

Die Verseifung kann zum Beispiel erfolgen, wie bei der Umsetzung von Verbindungen der Formel (VI) zu Verbindungen der Formel (IV) beschrieben.

Die Verbindungen der Formel (IV) sind bekannt oder können hergestellt werden, indem in Verbindungen der Formel (VI) der Benzyl-, Methyl- oder Ethylester verseift wird.

Die Umsetzung erfolgt im Allgemeinen in einem Lösungsmitteln, in Gegenwart einer Base, bevorzugt in einem Temperaturbereich von 0°C bis 40°C bei Normaldruck.

Basen sind beispielsweise Alkalihydroxide wie Lithium-, Natrium- oder Kaliumhydroxid, bevorzugt ist Lithiumhydroxid.

Lösungsmittel sind beispielsweise Halogenkohlenwasserstoffe wie Dichlormethan oder Trichlormethan, Ether wie Tetrahydrofuran oder Dioxan, oder Alkohole wie Methanol, Ethanol oder Iso-propanol, oder Dimethylformamid. Ebenso ist es möglich, Gemische der Lösungsmittel oder Gemische der Lösungsmittel mit Wasser einzusetzen. Besonders bevorzugt sind Tetrahydrofuran oder ein Gemisch aus Methanol und Wasser.

Die Verbindungen der Formel (VI) sind bekannt oder können hergestellt werden, indem Verbindungen der Formel 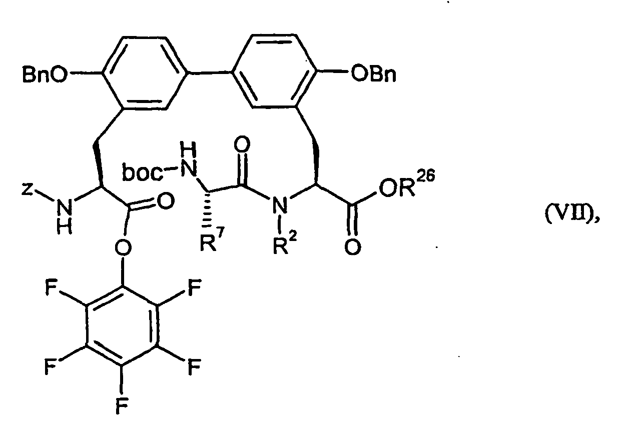 worin R², R⁷ und R²⁶ die oben angegebene Bedeutung haben,
in der ersten Stufe mit Säuren, wie für die zweite Stufe der Verfahren [A] und [B] beschrieben, und in der zweiten Stufe mit Basen umgesetzt werden.

In der zweiten Stufe erfolgt die Umsetzung mit Basen im Allgemeinen in einem Lösungsmitteln, bevorzugt in einem Temperaturbereich von 0°C bis 40°C bei Normaldruck.

Basen sind beispielsweise Alkalihydroxide wie Natrium- oder Kaliumhydroxid, oder Alkalicarbonate wie Cäsiumcarbonat, Natrium- oder Kaliumcarbonat, oder andere Basen wie DBU, Triethylamin oder Diisopropylethylamin, bevorzugt ist Triethylamin.

Lösungsmittel sind beispielsweise Halogenkohlenwasserstoffe wie Chloroform, Methylenchlorid oder 1,2-Dichlorethan, oder Tetrahydrofuran, oder Gemische der Lösungsmittel, bevorzugt ist Methylenchlorid oder Tetrahydrofuran.

Die Verbindungen der Formel (VII) sind bekannt oder können hergestellt werden, indem Verbindungen der Formel 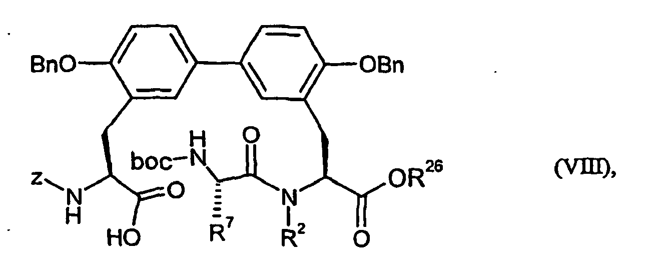 worin R², R⁷ und R²⁶ die oben angegebene Bedeutung haben,
mit Pentafluorphenol in Gegenwart von Dehydratisierungsreagenzien, wie für die erste Stufe der Verfahren [A] und [B] beschrieben, umgesetzt werden.

Die Umsetzung erfolgt bevorzugt mit DMAP und EDC in Dichlormethan in einem Temperaturbereich von -40°C bis 40°C bei Normaldruck.

Die Verbindungen der Formel (VIII) sind bekannt oder können hergestellt werden, indem Verbindungen der Formel 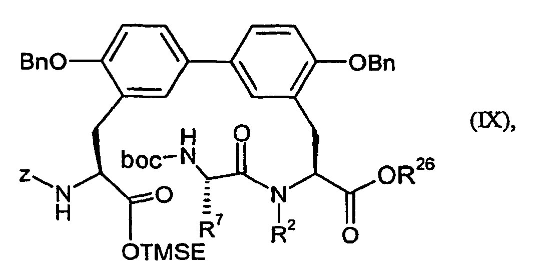 worin R², R⁷ und R²⁶ die oben angegebene Bedeutung haben,
mit Fluorid, insbesondere mit Tetrabutylammoniumfluorid, umgesetzt werden.

Die Umsetzung erfolgt im Allgemeinen in einem Lösungsmitteln, bevorzugt in einem Temperaturbereich von -10°C bis 30°C bei Normaldruck.

Inerte Lösungsmittel sind beispielsweise Halogenkohlenwasserstoffe wie Dichlormethan, oder Kohlenwasserstoffe wie Benzol oder Toluol, oder Ether wie Tetrahydrofuran oder Dioxan, oder Dimethylformamid. Ebenso ist es möglich, Gemische der Lösemittel einzusetzen. Bevorzugte Lösungsmittel sind Tetrahydrofuran und Dimethylformamid.

Die Verbindungen der Formel (IX) sind bekannt oder können hergestellt werden, indem Verbindungen der Formel 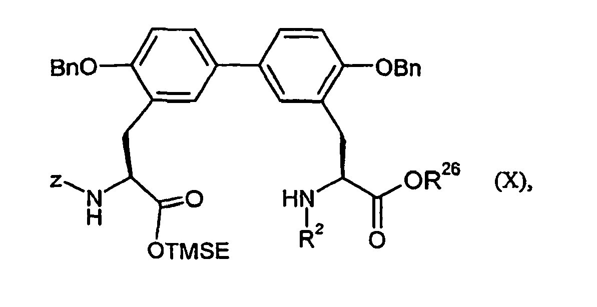 worin R² und R²⁶ die oben angegebene Bedeutung haben,
mit Verbindungen der Formel 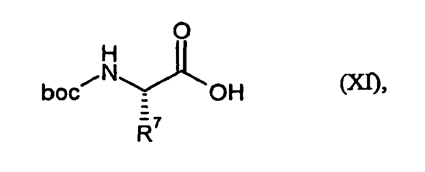 worin R⁷ die oben angegebene Bedeutung hat,
in Gegenwart von Dehydratisierungsreagenzien, wie für die erste Stufe der Verfahren [A] und [B] beschrieben, umgesetzt werden.

Die Verbindungen der Formel (X) sind bekannt oder können analog den im Beispielteil beschriebenen Verfahren hergestellt werden.

Die Verbindungen der Formel (XI) sind bekannt oder können analog bekannten Verfahren hergestellt werden.

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Die erfindungsgemäßen Verbindungen können aufgrund ihrer pharmakologischen Eigenschaften allein oder in Kombination mit anderen Wirkstoffen zur Behandlung und/oder Prophylaxe von Infektionskrankheiten, insbesondere von bakteriellen Infektionen, eingesetzt werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytocy), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen topisch anwendbaren Zubereitungen verhindert, gebessert oder geheilt werden können, seien beispielsweise genannt:
Infektionskrankheiten beim Menschen wie z. B. septische Infektionen, Knochen- und Gelenkinfektionen, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, septische Arthritis, Mastitis, Tonsillitis, Genital-Infektionen und Augeninfektionen.

Außer beim Menschen können bakterielle Infektionen auch bei anderen Spezies behandelt werden. Beispielhaft seien genannt:
Schwein: Coli-diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsie, Yersinia, erweitert.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise- zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei parenteraler Applikation Mengen von etwa 5 bis 250 mg/kg Körpergewicht je 24 h zur Erzielung wirksamer Ergebnisse zu verabreichen. Bei oraler Applikation beträgt die Menge etwa 5 bis 100 mg/kg Körpergewicht je 24 h.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

**Verwendete Abkürzungen:**
- abs.: absolut
- aq.: wässrig
- Bn: Benzyl
- boc: *tert-*Butoxycarbonyl
- CDCl₃: Chloroform
- CH: Cyclohexan
- d: dublett (im ¹H-NMR)
- dd: dublett von dublett (im ¹H-NMR)
- DC: Dünnschichtchromatographie
- DCC: Dicyclohexylcarbodiimid
- DIC: Diisopropylcarbodiimid
- DIEA: Diisopropylethylamin (Hünig-Base)
- DMSO: Dimethylsulfoxid
- DMAP: 4-*N*,*N-*Dimethylaminopyridin
- DMF: Dimethylformamid
- d. Th.: der Theorie
- EDC: *N'*-(3-Dimethylaminopropyl)*-N-*ethylcarbodiimid x HCl
- EE: Ethylacetat (Essigsäureethylester)
- ESI: Elektrospray-Ionisation (bei MS)
- Fmoc: 9-Fluorenylmethoxycarbonyl
- ges.: gesättigt
- HATU: *O-*(7-Azabenzotriazol-1-yl)-*N,N*,*N'*,*N'*-tetramethyluronium- hexafluorophosphat
- HBTU: *O-*(Benzotriazol-1-yl)-*N,N*,*N'*;*N'*-tetramethyluronium- hexafluorophosphat
- HOBt: 1-Hydroxy-1H-benzotriazol x H₂O
- h: Stunde(n)
- HPLC: Hochdruck-, Hochleistungsflüssigchromatographie
- LC-MS: Flüssigchromatographie-gekoppelte Massenspektroskopie
- m: multiplett (im ¹H-NMR)
- min: Minute
- MS: Massenspektroskopie
- NMR: Kernresonanzspektroskopie
- MTBE: Methyl-*tert*-butylether
- Pd/C: Palladium/Kohle
- proz.: Prozent
- q: quartett (im ¹H-NMR)
- R_{f}: Retentionsindex (bei DC)
- RP: Reverse Phase (bei HPLC)
- RT: Raumtemperatur
- Rₜ: Retentionszeit (bei HPLC)
- s: singulett (im ¹H-NMR)
- t: triplett (im ¹H-NMR)
- TBS: *tert*-Butyldimethylsilyl
- TFA: Trifluoressigsäure
- THF: Tetrahydrofuran
- TMSE: 2-(Trimethylsilyl)-ethyl
- TPTU: 2-(2-Oxo-1(2H)-pyridyl)-1,1,3,3-tetramethyluroniumtetrafluoroborat
- Z: Benzyloxycarbonyl

### LC-MS- und HPLC-Methoden:

**Methode 1 (HPLC)**: Instrument: HP 1100 mit DAD-Detektion; Säule: Kromasil RP-18, 60 mm x 2 mm, 3.5 µm; Eluent A: 5 ml Perchlorsäure/l Wasser, Eluent B: Acetonitril; Gradient: 0 min 2%B, 0.5 min 2%B, 4.5 min 90%B, 6.5 min 90%B; Fluss: 0.75 ml/min; Ofen: 30°C; UV-Detektion: 210 nm.
**Methode 2 (LC-MS)**: Instrument Micromass Platform LCZ; Säule Symmetry C18, 50 mm x 2.1 mm, 3.5 µm; Temperatur: 40°C; Fluss: 0.5 ml/min; Eluent A: Acetonitril + 0.1% Ameisensäure, Eluent B: Wasser + 0.1% Ameisensäure, Gradient: 0.0 min 10%A → 4 min 90%A →6 min 90%A.
**Methode 3 (LC-MS)**: Instrument: Waters Alliance 2790 LC; Säule: Symmetry C18, 50 mm x 2.1 mm, 3.5 µm; Eluent A: Wasser + 0.1% Ameisensäure, Eluent B: Acetonitril + 0.1% Ameisensäure; Gradient: 0.0 min 5%B → 5.0 min 10%B → 6.0 min 10%B; Temperatur: 50°C; Fluss: 1.0 ml/min; UV-Detektion: 210 nm.
**Methode 4 (LC-MS):** ZMD Waters; Säule: Inertsil ODS3 50 mm x 2.1 mm, 3 µm; Temperatur: 40°C; Fluss: 0.5 ml/min; Eluent A: Wasser + 0.05% Ameisensäure, Eluent B: Acetonitril + 0.05% Ameisensäure, Gradient: 0.0 min 5%B → 12 min → 100%B → 15 min 100%B.
**Methode 5 (LC-MS):** MAT 900, Finnigan MAT, Bremen; Säule: X-terra 50mm x 2.1 mm, 2.5 µm; Temperatur: 25°C; Fluss: 0.5 ml/min; Eluent A: Wasser + 0.01% Ameisensäure, Eluent B: Acetonitril + 0.01% Ameisensäure, Gradient: 0.0 min 10%B → 15 min → 90%B → 30 min 90%B.
**Methode 6 (LC-MS):** TSQ 7000, Finnigan MAT, Bremen; Säule: Inertsil ODS3 50 mm x 2.1 mm, 3 µm; Temperatur: 25°C; Fluss: 0.5 ml/min; Eluent A: Wasser + 0.05% Ameisensäure, Eluent B: Acetonitril + 0.05% Ameisensäure, Gradient: 0.0 min 15%B → 15 min → 100%B → 30 min 100%B.
**Methode 7 (LC-MS)**: 7 Tesla Apex II mit externer Elektrospray-Ionenquelle, Bruker Daltronics; Säule: X-terra C18 50 mm x 2.1 mm, 2.5 µm; Temperatur: 25°C; Fluss: 0.5 ml/min; Eluent A: Wasser +0.1% Ameisensäure, Eluent B: Acetonitril +0.1% Ameisensäure, Gradient: 0.0 min 5%B → 13 min → 100%B → 15 min 100%B.
**Methode 8 (LC-MS)**: Gerätetyp MS: Micromass ZQ; Gerätetyp HPLC: Waters Alliance 2795; Säule: Merck Chromolith SpeedROD RP-18e 50x4.6mm; Eluent A: Wasser + 500 µl 50%ige Ameisensäure / 1; Eluent B: Acetonitril + 500 µl 50%ige Ameisensäure / 1; Gradient: 0.0 min 10%B→ 2.0 min 95%B→ 4.0 min 95%B; Ofen: 35 °C; Fluss: 0.0 min 1.0 ml/min→ 2.0 min 3.0 ml/min→ 4.0 min 3.0 ml/min; UV-Detektion: 210 nm.
**Methode 9 (LC-MS**): Instrument: Micromass Platform LCZ mit HPLC Agilent Serie 1100; Säule: Grom-SIL120 ODS-4 HE, 50 mm x 2.0 mm, 3 µm; Eluent A: 1 l Wasser + 1 ml 50%ige Ameisensäure, Eluent B: 1l Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 210 nm.
**Methode 10 (LC-MS):** Gerätetyp MS: Micromass ZQ; Gerätetyp HPLC: Waters Alliance 2795; Säule: Merck Chromolith SpeedROD RP-18e 50x4.6mm; Eluent A: Wasser + 500 µl 50%ige Ameisensäure / l; Eluent B: Acetonitril + 500 µl 50%ige Ameisensäure / 1; Gradient: 0.0 min 10%B→ 3.0 min 95%B→ 4.0 min 95%B; Ofen: 35 °C; Fluss: 0.0 min 1.0 ml/min→ 3.0 min 3.0 ml/min→ 4.0 min 3.0 ml/min; UV-Detektion: 210 nm.
**Methode 11 (LC-MS)**: Gerätetyp MS: Micromass ZQ; Gerätetyp HPLC: Waters Alliance 2790; Säule: Uptisphere C 18, 50 mm x 2.0 mm, 3.0 µm; Eluent B: Acetonitril + 0.05% Ameisensäure, Eluent A: Wasser + 0.05% Ameisensäure; Gradient: 0.0 min 5%B→ 2.0 min 40%B → 4.5 min 90%B→ 5.5 min 90%B; Ofen: 45 °C; Fluss: 0.0 min 0.75 ml/min → 4.5 min 0.75 ml/min→ 5.5 min 1.25 ml/min; UV-Detektion: 210 nm.
**Methode 12 (LC-MS)**: Gerätetyp MS: Micromass ZQ; Gerätetyp HPLC: Waters Alliance 2795; Säule: Phenomenex Synergi 2µ Hydro- RP Mercury 20x4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A (Fluss: 1 ml/min) → 2.5 min 30%A (Fluss: 2 ml/min)→ 3.0 min 5%A (Fluss: 2 ml/min) → 4.5 min 5%A (Fluss: 2 ml/min); Ofen: 50°C; UV-Detektion: 210 nm.
**Methode 13 (LC-MS):** Gerätetyp MS: Micromass ZQ; Gerätetyp HPLC: HP 1100 Series; UV DAD; Säule: Grom-Sil 120 ODS-4 HE 50x2 mm, 3.0 µm; Eluent A: Wasser + 500 µl 50%ige Ameisensäure / I, Eluent B: Acetonitril + 500 µl 50%ige Ameisensäure / 1; Gradient: 0.0 min 70%B → 4.5 min 90%B; Ofen: 50 °C, Fluss: 0.8 ml/min, UV-Detektion: 210 nm.
**Methode 14 (LC-MS):** Instrument: Micromass Quattro LCZ, mit HPLC Agilent Serie 1100; Säule: Grom-SIL120 ODS-4 HE, 50 mm x 2.0 mm, 3 µm; Eluent A: 1 l Wasser + 1 ml 50%ige Ameisensäure, Eluent B: 1 l Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.
**Methode 15 (LC-MS):** Gerätetyp MS: Micromass ZQ; Gerätetyp HPLC: Waters Alliance 2790; Säule: Grom-Sil 120 ODS-4 HE 50x2 mm, 3.0 µm; Eluent A: Wasser + 500µl 50%ige Ameisensäure; Eluent B: Acetonitril + 500µl 50%ige Ameisensäure / 1; Gradient: 0.0min 5%B→ 2.0min 40%B→ 4.5min 90%B→ 5.5min 90%B; Ofen: 45 °C; Fluss: 0.0min 0.75ml/min→ 4.5min 0.75ml 5.5min→ 5.5min 1.25ml; UV-Detektion: 210 nm.
**Methode 16 (HPLC):** Instrument: HP 1100 mit DAD-Detektion; Säule: Kromasil RP-18, 60 mm x 2 mm, 3.5 µ.m; Eluent A: 5 ml Perchlorsäure/l Wasser, Eluent B: Acetonitril; Gradient: 0 min 2%B, 0.5 min 2%B, 4.5 min 90%B, 15 min 90%B; Fluss: 0.75ml/min; Ofen: 30°C; UV-Detektion: 210 nm.
**Methode 17 (LC-MS)**: Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.
**Methode 18 (LC-MS):** Instrument: Micromass Platform LCZ mit HPLC Agilent Serie 1100; Säule: Phenomenex Synergi 2µ Hydro-RP Mercury 20 mm x 4 mm; Eluent A: 1 l Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.
**Methode 19 (LC-MS):** Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.
**Methode 20 (LC-MS):** Instrument: Micromass Platform LCZ mit HPLC Agilent Serie 1100; Säule: ThermoHypersil-Keystone HyPurity Aquastar, 50 mm x 2.1 mm, 3 µm; Eluent A: 1 l Wasser + 1 ml 50%ige Ameisensäure, Eluent B: 1 l Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 210 nm.
**Methode 21 (Präparative HPLC/RP-HPLC)**: Säule: RP18 Phenomenex Luna C18(2) (New Column), 250 mm x 21.2 mm, 5µm (Firma Phenomenex, Aschaffenburg, Deutschland), Eluent: Acetonitril - Wasser Gradient unter Zusatz von 0.2% Diethylamin.
**Methode 22 (HPLC):** Instrument: HP 1100 mit DAD-Detektion; Säule: Kromasil RP-18, 60 mm x 2 mm, 3.5 µm; Eluent A: 5 ml Perchlorsäure/l Wasser, Eluent B: Acetonitril; Gradient: 0 min 2%B, 0.5 min 2%B, 4.5 min 90%B, 9 min 90%B; Fluss: 0.75 ml/min; Ofen: 30°C; UV-Detektion: 210 nm.
**Methode 23 (LC-MS):** Instrument: Micromass Platform LCZ mit HPLC Agilent Serie 1100; Säule: Thermo Hypersil GOLD-3µ 20 x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

### Chemische Synthese der Beispiele

Synthese der Ausgangsverbindungen:
Synthese von substituierten Phenylalaninderivaten am Beispiel von (-)-3-(2-Benzyloxy-5-iodophenyl)-2(*S*)-*tert*-butoxycarbonylamino-propionsäure [(-)-6A] 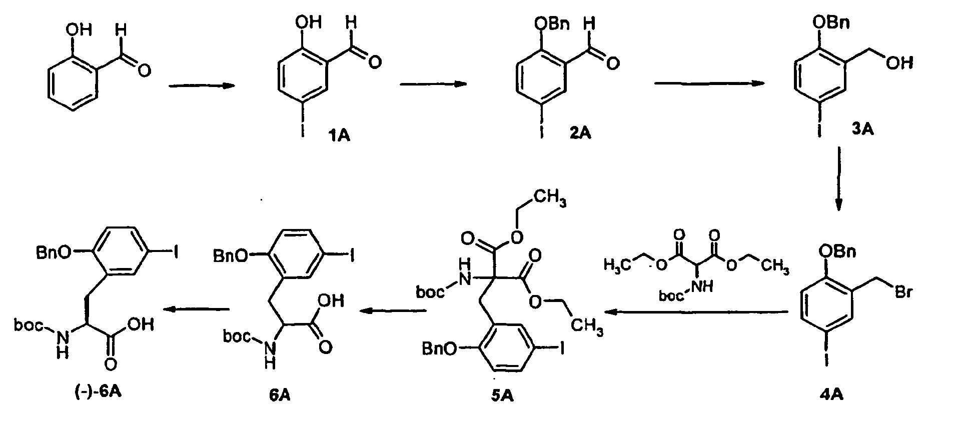
Synthese geschützter Hydroxyornithinderivate am Beispiel von 5-Benzyloxycarbonylamino-2(*S*)-*tert*-butoxycarbonylamino-4(*R*)-(*tert*-butyldimethylsilyloxy)-pentansäure (14A) 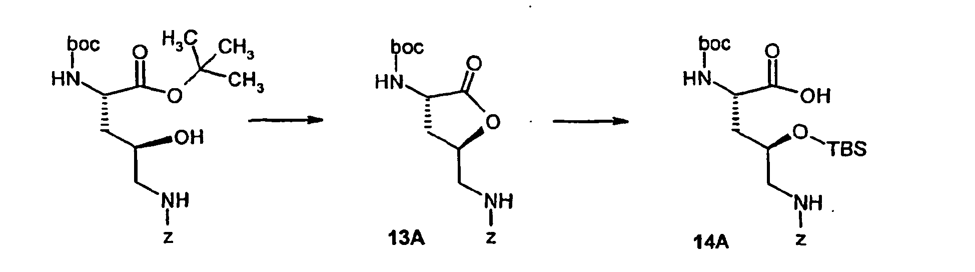
Synthese von substituierten Phenylalaninderivaten am Beispiel von Methyl-2-(benzyloxy)*-N-*[(benzyloxy)carbonyl]-5-brom-L-phenylalaninat] (56A) 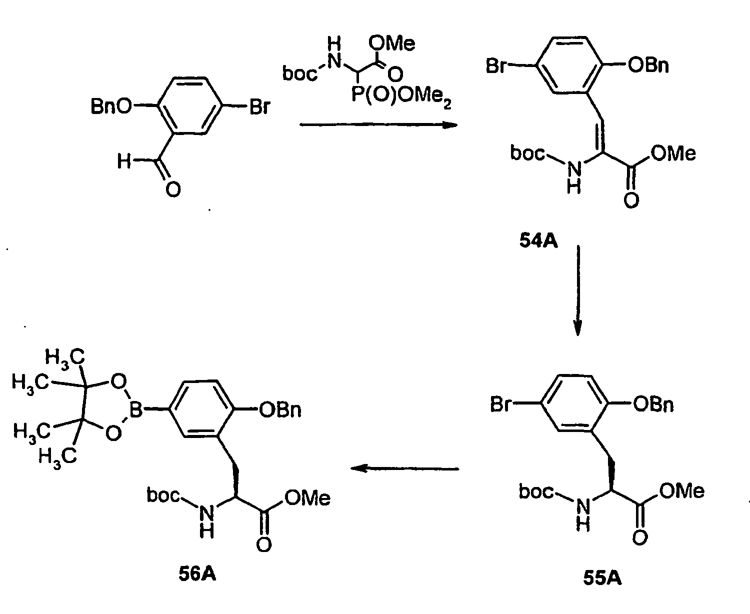
Synthese von geschützten Biphenyl-bisaminosäuren am Beispiel von 2(*S*)-Benzyloxy-carbonylamino-3-[4,4'-bis-benzyloxy-3'-(2(*S*')-benzyloxycarbonyl-2(*S*)-*tert*-butoxycarbonyl-aminoethyl)-biphenyl-3-yl]-propionsäure-2(*S*)-trimethylsilanyl-ethylester (12A)

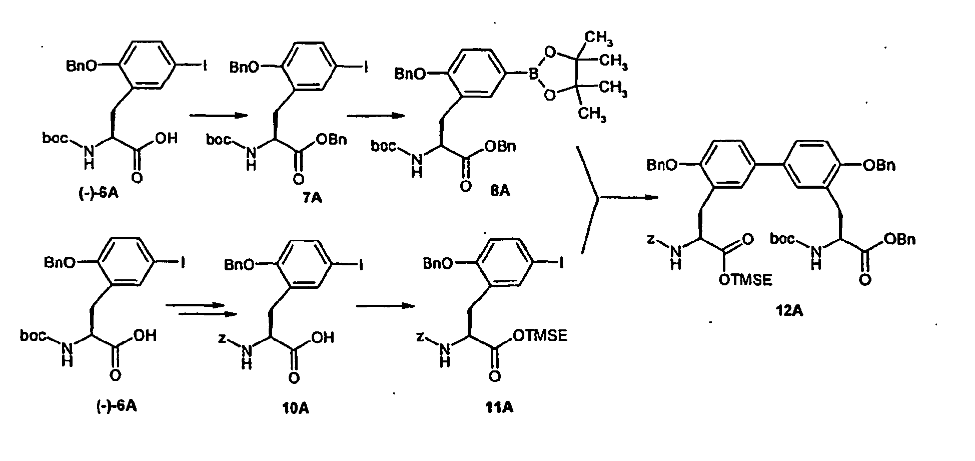

Cyclisierung der Biphenyl-bisaminosäuren

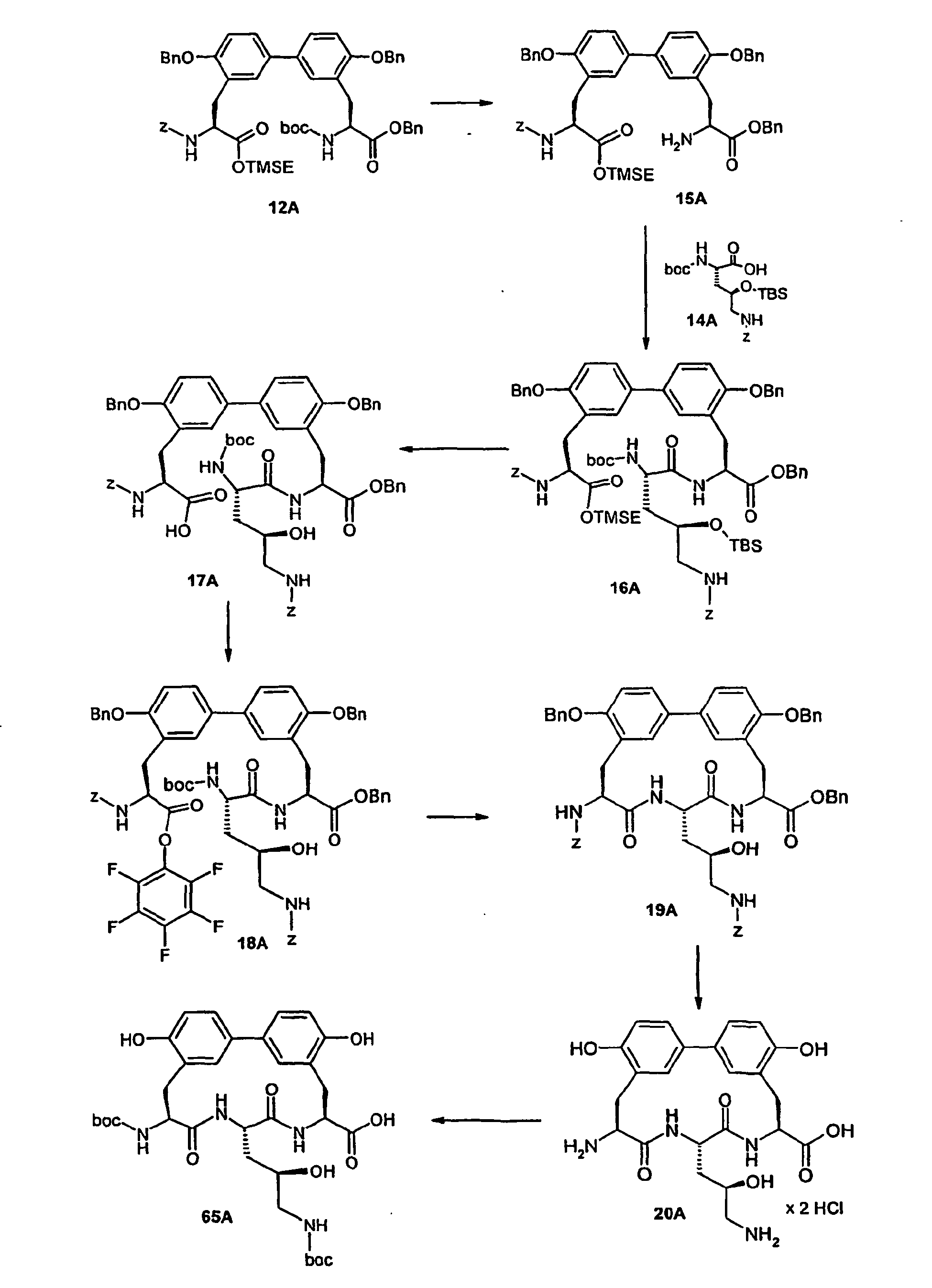

### Ausgangsverbindungen

### Beispiel 1A

2-Hydroxy-5-iod-benzaldehyd 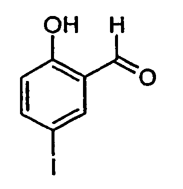 Zu einer Lösung von 188 g (1.54 mol) Salicylaldehyd in 1 l wasserfreiem Dichlormethan in einem ausgeheizten Kolben wird eine Lösung von 250 g (1.54 mol) Iodchlorid in 600 ml wasserfreiem Dichlormethan unter Argon über 2 h zugetropft. Nach 3 Tagen Rühren bei RT wird eine gesättigte wässrige Natriumsulfit-Lösung unter kräftigem Rühren hinzugegeben. Die organische Phase wird abgetrennt, einmal mit Wasser und gesättigter wässriger Natriumchlorid-Lösung gewaschen und über Natriumsulfat getrocknet. Das Lösungsmittel wird eingedampft und der Rückstand aus Essigsäureethylester umkristallisiert. Man erhält 216 g (57% d. Th.) des Produktes.
LC-MS (ESI, Methode 4): m/z = 246 (M-H)-.
¹H-NMR (400 MHz, CDCl₃): δ = 6.7 (d, 1H), 7.77 (dd, 1H), 7.85 (d, 1H), 9.83 (s, 1H), 10.95 (s, 1H).

### Beispiel 2A

2-Benzyloxy-5-iodbenzaldehyd 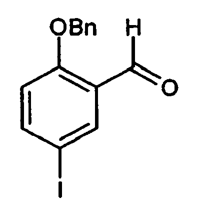 Zu einer Lösung von 100 g (0.40 mol) 2-Hydroxy-5-iodbenzaldehyd (Beispiel 1A) in 1.5 1 Dimethylformamid werden 67.2 g (0.48 mol) Kaliumcarbonat und nach wenigen Minuten 51 ml (0.44 mol) Benzylchlorid hinzugegeben. Das Reaktionsgemisch wird 24 h bei 120°C unter Rückfluss gerührt. Nach weiteren 24 h Rühren bei RT und Zugabe von 1.5 l Wasser kristallisiert ein Feststoff aus. Der Niederschlag wird abgesaugt, zweimal mit Wasser gewaschen und im Vakuum getrocknet. Der Feststoff wird aus 230 ml Ethanol umkristallisiert. Man erhält 122.9 g (90% d. Th.) des Produktes.
LC-MS (ESI, Methode 4): m/z = 338 (M+H)⁺.
¹H-NMR (400 MHz, CDCl₃): δ = 5.18 (s, 2H), 6.84 (d, 1H), 7.33-7.45 (m, 5H), 7.78 (dd, 1H), 8.12 (d, 1H), 10.4 (s, 1H).

### Beispiel 3A

(2-Benzyloxy-5-iod-phenyl)-methanol 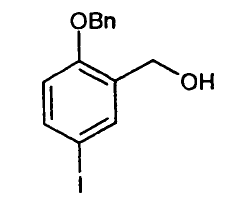 Zu einer auf 0°C gekühlten Lösung von 33.98 g (100.5 mmol) 2-Benzyloxy-5-iod-benzaldehyd (Beispiel 2A) in 200 ml Dichlormethan werden 100 ml einer 1M Diisobutylaluminiumhydrid-Lösung in Dichlormethan zugegeben. Nach 2 h Rühren bei 0°C wird unter Kühlung eine gesättigte Kaliumnatriumtartrat-Lösung hinzugegeben (stark exotherme Reaktion) und das Reaktionsgemisch 2 h weiter gerührt. Nach Abtrennung der Phasen wird die organische Phase zweimal mit Wasser und einmal mit gesättigter wässriger Natriumchlorid-Lösung gewaschen und über Natriumsulfat getrocknet. Das Lösungsmittel wird im Vakuum abgedampft. Man erhält 31.8 g (93% d. Th.) des Produktes.
¹H-NMR (400 MHz, CDCl₃): δ = 2.17 (t, 1H), 4.68 (d, 2H), 5.1 (s, 2H), 6.72 (d, 1H), 7.32-7.42 (m, 5H), 7.54 (dd, 1H), 7.63 (d, 1H).

### Beispiel 4A

1-Benzyloxy-2-brommethyl-4-iodbenzol 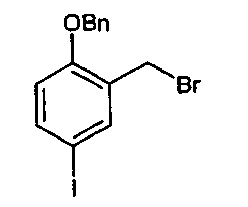 Zu einer Lösung von 35 g (103 mmol) (2-Benzyloxy-5-iod-phenyl)-methanol (Beispiel 3A) in 350 ml Toluol werden bei 40°C 3.3 ml (35 mmol) Phosphortribromid hinzugetropft. Innerhalb von 15 min wird die Temperatur des Reaktionsgemisches auf 100°C erhöht und weitere 10 min bei dieser Temperatur gerührt. Nach Abkühlung werden die beiden Phasen getrennt. Die organische Phase wird zweimal mit destilliertem Wasser und einmal mit gesättigter wässriger Natriumchlorid-Lösung gewaschen. Die organische Phase wird über Natriumsulfat getrocknet und eingedampft. Die Ausbeute beträgt 41 g (99% d. Th.).
¹H-NMR (300 MHz, CDCl₃): δ = 4.45 (s, 2H), 5.06 (s, 2H), 7.30 (m, 8H).

### Beispiel 5A

2-(2-Benzyloxy-5-iod-benzyl)-2-*tert*-butoxycarbonylamino-malonsäurediethylester 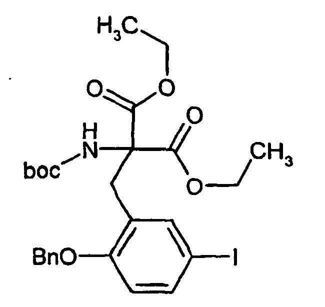 Zu einer Lösung von 28 g (101.7 mmol) 2-[*N-*(*tert*-Butoxycarbonyl)amino]malonsäure-diethylester und 7.9 ml (101.7 mmol) Natriumethylat in 300 ml Ethanol werden 41 g (101.7 mmol) von 1-Benzyloxy-2-brommethyl-4-iodbenzol (Beispiel 4A) hinzugegeben. Nach 3 h Rühren bei RT saugt man das ausgefallene Produkt ab. Nach Trocknung im Vakuum werden 55 g (90% d. Th.) Produkt isoliert.
1H-NMR (400 MHz, CDCl₃): δ = 1.12 (t, 6 H), 1.46 (s, 9H), 3.68 (s, 2H), 3.8-3.9 (m, 2H), 4.15-4.25 (m, 2H), 5.0 (s, 2H), 5.7 (s, 1H), 6.58 (d, 1H), 7.28-7.4 (m, 6H), 7.4 (dd, 1H).

### Beispiel 6A

(+/-)-3-(2-Benzyloxy-5-iod-phenyl)-2-*tert*-butoxycarbonylamino-propionsäure 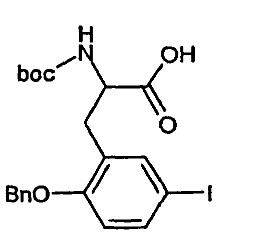 Zu einer Suspension von 58 g (97 mmol) 2-(2-Benzyloxy-5-iod-benzyl)-2-*tert*-butoxy-carbonylamino-malonsäurediethylester (Beispiel 5A) in 800 ml eines Gemisches von Ethanol und Wasser (7:3) werden 400 ml 1N Natronlauge hinzugegeben. Nach 3 h unter Rückfluss wird der pH-Wert der Reaktionsmischung nach Abkühlung auf Raumtemperatur mit konz. Salzsäure auf ca. pH 2 eingestellt. Die Reaktionsmischung wird eingedampft. Der Rückstand wird in MTBE und Wasser aufgenommen. Die wässrige Phase wird dreimal mit MTBE extrahiert. Die vereinigten organischen Phasen werden über Natriumsulfat getrocknet, filtriert und eingeengt. Nach Trocknung im Vakuum erhält man 47 g (97% d. Th.) des Produktes.
¹H-NMR (400 MHz, DMSO-d₆): δ = 1.32 (s, 9H), 2.68 (dd, 1H), 3.18 (dd, 1H), 4.25 (m, 1H), 5.15 (s, 2H), 6.88 (d, 1 H), 7.08 (d, 1H), 7.30-7.40 (m, 3 H), 7.45-7.55 (m, 3 H).

### Beispiel (-)-6A

3-(2-Benzyloxy-5-iod-phenyl)-2(*S*)-*tert*-butoxycarbonylamino-propionsäure 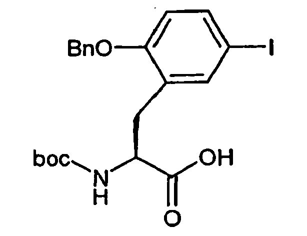 Das Racemat aus Beispiel 6A [(+/-)-3-(2-Benzyloxy-5-iod-phenyl)-2(*S*)-*tert*-butoxycarbonylamino-propionsäure] wird an einer chiralen stationären Kieselgelphase, basierend auf dem Selektor aus Poly(*N-*Methacryloyl-*L*-Leucin-dicyclopropylmethylamid), mit einem Gemisch aus *i-*Hexan/Ethylacetat als Elutionsmittel getrennt. Das zuerst eluierte Enantiomer (98.9% ee) ist in Dichlormethan rechtsdrehend ([α] D²¹: + 3.0°, c = 0.54, Dichlormethan) und entspricht dem (*R*)-Enantiomer Beispiel (+)-6A, wie durch Einkristallröntgenstrukturanalyse bestimmt wurde. Die Reinheit des zweiten, linksdrehenden Enantiomers Beispiel (-)-6A, d.h. des (*S*)-Enantiomers, beträgt > 99% ee.

### Beispiel 7A

3-(2-Benzyloxy-5-iod-phenyl)-2(*S*)-*tert*-butoxycarbonylamino-propionsäurebenzylester 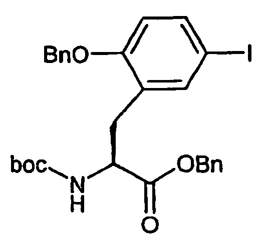 Unter Argon werden 10 g (20.11 mmol) (-)-3-(2-Benzyloxy-5-iod-phenyl)-2(*S*)-*tert*-butoxycarbonylamino-propionsäure (Beispiel (-)-6A) in 200 ml Acetonitril gelöst. Dazu werden 246 mg (2.01 mmol) 4-Dimethylaminopyridin und 4.16 ml (40.22 mmol) Benzylalkohol hinzugefügt. Die Mischung wird auf -10°C abgekühlt und mit 4.63 g (24.13 mmol) EDC versetzt. Man lässt alles langsam auf RT kommen und rührt über Nacht. Nach ca. 16 h wird das Gemisch im Vakuum einrotiert und der Rückstand säulenchromatographisch an Silicagel (Laufmittel: Dichlormethan) gereinigt. Ausbeute: 10.65 g (88% d. Th.).
HPLC (Methode 1): Rₜ = 6.03 min; LC-MS (Methode 3): Rₜ = 4.70 min
MS (DCI): m/z = 605 (M±NH₄)⁺.
¹H-NMR (200 MHz, CDCl₃): δ = 1.38 (s, 9H), 2.97 (dd, 1H), 3.12 (dd, 1H), 4.50-4.70 (m, 1H), 5.00-5.10 (m, 4H), 5.22 (d, 1H), 6.64 (d, 1H), 7.28-7.36 (m, 7H), 7.37-7.52 (m, 5H).

### Beispiel 8A

3-[2-Benzyloxy-5-(4,4,5,5-tetramethyl-[1,3,2]dioxaborolan-2-yl)-phenyl]-2(*S*)-*tert*-butoxy-carbonylamino-propionsäurebenzylester 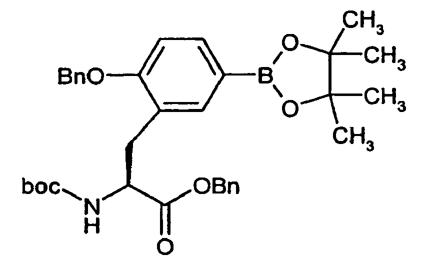 Zu einer Lösung von 10.30 g (17.53 mol) 3-(2-Benzyloxy-5-iod-phenyl)-2(*S*)-*tert*-butoxycarbonylamino-propionsäurebenzylester (Beispiel 7A) in 70 ml DMSO werden 5.15 g (52.60 mmol) Kaliumacetat zugegeben. Die Mischung wird deoxygeniert, indem durch die kräftig gerührte Lösung 15 min lang Argon durchgeleitet wird. Dann werden 5.17 g (20.16 mmol) Bis(pinacolato)diboran und 515 mg (0.70 mmol) Bis(diphenylphosphino)ferrocenpalladium(II)chlorid zugegeben. Unter leichtem Argonstrom wird auf 80°C erhitzt und nach 6 h wieder abgekühlt. Die Mischung wird über Silicagel (Laufmittel: Dichlormethan) filtriert. Der Rückstand wird säulenchromatographisch an Silicagel (Laufmittel: Cyclohexan:Ethylacetat 4:1) gereinigt.
Ausbeute: 8.15 g (79% d. Th.).
HPLC (Methode 1): Rₜ = 6.26 min.
LC-MS (Methode 2): Rₜ = 5.93 und 6.09 min.
MS (EI): m/z = 588 (M+H)⁺.
¹H-NMR (200 MHz, CDCl₃): δ = 1.26 (s, 6H), 1.33 (s, 9H), 1.36 (s, 6H), 2.91-3.10 (m, 1H), 3.12-3.28 (m, 1H), 4.49-4.68 (m, 1H), 5.05 (dd, 2H), 5.11 (dd, 2H), 5.30 (d, 1H), 6.90 (d, 1H), 7.27-7.37 (m, 7H), 7.38-7.42 (m, 3H), 7.55-7.62 (m, 1H), 7.67(dd, 1H).

### Beispiel 9A

2(*S*)-Amino-3-(2-benzyloxy-5-iod-phenyl)-propionsäure-Hydrochlorid 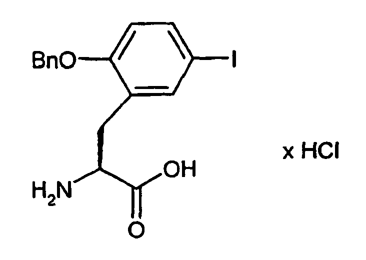 12 g (24.13 mmol) 3-(2-Benzyloxy-5-iod-phenyl)-2(*S*)-*tert*-butoxycarbonylamino-propionsäure (Beispiel (-)-6A) werden unter Argon in 60 ml einer 4M Chlorwasserstoff-Dioxan-Lösung gegeben und 2 h bei RT gerührt. Die Reaktionslösung wird eingeengt und im Hochvakuum getrocknet.
Ausbeute: 10.47 g (100% d. Th.).
HPLC (Methode 1): Rₜ = 4.10 min.
MS (EI): m/z= 398 (M+H-HCl)⁺.
¹H-NMR (200 MHz, CDCl₃): δ = 3.17-3.31 (m, 1H), 3.33-3.47 (m, 1H), 4.22 (t, 1H), 5.13 (s, 2H), 6.69 (d, 1 H), 7.24-7.40 (m, 2H), 7.41-7.45 (m, 2H), 7.48 (d, 1H), 7.52 (d, 1H), 7.60 (d, 1H), 8.66 (br.s, 2H).

### Beispiel 10A

2(*S*)-Benzyloxycarbonylamino-3-(2-benzyloxy-5-iod-phenyl)-propionsäure 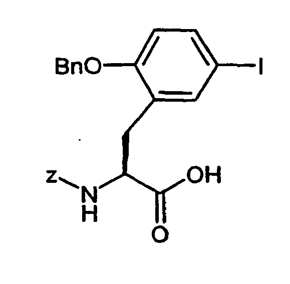 Eine Lösung aus 10.46 g (24.13 mmol) 2(*S*)-Amino-3-(2-benzyloxy-5-iod-phenyl)-propionsäure-Hydrochlorid (Beispiel 9A) in DMF wird mit 9.25 ml (53.09 mol) *N,N-*Diisopropylethylamin versetzt. Dazu gibt man 6.615 g (26.54 mmol) *N-*(Benzyloxycarbonyl)succinimid (Z-OSuc) zu. Die resultierende Lösung wird über Nacht gerührt und dann im Vakuum einrotiert. Der Rückstand wird in Dichlormethan aufgenommen und jeweils zweimal mit 0.1N Salzsäurelösung und gesättigter wässriger Natriumchlorid-Lösung ausgeschüttelt. Die organische Phase wird getrocknet, filtriert und eingeengt. Die Mischung wird durch Säulenchromatographie an Silicagel (Laufmittel: Cyclohexan/Diethylether 9:1 bis 8:2) gereinigt.
Ausbeute: 8.30 g (65% d. Th.).
HPLC (Methode 1): Rₜ = 5.01 min.
MS (EI): m/z = 532 (M+H)⁺.
¹H-NMR (200 MHz, DMSO-d₆): δ = 3.14-3.3 (m, 2 H), 4.25-4.45 (m, 1H), 4.97 (s, 2H), 5.14 (s, 2H), 6.88 (d, 1 H), 7.20-7.56 (m, 12 H), 7.62 (d, 1 H), 12.73 (br.s, 1H).

### Beispiel 11A

2(*S*)-Benzyloxycarbonylamino-3-(2-benzyloxy-5-iod-phenyl)-propionsäure-(2-trimethylsilyl)-ethylester 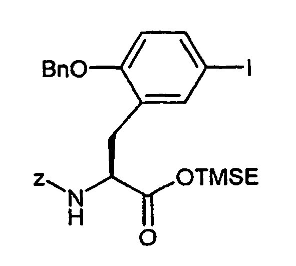 8.35 g (15.7 mmol) 2(*S*)-Benzyloxycarbonylamino-3-(2-benzyloxy-5-iod-phenyl)-propionsäure (Beispiel 10A) werden in 150 ml THF vorgelegt und mit 2.14 g (18.07 mmol) 2-Trimethylsilylethanol und 250 mg (2.04 mmol) 4-Dimethylaminopyridin versetzt. Die Mischung wird auf 0° abgekühlt und mit 2.38 g (2.95 ml, 18.86 mmol) *N,N'*-Diisopropylcarbodiimid, gelöst in 40 ml THF, versetzt. Es wird über Nacht bei RT gerührt und zur Aufarbeitung im Vakuum einrotiert. Der Rückstand wird in Dichlormethan aufgenommen und jeweils zweimal mit 0.1N Salzsäurelösung und gesättigter wässriger Natriumchlorid-Lösung ausgeschüttelt. Die organische Phase wird getrocknet, filtriert und eingeengt. Die Mischung wird säulenchromatographisch (Silicagel, Laufmittel: Cyclohexan/Diethylether 9:1 bis 8:2) gereinigt.
Ausbeute: 8.2 g (83% d. Th.).
HPLC (Methode 1): Rₜ = 6.42 min
MS (EI): m/z = 532 (M+H)⁺.
¹H-NMR (300 MHz, CDCl₃): δ = 0.01 (s, 9H), 0.88 (t, 2H), 2.96 (dd, 1H), 3.13 (dd, 1H), 4.04-4.17 (m, 2H), 4.51-4.62 (m, 1H), 4.95-5.05 (m, 4H), 5.44 (d, 1H), 6.64 (d, 1H), 7.25-7.33 (m, 7 H), 7.37 (dd, 4H), 7.45 (dd, 1H).

### Beispiel 12A

2(*S*)-Benzyloxycarbonylamino-3-[4,4'-bis-benzyloxy-3'-(2(*S*)-benzyloxycarbonyl-2-*tert-*butoxycarbonylamino-ethyl)-biphenyl-3-yl]-propionsäure-2-(trimethylsilyl)-ethylester 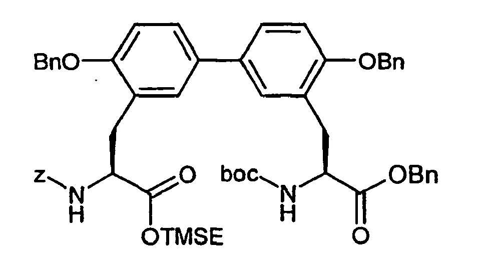 Zu einer Lösung von 0.316 g (0.5 mmol) 2(*S*)-Benzyloxycarbonylamino-3-(2-benzyloxy-5-iod-phenyl)-propionsäure-(2-trimethylsilyl)-ethylester (Beispiel 11A) in 2.5 ml entgastem DMF werden unter Argon bei RT 45.8 mg (0.05 mmol) Bis(diphenylphosphino)ferrocen-palladium(II)chlorid (PdCl₂(dppf)) und 0.325 g (1.0 mmol) Cäsiumcarbonat hinzugegeben. Das Reaktionsgemisch wird auf 40°C erhitzt. Innerhalb von 30 min wird eine Lösung von 0.294 g (0.5 mmol) 3-[2-Benzyloxy-5-(4,4,5,5-tetramethyl-[1,3,2]dioxaborolan-2-yl)-phenyl]-2(*S*)-*tert*-butoxy-carbonylamino-propionsäurebenzylester (Beispiel 8A) in 2.5 ml entgastem DMF zugetropft. Das Reaktionsgemisch wird 4 h bei 40°C und weitere 2 h bei 50°C gerührt. Das Lösungsmittel wird eingedampft und der Rückstand in Essigsäureethylester aufgenommen. Die organische Phase wird zweimal mit Wasser ausgeschüttelt, über Natriumsulfat getrocknet und eingeengt. Das Rohprodukt wird durch Kieselgelchromatographie mit Dichlormethan/Essigsäureethylester (30/1) gereinigt. Man erhält 0.320 g (66% d. Th.) des Produktes.
HPLC (Methode 1): Rₜ = 7.65 min
MS (EI): m/z = 987 (M+Na), 965 (M+H)⁺.
¹H-NMR (200 MHz, CDCl₃): δ = 0.00 (s, 9H), 0.90 (t, 2H), 1.37 (s, 9H), 3.02-3.35 (m, 4H) 4.06-4.25 (m, 2H), 4.55-4.73 (m, 2H), 4.98-5.18 (m, 8H), 5.40 (d, 1H), 5.63 (d, 1H), 6.88-7.00 (m, 2H), 7.19-7.39 (m, 20H), 7.42-7.53 (m, 4H).

### Beispiel 13A

Benzyl-({(2*R,*4*S*)-4-[(*tert*-butoxycarbonyl)amino]-5-oxotetrahydrofuran-2-yl}methyl)carbamat 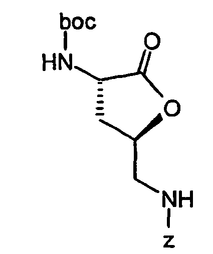 Eine Lösung von 7.60 g (17.3 mmol) 5-Benzyloxycarbonylamino-2(*S*)-*tert*-butoxycarbonylamino-4(*R*)-hydroxy-pentansäure-*tert*-butylester (Org. Lett., 2001, 3, 20, 3153-3155) in 516 ml Dichlormethan und 516 ml Trifluoressigsäure wird 2 h bei RT gerührt. Das Lösungsmittel wird eingedampft. Das zurückbleibende Rohprodukt wird in 2.6 1 wasserfreiem Methanol gelöst, und unter Rühren bei 0°C werden 6.3 g (28.8 mmol) Di-*tert*-Butyldicarbonat und 7.3 ml (52.43 mmol) Triethylamin hinzugegeben. Nach 15 h wird die Reaktionslösung eingedampft und der Rückstand in 1 1 Essigsäureethylester aufgenommen. Nach Trennung der Phasen wird die organische Phase zweimal mit einer 5%-igen Zitronensäure-Lösung, zweimal mit Wasser und einmal mit gesättigter wässriger Natriumchlorid-Lösung ausgeschüttelt, über Natriumsulfat getrocknet und eingeengt. Das Rohprodukt wird durch Kieselgelchromatographie mit Toluol/Aceton (5/1) gereinigt. Man erhält 4.92 g (78% d. Th.) des Produktes.
LC-HR-FT-ICR-MS (Methode 7):
ber. für C₁₈H₂₈N₃O₆ (M+NH₄)⁺ 382.19726
gef. 382.19703.
¹H-NMR (400 MHz, CDCl₃): δ = 1.45 (s, 9H), 2.3-2.4 (m, 1H), 2.45-2.55 (m, 1H), 3.3-3.4 (m, 1H), 3.5-3.6 (m, 1H), 4.17-4.28 (m, 1H), 4.7-4.8 (m, 1H), 5.0-5.15 (m, 4H), 7.3-7.4 (m, 5H).

### Beispiel 14A

5-Benzytoxycarbonylamino-2(*S*)-*tert*-butoxycarbonylamino-4(*R*)-(*tert*-butyl-dimethylsilanyloxy)-pentansäure

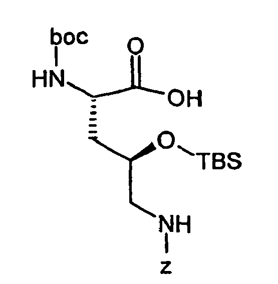

### Methode A:

Zu einer Lösung von 0.73 g (2 mmol) der Verbindung aus Beispiel 13A in 50 ml 1,4-Dioxan werden bei 0°C 2 ml 1M Natronlauge hinzugegeben. Die Reaktionslösung wird 2 h gerührt und dann eingedampft. Der Rückstand wird in 50 ml Dichlormethan aufgenommen. Zu dieser Lösung werden 1.12 ml (8 mmol) Triethylamin hinzugegeben und nach einer kurzen Zeit 1.38 ml (6 mmol) Trifluormethansulfonsäure-*tert*-butyl-dimethylsilylester zugetropft. Nach 3 h Rühren bei RT wird das Reaktionsgemisch mit Dichlormethan verdünnt. Die organische Phase wird mit 1N Natriumbicarbonat-Lösung gewaschen, über Natriumsulfat getrocknet und eingedampft. Das Rohprodukt wird in 7.4 ml 1,4-Dioxan gelöst und mit 36.2 ml 0.1N Natronlauge versetzt. Nach 3 h Rühren bei RT wird die Reaktionslösung eingedampft und der Rückstand in Wasser und Essigsäureethylester aufgenommen. Die organische Phase wird dreimal mit Essigsäureethylester extrahiert. Die vereinigten organischen Phasen werden über Natriumsulfat getrocknet und eingedampft. Man erhält 0.90 g (90% d. Th.) des Produktes.

### Methode B:

Eine Lösung von 14.0 g (38 mmol) 2(*S*)-*tert*-Butoxycarbonylamino-4(*R*)-hydroxy-5-nitro-pentansäure-benzylester (Beispiel 22A) in 840 ml Ethanol/Wasser 9/1 wird mit 1.96 g Palladium auf Kohle (10%ig) versetzt und unter Normaldruck 24 h bei RT hydriert. Es wird über Kieselgur filtriert, und das Filtrat wird mit 14.7 g (114 mmol) Diisopropylethylamin versetzt. Anschließend werden 11.4 g (45.6 mmol) *N-*(Benzyloxycarbonyloxy)-succinimid hinzugegeben, und es wird 4 h bei RT gerührt. Die Lösung wird eingeengt, der Rückstand in Dichlormethan aufgenommen und zweimal mit 0.1N Salzsäure ausgeschüttelt. Die organische Phase wird abgetrennt und mit 14.7 g (114 mmol) Diisopropylamin alkalisch gestellt. Die Lösung wird auf 0°C gekühlt, mit 30.1 g (114 mmol) Trifluormethansulfonsäure-dimethyl-*tert*-butylsilylester versetzt und bei RT 2.5 h gerührt.

Die organische Phase wird mit gesättigter Natriumhydrogencarbonat-Lösung gewaschen, über Natriumsulfat getrocknet und einrotiert. Der Rückstand wird in 50 ml Dioxan gelöst, mit 200 ml 0.1N Natronlauge versetzt und 3 h bei RT gerührt. Es wird mehrmals mit Essigsäureethylester extrahiert, die gesammelten organischen Phasen werden über Natriumsulfat getrocknet und im Vakuum eingeengt. Der Rückstand wird an Kieselgel chromatographiert (Laufmittel: Dichlormethan/Ethanol 20/1, 9/1). Man erhält 8.11 g (43% d. Th.) des Produktes.
MS (ESI): m/z = 497 (M+H)⁺.
¹H-NMR (300 MHz, DMSO-d₆): δ = 0.00 (s, 6H), 0.99 (s, 9H), 1.33 (s, 9H), 1.59 (m, 1H), 1.80 (m, 1H), 2.75-3.15 (m, 2H), 3.81 (m, 1H), 3.98 (m, 1H), 4.96 (m, 2H), 7.04 (d, 1H), 7.19 (m, 1H), 7.30 (m, 5H), 12.37 (br. s, 1H).

### Beispiel 15A

3-[3'-(2(*S*)-Amino-2-benzyloxycarbonyl-ethyl)-4,4'-bis-benzyloxy-biphenyl-3-yl]-2(*S*)-benzyl-oxycarbonylamino-propionsäure-2-(trimethylsilyl)-ethylester-Hydrochlorid 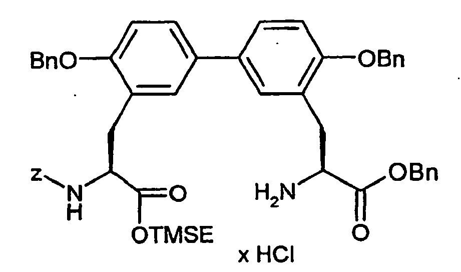 Zu einer auf 0°C gekühlten Lösung von 2.65 g (2.75 mmol) 2(*S*)-Benzyloxycarbonylamino-3-[4,4'-bis-benzyloxy-3'-(2(*S*)-benzyloxycarbonyl-2-*tert*-butoxycarbonylamino-ethyl)-biphenyl-3-yl]-propionsäure-2-(trimethylsilyl)-ethylester (Beispiel 12A) in 50 ml wasserfreiem Dioxan werden 50 ml einer 4M Chlorwasserstoff-Dioxan-Lösung über ca. 20 min hinzugegeben. Nach 3 h Rühren wird die Reaktionslösung eingedampft und im Hochvakuum getrocknet.
Ausbeute: 100% d. Th.
HPLC (Methode 1): Rₜ = 5.96 min.
MS (EI): m/z = 865 (M+H)⁺.

### Beispiel 16A

2(*S*)-[5-Benzyloxycarbonylamino-2(*S*)-*tert*-butoxycarbonylainino-4(*R*)-(*tert*-butyldimethylsilyloxy)-pentanoylamino]-3-{4,4'-bis-benzyloxy-3'-[2(*S*)-benzyloxycarbonylamino-2-(2-trimethylsilyl-ethoxycarbonyl)-ethyl]-biphenyl-3-yl}-propionsäurebenzylester 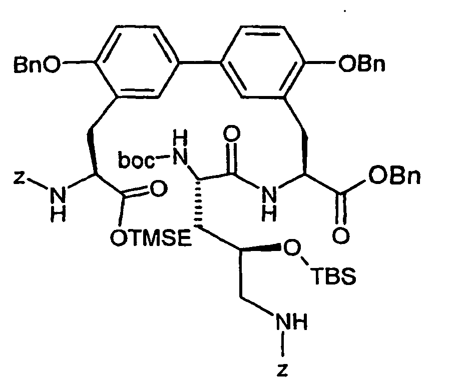 Zu einer auf 0°C gekühlten Lösung von 0.520 g (0.58 mmol) 3-[3'-(2(*S*)-Amino-2-benzyloxycarbonyl-ethyl)-4,4'-bis-benzyloxy-biphenyl-3-yl]-2(*S*)-benzyloxycarbonyl-amino-propionsäure-(2-trimethylsilyl)-ethylester-Hydrochlorid (Beispiel 15A) und 0.287 g (0.58 mmol) 5-Benzyloxycarbonylamino-2(*S*)-*tert*-butoxycarbonylamino-4(*R*)-(*tert*-butyldimethylsilyloxy)-pentansäure (Beispiel 14A) in 7.3 ml wasserfreiem DMF werden 0.219 g (0.58 mmol) HATU und 0.082 g (0.63 mmol) *N*,*N*-Diisopropylethylamin hinzugegeben. Nach 30 min Rühren bei 0°C werden zusätzliche 0.164 g (1.26 mmol) *N*,*N*-Diisopropylethylamin hinzugegeben. Das Reaktionsgemisch wird 15 h bei RT gerührt. Das Lösungsmittel wird dann eingedampft und der Rückstand in Essigsäureethylester aufgenommen. Die organische Phase wird dreimal mit Wasser und einmal mit gesättigter wässriger Natriumchlorid-Lösung gewaschen, über Natriumsulfat getrocknet und eingeengt. Das Rohprodukt wird durch Kieselgelchromatographie mit Dichlormethan/Essigsäureethylester (Gradient 30/1→20/1→10/1) gereinigt. Man erhält 533 mg (66% d. Th.) des Produktes.
LC-MS (ESI, Methode 6): m/z = 1342 (M+H)⁺, 1365 (M+Na)⁺.

### Beispiel 17A

2(*S*)-Benzyloxycarbonylamino-3-{4,4'-bis-benzyloxy-3'-[2(*S*)-benzylexycarbonyl-2-(5-benzyloxycarbonylamino-2(*S*)-*tert*-butoxycarbonylamino-4(*R*)-hydroxy-pentanoylamino)-ethyl]-biphenyl-3-yl}-propionsäure 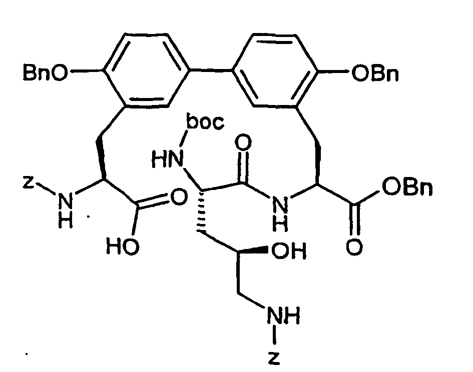 Zu einer Lösung von 800 mg (0.6 mmol) 2(*S*)-[5-Benryloxycarbonylamino-2(*S*)-*tert*-butoxycarbonylamino-4(*R*)(*tert*-butyldimethylsilyloxy)-pentanoylamino]-3-{4,4'-bis-benzyloxy-3'-[2(*S*)-benzyloxycarbonylarnino-2-(2-trimethylsilyl-ethoxycarbonyl)-ethyl]-biphenyl-3-yl}-propionsäurebenzylester (Beispiel 16A) in 26 ml absolutem DMF werden bei RT tropfenweise 1.8 ml 1N Tetrabutylammoniumfluorid in THF hinzugegeben. Nach 25 min bei RT wird auf 0°C gekühlt und mit viel Eiswasser versetzt. Es wird sofort mit Ethylacetat und etwas 1N Salzsäure-Lösung versetzt, die organische Phase mit Magnesiumsulfat getrocknet, eingeengt und 1 h im Hochvakuum getrocknet. Das Rohprodukt wird ohne weitere Reinigung umgesetzt.

| | |
|---|---|
| LC-MS (ESI, Methode 4): | m/z = 1129 (M+H)⁺. |
| LC-HR-FT-ICR-MS (Methode 7): | ber. für C₆₅H₆₉N₄O₁₄ (M+H)⁺ 1129.48048 gef. 1129.48123. |

### Beispiel 18A

2(*S*)-(5-Benzyloxycarbonylamino-2(*S*)-*tert*-butoxycarbonylamino-4(*R*)-hydroxy-pentanoylamino)-3-[4,4'-bis-benzyloxy-3'-(2(*S*)-benzyloxycarbonylamino-2-pentafluorphenyloxycarbonyl-ethyl)-biphenyl-3-yl]-propionsäurebenzylester 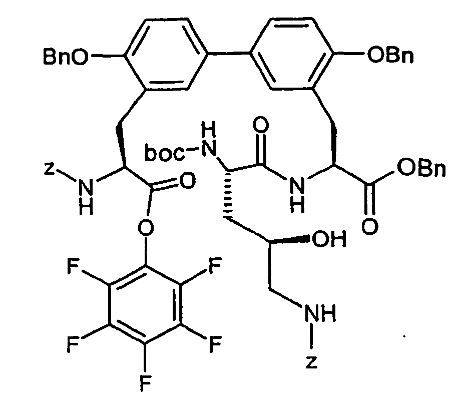 691 mg (Rohgemisch, ca. 0.6 mmol) 2(*S*)-Benzyloxycarbonylamino-3-{4,4'-bis-benzyloxy-3'-[2(*S*)-benzyloxycarbonyl-2-(5-benzyloxycarbonylamino-2(*S*)-*tert*-butoxycarbonylamino-4(*R*)hydroxy-pentanoylamino)-ethyl]-biphenyl-3-yl}-propionsäure (Beispiel 17A) werden in 25 ml Dichlormethan vorgelegt und mit 547.6 mg (2.98 mmol) Pentafluorphenol, gelöst in 6 ml Dichlormethan, versetzt. Man fügt 7.3 mg (0.06 mmol) DMAP hinzu und kühlt auf -25°C (Ethanol/Kohlendioxid-Bad). Bei -25°C werden 148 mg (0.774 mmol) EDC hinzugefügt. Die Mischung erwärmt sich über Nacht langsam auf RT. Die Reaktionsmischung wird im Vakuum eingeengt und im Hochvakuum kurz getrocknet. Das Rohprodukt wird ohne weitere Reinigung umgesetzt.

| | |
|---|---|
| LC-MS (ESI, Methode 5): | m/z = 1317 (M+Na)⁺, 1295 (M+H)⁺. |
| LC-HR-FT-ICR-MS (Methode 7): | ber. für C₇₁H₆₈F₅N₄O₁₄ (M+H)⁺ 1295.46467 gef. 1295.46430. |

### Beispiel 19A

5,17-Bis-benzyloxy-14(*S*)-benzyloxycarbonylamino-11(*S*)-(3-benzyloxycarbonylamino-2(*R*)-hydroxy-propyl)-10,13-dioxo-9,12-diaza-tricyclo[14.3.1.1^{2,6}]henicosa-1(19),2,4,6(21),16(20),17-hexaen-8(*S*)-carbonsäurebenzylester

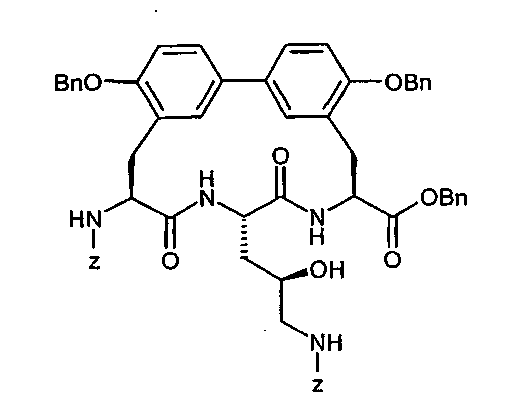

### Methode A:

Zu einer Lösung von 119.3 mg 2(*S*)-(5-Benzyloxycarbonylamino-2(*S*)-*tert*-butoxycarbonylamino-4(*R*)-hydroxy-pentanoylamino)-3-[4,4'-bis-benzyloxy-3'-(2(*S*)-benzyloxycarbonylamino-2-pentafluorphenyloxycarbonyl-ethyl)-biphenyl-3-yl]-propionsäurebenzylester (Beispiel 18A) in 2.7 ml 1,4-Dioxan werden 4 ml einer 4M Chlorwasserstoff-Dioxan-Lösung hinzugegeben. Bis zum Reaktionsende werden weitere 1.5 ml einer 4M Chlorwasserstoff-Dioxan-Lösung zugegeben. Die Reaktionslösung wird eingedampft und zweimal mit Chloroform codestilliert. Das Rohprodukt (LC-HR-FT-ICR-MS, Methode 7: ber. für C₆₆H₆₀F₅N₄O₁₂ (M+H)⁺ 1195.41224, gef. 1195.41419) wird in 100 ml Chloroform gelöst und über 3 h zu einer sehr gut gerührten Suspension von 200 ml Chloroform und 100 ml gesättigter wässriger Natriumhydrogencarbonat-Lösung hinzugetropft. Die Reaktionsmischung wird 2 h kräftig gerührt. Nach Trennung der zwei Phasen wird die wässrige Phase mit Chloroform extrahiert. Die vereinigten organischen Phasen werden mit 5%-iger wässriger Zitronensäure-Lösung gewaschen, über Magnesiumsulfat getrocknet und zur Trockne eingedampft. Das Rohprodukt wird mit Acetonitril gewaschen und im Hochvakuum getrocknet.
Ausbeute: 60.5 mg (65% d. Th.)
LC-MS (ESI, Methode 5): m/z= 1011 (M+H)⁺.

### Methode B:

771 mg (0.595 mmol) 2(*S*)-(5-Benzyloxycarbonylamino-2(*S*)-*tert*-butoxycarbonyl-amino-4(*R*)-hydroxy-pentanoylamino)-3-[4,4'-bis-benzyloxy-3'-(2(*S*)-benzyloxy-carbonyl-amino-2-pentafluorphenyloxycarbonyl-ethyl)-biphenyl-3-yl]-propionsäure-benzylester (Beispiel 18A) werden in 8 ml Dioxan gelöst und dann bei 0°C mit 16 ml einer 4N Chlorwasserstoff-Dioxan-Lösung tropfenweise versetzt. Nach 45 min erfolgt erneute Zugabe von 6 ml einer 4N Chlorwasserstoff-Dioxan-Lösung und nach 15 min nochmals 8 ml. Die Mischung wird 30 min bei 0°C gerührt, bevor die Reaktionslösung schonend eingeengt, mit Chloroform codestilliert (zweimal) und kurz im Hochvakuum getrocknet wird. Das Rohprodukt (732 mg, 0.59 mmol) wird in 1000 ml Chloroform gelöst und tropfenweise mit einer Lösung von 6 ml Triethylamin in 50 ml Chloroform versetzt. Es wird über Nacht bei RT gerührt. Zur Aufarbeitung wird das Gemisch schonend im Vakuum einrotiert und der Rückstand in Acetonitril verrührt. Die entstandenen Kristalle werden abgesaugt, mit Acetonitril gewaschen und im Hochvakuum getrocknet.
Ausbeute: 360 mg (60% d. Th.).
MS (EI): m/z = 1011 (M+H)⁺.
HPLC (Methode 1): Rₜ = 5.59 min.
¹H-NMR (400 MHz, DMSO-d₆): δ = 1.52-1.65 (m, 1H), 1.73-1.84 (m, 1H), 2.82-3.01 (m, 3H), 3.02-3.11 (m, 1H), 3.46 (s, 1H), 3.57-3.68 (m, 1H), 4.47-4.56 (m, 1H), 4.64-4.71 (m, 1H), 4.73-4.85 (m, 2H), 4.88-5.00 (m, 4H), 5.09 (s, 2H), 5.14-5.20 (m, 4H), 6.29 (d, 1H), 7.00-7.11 (m, 4H), 7.21-7.40 (m, 20H), 7.41-7.48 (m, 9H), 8.77 (d, 1H), 8.87 (d, 1H).

### Beispiel 20A

14(*S*)-Amino-11(*S*)-(3-amino-2(*R*)-hydroxy-propyl)-5,17-dihydroxy-10,13-dioxo-9,12-diazatricyclo[14.3.1.1^{2,6}]henicosa-1(19),2,4,6(21),16(20),17-hexaen-8(*S*)-carbonsäure-Dihydrochlorid (Biphenomycin B)

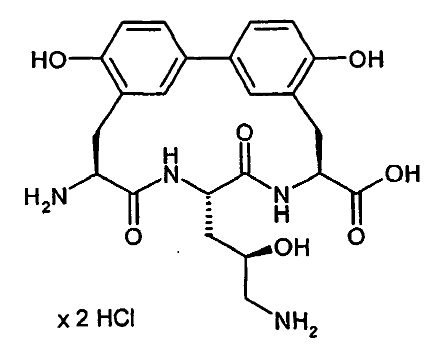

Es werden 200 mg (0.20 mmol) 5,17-Bis-benzyloxy-14(*S*)-benzyloxycarbonylamino-11(*S*)-(3-benzyloxycarbonylamino-2(*R*)-hydroxy-propyl)-10,13-dioxo-9,12-diaza-tricyclo[14.3.1.1^{2,6}]-henicosa-1(19),2,4,6(21),16(20),17-hexaen-8(*S*)-carbonsäure-benzylester (Beispiel 19A) in einem Gemisch aus 220 ml Essigsäure/Wasser/Ethanol 4:1:1 gegeben (Ethanol kann durch THF substituiert werden). Dazu gibt man 73 mg 10%ige Palladium/Kohle (10% Pd/C) und hydriert anschließend 15 h bei Normaldruck. Das Reaktionsgemisch wird über vorgewaschenem Kieselgur filtriert und das Filtrat im Vakuum einrotiert. Der Rückstand wird mit 4.95 ml 0.1N wässriger Salzsäure versetzt und eingeengt. Man verrührt den Rückstand mit 10 ml Diethylether und dekantiert ab. Der zurückgebliebene Feststoff wird im Hochvakuum getrocknet.
Ausbeute: 103 mg (95% d. Th.).
HPLC (Methode 1): Rₜ = 3.04 min;
LC-MS (Methode 2): Rₜ = 0.38 min
MS (EI): m/z =473 (M+H)⁺.
¹H-NMR (400 MHz, D₂O): δ = 2.06-2.20 (m, 1H), 2.74-2.89 (m, 1H), 2.94-3.05 (m, 1H), 3.12-3.25 (m, 2H), 3.53 (d, 1H), 3.61-3.72 (m, 1H), 3.97-4.07 (m, 1H), 4.53 (s, 1H), 4.61 (d, 1H), 4.76-4.91 (m, 12H), 7.01-7.05 (m, 2H), 7.07 (s, 1H), 7.40-7.45 (m, 2H), 7.51 (d, 1H).

### Beispiel 21A

2(*S*)-*tert*-Butoxycarbonylamino-5-nitro-4-oxo-pentansäurebenzylester 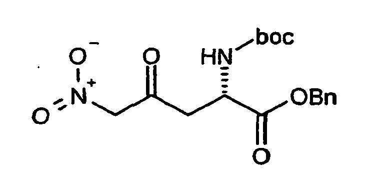 Eine Lösung A von 10 g (30.9 mmol) 2(*S*)-*tert*-Butoxycarbonylamino-bernsteinsäure-1-benzylester und 5.27 g (32.5 mmol) 1,1'-Carbonyldiimidazol in 100 ml Tetrahydrofuran wird 5 h bei RT gerührt. Zu einer Lösung B von 3.2 g (34.2 mmol) Kalium-*tert*-butylat in 100 ml Tetrahydrofuran werden bei 0°C 18.8 g (30.9 mmol) Nitromethan zugetropft. Die Lösung B wird unter Erwärmen auf RT nachgerührt, und anschließend wird bei RT Lösung A zugetropft. Die resultierende Mischung wird 16 h bei RT gerührt und mit 20%iger Salzsäure auf pH 2 eingestellt. Das Lösungsmittel wird eingedampft. Das zurückbleibende Rohprodukt wird in Essigsäureethylester/Wasser aufgenommen. Nach Trennung der Phasen wird die organische Phase zweimal mit Wasser ausgeschüttelt, über Natriumsulfat getrocknet und eingeengt. Man erhält 13 g (99% d.Th.) des Produktes.
MS (ESI): m/z = 334 (M+H)^{+ 1}H-NMR (300 MHz, DMSO-d₆): δ = 1.37 (s, 9H), 2.91 (m, 1H), 3.13 (m, 1H), 4.44 (m, 1H), 5.12 (s, 2H), 5.81 (m, 2H), 7.2-7.5 (m, 5H).

### Beispiel 22A

2(*S*)-*tert*-Butoxycarbonylamino-4(*R*)-hydroxy-5-nitro-pentansäurebenzylester 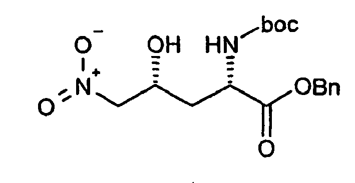 Eine Lösung von 11.3 g (30.8 mmol) 2(*S*)-*tert*-Butoxycarbonylamino-5-nitro-4-oxo-pentansäurebenzylester in 300 ml Tetrahydrofuran wird auf-78°C gekühlt, mit 30.8 ml einer 1M Lösung von L-Selectrid^{®} in Tetrahydrofuran tropfenweise versetzt und 1 h bei -78°C nachgerührt. Nach Erwärmen auf RT wird die Lösung vorsichtig mit gesättigter Ammoniumchlorid-Lösung versetzt. Die Reaktionslösung wird eingeengt und der Rückstand in Wasser und Essigsäureethylester aufgenommen. Die wässrige Phase wird dreimal mit Essigsäureethylester extrahiert. Die vereinigten organischen Phasen werden über Natriumsulfat getrocknet und eingedampft. Das Rohprodukt wird an Kieselgel 60 vorgereinigt (Laufmittel: Cyclohexan/Essigsäureethylester 10/1), die gesammelten Fraktionen werden eingeengt und mit Cyclohexan/Essigsäureethylester 5/1 ausgerührt. Die zurückbleibenden Kristalle werden abgesaugt und getrocknet. Man erhält 2.34 g (21% d. Th.) des gewünschten Diastereomers. Aus der Mutterlauge erhält man durch chromatographische Trennung an Lichrospher Diol 10 µM (Laufmittel: Ethanol/*iso*-Hexan 5/95) weitere 0.8 g (6.7% d.Th.) des Produktes.
MS (ESI): m/z = 369 (M+H)⁺.
¹H-NMR (300 MHz, DMSO-d₆): δ = 1.38 (s, 9H), 1.77 (m, 1H), 1.97 (m, 1H), 4.10-4.44 (m, 3H), 4.67 (m, 1H), 5.12 (m, 2H), 5.49 (d, 1H), 7.25-7.45 (m, 5H).

### Beispiel 23A

2(*S*)-[*S*-Benzyloxycarbonylamino-2(*S*)-*tert*-butoxycarbonylamino-pentanoylamino]-3-{4,4'-bis-benzyloxy-3'-[2(*S*)-benzyloxycarbonylamino-2-(2-trimethylsilyl-ethoxy-carbonyl)-ethyl]-biphenyl-3-yl}-propionsäurebenzylester 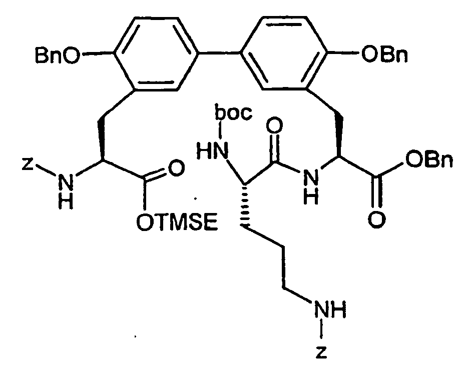 Die Herstellung erfolgt analog Beispiel 16A aus 0.47 g (0.51 mmol) der Verbindung aus Beispiel 15A und 0.19 g (0.51 mmol) *N*⁵-[(Benzyloxy)carbonyl]-*N*²-(*tert*-butoxycarbonyl)-L-omithin mit 0.19 g (0.51 mmol) HATU und 0.35 ml (1.65 mmol) *N*,*N*-Diisopropylethylamin in 5.55 ml trockenem DMF.
Ausbeute: 0.58 g (92% d.Th.)
LC-MS (Methode 10): Rₜ = 3.46 min
MS (ESI): m/z = 1212 (M+H)⁺

### Beispiel 24A

2(*S*)-Benzyloxycarbonylamino-3-{4,4'-bis-benzyloxy-3'-[2(*S*)-benzyloxycarbonyl-2-(5-benzyloxycarbonylamino)-2(*S*)-*tert*-butoxycarbonylamino-pentanoylarnino)-ethyl]-biphenyl-3-yl}-propionsäure

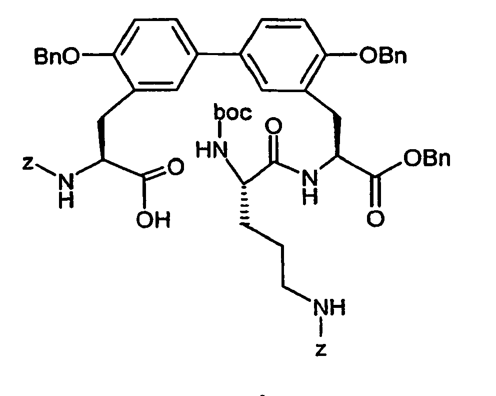

Die Herstellung erfolgt analog Beispiel 17A aus 0.82 g (0.68 mmol) der Verbindung aus Beispiel 23A mit 2 Äquivalenten (1.3 ml) Tetrabutylammoniumfluorid (1M in THF) in 30 ml getrocknetem DMF.
Ausbeute: 772 mg (94% d.Th.)
LC-MS (Methode 11): Rₜ= 1.62 min.
MS (ESI): m/z = 1112 (M+H)⁺

### Beispiel 25A

2(*S*)-(5-Benzyloxycarbonylamino-2(*S*)-*tert*-butoxycarbonylamino-pentanoylamino)-3-[4,4'-bis-benzyloxy-3'-(2(*S*)-benzyloxycarbonylamino-2-pentafluorphenyloxycarbonylethyl)-biphenyl-3-yl]-propionsäurebenzylester 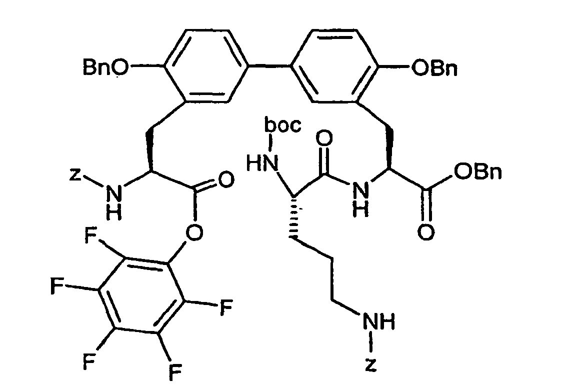 Die Herstellung erfolgt analog Beispiel 18A aus 422 mg (0.38 mmol) der Verbindung aus Beispiel 24A und 349 mg (1.9 mmol) Pentafluorphenol mit 80 mg (0.42 mmol) EDC und 4.63 mg (0.04 mmol) DMAP in 4 ml Dichlormethan.
Ausbeute: 502 mg (95% d.Th.)
LC-MS (Methode 11): Rₜ = 3.13 min.
MS (ESI): m/z = 1278 (M+H)⁺

### Beispiel 26A

2(*S*)-(5-Benzyloxycarbonylamino-2(*S*)-amino-pentanoylamino)-3-[4,4'-bis-benzyloxy-3'-(2-(*S*)-benzyloxycarbonylamino-2-pentafluorphenyloxycarbonyl-ethyl)-biphenyl-3-yl]-propionsäurebenzylester-Hydrochlorid 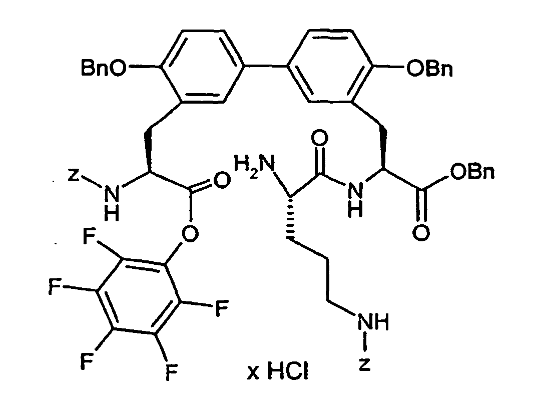 215 mg (0.17 mmol) der Verbindung aus Beispiel 25A werden in einem Eisbad unter Rühren mit 5 ml einer 4N Chlorwasserstoff-Dioxan-Lösung versetzt. Man lässt eine Stunde rühren und dampft alles im Vakuum bis zur Gewichtskonstanz ein.
Ausbeute: 200 mg (92% d.Th.)
LC-MS (Methode 11): Rₜ = 4.25 min.
MS (ESI): m/z = 1178 (M-HCl+H)⁺

### Beispiel 27A

5,17-Bis-benzyloxy-14(*S*)-benzyloxycarbonyl-amino-11(*S*)-(3-benzyloxycarbonylamino-propyl)-10,13-dioxo-9,12-diaza-tricyclo[14.3.1.1^{2.6}]-henicosa-1(19),2,4,6(21),16(20),17-hexaen-8(*S*)-carbonsäurebenzylester 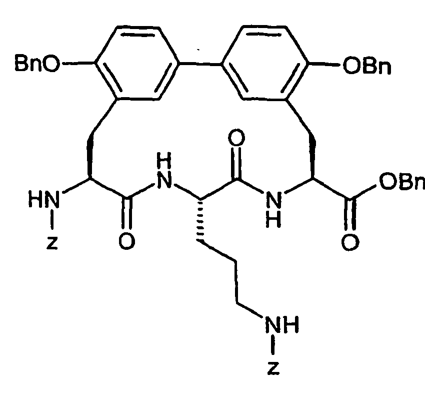 1.35 g (0.91 mmol) der Verbindung aus Beispiel 26A werden in 3 1 Chloroform vorgelegt und unter kräftigem Rühren innerhalb von 20 min bei RT mit 2.54 ml (18.2 mmol) Triethylamin in 50 ml Chloroform versetzt. Man lässt über Nacht nachrühren und dampft alles im Vakuum zur Trockne ein. Den Rückstand verrührt man mit 5 ml Acetonitril, filtriert und trocknet den Rückstand bis zur Gewichtskonstanz.
Ausbeute: 890 mg (93% d.Th.)
LC-MS (Methode 11): Rₜ = 5.10 min.
MS (ESI): m/z = 994 (M+H)⁺

### Beispiel 28A

(8*S*,11*S*,14*S*)-14-Amino-11-(3-aminopropyl)-5,17-dihydroxy-10,13-dioxo-9.12-diazatricyclo-[14.3.1.1^{2.6}]-henicosa-1(20),2(21),3,5,6,18-hexaen-8-carbonsäure-Dihydrochlorid 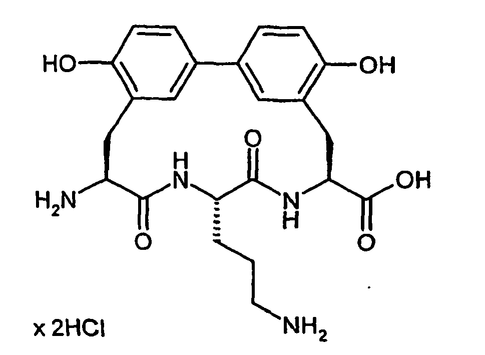 50 mg (0.05 mmol) der Verbindung aus Beispiel 27A werden in 50 ml Eisessig / Wasser / Ethanol (4/1/1) suspendiert, mit 30 mg Pd/C (10%-ig)-Katalysator versetzt und 20 Stunden bei RT hydriert. Nach Abfiltrieren des Katalysators über Kieselgur dampft man das Filtrat im Vakuum zur Trockne ein und versetzt unter Rühren mit 2.5 ml 0.1N Salzsäure. Man dampft im Vakuum zur Trockne ein und trocknet bis zur Gewichtskonstanz.
Ausbeute: 17 mg (63% d. Th.)
DC (Methanol / Dichlormethan / 25%-iger Ammoniak = 5 / 3 / 2): R_{f}= 0.6
LC-MS (Methode 3): Rₜ = 0.28 min.
MS (ESI): m/z = 457 (M-2HCl+H)⁺

### Beispiel 29A

(8*S*,11*S*,14*S*)-14-[(*tert*-Butoxycarbonyl)-amino-11-[3-[(*tert*-butoxycarbonyl)amino]propyl}-5,17-dihydroxy-10,13-dioxo-9,12-diazatricyclo[14.3.1.1^{2.6}]-henicosa-1(20),2(21),3,5,16,18-hexaen-8-carbonsäure 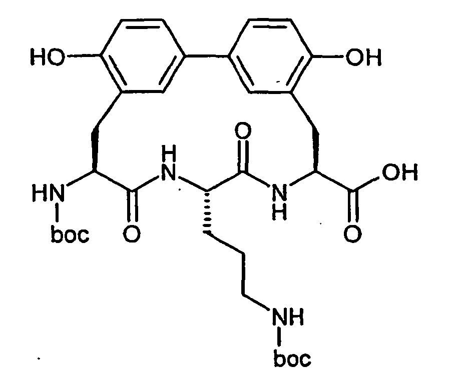 600 mg (1.13 mmol) der Verbindung aus Beispiel 28A werden in 6 ml (5.66 mmol) 1N Natronlauge gelöst und bei Raumtemperatur unter Rühren mit 740.8 mg (3.39 mmol) Di-*tert*-butyl-dicarbonat, gelöst in 5 ml Methanol, versetzt. Nach einer Stunde ist die Reaktion beendet (DC-Kontrolle, Laufmittel: Dichiormethan/Methanol/Ammoniak = 80/20/2). Durch Zutropfen von 0.1N Salzsäure stellt man pH = 3 ein. Man extrahiert dreimal mit je 20 ml Essigsäureethylester, trocknet mit Natriumsulfat und dampft im Vakuum bis zur Gewichtskonstanz ein.
Ausbeute: 622 mg (84% d.Th.)
LC-MS (Methode 10): Rₜ = 1.96 min.
MS (ESI): m/z = 656 (M+H)⁺

### Beispiel 30A

2-(Benzyloxy)-*N*-(*tert*-butoxycarbonyl)-5-iod-*N*-methyl-L-phenylalanin 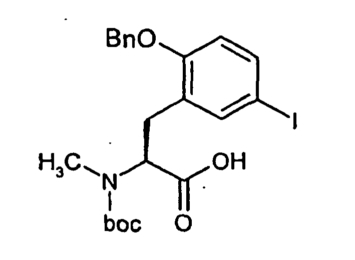 Unter Argonatmosphäre werden 500 mg (1 mmol) der Verbindung aus Beispiel (-)-6A in 20 ml THF gelöst, mit 90.5 mg (3.02 mmol) Natriumhydrid und 0.51 ml (1141.6 mg; 8.04 mmol) Methyliodid (80%-ig) versetzt und über Nacht bei Raumtemperatur gerührt. Man verdünnt mit 25 ml Essigsäureethylester und 25 ml Wasser und stellt mit 0.1N Salzsäure auf pH = 9 ein. Man engt im Vakuum auf ein kleines Volumen ein. Man versetzt mit 10 ml Essigsäureethylester und 10 ml Wasser, schüttelt alles heftig und trennt die organische Phase ab. Nach Trocknen mit Natriumsulfat und Einengen im Vakuum erhält man 140 mg Produkt (19% d. Th.). Die wässrige Phase säuert man an (pH = 3) und schüttelt sie dreimal mit 20 ml Essigsäureethylester aus. Nach Einengen und Trocknen im Vakuum erhält man 351 mg Produkt (68% d. Th.).
LC-MS (Methode 9): Rₜ = 3.9 min.
MS (EI): m/z = 511 (M+M)⁺

### Beispiel 31A

Benzyl-2-(benzyloxy)-*N*-(*tert*-butoxycarbonyl)-5-iod-*N*-methyl-L-phenylalaninat 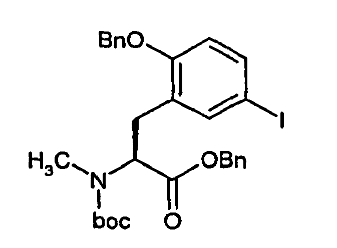 Die Herstellung erfolgt analog zu Beispiel 7A aus 350 mg (0.68 mmol) der Verbindung aus Beispiel 30A, 8.29 mg (0.07 mmol) DMAP, 148 mg (1.37 mmol) Benzylalkohol und 157.46 mg (0.82 mmol) EDC in 3 ml Acetonitril.
Ausbeute: 382 mg (93% d. Th.)
LC-MS (Methode 9): Rₜ = 4.8 min.
MS (EI): m/z = 601 (M+H)⁺

### Beispiel 32A

Benzyl-2-(benryloxy)-*N*-(*tert*-butoxycarbonyl)-*N*-methyl-5-(4,4,5,5-tetramethyl-1,3,2-dioxaborolan-2-yl)-L-phenylalaninat 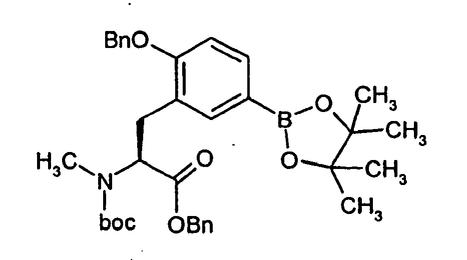 Analog zu Beispiel 8A werden 380 mg (0.63 mmol) der Verbindung aus Beispiel 31A in einem ausgeheizten Kolben in 4 ml DMF vorgelegt und unter Rühren bei Raumtemperatur mit 184.5 mg (0.73 mmol) 4,4,4',4',5,5,5',5'-Octamethyl-2,2'-bi-1,3,2-dioxaborolan, 186 mg (1.9 mmol) Kaliumacetat und 23.15 mg (0.03 mmol) Bis(diphenylphosphino)-ferrocen-palladium(II)chlorid versetzt. Man lässt 4 h bei 80°C reagieren. Nach der Aufarbeitung und Chromatographie (Kieselgel 60, Laufmittel: Cyclohexan/Essigsäureethylester = 4/1) erhält man das Produkt.
Ausbeute: 196 mg
LC-MS (Methode 9): Rₜ = 4.9 min.
MS (EI): m/z = 601 (M+H)⁺

### Beispiel 33A

2(*S*)-Benzyloxycarbonylamino-3-[4,4'-bis-benzyloxy-3'-(2(*S*)-benzyloxy-carbonyl-(2-*tert-*butoxycarbonyl-2-methyl)amino-ethyl)-biphenyl-3-yl]-propionsäure-2-(trimethylsilyl)-ethylester 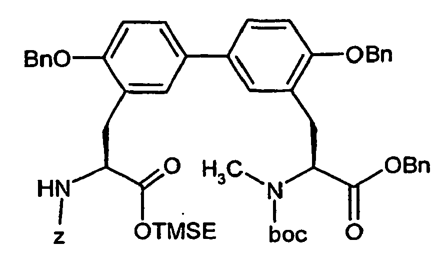 Die Herstellung erfolgt analog Beispiel 12A aus 190 mg (0.32 mmol) der Verbindung aus Beispiel 32A, 199.5 mg (0.32 mmol) der Verbindung aus Beispiel 11A, 195.5 mg (0.63 mmol) Cäsiumcarbonat und 23.15 mg (0.03 mmol) Bis(diphenylphosphino)ferrocen-palladium(II)chlorid in 1.5 ml DMF unter Argonatmosphäre.
Ausbeute: 212 mg (66% d. Th.)
LC-MS (Methode 13): Rₜ = 4.86 min.
MS (EI): m/z= 978 (M+H)⁺

### Beispiel 34A

2(*S*)-Benzyloxycarbonylamino-3-[4,4'-bis-benzyloxy-3'-(2(*S*)-benzyloxy-carbonyl-2-methylaminoethyl-biphenyl-3-yl]-propionsäure-2-(trimethylsilyl)-ethylester Hydrochlorid 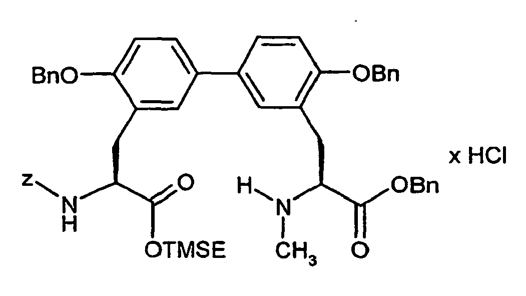 Die Herstellung erfolgt analog Beispiel 15A aus 930 mg (0.95 mmol) der Verbindung aus Beispiel 33A und 22.14 ml einer 4M Chlorwasserstoff-Dioxan-Lösung in 15 ml Dioxan.
Ausbeute: 915 mg (78% d. Th.)
LC-MS (Methode 13): Rₜ = 2.53 min.
MS (EI): m/z = 878 (M-HCl+H)⁺

### Beispiel 35A

2(*S*)-{Methyl-[5-benzyloxycarbonylamino-2(*S*)-*tert*-butoxycarbonylamino-4(*R*)-(*tert*-butyl-dimethylsilyloxy)-pentanoyl]amino}-3-{4,4'-bis-benzyloxy-3`-[2(*S*)-benzyloxycarbonylamino-2-(2-trimethylsilyl-ethoxycarbonyl)ethyl]-biphenyl-3-yl}-propionsäurebenzylester 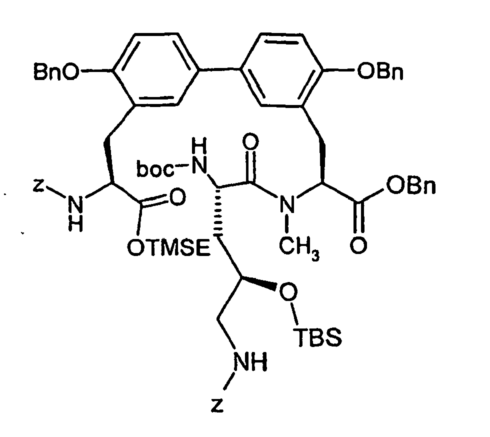 Die Herstellung erfolgt analog Beispiel 16A aus 922 mg (1.01 mmol) der Verbindung aus Beispiel 34A, 0.5 g (1.01 mmol) der Verbindung aus Beispiel 14A, 421 mg (1.11 mmol) HATU und 0.7 ml (518 mg; 3.27 mmol) DIEA in 4.2 ml DMF.
Ausbeute: 703 mg (51% d. Th.)
LC-MS (Methode 8): Rₜ = 3.17 min.
MS (EI): m/z = 1356 (M+H)⁺

### Beispiel 36A

2(*S*)-Benzyloxycarbonylamino-3-{4,4'-bis-benzyloxy-3'-[2(*S*)-benzyloxycarbonyl-2-{methyl-(5-benzyloxycarbonylamino-2(*S*)-*tert*-butoxycarbonylainino-4(*R*)-hydroxy-pentanoyl)amino}-ethyl]-biphenyl-3-yl}-propionsäure 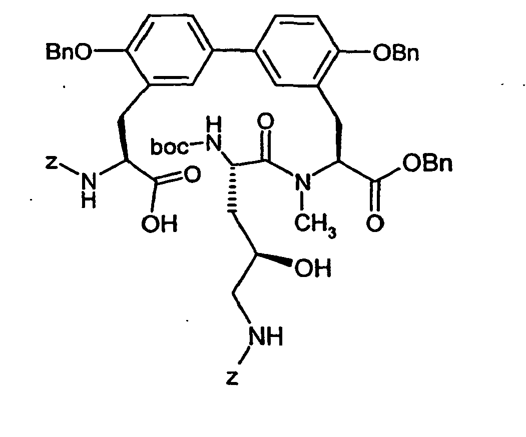 Die Herstellung erfolgt analog Beispiel 17A aus 360 mg (0.27 mmol) der Verbindung aus Beispiel 35A und 0.8 ml (3 Äquivalenten) 1M Tetrabutylammoniumfluorid-Lösung (THF) in 20 ml DMF.
Ausbeute: 159 mg (53% d. Th.)
LC-MS (Methode 12): Rₜ = 3.19 min.
MS (EI): m/z = 1142 (M+H)⁺

### Beispiel 37A

2(*S*)-[Methyl-(5-benzyloxycarbonylamino)-2(*S*)-*tert*-butoxycarbonylamino-4(*R*)-hydroxypentanoyl]amino-3-[4,4'-bis-benzyloxy-3'-(2(*S*)-benzyloxycarbonylamino-2-pentafluorphenyloxycarbonyl-ethyl)-biphenyl-3-yl]-propionsäurebenzylester 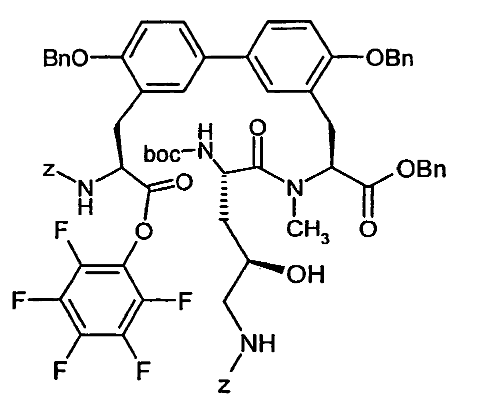 Die Herstellung erfolgt analog Beispiel 18A aus 330 mg (0.29 mmol) der Verbindung aus Beispiel 36A, 265.6 mg (1.44 mmol) Pentafluorphenol, 3.53 mg (0.03 mmol) DMAP und 60.87 mg (0.32 mmol) EDC in 10 ml Dichlormethan.
Ausbeute: 271 mg (69% d. Th.)
LC-MS (Methode 12): Rₜ = 3.38 min.
MS (EI): m/z = 1308 (M+H)⁺

### Beispiel 38A

2(*S*)-[Methyl-(5-benzyloxycarbonylamino)-2(*S*)-amino-4(*R*)-hydroxy-pentanoyl]amino-3-[4,4'-bis-benzyloxy-3'-(2(*S*)-benzyloxycarbonylamino-2-pentafluor-phenyloxycarbonyl-ethyl)-biphenyl-3-yl]-propionsäurebenrylester Hydrochlorid 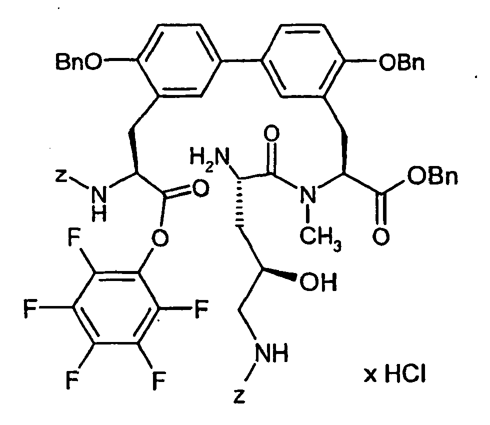 130 mg (0.1 mmol) der Verbindung aus Beispiel 37A werden in 0.5 ml Dioxan gelöst und vorsichtig mit 5 ml einer 4N Chlorwasserstoff-Dioxan-Lösung versetzt (Eisbad). Nach 30 Minuten lässt man bei Raumtemperatur noch 2 h weiterreagieren. Man dampft alles im Vakuum zur Trockne ein und trocknet im Hochvakuum bis zur Gewichtskonstanz.
Ausbeute: 130 mg (70% d. Th.)
LC-MS (Methode 15): Rₜ = 2.68 min.
MS (EI): m/z = 1208 (M-HCl+H)⁺

### Beispiel 39A

Benzyl-(8*S*,11*S*,14*S*)-5,17-bis(benzyloxy)-14-{[(benzyloxy)carbonyl]amino}-11-((2*R*)-3-{[(benzyloxy)carbonyl]amino}-2-hydroxypropyl-9-methyl-10,13-dioxo-9,12-diazatricyclo[14.3.1.12.6]-henicosa-1 (20),2(21),3,5,16,18-hexaen-8-carboxylat 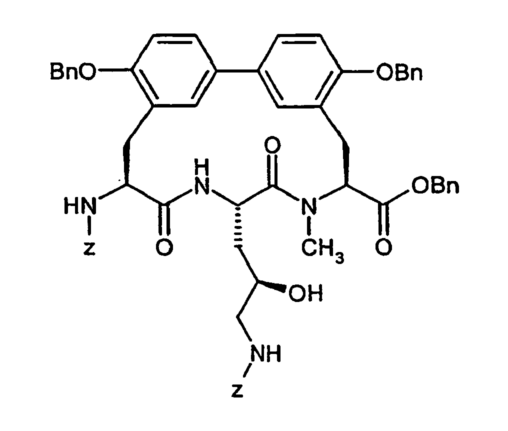 130 mg (0.1 mmol) der Verbindung aus Beispiel 38A werden in 220 ml trockenem Chloroform vorgelegt. Bei Raumtemperatur versetzt man unter Rühren innerhalb von 20 Minuten mit 23 ml (20 Äquiv.) Triethylamin in 5 ml Dichlormethan. Man lässt über Nacht nachrühren. Anschließend wird alles im Vakuum zur Trockne eingedampft. Der Rückstand wird mit Acetonitril ausgerührt. Nach dem Trocknen des Rückstandes gewinnt man 44 mg Produkt. Aus der Mutterlauge wird durch RP-HPLC noch weiteres Produkt gewonnen (30 mg).
Ausbeute: 74 mg (69% d. Th.)
LC-MS (Methode 15): Rₜ = 3.13 min.
MS (EI): m/z =1024 (M+14)⁺

### Beispiel 40A

(8*S*,11*S*,14*S*)-14-Amino-11-[(2*R*)-3-amino-2-hydroxypropyl]-5,17-dihydroxy-9-methyl-10,13-dioxo-9,12-diazatricyclo[14.3.1.12,6]henicosa-1(20),2(21),3,5,16,18-hexaen-carbonsäure-Di(hydrotrifluoracetat) 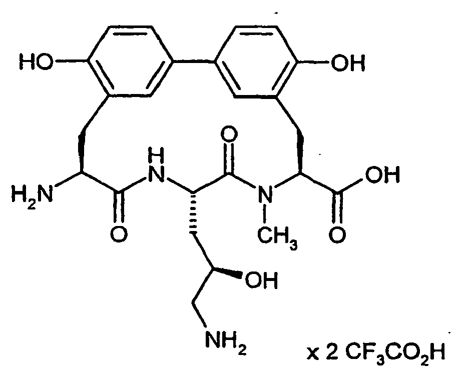 33 mg (0.032 mmol) der Verbindung aus Beispiel 39A werden mit verdünnter Trifluoressigsäure vorsichtig behandelt. Die entstandene klare Lösung wird anschließend lyophilisiert.
Ausbeute: 23 mg (quantitativ)
LC-MS (Methode 15): Rₜ = 0.92 min.
MS (EI): m/z = 486 (M-2CF₃CO₂H+H)⁺

### Beispiel 41A

(8*S*,11*S*,14*S*)-5,17-Bis(benzyloxy)-14-{[benzyloxycarbonyl]amino}-11-(2*R*)-3-{[benzyloxycarbonyl]-amino}-2-hydroxypropyl-9-methyl-10,13-dioxo-9,12-diazatricyclo[14.3.1.12,6]henicosa-1(20),2(21),3,5,16,18-hexaen-8-carbonsäure 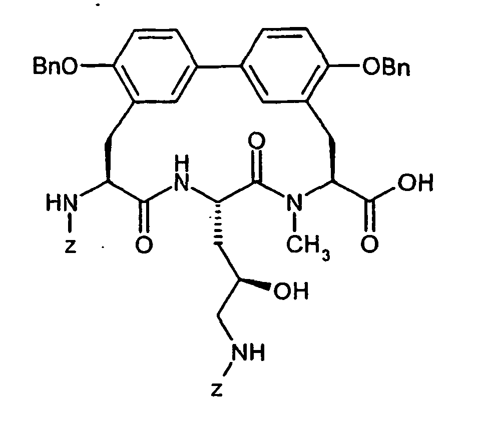 37 mg (0.04 mmol) der Verbindung aus Beispiel 39A werden in 2 ml THF gelöst, mit 0.14 ml 1N Lithiumhydroxid-Lösung versetzt und 3 h bei Raumtemperatur gerührt. Anschließend säuert man mit 1N Salzsäure an und dampft alles im Hochvakuum zur Trockne ein.
Ausbeute: 33 mg (71% d. Th.)
LC-MS (Methode 12): Rₜ = 2.90 min.
MS (EI): m/z = 934 (M+H)⁺

Analog zu oben aufgeführten Vorschriften werden die in der folgenden Tabelle aufgeführten Beispiele 42A bis 48A aus den entsprechenden Edukten hergestellt:

| **Bsp.- Nr.** | **Struktur** | **Hergestellt analog** | **Analytische Daten** |
|---|---|---|---|
| **42A** | 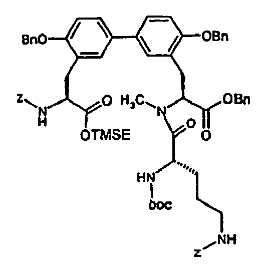 | 16A mit *N⁵-* [(Benzyloxy)- carbonyl]-*N*²- (*tert*-butoxy- carbonyl)-*L*- ornithin | LC-MS (Methode 13): Rₜ = 4.85 min. MS (EI): m/z = 1226 (M+H)⁺ |
| **43A** | 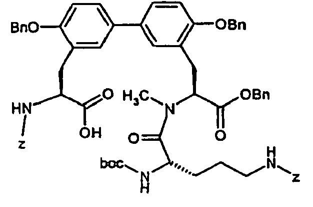 | 17A | LC-MS (Methode 13): Rₜ = 2.04 min. MS (EI): m/z = 1126 (M+H)⁺ **-** |
| **44A** | 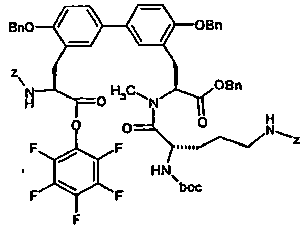 | 18A | LC-MS (Methode 13): Rₜ = 3.79 min. MS (EI): m/z = 1292 (M+H)⁺ |
| **45A** | 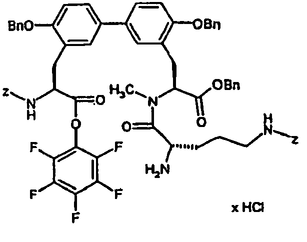 | 26A | LC-MS (Methode 13): Rₜ = 3.72 min. MS (EI): m/z = 1192 (M-HCl+H)⁺ |
| **46A** | 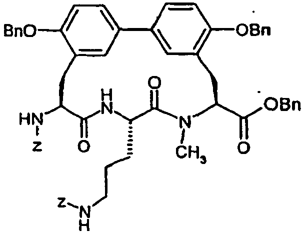 | 39A | LC-MS (Methode 13): Rₜ = 4.39 min. MS (EI): m/z = 1008 (M+H)⁺ |
| **47A** | 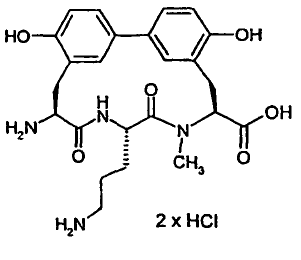 | 40A | LC-MS (Methode 12): Rₜ = 0.53 min. MS (EI): m/z = 470 (M-2HCl+H)⁺ |
| **48A** | 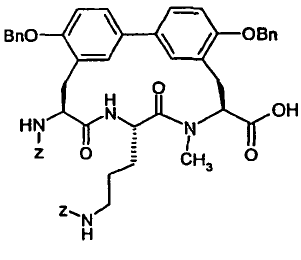 | 41A | LC-MS (Methode 14): Rₜ = 3.64 min. MS (EI): m/z = 918 (M+H)⁺ |

### Beispiel 49A

2-(Trimethylsilyl)ethyl-(2*Z*)-3-[2-(benzyloxy)-5-bromphenyl]-2-{[(benzyloxy)-carbonyl]-amino}acrylat 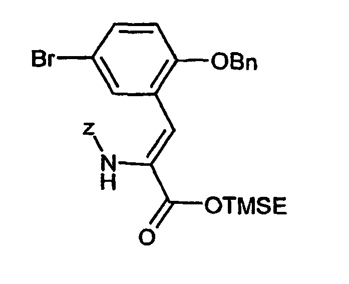 7.5 g (25.8 mmol) 2-(Benzyloxy)-5-brombenzaldehyd (Synthesis. 1992, 10, 1025-30) und 11.8 g (28.3 mmol) 2-(Trimethylsilyl)ethyl-{[(benzyloxy)carbonyl]amino}-(dimethoayphosphoryl)acetat (Tetrahedron, 1999, 55, 10527-36) werden in 150 ml THF vorgelegt und unter Aceton/Trockeneis-Kühlung bei -78°C mit 3.26 g (28.3 mmol) 1,1,3,3-Tetramethylguanidin versetzt. Es wird langsam auf RT erwärmt und für weitere 12 h bei RT gerührt. Das Lösungsmittel wird im Vakuum abdestilliert, das Rohprodukt mit Essigsäureethylester aufgenommen und je einmal mit gesättigter Natriumhydrogencarbonat- und gesättigter Natriumchloridlösung gewaschen. Die organische Phase wird über Magnesiumsulfat getrocknet, filtriert und im Vakuum zur Trockne eingeengt. Das Rohprodukt wird aus Essigsäureethylester/Cyclohexan (1:20) umkristallisiert.
Ausbeute: 13 g (88% d.Th.)
HPLC (Methode 16): Rₜ = 6.06 min.
MS (DCI(NH₃)): m/z = 599 (M+NH₄)⁺

### Beispiel 50A

(8*S*,11*S*,14*S*)-5,17-Bis(benzyloxy)-14-{[(benzyloxy)carbonyl]amino}-11-(3-{[(benzyloxy)carbonyl]amino}propyl)-10,13-dioxo-9,12-diazatricyclo [14.3.1.1^{2.6}]henicosa-1(20),2(21),3,5,16,18-hexaen-8-carbonsäure 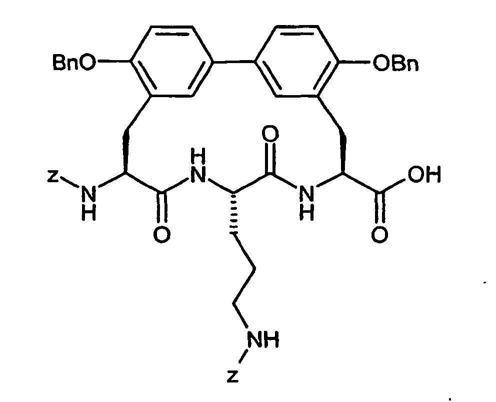 200 mg (0.2 mmol) der Verbindung aus Beispiel 27A werden in 8 ml THF und 4 ml DMF vorgelegt und unter Rühren mit 0.8 ml einer 1M wässrigen Lithiumhydroxid-Lösung (4 Äquivalente) versetzt. Nach 2 h Rühren bei Raumtemperatur entsteht ein Gel. Man versetzt mit 0.8 ml 1N Salzsäure und noch etwas Wasser. Anschließend dampft man alles im Vakuum zur Trockne ein, rührt mit Wasser aus, filtriert den Niederschlag und trocknet ihn.
Ausbeute: 140 mg (77% d.Th.)
LC-MS (Methode 10): Rₜ = 2.83 min.
MS (EI): m/z = 904 (M+H)⁺

### Beispiel 51A

(8*S*,11*S*,14*S*)-14-[(*tert*-Butoxycarbonyl)amino]-11-{3-[(*tert*-butoxycarbonyl)-amino]propyl}-5,17-dihydroxy-9-methyl-10,13-dioxo-9,12-diaza-tricyclo[14.3.1.1^{2,6}]henicosa-1(20),2(21),3,5,16,18-hexaen-8-carbonsäure 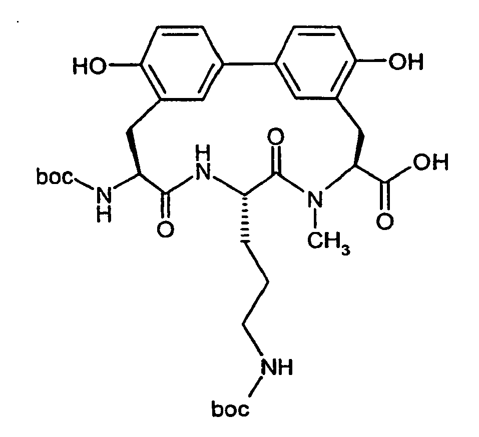 11 mg (0.02 mmol) der Verbindung aus Beispiel 47A werden in 0.5 ml Wasser gelöst, mit 12.27 mg (0.08 mmol) Natriumcarbonat versetzt, im Eisbad gekühlt und unter Rühren mit 13.25 mg (0.06 mmol) Di-*tert*-butyl-dicarbonat in 0.2 ml Methanol versetzt. Man rührt über Nacht bei RT, dampft im Vakuum zur Trockne ein, löst in 0.5 ml Wasser, säuert mit 1N Salzsäure bis pH = 2 an und extrahiert die entstandene Suspension mit Essigsäureethylester. Nach dem Trocknen mit Natriumsulfat dampft man im Vakuum zur Trockne ein.
Ausbeute: 10 mg (51% d.Th.)
LC-MS (Methode 12): Rₜ = 1.92 min.
MS (EI): m/z = 670 (M+M)⁺

### Beispiel 52A

(8*S*,11*S*,14[(14-[(*tert*-Butoxycarbonyl)amino]-11-{(2*R*)-3-[(*tert*-butoxy-carbonyl)amino]-2-hydroxypropyl}-5,17-dihydroxy-9-methyl-10,13-dioxo-9,12-diazatricyclo[14.3.1.1^{2,6}]henicosal-(20),2(21),3,5,16,18-hexaen-8-carbonsäure 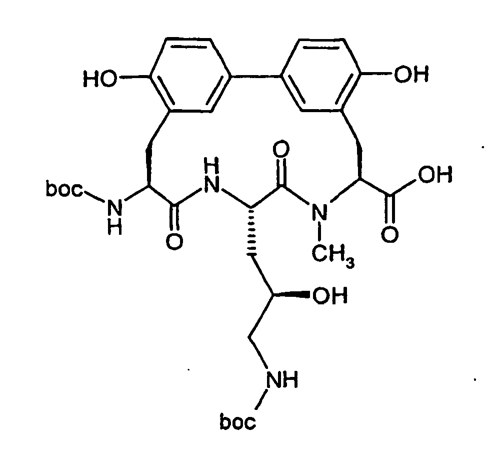 90 mg (0.16 mmol) der Verbindung aus Beispiel 40A werden in 2.5 ml Wasser gelöst, mit 85.3 mg (0.8 mmol) Natriumcarbonat versetzt, im Eisbad gekühlt und mit 105.3 mg (0.48 mmol) Di-(*tert-*butyl)-dicarbonat in 1.2 ml Methanol versetzt. Man lässt über Nacht bei Raumtemperatur rühren, engt im Vakuum auf ein kleines Volumen ein und säuert mit 1N Salzsäure bis pH = 2 an. Der anfallende Niederschlag wird abfiltriert und getrocknet.
Ausbeute: 89 mg (73% d.Th.)
LC-MS (Methode 12): Rₜ = 1.8 min.
MS (EI): m/z = 686 (M+H)⁺

### Beispiel 53A

2-(Trimethylsilyl)ethyl-2-(benzyloxy)-*N*-[(benzyloxy)carbonyl]-5-brom-L-phenylalaninat 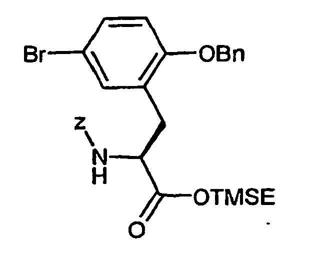 930 mg (1.6 mmol) der Verbindung aus Beispiel 49A werden in 100 ml Ethanol und 10 ml Dioxan gelöst. Unter Argonatmosphäre gibt man 20 mg (+)-1,2-Bis((2*S*,5*S*)-2,5-diethylphospholano)benzol(cyclooctadien)rhodium(I)trifluoromethansulfonat hinzu und belässt die Lösung 15 min im Ultraschallbad. Anschließend wird für 5 d unter einem Wasserstoffdruck von 3 bar hydriert. Es wird über Kieselgel filtriert und sorgfältig mit Ethanol nachgewaschen. Das Filtrat wird im Vakuum eingeengt und das Rohprodukt am Hochvakuum getrocknet.
Ausbeute: 900 mg (96% d.Th.)
ee = 98.8% (Chiralcel OD (Daicel); Elutionsmittel: i-Hexan und Ethanol (5/1 vol/vol) unter Zusatz von 0.2 Vol% Diethylamin)
HPLC (Methode 16): Rₜ = 6.08 min.
MS (DCI(NH₃)): m/z = 601 (M+NH₄)⁺

### Beispiel 54A

Methyl-(22)-3-[2-(benzyloxy)-5-bromphenyl]-2-{[(benzyloxy)carbonyl]amino}-acrylat 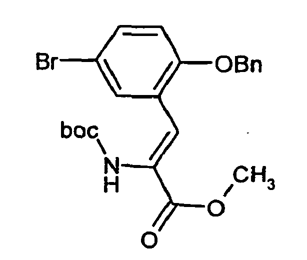 Die Herstellung erfolgt analog Beispiel 49A aus 7.5 g (25.8 mmol) 2-(Benzyloxy)-5-brombenzaldehyd und 8.4 g (28.3 mmol) 2-(Trimethylsilyl)ethyl-{[(benzyloxy)carbonyl]amino)-(dimethoxyphosphoryl)acetat (J. Prakt. Chem., 2000, 342, 736-44) mit 3.3 g (28.3 mmol) 1,1,3,3-Tetramethylguanidin in 150 ml THF.
Ausbeute: 10 g (87% d.Th.)
HPLC (Methode 16): Rₜ = 5.42 min.
MS (DCI(NH₃)): m/z = 479 (M+NH₄)⁺

### Beispiel 55A

Methyl-2-(benzyloxy)-*N*-[(benzyloxy)carbonyl]-5-brom-L-phenylalaninat 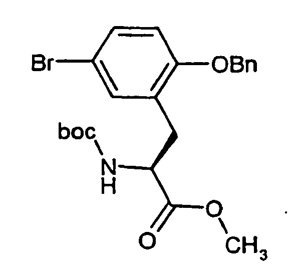 Die Herstellung erfolgt analog Beispiel 53A aus 1.96 g (4.2 mmol) der Verbindung aus Beispiel 54A und 15 mg (+)-1,2-Bis((2*S*,5*S*)-2,5-diethylphospholano)-benzol(cyclooctadien)-rhodium(I)-trifluoromethansulfonat in 100 ml Ethanol und 20 ml Dioxan.
Ausbeute: 1.96 g (99% d.Th.)
ee = 97.6% (Chiralcel OD (Daicel); Elutionsmittel: i-Hexan und Ethanol (5/1 vol/vol)) unter Zusatz von 0.2 Vol% Diethylamin)
LC-MS (Methode 17): Rₜ = 3.05 min.
MS (DCI(NH₃)): m/z = 481 (M+NH₄)⁺
¹H-NMR (300 MHz, DMSO-d₆): δ = 1.32 (s, 9H), 2.72 (m_{c}, 1H), 3.17 (m_{c}, 1H), 3.60 (s, 3H), 4.32 (m_{c}, 1H), 5.13 (s, 2H), 7.01 (m_{c}, 1H), 7.22 (m_{c}, 1H), 7.28-7.58 (m_{c}, 6H).

### Beispiel 56A

Methyl-2-(benzyloxy)-*N*-(*tert*-butoxycarbonyl)-5-(4,4,5,5-tetramethyl-1,3,2-dioxaborolan-2-yl)-L-phenylalaninat 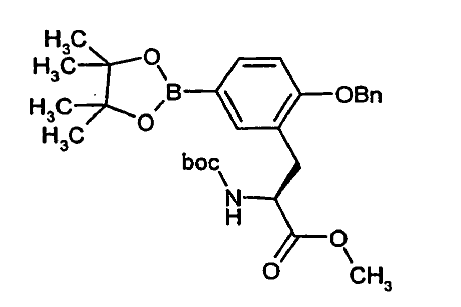 Zu einer Lösung von 0.36 g (0.77 mmol) der Verbindung aus Beispiel 55A in 5 ml DMF werden 0.23 g (2.31 mmol) Kaliumacetat und 4 mg (0.08 mmol) Kaliumhydroxid gegeben. Die Mischung wird deoxygeniert, indem durch die kräftig gerührte Lösung 15 min lang Argon durchgeleitet wird. Dann werden 0.25 g (1.0 mmol) 4,4,4',4',5,5,5',5'-Octamethyl-2,2'-bi-1,3,2-dioxaborolan und 0.023 g (0.03 mmol, 0.04 Äquivalente) Bis-(diphenylphosphino)ferrocenpalladium(II)chlorid zugegeben. Unter leichtem Argonstrom wird auf 60°C erhitzt und bei dieser Temperatur 1.5 h gerührt. Anschließend wird 30 min bei 80°C gerührt und dann auf RT abgekühlt. Das Lösungsmittel wird im Vakuum abdestilliert, das Rohprodukt mit Essigsäureethylester aufgenommen und zweimal mit gesättigter Natriumchlorid-lösung gewaschen. Die organische Phase wird über Magnesiumsulfat getrocknet, filtriert und im Vakuum zur Trockne eingeengt. Der Rückstand wird chromatographisch gereinigt (RP-HPLC, Acetonitril, Wasser).
Ausbeute: 0.219 g (56% d.Th.)
MS (EI): m/z = 512 (M+H)⁺
¹H-NMR (400 MHz, DMSO-d₆): δ = 1.27 (m_{c}, 12H), 1.29 (s, 9H), 2.75 (m_{c}, 1H), 3.19 (m_{c}, 1H), 3.57 (s, 3H), 4.30 (m_{c}, 1H), 5.19 (m_{c}, 2H), 7.04 (m_{c}, 1H), 7.24 (m_{c}, 1 H), 7.28-7.58 (m, 6H).

### Beispiel 57A

2-(Trimethylsilyl)ethyl-2-(benzyloxy)-*N*-[(benzyloxy)carbonyl]-5-(4,4,5,5-tetramethyl-1,3,2-dioxaborolan-2-yl)-*L*-phenylalaninat 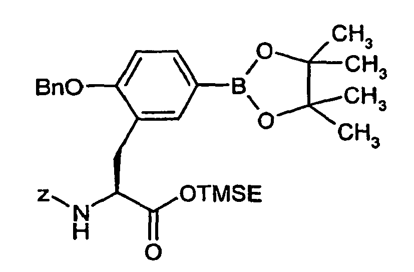 Die Herstellung erfolgt analog Beispiel 8A aus 2.0 g (3.17 mmol) der Verbindung aus Beispiel 11A, 0.924 g (3.64 mmol) 4,4,4,4',5,5,5',5'-Octamethyl-2,2'-bi-1,3,2-dioxaborolan, 0.932 g (9.50 mmol) Kaliumacetat und 0.116 g (0.160 mmol, 0.05 Äquivalenten) Bis-(diphenylphosphino)ferrocenpalladium(II)chlorid in 30 ml Dimethylsulfoxid.
Ausbeute: 1.12 g (56% d. Th.)
LC-MS (Methode 13): Rₜ = 4.50 min
MS (EI): m/z = 632 (M+M)⁺
¹H-NMR (200 MHz, CDCl₃): δ = 0.92 (dd, 2H), 1.31 (s, 12H), 2.95-3.95 (m, 2H), 4.11 (m_{c}, 2H), 4.55 (11 (m_{c}, 1H), 4.99 (s, 2H), 5.08 (s, 2H), 5.53 (d, 1H), 6.90 (d, 1H), 7.15-7.47 (m, 10 H), 7.58 (d, 1H), 7.67 (dd, 1H).

### Beispiel 58A

2-(Trimethylsilyl)ethyl-(2*S*)-2-{[(benzyloxy)carbonyl]amino}-3-(4,4'-bis(benzyloxy)-3'-{(2*S*)-2-[(*tert*-butoxycarbonyl)amino]-3-methoxy-3-oxopropyl}biphenyl-3-yl)propanoat

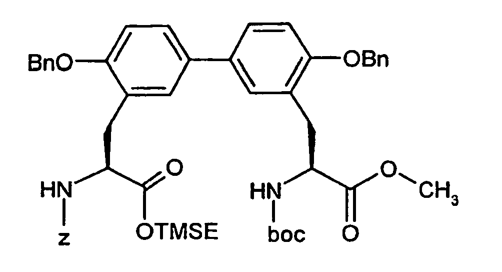

### Methode A:

Die Herstellung erfolgt analog Beispiel 12A aus 0.46 g (0.79 mmol) der Verbindung aus Beispiel 53A, 0.41 g (0.79 mmol) der Verbindung aus Beispiel 56A, 0.52 g (1.58 mmol) Cäsiumcarbonat und 0.023 g (0.032 mmol, 0.04 Äquivalenten) Bis-(diphenylphosphino)ferrocenpalladium(II)chlorid in 12 ml DMF.
Ausbeute: 0.34 g (48% d. Th.)

### Methode B:

Die Herstellung erfolgt analog Beispiel 53A aus 0.59 g (0.67 mmol) der Verbindung aus Beispiel 69A und 10 mg (+)-1,2-Bis((2*S*,5*S*)-2,5-diethylphospholano)-benzol(cyclooctadien)-rhodium(I)trifluoromethansulfonat in 100 ml Ethanol und 30 ml Dioxan.
Ausbeute: 0.60 g (99% d.Th.)
ee = 99.5% (chirale stationäre Kieselgelphase, basierend auf dem Selektor Poly(N-methacryloyl-L-leucin-L-menthylamid); Elutionsmittel: i-Hexan und Ethylacetat (2/1 vol/vol))
HPLC (Methode 16): Rₜ = 6.54 min
MS (EI): m/z = 890 (M+H)⁺
¹H-NMR (400 MHz, DMSO-d₆): δ = 0.00 (s, 9H), 0.83 (m_{c}, 2H), 1.31 (s, 9H), 2.86 (m, 2H), 3.25 (m, 2H), 3.62 (s, 3H), 4.09 (m, 2H), 4.41 (m_{c}**,** 1H), 4.98 (m_{c}, 2H), 5.22 (m, 4H), 7.12 (m, 2H), 7.29 (m, 2H), 7.33 - 7.59 (m, 20 H), 7.78 (d, 1H).

Analog zu oben aufgeführten Vorschriften werden die in der folgenden Tabelle aufgeführten Beispiele 59A bis 64A aus den entsprechenden Edukten hergestellt:

| **Beispiel- Nr.** | **Struktur** | **Hergestellt analog** | **Analytische Daten** |
|---|---|---|---|
| **59A** | 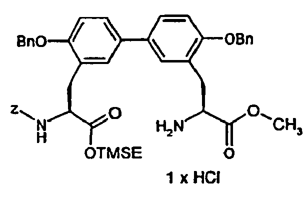 | 15A | LC-MS (Methode 12): Rₜ= 2.50 min. MS (EI): m/z = 789 (M-HCl+H)⁺ |
| **60A** | 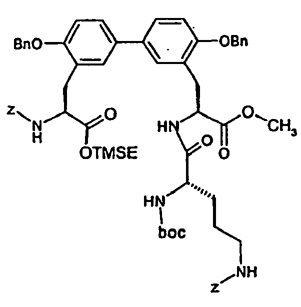 | 16A | LC-MS (Methode 13): Rₜ = 3.51 min. MS (EI): m/z = 1137 (M+H)⁺ |
| **61A** | 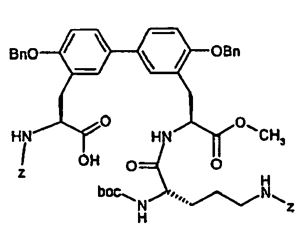 | 17A | LC-MS (Methode 13): Rₜ = 3.20 min. MS (EI): m/z = 1037 (M+H)⁺ |
| **62A** | 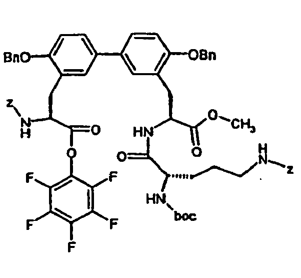 | 18A | LC-MS (Methode 19): Rₜ = 3.43 min. MS (EI): m/z = 1203 (M+H)⁺ |
| **63A** | 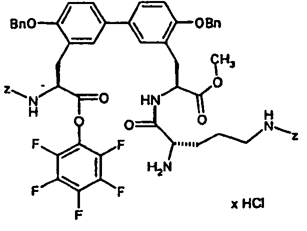 | 26A | LC-MS (Methode 12): Rₜ = 2.83 min. MS (EI): m/z =1103 (M-HCl+H)⁺ |
| **64A** | 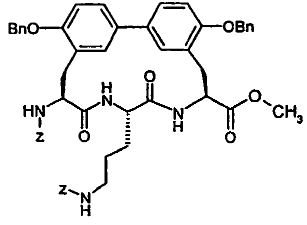 | 39A | LC-MS (Methode 12): Rₜ = 3.10 min. MS (EI): m/z = 919 (M+H)⁺ |

### Beispiel 65A

(8*S*,11*S*,14*S*)-14-[(*tert*-Butoxycarbonyl)amino]-11-{(2*R*)-3-[(*tert*-butoxycarbonyl)amino]-2-hydroxypropyl}-5,17-dihydroxy-10,13-dioxo-9,12-diazatricyclo[14.3.1.1^{2,6}]henicosa-1(20),2(21),3,5,16,18-hexaen-8-carbonsäure 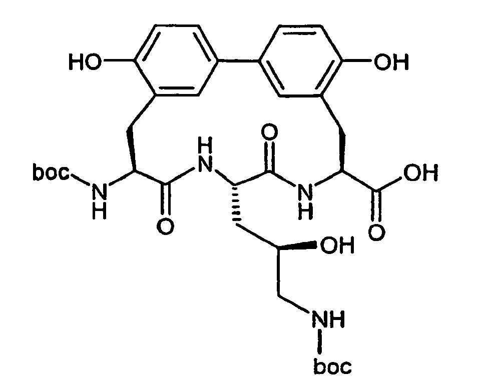 50 mg (0.09 mmol) der Verbindung aus Beispiel 20A werden in einem Gemisch von 8 ml Methanol/Wasser (9:1) vorgelegt. Dazu gibt man 1 ml 1N Natriumhydrogencarbonatlösung und anschließend 80 mg (0.37 mmol) Di-*tert*-butyldicarbonat in 2 ml Methanol/Wasser (9:1). Es wird über Nacht bei RT gerührt. Die Lösung wird zur Aufarbeitung mit 60 ml Ethylacetat und 30 ml Wasser versetzt. Die organische Phase wird einmal mit 0.1 normaler Salzsäure gewaschen, getrocknet und im Vakuum einrotiert.
Ausbeute: 49 mg (79% d. Th.).
LC-MS (Methode 3): Rₜ = 2.56 min.
MS (EI): m/z = 673 (M+H)⁺.
LC-HR-FT-ICR-MS:
ber. für C₃₃H₄₄N₄O₁₁ (M+H)⁺ 673.3079
gef. 673.3082.

### Beispiel 66A

Benzyl-(8*S*,11*S*,14*S*)-5,17-bis(benzyloxy)-14-{[(benzyloxy)carbonyl]amino}-11-((2*R*)-3-{[(benzyloxy)carbonyl]amino}-2-{[*tert*-butyl(dimethyl)silyl]oxy}propyl)-10,13-dioxo-9,12-diazatricyclo[14.3.1.1^{2,6}] henicosa-1(20),2(21),3,5,16,18-hexaen-8-carboxylat 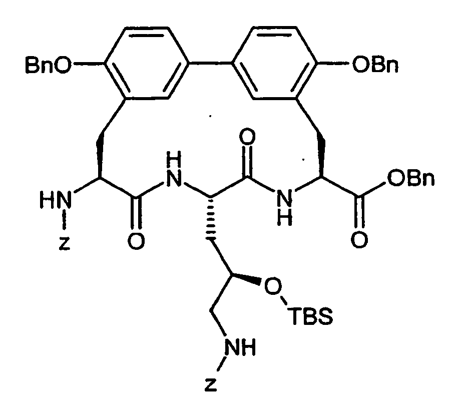 200 mg (0.20 mmol) der Verbindung aus Beispiel 19A werden in 50 ml absolutem DMF gelöst und bei 0°C mit 210 mg (0.79 mmol) Trifluormethansulfonsäure-*tert*-butyldimethylsilylester, 0.11 ml (0.79 mmol) Triethylamin und 20 mg (0.20 mmol) DMAP versetzt. Es wird 2 d bei RT gerührt. Nach Zugabe von 20 ml Dichlormethan wäscht man die Lösung vorsichtig mit 10 ml gesättigter Natriumhydrogencarbonat-Lösung und 10 ml Wasser. Die organische Phase wird zur Trockne eingeengt und der Rückstand im Hochvakuum getrocknet.
Ausbeute: 215 mg (96% d. Th.)
LC-MS (Methode 12): Rₜ = 3.43 min.
MS (EI): m/z = 1125 (M+H)⁺

### Beispiel 67A

(8*S*,11*S*,14*S*)-5,17-Bis(benzyloxy)-14-{[(benzyloxyl)carbonyl]amino}-11-((2*R*)-3-{[(benzyloxy)carbonyl]amino}-2-{[*tert*-butyl(dimethyl)silyl]oxy}propyl)-10,13-dioxo-9,12-diazatricyclo[14.3.1.1^{2,6}]henicosa-1(20),2(21),3,5,16,18-hexaen-8-carbonsäure 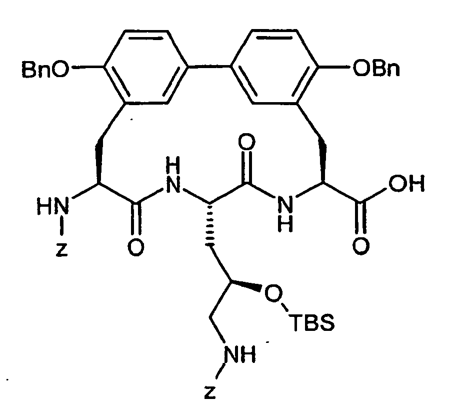 210 mg (0.19 mmol) der Verbindung aus Beispiel 66A werden in 2 ml THF gelöst und mit je 1 ml Wasser und Methanol versetzt. Nach Zugabe von 13 mg (0.56 mmol) Lithiumhydroxid wird 12 h bei RT gerührt. Anschließend wird die Reaktionslösung mit 30 ml Wasser verdünnt und durch Zugabe von 1N Salzsäure auf pH = 3 gestellt. Der Niederschlag wird abfiltriert und im Hochvakuum getrocknet.
Ausbeute: 192 mg (99% d. Th.)
LC-MS (Methode 12): Rₜ = 3.24 min.
MS (EI): m/z = 1135 (M+H)⁺

### Beispiel 68A

Methyl-(2*Z*)-3-[2-(benzyloxy)-5-(4,4,5,5-tetramethyl-1,3,2-dioxaborolan-2-yl)phenyl]-2-[(*tert-*butoxycarbonyl)amino]acrylat 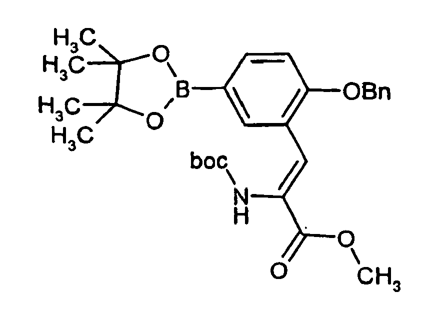 Die Herstellung erfolgt analog Beispiel 8A aus 1.0 g (2.16 mmol) der Verbindung aus Beispiel 54A, 0.63 g (2.5 mmol) 4,4,4',4-,5,5,5',5'-Octamethyl-2,2'-bi-1,3,2-dioxaborolan, 0.64 g (6.50 mmol) Kaliumacetat und 0.063 g (0.087 mmol, 0.04 Äquivalenten) Bis-(diphenylphosphino)ferrocenpalladium(II)chlorid in 14 ml Dimethylsulfoxid.
Ausbeute: 0.832 g (76% d.Th.)
LC-MS (Methode 12): Rₜ = 2.96 min.
MS (EI): m/z = 510 (M+H)⁺

### Beispiel 69A

2-(Trimethylsilyl)ethyl-(2*Z*)-2-{[(benzyloxy)carbonyl]amino}-3-(4,4'-bis(benzyl-oxy)3'-{(1*Z*)-2-[(*tert*-butoxycarbonyl)amino]-3-methoxy-3-oxoprop-1-en-1-yl}-biphenyl-3-yl)acrylat 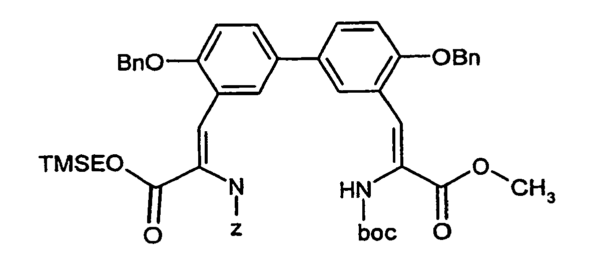 Die Herstellung erfolgt analog Beispiel 12A aus 0.42 g (0.82 mmol) der Verbindung aus Beispiel 68A, 0.48 g (0.82 mmol) der Verbindung aus Beispiel 49A, 0.54 g (1.65 mmol) Cäsiumcarbonat und 0.024 g (0.033 mmol, 0.04 Äquivalenten) Bis-(diphenylphosphino)ferrocenpalladium(II)chlorid in 12 ml DMF.
Ausbeute: 0.47 g (64% d. Th.)
HPLC (Methode 16): Rₜ = 6.57 min
MS (EI): m/z = 886 (M+M)⁺

### Beispiel 70A

2-(Benzyloxy)-*N*-(*tert*-butoxycarbonyl)-5-iod-*N*-ethyl-L-phenylalanin 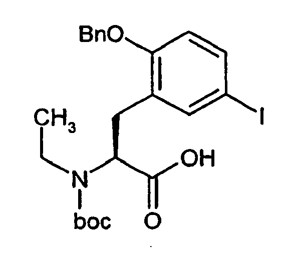 Unter Argonatmosphäre werden 1.0 g (2.01 mmol) der Verbindung aus Beispiel (-)-6A in 40 ml THF gelöst, mit 241 mg (6.03 mmol) Natriumhydrid (60%ig Dispersion in Mineralöl), 1.0 g (6.03 mmol) Kaliumiodid und 1.29 ml (2509 mg; 16.1 mmol) Ethyliodid versetzt und über Nacht bei Raumtemperatur gerührt. Das Ansatz wird im Vakuum eingeengt. Das Rohprodukt wird in Essigsäureethylester aufgenommen, die organische Phase wird mehrmals mit Wasser gewaschen, über Natriumsulfat getrocknet und im Vakuum eingeengt. Das Rohprodukt wird chromatographisch mittels RP-HPLC (Laufmittel Acetonitril/Wasser Gradient) gereinigt.
Ausbeute: 470 mg (44% d. Th.).
LC-MS (Methode 12): Rₜ = 2.79 min.
MS (EI): m/z = 526 (M+H)⁺

### Beispiel 71A

Benzyl-2-(benzyloxy)-*N*-(*tert*-butoxycarbonyl)-5-iod-*N*-ethyl-L-phenylalaninat 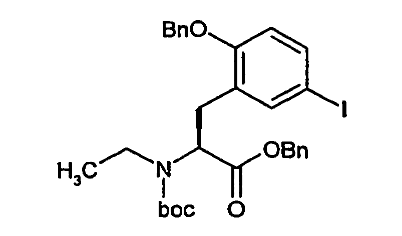 Die Herstellung erfolgt analog zu Beispiel 7A aus 420 mg (0.68 mmol) der Verbindung aus Beispiel 70A, 9.77 mg (0.08 mmol) DMAP, 173 mg (1.6 mmol) Benzylalkohol und 184 mg (0.96 mmol) EDC in 8 ml Acetonitril.
Ausbeute: 375 mg (76% d. Th.)
LC-MS (Methode 12): Rₜ = 3.26 min.
MS (EI): m/z = 616 (M+H)⁺
¹H-NMR (300 MHz, CDCl₃): δ = 0.80 (m_{c}, 3H), 1.4 (m_{c}, 9H) 2.75 (m_{c}, 1H), 3.07 (m_{c}, 1H), 3.22 (m_{c}, 1H), 3.47 (m_{c}, 1H), 4.23 (m_{c}, 1H), 5.06 (s, 2H), 5.15 (m_{c}, 2H), 6.65 (d, 1H), 7.25-7.5 (m, 12H).

### Beispiel 72A

2(*S*)-Benzyloxycarbonylamino-3-[4,4'-bis-benzyloxy-3'-(2(*S*)-benzyloxy-carbonyl-(2-*tert*-butoxycarbonyl-2-ethyl)amino-ethyl)-biphenyl-3-yl]-propionsäure-2-(trimethylsily1)-ethylester 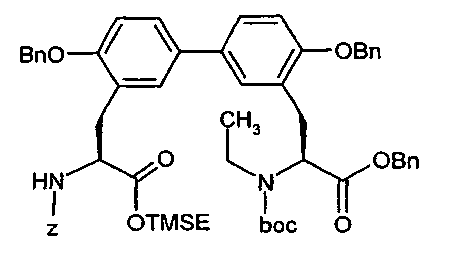 Die Herstellung erfolgt analog Beispiel 12A aus 343 mg (0.54 mmol) der Verbindung aus Beispiel 57A, 334 mg (0.54 mmol) der Verbindung aus Beispiel 71A, 354 mg (1.09 mmol) Cäsiumcarbonat und 40 mg (0.05 mmol) Bis(diphenylphosphino)ferrocen-palladium(II)chlorid in 8 ml DMF unter Argonatmosphäre.
Ausbeute: 216 mg (40% d. Th.)
LC-MS (Methode 12): Rₜ= 3.54 min.
MS (EI): m/z = 893 (M-boc+H)⁺

### Beispiel 73A

2(*S*)-Benzyloxycarbonylamino-3-[4,4'-bis-benzyloxy-3'-(2(*S*)-benzyloxy-carbonyl-2-ethylamino-ethyl-biphenyl-3-yl]-propionsäure-2-(trimethylsilyl)-ethylester Hydrochlorid 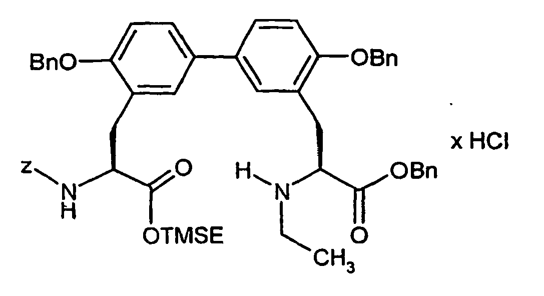 Die Herstellung erfolgt analog Beispiel 15A aus 210 mg (0.211 mmol) der Verbindung aus Beispiel 72A und 15 ml einer 4N Chlorwasserstoff-Dioxan-Lösung in 4 ml Dioxan.
Ausbeute: quantitativ
LC-MS (Methode 12): Rₜ = 3.01 min.
MS (EI): m/z = 893 (M-HCl+H)⁺

Analog zu oben aufgeführten Vorschriften werden die in der folgenden Tabelle aufgeführten Beispiele 74A bis 78A aus den entsprechenden Edukten hergestellt:

| **Bsp.- Nr.** | **Struktur** | **Hergestellt analog** | **Analytische Daten** |
|---|---|---|---|
| **74A** | 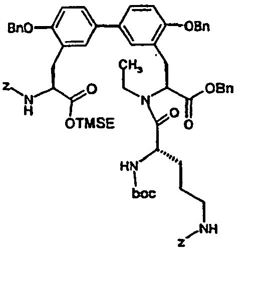 | 16A mit *N*⁵-[(Ben-zyloxy)-carbonyl]-*N*²-(*tert*-butoxy-carbonyl)-*L*-ornithin | LC-MS (Methode 17): Rₜ = 3.63 min. MS (EI): m/z = 1241 (M+H)⁺ |
| **75A** | 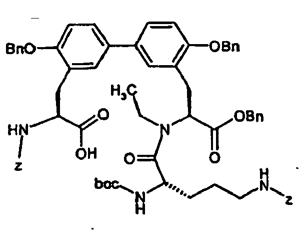 | 17A | LC-MS (Methode 17): Rₜ = 3.38 min. MS (EI): m/z = 1149 (M+M)⁺ |
| **76A** | 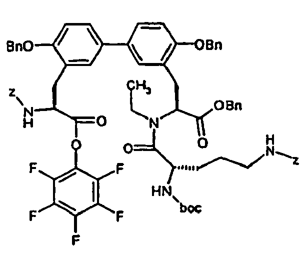 | 18A | LC-MS (Methode 17): Rₜ = 3.58 min. MS (EI): m/z = 1315 (M+H)⁺ |
| **77A** | 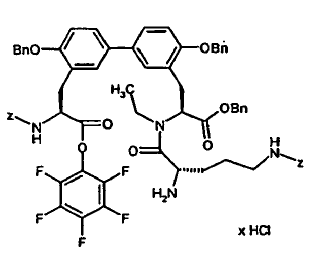 | 26A | |
| **78A** | 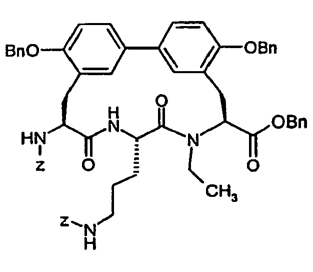 | 39A | LC-MS (Methode 17): Rₜ = 3.39 min. MS (EI): m/z = 931 (M+M)⁺ |

### Beispiel 79A

Benzyl-{(1*S*)-4-[(*tert*-butoxycarbonyl)amino]-1-[({2-[(*tert*-butoxycarbonyl)amino]ethyl}amino)-carbonyl]butyl}carbamat 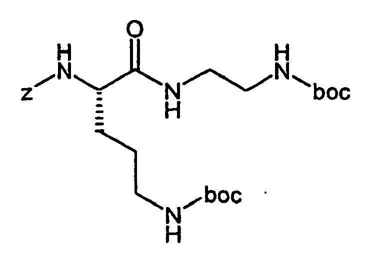 Unter Argon werden 300 mg (0.82 mmol) *N*²-[(Benzyloxy)carbonyl]-*N*⁵-(*tert*-butoxycarbonyl)-*L-*ornithin und 171 mg (1.06 mmol) *tert*-Butyl-(2-aminoethyl)carbamat in 6 ml Dimethylformamid gelöst. Bei 0°C (Eisbad) werden dann 204 mg (1.06 mmol) EDC und 33 mg (0.25 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum getrocknet
Ausbeute: 392 mg (94% d. Th.)
LC-MS (Methode 17): Rₜ = 2.36 min.
MS (EI): m/z = 509 (M+H)⁺

### Beispiel 80A

Benzyl-{(1*S*)-4-[(*tert*-butoxycarbonyl)amino]-1-[({3-[(*tert*-butoxycarbonyl)amino]-2-hydroxypropyl}amino)carbonyl]butyl}carbamat 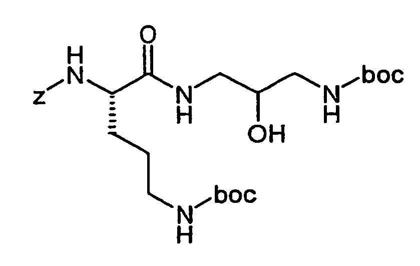 Die Herstellung erfolgt analog zu Beispiel 79A aus 300 mg (0.82 mmol) *N*²-[(Benzyloxy)carbonyl]-*N*⁵-(*tert*-butoxycarbonyl)-*L*-ornithin und 202 mg (1.06 mmol) *tert*-Butyl-(3-amino-2-hydroxypropyl)carbamat in 6 ml Dimethylformamid unter Zusatz von 204 mg (1.06 mmol) EDC und 33 mg (0.25 mmol) HOBt.
Ausbeute: 412 mg (93% d. Th.)
LC-MS (Methode 19): Rₜ = 2.23 min.
MS (EI): m/z = 539 (M+H)⁺

### Beispiel 81A

*N*⁵-(*tert*-Butoxycarbonyl)-*N*-{2-[(*tert*-butoxycarbonyl)amino]ethyl}-*L*-ornithinamid 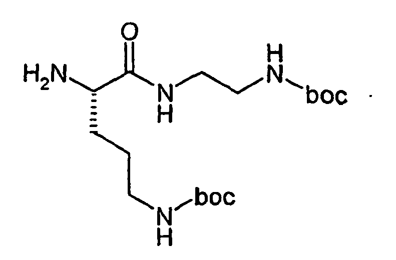 Eine Lösung von 390 mg (0.77 mmol) Benzyl-{(1*S*)-4-[(*tert*-butoxycarbonyl)amino]-1-[({2-[(*tert-*butoxycarbonyl)amino]ethyl}amino)carbonyl]butyl}carbamat (Beispiel 79A) in 50 ml Ethanol wird nach Zugabe von 40 mg Palladium auf Aktivkohle (10%ig) 4 h bei RT und Normaldruck hydriert. Es wird über Kieselgur filtriert und der Rückstand mit Ethanol gewaschen. Das Filtrat wird im Vakuum zur Trockne eingeengt. Das Produkt wird ohne weitere Reinigung umgesetzt.
Ausbeute: 263 mg (91% d. Th.)
MS (ESI): m/z = 375 (M+H)⁺; 397 (M+Na)⁺.

### Beispiel 82A

*N*⁵-(*tert*-Butoxycarbonyl)-*N*-{3-[(*tert*-butoxycarbonyl)amino]-2-hydroxypropyl}-L-ornithinamid 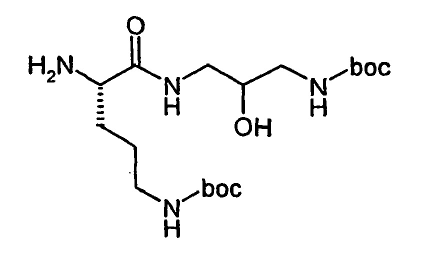 Die Herstellung erfolgt analog Beispiel 81A aus 412 mg (0.76 mmol) Benzyl-{(1*S*)-4-[(*tert-*butoxycarbonyl)amino]-1-[({3-[(*tert*-butoxycarbonyl)amino]-2-hydroxypropyl}amino)-carbonyl]-butyl}carbamat (Beispiel 80A) in 50 ml Ethanol unter Zusatz von 41 mg Palladium auf Aktivkohle (10%ig). Das Produkt wird ohne weitere Reinigung umgesetzt.
Ausbeute: 306 mg (99% d. Th.)
MS (ESI): m/z = 405 (M+H)⁺.

### Beispiel 83A

*tert*-Butyl-[(1*S*)-4-[(*tert*-butoxycarbonyl)amino]-1-(hydroxymethyl)butyl]carbamat 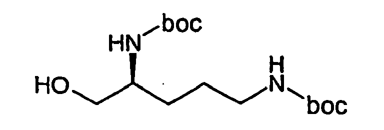 Eine Lösung von 300 mg (0.90 mmol) *N²*,*N⁵*-Bis(*tert*-butoxycarbonyl)*-L-*ornithin in 10 ml Tetrahydrofuran wird bei -10°C mit 91 mg (0.90 mmol) 4-Methylmorpholin und 98 mg (0.90 mmol) Chlorameisensäureethylester versetzt und 30 min gerührt. Bei dieser Temperatur werden 1.81 ml (1.81 mmol) einer 1M Lösung von Lithiumaluminiumhydrid in Tetrahydrofuran langsam zugetropft. Es wird langsam auf RT erwärmt und für 12 h bei RT gerührt. Unter Eiskühlung gibt man vorsichtig 0.1 ml Wasser und 0.15 ml 4.5%ige Natriumhydroxid-Lösung hinzu und rührt weitere 3 h bei RT. Der Ansatz wird filtriert und das Filtrat wird im Vakuum eingeengt. Der Rückstand wird in Essigsäureethylester gelöst, mit Wasser gewaschen, über Magnesiumsulfat getrocknet und erneut im Vakuum zur Trockne eingeengt. Das Produkt wird ohne weitere Reinigung umgesetzt.
Ausbeute: 239 mg (83% d. Th.)
MS (ESI): m/z = 319 (M+H)⁺; 341 (M+Na)⁺.

### Beispiel 84A

(2*S*)-2,5-Bis[(*tert*-butoxycarbonyl)amino]pentyl-methansulfonat 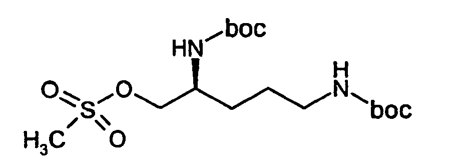 Eine Lösung von 240 mg (0.75 mmol) *tert*-Butyl-[(1*S*)-4-[(*tert*-butoxycarbonyl)amino]-1-(hydroxymethyl)butyl]carbamat (Beispiel 83A) in 20 ml Dichlormethan wird mit 103 mg (0.90 mmol) Methansulfonsäurechlorid und 0.21 ml (1.5 mmol) Triethylamin versetzt und für 16 h bei RT gerührt. Es wird mit Dichlormethan verdünnt und zweimal mit 0.1N Salzsäure gewaschen. Die organische Phase wird über Magnesiumsulfat getrocknet und im Vakuum bis zur Trockne eingeengt. Das Produkt wird ohne weitere Reinigung umgesetzt.
Ausbeute: 218 mg (73% d. Th.)
MS (ESI): m/z = 419 (M+Na)⁺.

### Beispiel 85A

*tert*-Butyl-{(4*S*)-5-azido-4-[(*tert*-butoxycarbonyl)amino]pentyl}carbamat 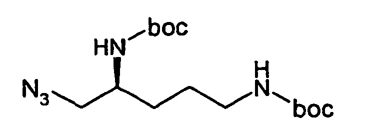 Eine Lösung von 218 mg (0.55 mmol) (2*S*)-2,5-Bis[(*tert*-butoxycarbonyl)amino]pentylmethansulfonat (Beispiel 84A) in 15 ml Dimethylformamid wird mit 36 mg (0.55 mmol) Natriumazid versetzt und 12 h bei 70°C gerührt. Ein Großteil des Lösungsmittel wird im Vakuum abdestilliert und der Rückstand wird mit Essigsäureethylester verdünnt. Es wird mehrmals mit gesättigter Natriumhydrogencarbonat-Lösung gewaschen, über Magnesiumsulfat getrocknet und im Vakuum zur Trockne eingeengt. Das Produkt wird ohne weitere Reinigung umgesetzt.
Ausbeute: 188 mg (99% d. Th.)
MS (ESI): m/z = 344 (M+H)⁺.

### Beispiel 86A

*tert*-Butyl-{(4*S*)-5-amino-4-[(*tert*-butoxycarbonyl)amino]pentyl}carbamat 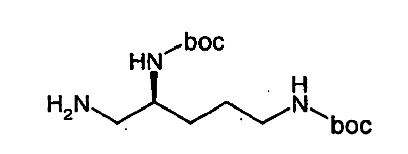 Eine Lösung von 188 mg (0.55 mmol) *tert*-Butyl-{(4*S*)-5-azido-4-[(*tert*-butoxycarbonyl)amino]-pentyl}carbamat (Beispiel 85A) in Ethanol wird nach Zugabe von 20 mg Palladium auf Aktivkohle (10%ig) 12 h bei RT und Normaldruck hydriert. Es wird über Kieselgur filtriert und der Rückstand mit Ethanol gewaschen. Das Filtrat wird im Vakuum zur Trockne eingeengt. Das Produkt wird ohne weitere Reinigung umgesetzt.
Ausbeute: 102 mg (59% d. Th.)
MS (ESI): m/z = 318 (M+H)⁺; 340 (M+Na)⁺.

### Beispiel 87A

Benzyl-[(1*S*)-3-[(*tert*-butoxycarbonyl)amino]-1-(hydroxymethyl)propyl]carbamat 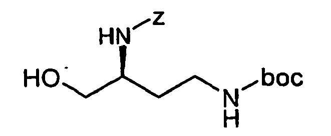 Die Herstellung erfolgt analog Beispiel 83A aus 300 mg (0.85 mmol) (2*S*)-2-{[(Benzyloxy)-carbonyl]amino}-4-[(*tert*-butoxycarbonyl)amino]butansäure in 10 ml Tetrahydrofuran mit 86 mg (0.85 mmol) 4-Methylmorpholin, 92 mg (0.85 mmol) Chlorameisensäureethylester und 1.7 ml (1.70 mmol) einer 1M Lösung von Lithiumaluminiumhydrid in Tetrahydrofuran. Das Produkt wird ohne weitere Reinigung umgesetzt.
Ausbeute: 229 mg (80% d. Th.)
LC-MS (Methode 12): Rₜ = 1.83 min.
MS (EI): m/z = 339 (M+H)⁺; 239 (M-C₅H₈O₂+H)⁺.

### Beispiel 88A

*tert*-Butyl-[(3*S*)-3-amino-4-hydroxybutyl]carbamat Hydrochlorid 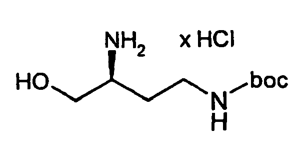 Die Herstellung erfolgt analog Beispiel 81A aus 229 mg (0.68 mmol) Benzyl-[(1*S*)-3-[(*tert-*butoxycarbonyl)amino]-1-(hydroxymethyl)propyl]carbamat (Beispiel 87A) in 50 ml Ethanol unter Zusatz von 23 mg Palladium auf Aktivkohle (10%ig). Das Rohprodukt wird in 1 ml 1N Salzsäure gerührt und im Vakuum eingedampft und im Hochvakuum bis zur Gewichtskonstanz getrocknet.
Ausbeute: 183 mg (90% d. Th.)
MS (ESI): m/z = 205 (M-HCl+H)⁺.

### Beispiel 89A

*tert*-Butyl-{(3*S*)-3-[(*tert*-butoxycarbonyl)amino]-4-hydroxybutyl}carbamat 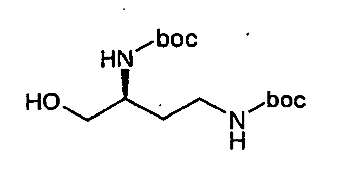 Die Herstellung erfolgt analog Beispiel 83A aus 300 mg (0.60 mmol) (2*S*)-2,4-Bis[(*tert-*butoxycarbonyl)amino]butansäure - *N-*Cyclohexylcyclohexanamin (1:1) in 10 ml Tetrahydrofuran mit 61 mg (0.60 mmol) 4-Methylmorpholin, 65 mg (0.60 mmol) Chlorameisensäureethylester und 1.2 ml (1.20 mmol) einer 1M Lösung von Lithiumaluminiumhydrid in Tetrahydrofuran. Das Produkt wird ohne weitere Reinigung umgesetzt.
Ausbeute: 174 mg (95% d. Th).
MS (ESI): m/z = 305 (M+H)⁺.

### Beispiel 90A

(2*S*)-2,4-Bis[(*tert*-butoxycarbonyl)amino]butyl-methansulfonat 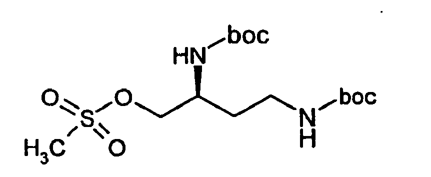 Die Herstellung erfolgt analog Beispiel 84A aus 250 mg (0.81 mmol) *tert*-Butyl-{(3*S*)-3-[(*tert-*butoxycarbonyl)amino]-4-hydroxybutyl}carbamat (Beispiel 89A) in 20 ml Dichlormethan mit 110 mg (0.97 mmol) Methansulfonsäurechlorid und 0.23 ml (1.6 mmol) Triethylamin. Das Produkt wird ohne weitere Reinigung umgesetzt.
Ausbeute: 200 mg (64% d. Th.')
MS (ESI): m/z = 383 (M+H)⁺; 400 (M+Na)⁺.

### Beispiel 91A

*tert*-Butyl-{(3*S*)-4-azido-3-[(*tert*-butoxycarbonyl)amino] butyl}carbarnat 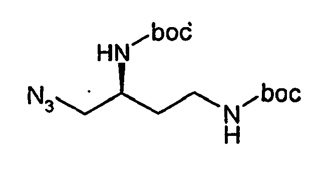 Die Herstellung erfolgt analog Beispiel 85A aus 200 mg (0.52 mmol) (2*S*)-2,4-Bis[(*tert-*butoacycarbonyl)amino]butyl-methansulfonat (Beispiel 90A) in 15 ml Dimethylformamid mit 34 mg (0.52 mmol) Natriumazid. Das Produkt wird ohne weitere Reinigung umgesetzt.
Ausbeute: 171 mg (99% d. Th.)

### Beispiel 92A

*tert*-Butyl- {(3*S*)-4-amino-3-[(*tert*-butoxycarbonyl)amino]butyl}carbamat 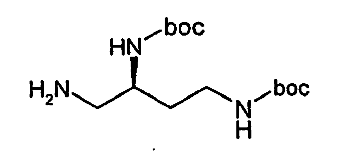 Die Herstellung erfolgt analog Beispiel 86A aus 171 mg (0.52 mmol) *tert*-Butyl-{(3*S*)-4-azido-3-[(*tert*-butoxycarbonyl)amino]butyl}carbamat (Beispiel 91A) in 10 ml Ethanol unter Zusatz von 20 mg Palladium auf Aktivkohle (10%ig). Das Produkt wird ohne weitere Reinigung umgesetzt.
Ausbeute: 117 mg (75% d. Th.)
MS (ESI): m/z = 304 (M+H)⁺; 326 (M+Na)⁺.

### Beispiel 93A

Benzyl-((4*S*)-4-[(*tert*-butoxycarbonyl)amino]-5-{[(1*S*)-3-[(*tert*-butoxycarbonyl)amino]-1-(hydroxymethyl)propyl]amino}-5-oxopentyl)carbamat 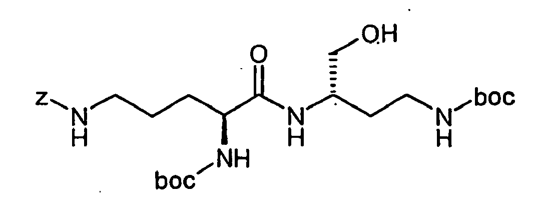 Die Herstellung erfolgt analog zu Beispiel 79A aus 140 mg (0.38 mmol) *N⁵*-[(Benzyloxy)carbonyl]-*N²*-(*tert*-butoxycarbonyl)*-L-*ornithin und 120 mg (0.50 mmol) *tert*-Butyl-[(3*S*)-3-amino-4-hydroxybutyl]carbamat Hydrochlorid (Beipiel 88A) in 6 ml Dimethylformamid unter Zusatz von 96 mg (0.50 mmol) EDC, 16 mg (0.12 mmol) HOBt und 0.17 ml (1.00 mmol) Diisopropylethylamin. Das Produkt wird mittels präparativer RP-HPLC gereinigt (Laufmittel Wasser / Acetonitril Gradient: 90:10 → 10:90).
Ausbeute: 50 mg (23% d. Th.)
LC-MS (Methode 19): Rₜ = 2.24min.
MS (EI): m/z = 553 (M+H)⁺

### Beispiel 94A

*N²*-(*tert*-Butoxycarbonyl)*-N-*[(1*S*)-3-[(*tert*-butoxycarbonyl)amino]-1-(hydroxymethyl)propyl]*-L-*ornithinamid 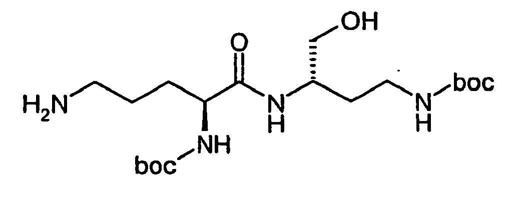 Die Herstellung erfolgt analog Beispiel 81A aus 50 mg (0.09 mmol) Benzyl-((4*S*)-4-[(*tert-*butoxycarbonyl)amino]-5-{[(1*S*)-3-[(*tert*-butoxycarbonyl)amino]-1-(hydroxymethyl)propyl]-amino}-5-oxopentyl)carbamat (Beispiel 93A) in 50 ml Ethanol unter Zusatz von 5 mg Palladium auf Aktivkohle (10%ig). Das Produkt wird ohne weitere Reinigung umgesetzt.
Ausbeute: 37 mg (98% d. Th.)
MS (ESI): m/z = 419 (M+H)⁺

### Beispiel 95A

Benzyl-{2-[((2*S*,4*R*)-5-{[(benzyloxy)carbonyl]amino}-2-[(*tert*-butoxycarbonyl)amino]-4-([*tert-*butyl(dimethyl)silyl]oxy}pentanoyl)amino]ethyl}carbamat 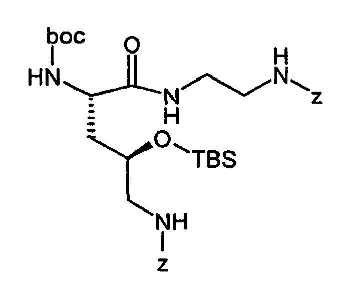 85 mg (0.17 mmol) 5-Benzyloxycarbonylamino-2(*S*)-*tert*-butoxycarbonylamino-4(*R*)-(*tert*-butyl-dimethylsilanyloxy)-pentansäure (Beispiel 14A), 67.1 mg (0.29 mmol) Benzyl-(2-aminoethyl)-carbamat Hydrochlorid und 0.05 ml (0.29 mmol) Diisopropylethylamin werden in 3 ml DMF gelöst und auf 0°C gekühlt. Man versetzt mit 55.8 mg (0.29 mmol) EDC und 7.6 mg (0.06 mmol) HOBT, läßt auf Raumtemperatur erwärmen und rührt über Nacht nach. Anschließend engt man im Vakuum ein und reinigt per Chromatographie an Kieselgel (Dichlormethan/Methanol 20:1)
Ausbeute: 73 mg (59% d. Th.).
LC-MS (Methode 12): Rₜ = 3.04 min.
MS (EI): m/z = 673 (M+H)⁺.

### Beispiel 96A

Benzyl-{2-[((2*S*,4*R*)-2-amino-5-([(benzyloxy)carbonyl]amino)-4-{[*tert*-butyl(dimethyl)silyl]oxy}-pentanoyl)amino]ethyl) carbamat Hydrochlorid 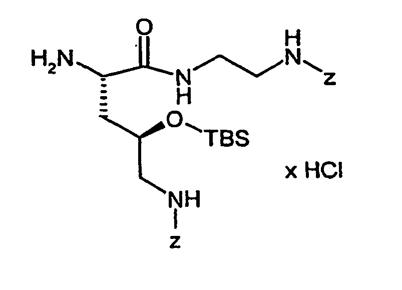 53 mg (0.08 mmol) der Verbindung aus Beispiel 95A werden auf 0°C gekühlt und mit 1 ml 4N Chlorwasserstoff in Dioxan versetzt. Nach 1 h engt man im Vakuum ein und erhält die Titelverbindung.
Ausbeute: 41 mg (90% d. Th.).
LC-MS (Methode 17): Rₜ = 1.60 min.
MS (EI): m/z = 459 (M-HCl+H)⁺.

### Beispiel 97A

(2*S*)-2- {[(Benzyloxy)carbonyl]amino}-4-[(*tert*-butoxycarbonyl)amino]butansäure 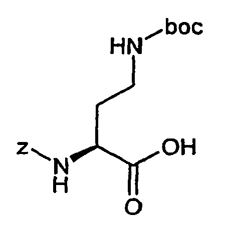 500 mg (1.98 mmol) (2*S*)-4-Amino-2-{[(benzyloxy)carbonyl]amino}butansäure werden in 5 ml Wasser und 5 ml 1N Natronlauge gelöst. 649 mg (2.97 mmol) Di-*tert*-butyldicarbonat in 2 ml Methanol wird zugegeben. Man erhitzt im Wasserbad kurz auf 30°C und rührt über Nacht bei Raumtemperatur nach. Nach Einengen im Vakuum reinigt man per Chromatographie an Kieselgel (Dichlormethan/Methanol/lconzentrierte Ammoniak-Lösung 85:15:3) und erhält so die Titelverbindung.
Ausbeute: 740 mg (99% d. Th.).
LC-MS (Methode 17): Rₜ = 2.08 min.
MS (EI): m/z = 353 (M+H)⁺.

### Beispiel 98A

Benzyl-{(1*S*)-3-[(*tert*-butoxycarbonyl)amino]-1-[({3-[(*tert*-butoxycarbonyl)amino]-2-hydroxypropyl}amino)carbonyl]propyl}carbamat 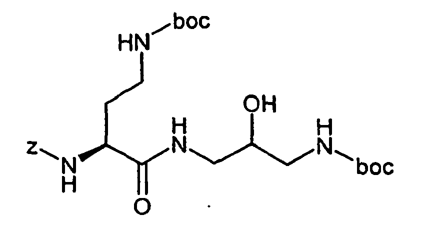 44 mg (0.12 mmol) der Verbindung aus Beispiel 97A und 40 mg (0.21 mmol) *tert*-Butyl-(3-amino-2-hydroxypropyl)carbamat werden in 1 ml DMF gelöst, auf 0°C gekühlt und nacheinander mit 40.3 mg (0.21 mmol) EDC und 5.51 mg (0.04 mmol) HOBt versetzt. Man läßt auf Raumtemperatur erwärmen und rührt über Nacht nach. Nach Einengen im Vakuum reinigt man per Chromatographie an Kieselgel (Dichlormethan/Methanol 20:1) und erhält so die Titelverbindung.
Ausbeute: 38 mg (59% d. Th.).
LC-MS (Methode 17): Rₜ = 2.25 min.
MS (EI): m/z = 525 (M+H)⁺.

### Beispiel 99A

*tert*-Butyl-[3-({(2*S*)-2-amino-4-[(*tert*-butoxycarbonyl)amino]butanoyl}amino)-2-hydroxypropyl]-carbamat 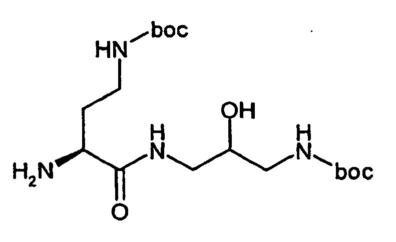 38 mg (0.12 mmol) der Verbindung aus Beispiel 98A werden in 10 ml Methanol gelöst und mit 10 mg Palladium auf Aktivkohle (10%ig) versetzt. Man hydriert für 2 h unter Normaldruck, filtriert über Kieselgur und engt die Mutterlauge im Vakuum ein.
Ausbeute: 26 mg (75% d. Th.).
LC-MS (Methode 12): Rₜ = 1.10 min.
MS (EI): m/z = 391 (M+H)⁺.

### Beispiel 100A

Benzyl-[(1*S*)-2-({3-[(*tert*-butoxycarbonyl)amino]-2-hydroxypropyl}amino)-1-(hydroxymethyl)-2-oxoethyl]carbamat 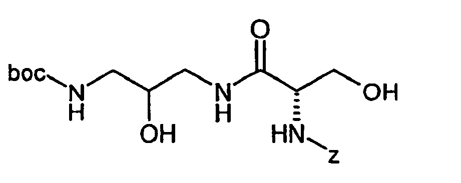 Zu einer Lösung von 207.5 mg (0.867 mmol) *N-*[(Benzyloxy)carbonyl]*-L-*serin in 10 ml wasserfreiem DMF werden 362.8 mg (0.954 mmol) HATU und 123.3 mg (0.954 mmol) *N,N*-Diisopropylethylamin hinzugegeben. Nach 15 min Rühren bei RT werden 165 mg (0.867 mmol) *tert-*Butyl-(3-amino-2-hydroxypropyl)carbamat hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 175 mg (49% d. Th).
LC-MS (Methode 12): Rₜ = 1.56 min.
MS (EI): m/z = 412 (M+H)⁺.

### Beispiel 101A

*N-*{3-[(*tert*-Butoxycarbonyl)amino]-2-hydroxypropyl}*-L-*serinamid 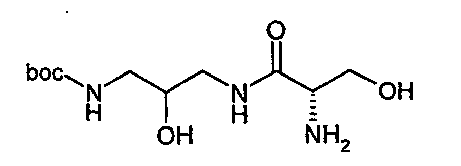 Es werden 131 mg (0.318mmol) Benzyl-[(1*S*)-2-({3-[(*tert*-butoxycarbonyl)amino]-2-hydroxypropyl}amino)-1-(hydroxymethyl)-2-oxoethyl]carbamat (Beispiel 100A) in 20 ml Ethanol gelöst. Dazu gibt man 20 mg Palladium auf Aktivkohle (10%ig) und hydriert anschließend 15 h bei Normaldruck. Das Reaktionsgemisch wird über vorgewaschenem Kieselgur filtriert und das Filtrat im Vakuum einrotiert. Das Rohprodukt wird ohne weitere Reinigung umgesetzt.
Ausbeute: 63 mg (71 % d. Th.).
LC-MS (Methode 19): Rₜ = 0.53 min
MS (EI): m/z= 278 (M+H)⁺.

### Beispiel 102A

Benzyl-(2-{[*N⁵*-[{[(benzyloxy)carbonyl]amino}(imino)methyl]-*N²*-(*tert*-butoxycarbonyl)*-L-*ornithyl]amino}ethyl)carbamat 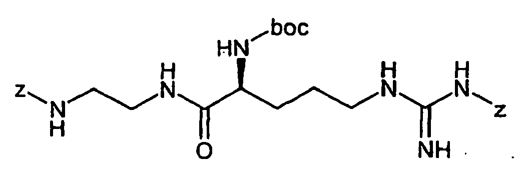 Zu einer Lösung von 408.4 mg (1 mmol) *N⁵*-[{[(Benzyloxy)carbonyl]amino}(imino)methyl]-*N²-*(*tert*-butoxycarbonyl)*-L-*ornithin in 15 ml) wasserfreiem DMF werden 419.3 mg (1.1 mmol) HATU und 258.5 mg (2 mmol) *N,N*-Diisopropylethylamin hinzugegeben. Nach 15 min Rühren bei RT werden 253.76 mg (1.1 mmol) Benzyl-(2-aminoethyl)carbamat Hydrochlorid hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute: 334 mg (41% d. Th.)
LC-MS (Methode 17): Rₜ = 1.94
MS (EI): m/z = 585 (M+H)⁺.

### Beispiel 103A

Benzyl-[(6*S*)-6-amino-7,12-dioxo-14-phenyl-13-oxa-2,8,11-triazatetradecan-1-imidoyl]carbamat-Hydrochlorid 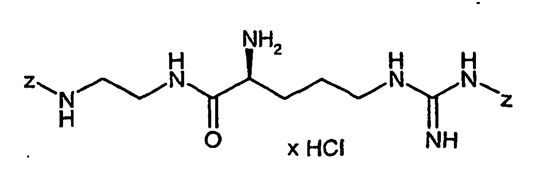 Eine Mischung von 334 mg (0.417 mmol) Benzyl-(2-{[1*N⁵*-[{[(benzyloxy)carbonyl]amino}-(imino)methyl]-*N²*-(*tert*-butoxycarbonyl)*-L-*ornithyl]amino}ethyl)carbamat (Beispiel 102A) und 17 ml einer 4M Chlorwasserstoff-Dioxan-Lösung wird 4 h bei RT gerührt. Die Reaktionslösung wird eingeengt, mehrmals mit Dichlormethan coevaporiert und im Hochvakuum getrocknet. Das Rohprodukt wird ohne weitere Reinigung umgesetzt.
Ausbeute: quant.
LC-MS (Methode 12): Rₜ = 1.05 min.
MS (EI): m/z= 485 (M-HCl+H)⁺.

### Beispiel 104A

Benzyl-[2-({(3*S*)-3-{[(benzyloxy)carbonyl]amino}-6-[(*tert*-butoxycarbonyl)amino]hexanoyl}-amino)ethyl]carbamat 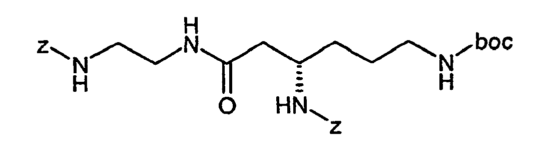 Zu einer Lösung von 500 mg (1.31 mmol) (3*S*)-3-{[(Benzyloxy)carbonyl]amino}-6-[(*tert-*butoxycarbonyl)amino]hexansäure in 25 ml wasserfreiem DMF werden 549.7 mg (1.446 mmol) HATU und 339.7 mg (2.629 mmol) *N,N-*Diisopropylethylamin hinzugegeben. Nach 15 min Rühren bei RT werden 333.5 mg (1.446 mmol) Benzyl-(2-aminoethyl)carbamat Hydrochlorid hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt Das Rohprodukt wird durch präparative HPLC aufgereinigt.
Ausbeute 556.6 mg (44% d. Th.)
LC-MS (Methode 17): Rₜ = 2.41 min.
MS (EI): m/z= 557 (M+H)⁺.

### Beispiel 105A

Benzyl-((1*S*)-4-amino-1-{2-[(2-{[(benzyloxy)carbonyl]amino}ethyl)amino]-2-oxoethyl}butyl)-carbamat Hydrochlorid 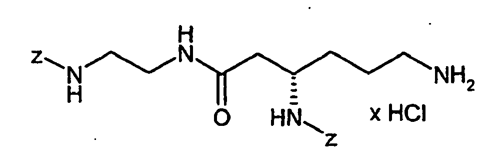 Zu einer Lösung von 320 mg (0.287 mmol) Benzyl-[2-({(3*S*)-3-{[(benzyloxy)carbonyl]amino}-6-[(*tert*-butoxycarbonyl)amino]hexanoyl]amino)ethyl]carbamat (Beispiel 104A) in 2 ml Dioxan werden bei 0°C 8 ml einer 4M Chlorwasserstoff-Dioxan-Lösung hinzugegeben. Nach 1 h bei RT wird die Reaktionslösung im Vakuum eingeengt, mehrmals mit Dichlormethan coevaporiert und im Hochvakuum getrocknet. Das Rohprodukt wird ohne weitere Reinigung umgesetzt.
Ausbeute: quant.
LC-MS (Methode 20): Rₜ = 2.84 min.
MS (EI): m/z = 457 (M-HCl+H)⁺.

### Beispiel 106A

Benzyl-{2-[((3*S*)-3-{[(benzyloxy)carbonyl]amino}-6-{[*N⁵*-[(benzyloxy)carbonyl]-*N²*-(*tert-*butoxycarbonyl)*-L-*ornithyl]amino}hexanoyl)amino]ethyl}carbamat 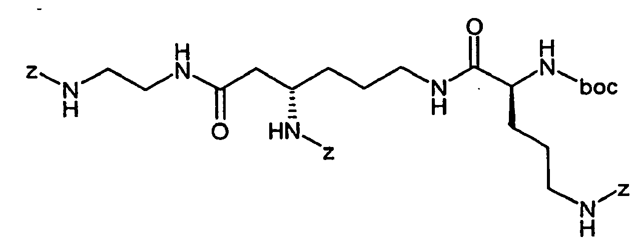 Zu einer Lösung von 78.4 mg (0.214 mmol) *N⁵*-[(Benzyloxy)carbonyl]-*N²*-(*tert*-butoxycarbonyl)*-L-*ornithin in 5 ml wasserfreiem DMF werden 89.5 mg (0.235 mmol) HATU und 55.3 mg (0.428 mmol) *N,N*-Diisopropylethylamin hinzugegeben. Nach 15 min Rühren bei RT wird eine Lösung von 116 mg (0.235 mmol) Benzyl-((1*S*)-4-amino-1-{2-[(2-{[(benzyloxy)carbonyl]amino}-ethyl)amino]-2-oxoethyl}butyl)carbamat Hydrochlorid (Beispiel 105A) in 5 ml wasserfreiem DMF hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 48 mg (28% d. Th.)
LC-MS (Methode 12): Rₜ = 2.33 min.
MS (EI): m/z = 805 (M+H)⁺.

### Beispiel 107A

Benzyl-((4*S*,10*S*)-4-amino-10-{[(benzyloxy)carbonyl]amino}-5,12,17-trioxo-19-phenyl-18-oxa-6,13,16-triazanonadec-1-yl)carbamat Hydrochlorid 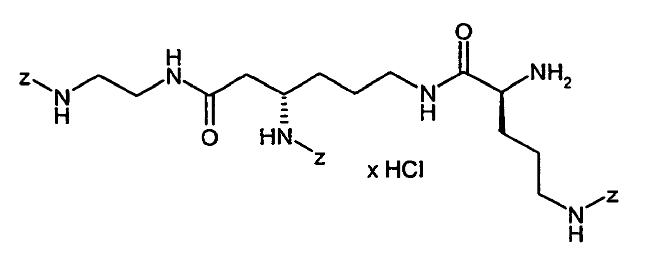 Zu einer Lösung von 48 mg (0.060 mmol) Benzyl-{2-[((3*S*)-3-{[(benzyloxy)carbonyl]amino}-6-{[1*N⁵*-[(benzyloxy)carbonyl]-*N²*-(*tert*-butoxycarbonyl)*-L-*ornithyl]amino}hexanoyl)amino]ethyl}-carbamat (Beispiel 106A) in 1 ml Dioxan werden bei RT 2.5 ml ml einer 4M Chlorwasserstoff Dioxan-Lösung hinzugegeben. Nach 4 h bei RT wird die Reaktionslösung im Vakuum eingeengt, mehrmals mit Dichlormethan coevaporiert und im Hochvakuum getrocknet. Das Rohprodukt wird ohne weitere Reinigung umgesetzt.
Ausbeute: quant.
LC-MS (Methode 12): Rₜ = 1.69 min.
MS (EI): m/z = 705 (M-HCl+H)⁺.

### Beispiel 108A

Benzyl-{(4*S*)-4-[(*tert*-butoxycarbonyl)amino]-5-[(2,3-dihydroxypropyl)amino]-5-oxopentyl}-carbamat 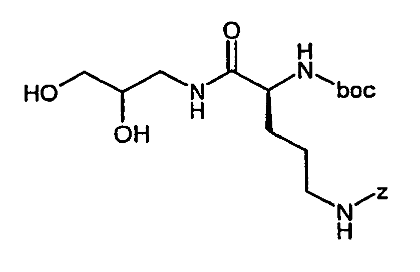 Zu einer Lösung von 366 mg (1mmol) *N⁵*-[(Benzyloxy)carbonyl]-*N²*-(*tert*-butoxycarbonyl)*-L-*ornithin in 15 ml wasserfreiem DMF werden 658.5 mg (1.8 mmol) HATU und 129.2 mg (1 mmol) *N,N*-Diisopropylethylamin hinzugegeben. Nach 15 min Rühren bei RT werden 182.2 mg (1 mmol) 3-Aminopropan-1,2-diol hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 135 mg (30% d. Th.)
LC-MS (Methode 12): Rₜ = 1.79 min.
MS (EI): m/z = 440 (M+H)⁺.

### Beispiel 109A

Benzyl-{(4*S*)-4-amino-5-[(2,3-dihydroxypropyl)amino]-5-oxopentyl}carbamat Hydrochlorid 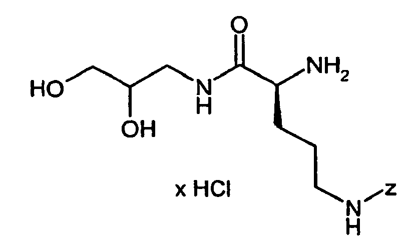 Zu einer Lösung von 135 mg (0.31 mmol) Benzyl-{(4*S*)-4-[(*tert*-butoxycarbonyl)amino]-5-[(2,3-dihydroxypropyl)amino]-5-oxopentyl}carbamat (Beispiel 108A) in 0.5 ml Dioxan werden bei 0°C 4 ml einer 4M Chlorwasserstoff-Dioxan-Lösung hinzugegeben. Nach 1 h bei RT wird die Reaktionslösung im Vakuum eingeengt, mehrmals mit Dichlormethan coevaporiert und im Hochvakuum getrocknet. Das Rohprodukt wird ohne weitere Reinigung umgesetzt.
Ausbeute: quant.
LC-MS (Methode 12): Rₜ = 1.01 min.
MS (EI): m/z = 340 (M-HCl+H)⁺.

### Beispiel 110A

Benzyl-[2-({(2*S*)-4-{[(benzyloxy)carbonyl]amino}-2-[(*tert*-butoxycarbonyl)amino]butanoyl}-amino)ethyl]carbamat 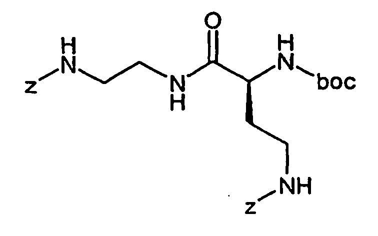 Zu einer Lösung von 410.8 mg (0.770 mmol) (2*S*)-4-{[(Benzyloxy)carbonyl]amino}-2-[(*tert-*butoxycarbonyl)amino]butansäure - *N-*Cyclohexylcyclohexanamin (1:1) in 25 ml wasserfreiem DMF werden 321.9 mg (0.847 mmol) HATU und 199.3 mg (1.534 mmol) *N,N*-Diisopropylethylamin hinzugegeben. Nach 15 min Rühren bei RT werden 195.3 mg (0.847 mmol) Benzyl-(2-aminoethyl)carbamat Hydrochlorid hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 270 mg (66% d. Th).)
LC-MS (Methode 12): Rₜ = 2.19 min.
MS (EI): m/z = 529 (M+H)⁺.

### Beispiel 111A

Benzyl-{2-[((2*S*)-2-amino-4-{[(benzyloxy)carbonyl]amino}butanoyl)amino]ethyl}carbamat Hydrochlorid 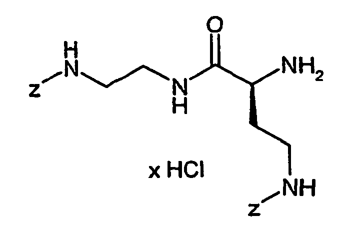 Eine Mischung von 270 mg (0.511 mmol) Benzyl-[2-({(2*S*)-4-{[(benzyloxy)carbonyl]amino}-2-[(*tert*-butoxycarbonyl)amino]butanoyl}amino)ethyl]carbamat (Beispiel 110A) und 9 ml einer 4M Chlorwasserstoff Dioxan-Lösung wird 2 h bei RT gerührt. Die Reaktionslösung wird eingeengt, mehrmals mit Dichlormethan coevaporiert und im Hochvakuum getrocknet. Das Rohprodukt wird ohne weitere Reinigung umgesetzt.
Ausbeute: quant.
LC-MS (Methode 17): Rₜ = 1.58 min.
MS (EI): m/z = 429 (M-HCl+H)⁺.

### .Beispiel 112A

Benzyl-{2-[((2*S*)-4-{[(benzyloxy)carbonyl]amino}-2-{[*N-*[(benzyloxy)carbonyl]-*N*²-(*tert-*butoxycarbonyl)*-L-*ornithyl]amino}butanoyl)amino]ethyl}carbamat 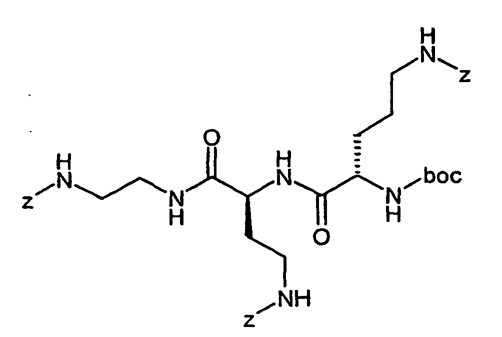 Zu einer Lösung von 155.4 mg (0.770 mmol) *N⁵*-[(Benzyloxy)carbonyl]-*N²*-(*tert*-butoxycarbonyl)-L-ornithin in 5 ml wasserfreiem DMF werden 217.0 mg (0.467 mmol) HATU und 109.7 mg (1.534 mmol) *N,N*-Diisopropylethylamin hinzugegeben. Nach 15 min Rühren bei RT wird eine Lösung von 195.3 mg (0.849 mmol) Benzyl-{2-[((2*S*)-2-amino-4-{[(benzyloxy)carbonyl]amino}-butanoyl)amino]ethyl}carbamat Hydrochlorid (Beispiel 111A) in 5 ml wasserfreiem DMF hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 71 mg (21 % d. Th.)
LC-MS (Methode 12): Rₜ = 2.42 min.
MS (EI): m/z = 777 (M+H)⁺

### Beispiel 113A

Benzyl-[(4*S,*7*S*)-4-amino-7-(2-{[(benzyloxy)carbonyl]amino}ethyl)-5,8,13-trioxo-15-phenyl-14-oxa-6,9,12-triazapentadec-1-yl]carbarnat Hydrochlorid 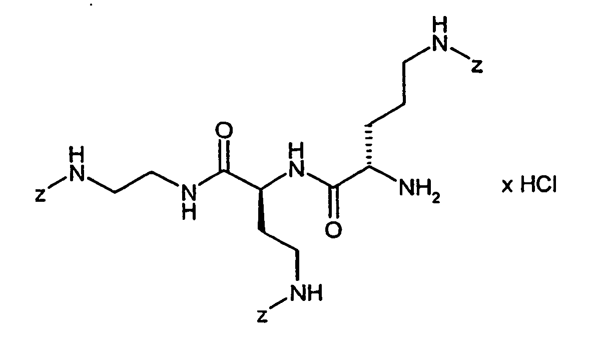 Zu einer Lösung von 71 mg (0.091 mmol) Benzyl-{2-[((2*S*)-4-{[(benzyloxy)carbonyl]amino}-2--{[*N⁵*-[(benzyloxy)carbonyl]-*N²*-(*tert*-butoxycarbonyl)*-L-*ornithyl]amino}butanoyl)amino]ethyl}-carbamat (Beispiel 112A) in 1.5 ml Dioxan werden bei RT 3.7 ml einer 4M Chlorwasserstoff-Dioxan-Lösung hinzugegeben. Nach 4 h bei RT wird die Reaktionslösung im Vakuum eingeengt, mehrmals mit Dichlormethan coevaporiert und im Hochvakuum getrocknet Das Rohprodukt wird ohne weitere Reinigung umgesetzt.
Ausbeute: quant.
LC-MS (Methode 12): Rₜ = 1.70 min.
MS (EI): m/z = 677 (M-HCl+H)⁺

### Beispiel 114A

Benzyl-{(1*S*)-4-[(*tert*-butoxycarbonyl)amino]-1-[2-({2-[(*tert*-butoxycarbonyl)amino]ethyl}amino)-2-oxoethyl]butyl}carbamat 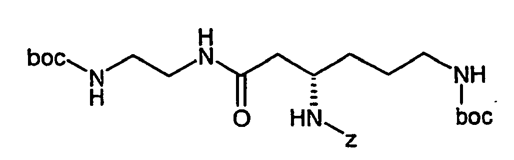 Zu einer Lösung von 760.9 mg (2 mmol) (3*S*)-3-{[(Benzyloxy)carbonyl]amino}-6-[(*tert-*butoxycarbonyl)amino]hexansäure in 25 ml wasserfreiem DMF werden 836.5 mg (2.2 mmol) HATU und 517.0 mg (4 mmol) *N,N*-Diisopropylethylamin hinzugegeben. Nach 15 min Rühren bei RT werden 352.5 mg (2.2 mmol) *tert*-Butyl-(2-aminoethyl)carbamat Hydrochlorid hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt
Ausbeute 400 mg (38% d. Th.)
LC-MS (Methode 19): Rₜ = 2.33 min.
MS (EI): m/z = 523 (M+H)⁺.

### Beispiele 115A

*tert*-Butyl-[(4*S*)-4-amino-6-({2-[(*tert*-butoxycarbonyl)amino]ethyl}amino)-6-oxohexyl]carbamat 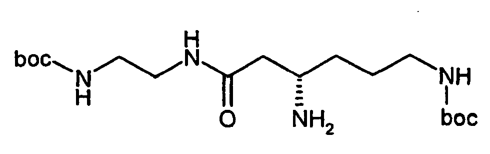 Es werden 400 mg (0.765 mmol) Benzyl-{(1*S*)-4-[(*tert*-butoxycarbonyl)amino]-1-[2-({2-[(*tert-*butoxycarbonyl)amino]ethyl}amino)-2-oxoethyl]butyl}carbamat (Beispiel 114A) in 50 ml Ethanol gelöst. Dazu gibt man 80 mg Palladium auf Aktivkohle (10%ig) und hydriert anschließend 15 h bei Normaldruck. Das Reaktionsgemisch wird über vorgewaschenem Kieselgur filtriert und das Filtrat im Vakuum einrotiert. Das Rohprodukt wird ohne weitere Reinigung umgesetzt.
Ausbeute: quant.
LC-MS (Methode 17): Rₜ = 1.42 min
MS (EI): m/z = 389 (M+H)⁺.

Analog zu der oben aufgeführten Vorschrift von Beispiel 83A werden die in der folgenden Tabelle aufgeführten Beispiele 116A und 117A aus den entsprechenden Ausgangsverbindungen hergestellt:

| **Bsp.-Nr.** | **Struktur** | **Hergestellt aus** | **Analytische Daten** |
|---|---|---|---|
| 116A | 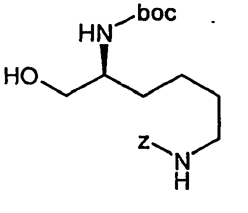 | *N⁶*-[(Benzyloxy)- carbonyl]-*N²*-(*tert*-butoxycarbonyl)*-L-*lysinlysin | LC-MS (Methode 12): Rₜ = 1.94 min. MS (EI):m/z=367(M+H)⁺ |
| 117A | 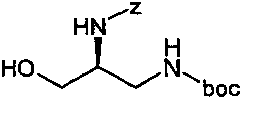 | *N-*[(Benzyloxy)-carbonyl]-3-[(*tert*-butoxycarbonyl)-amino]*-L-*alanin | LC-MS (Methode 19): Rₜ = 1.98 min. MS (EI): m/z = 325 (M+H)⁺ |

### Beispiel 118A

Benzyl-[(1*S*)-2-amino-1-(hydroxymethyl)ethyl]carbamat Hydrochlorid 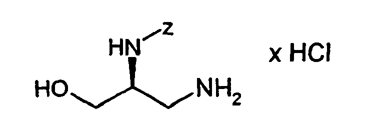 Eine Mischung von 269 mg (0.83 mmol) Benzyl-*tert*-butyl[(2*S*)-3-hydroxypropan-1,2-diyl]biscarbamat (Beispiel 117A) und 5 ml einer 4M Chlorwasserstoff-Dioxan-Lösung wird 2 h bei RT gerührt. Die Reaktionslösung wird eingeengt, mehrmals mit Dichlormethan coevaporiert und im Hochvakuum getrocknet. Das Rohprodukt wird ohne weitere Reinigung umgesetzt.
Ausbeute: 212 mg (98% d. Th.)
LC-MS (Methode 12): Rₜ = 0.55 min.
MS (EI): m/z = 225 (M-HCl+H)⁺.

### Beispiel 119A

Benzyl-{(1*S*)-1-[({(2*S*)-2,5-bis[(*tert*-butoxycarbonyl)amino]pentyl)amino)carbonyl]-4-[(*tert-*butoxycarbonyl)amino]butyl} carbamat 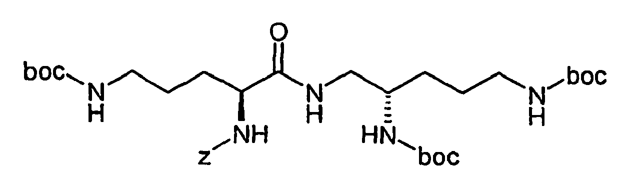 Die Herstellung erfolgt analog zu Beispiel 79A aus 120 mg (0.33 mmol) *N⁵*-(*tert*-Butoxycarbonyl)-*N²*-[(benzyloxy)carbonyl]*-L-*ornithin und 136 mg (0.43 mmol) *tert*-Butyl-{(4*S*)-5-amino-4-[(*tert-*butoxtycarbonyl)amino]pentyl}carbarmat (Beispiel 86A) in 6 ml Dimethylformamid unter Zusatz von 82 mg (0.43 mmol) EDC und 13 mg (0.1 mmol) HOBt. Das Produkt wird mittels präparativer RP-HPLC gereinigt (Laufmittel Wasser / Acetonitril Gradient: 90: 10→ 5:95).
Ausbeute: 132 mg (61 % d. Th.)
LC-MS (Methode 17): Rₜ = 2.68 min.
MS (EI): m/z = 666 (M+H)⁺

### Beispiel 120A

*tert*-Butyl-[(4*S*)-4-amino-5-({(2*S*)-2,5-bis[(*tert*-butoxycarbonyl)amino]pentyl}amino-5-oxopentyl]carbamat 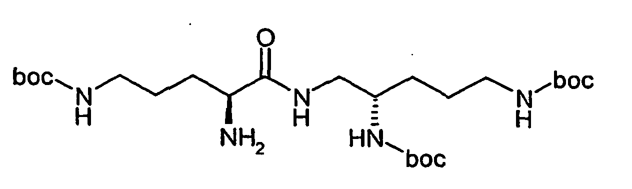 Die Herstellung erfolgt analog Beispiel 81A aus 132 mg (0.20 mmol) Benzyl-{(1*S*)-1-[({(2*S*)-2,5-bis[(*tert*-butoxycarbonyl)amino)pentyl}amino)carbonyl)-4-[(*tert*-butoxycarbony])amino]butyl}-carbamat (Beispiel 119A) in 50 ml Ethanol unter Zusatz von 13 mg Palladium auf Aktivkohle (10%ig). Das Produkt wird ohne weitere Reinigung umgesetzt.
Ausbeute: quant.
MS (ESI): m/z = 532 (M+H)⁺

### Beispiel 121A

Benzyl-[2-({(2*S*)-S-{(benzyloxy)carbonyl]amino}-2-[(*tert*-butoxycarbonyl)amino]pentanoyl}-amino)ethyl]carbamat 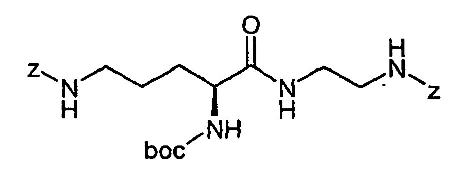 Die Herstellung erfolgt analog zu Beispiel 79A aus 300 mg (0.82 mmol) *N⁵*-[(Benzyloxy)carbonyl]-*N²*-(*tert*-butoxycarbonyl)*-L-*ornithin und 246 mg (1.06 mmol) Benzyl-(2-aminoethyl)carbamat Hydrochlorid in 6 ml Dimethylformamid unter Zusatz von 204 mg (1.06 mmol) EDC, 33 mg (0.25 mmol) HOBt und 148 mg (1.15 mmol) *N,N*-Diisopropylethylamin.
Ausbeute: 397 mg (89% d. Th.)
LC-MS (Methode 12): Rₜ = 2.20 min.
MS (EI): m/z = 543 (M+H)⁺

### Beispiel 122A

Benzyl-{(4*S*)-4-amino-5-[(2-([(benzyloxy)carbonyl]amino)ethyl)amino]-5-oxopentyl}carbamat Hydrochlorid 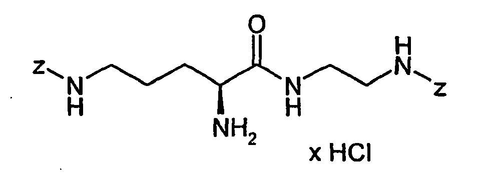 Eine Mischung von 400 mg (0.73 mmol) Benzyl-[2-({(2*S*)-S-{[(benzyloxy)carbonyl]amino}-2-[(*tert*-butoxycarbonyl)amino]pentanoyl}amino)ethyl]carbamat (Beispiel 121A) und 1 ml einer 4M Chlorwasserstoff Dioxan-Lösung wird 2 h bei RT gerührt. Die Reaktionslösung wird eingeengt, mehrmals mit Dichlormethan coevaporiert und im Hochvakuum getrocknet. Das Rohprodukt wird ohne weitere Reinigung umgesetzt.
Ausbeute: quant
LC-MS (Methode 19): Rₜ = 1.61 min.
MS (EI): m/z = 443 (M-HCl+H)⁺.

### Beispiel 123A

*N⁵*-[(Benzyloxy)carbonyl]-*N²*-(*tert*-butoxycarbonyl)*-L-*omithyl-*N⁵*-[(benzyloxy)carbonyl]*-N-*(2-{[(benzyloxy)carbonyl]amino}ethyl)*-L-*ornithinamid 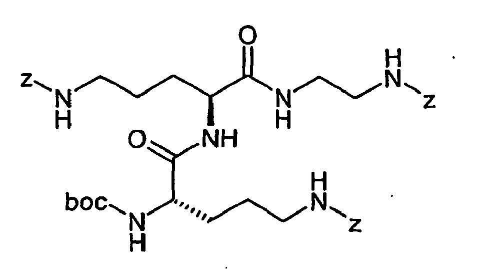 Zu einer Lösung von 320 mg (0.88 mmol) *N⁵*-[(Benzyloxy)carbonyl]-*N²*-(*tert*-butoxycarbonyl)*-L-*ornithin und 350 mg (0.73 mmol) Benzyl-{(4*S*)-4-amino-5-[(2-{[(benzyloxy)carbonyl]amino}-ethyl)amino]-5-oxopentyl}carbamat (Beispiel 122A) in 5 ml wasserfreiem DMF werden bei 0°C 350 mg (0.92 mmol) HATU und 330 mg (2.56 mmol) *N,N*-Diisopropylethylamin hinzugegeben. Das Reaktionsgemisch wird 15 h bei RT gerührt. Das Lösungsmittel wird dann eingedampft und der Rückstand mit Wasser ausgerührt, abfiltriert und im Hochvakuum getrocknet.
Ausbeute 480 mg (68% d. Th.)
LC-MS (Methode 19): Rₜ = 2.61 min.
MS (EI): m/z = 791 (M+H)⁺.

### Beispiel 124A

*N⁵*-[(Benzyloxy)carbonyl]*-L-*ornithyl-*N⁵*-[(benzyloxy)carbonyl]*-N-*(2-{[(benzyloxy)carbonyl]-amino} ethyl)*-L-*ornithinamid Hydrochlorid 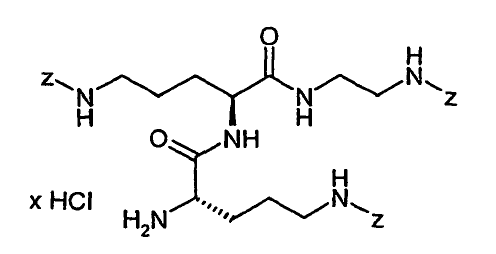 Die Herstellung erfolgt analog zu Beispiel 122A aus 70 mg (0.09 mmol *N⁵*-[(Benzyloxy)carbonyl]-*N²*-(*tert*-butoxycarbonyl)*-L-*ornithyl-*N⁵*-[(benzyloxy)carbonyl]*-N-*(2-{[(benzyloxy)carbonyl]-amino}ethyl)*-L-*ornithinamid (Beispiel 123A) in 0.68 ml 4M Chlorwasserstofflösung in Dioxan.
Ausbeute: 65 mg (98% d. Th.)
MS (ESI): m/z = 691 (M-HCl+H)⁺

### Beispiel 125A

Benzyl-[2-({(2*S*)-2,5-bis[(*tert*-butoxycarbonyl)amino]pentyl}amino)-2-oxoethyl]carbamat 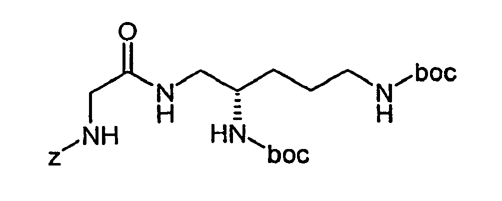 Die Herstellung erfolgt analog zu Beispiel 79A aus 92 mg (0.44 mmol) *N-*[(Benzyloxy)carbonyl]glycin und 181 mg (0.57 mmol) *tert*-Butyl-{(4*S*)-5-amino-4-[(*tert*-butoxycarbonyl)amino]pentyl}carbamat (Beispiel 86A) in 6 ml Dimethylformamid unter Zusatz von 110 mg (0.57 mmol) EDC und 18 mg (0.13 mmol) HOBt. Das Produkt wird mittels präparativer RP-HPLC gereinigt (Laufmittel Wasser / Acetonitril Gradient: 90:10 → 5:95).
Ausbeute: 105 mg (47% d. Th.)
LC-MS (Methode 12): Rₜ = 2.12 min.
MS (EI): m/z = 509 (M+H)⁺

### Beispiel 126A

*tert*-Butyl-{(4*S*)-5-[(aminoacetyl)amino]-4-[(*tert*-butoxycarbonyl)amino]pentyl}carbamat 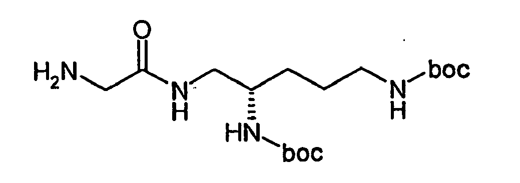 Die Herstellung erfolgt analog Beispiel 81A aus 105 mg (0.21 mmol) Benzyl-[2-({(2*S*)-2,5-bis[(*tert*-butoxycarbonyl)amino]pentyl}amino)-2-oxoethyl]carbamat (Beispiel 125A) in 50 ml Ethanol unter Zusatz von 11 mg Palladium auf Aktivkohle (10%ig). Das Produkt wird ohne weitere Reinigung umgesetzt.
Ausbeute: 64 mg (83% d. Th.)
MS (ESI): m/z = 375 (M+H)⁺

### Beispiel 127A

Benzyl-[(1*S*)-1-[(benzyloxy)methyl]-2-({(2*S*)-2,5-bis[(*tert*-butoxycarbonyl)amino]pentyl}amino)-2-oxoethyl]carbamat 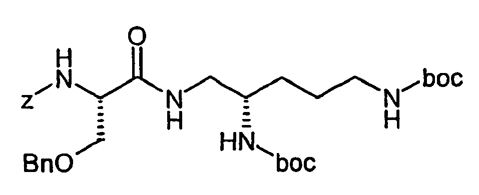 Die Herstellung erfolgt analog zu Beispiel 79A aus 150 mg (0.46 mmol) O-Benzyl*-N-*[(benzyloxy)carbonyl]*-L-*serin und 188 mg (0.59 mmol) *tert*-Butyl-{(4*S*)-5-amino-4-[(*tert-*butoxycarbonyl)amino]pentyl}carbamat (Beispiel 86A) in 6 ml Dimethylformamid unter Zusatz von 114 mg (0.57 mmol) EDC und 18 mg (0.13 mmol) HOBt. Das Produkt wird mittels präparativer RP-HPLC gereinigt (Laufmittel Wasser / Acetonitril Gradient: 90; 10→ 5:95).
Ausbeute: 129 mg (45% d. Th.)
LC-MS (Methode 17): Rₜ = 2.81 min.
MS (EI): m/z = 629 (M+H)⁺

### Beispiel 128A

*tert*-Butyl-{(4*S*)-S-{[(2*S*)-2-amino-3-hydroxypropanoyl]amino}-4-[(*tert*-butoxycarbonyl)amino]-pentyl}carbamat 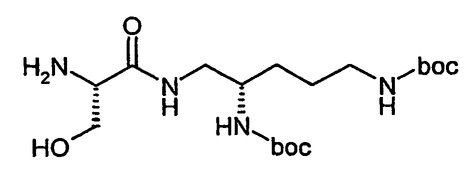 Eine Lösung von 128 mg (0.77 mmol) Benzyl-[(1*S*)-1-[(benzyloxy)methyl]-2-({(2*S*)-2,5-bis[(*tert-*butoxycarbonyl)amino]pentyl}amino)-2-oxoethyl]carbamat (Beispiel 127A) in 50 ml Ethanol wird nach Zugabe von 13 mg Palladium auf Aktivkohle (10%ig) 48 h bei RT und Normaldruck hydriert. Es wird über Kieselgur filtriert und der Rückstand mit Ethanol gewaschen. Das Filtrat wird im Vakuum zur Trockne eingeengt. Das Produkt wird mittels präparativer RP-HPLC gereinigt (Laufmittel Wasser / Acetonitril Gradient: 90:10→ 5:95).
Ausbeute: 22 mg (27% d. Th.)
LC-MS (Methode 19): Rₜ = 1.43 min.
MS (EI): m/z = 405 (M+H)⁺

### Beispiel 129A

9H-Fluoren-9-ylmethyl-{(1*S*)-4-amino-1-[({(2*S*)-2,5-bis[(*tert*-butoxycarbonyl)amino]pentyl)-amino)carbonyl]-4-oxobutyl} carbamat 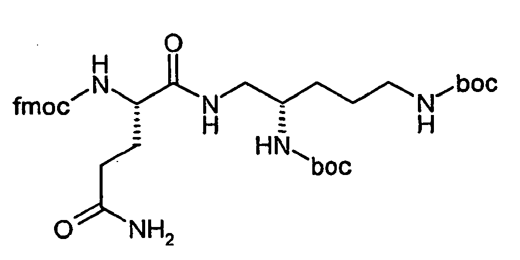 Zu einer Lösung von 100 mg (0.27 mmol) *N²*-[(9H-Fluoren-9-ylmethoxy)carbonyl]*-L-*glutamin und 112 mg (0.35 mmol) *tert*-Butyl-{(4*S*)-5-amino-4-[(*tert*-butoxycarbonyl)amino]pentyl}carbamat (Beispiel 86A) in 5 ml wasserfreiem DMF werden bei 0°C 129 mg (0.34 mmol) HATU und 133 mg (0.95 mmol) *N,N*-Diisopropylethylamin hinzugegeben. Das Reaktionsgemisch wird 15 h bei RT gerührt. Das Lösungsmittel wird dann eingedampft und der Rückstand mit Wasser ausgerührt, abfiltriert und im Hochvakuum getrocknet.
Ausbeute 45 mg (25% d. Th.)
LC-MS (Methode 12): Rₜ = 2.27 min.
MS (EI): m/z = 668 (M+M)⁺.

### Beispiel 130A

*tert*-Butyl-[(1*S*)-4-[(*tert*-butoxycarbonyl)amino]-1-({[(2*S*)-2,5-diamino-5-oxopentanoyl]amino}-methyl)butyl]carbamat 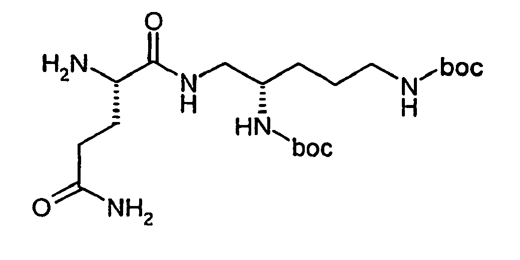 Eine Lösung von 33 mg (0.05 mmol) 9H-Fluoren-9-ylmethyl-{(1*S*)-4-amino-1-[({(2*S*)-2,5-bis[(*tert*-butoxycarbonyl)amino]pentyl}amino)carbonyl]-4-oxobutyl}carbamat (Beispiel 129A) in 1ml Dimethylformamid wird nach Zugabe von Zusatz von 4 mg (0.05 mmol) Piperidin 45 min bei Raumtemperatur gerührt. Das Lösungsmittel wird eingedampft und das Rohprodukt wird ohne weitere Reinigung umgesetzt.
Ausbeute: quant.
MS (ESI): m/z = 446 (M+H)⁺

Analog zu der oben aufgeführten Vorschrift von Beispiel 79A werden die in der folgenden Tabelle aufgeführten Beispiele 131A bis 135 aus den entsprechenden Edukten hergestellt:

| **Bsp.-Nr.** | **Struktur** | **Hergestellt aus** | **Analytische Daten** |
|---|---|---|---|
| **131A** | 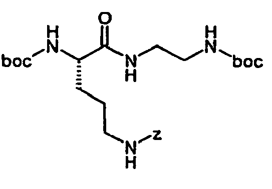 | *N⁵*-[(Benzyloxy)-carbonyl]-*N²*-(*tert*-butoxycarbonyl)*-L-*ornithin und *tert*-Butyl-(2-aminoethyl)-carbamat | LC-MS (Methode 19): Rₜ = 2.33 min. MS (EI): m/z = 509 (M+H)⁺ |
| **132A** | 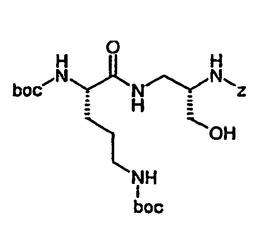 | *N²,N⁵-*Bis(*tert-* butoxycarbonyl)*-L-*ornithin und Beispiel 118A | LC-MS (Methode 19): Rₜ = 2.20 min. MS (EI): m/z = 539 (M+H)⁺ |
| **133A** | 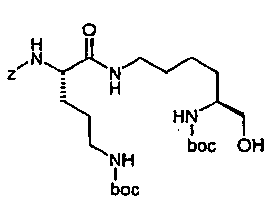 | *N²*-[(Benzyloxy)-carbonyl]-*N⁵*-(*tert*-butoxycarbonyl)*-L-*ornithin und Beispiel 142A | LC-MS (Methode 19): Rₜ = 2.31 min. MS (EI): m/z = 581 (M+H)⁺ |
| **134A** | 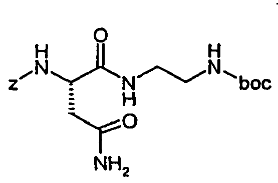 | *N²*-[(Benzyloxy)-carbonyl]*-L-*asparagin und *tert*-Butyl-(2-aminoethyl)-carbamat | LC-MS (Methode 19): Rₜ = 1.75 min. MS (EI): m/z = 409 (M+H)⁺ |
| 135A | 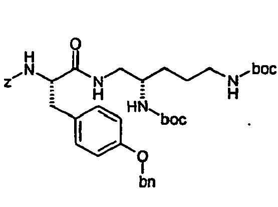 | *O*-Benzyl*-N-*[(benzyloxy)carbonyl]-*L*-tyrosin und Beispiel 86A | LC-MS (Methode 12): Rₜ = 2.79 min. MS (EI): m/z = 705 (M+H)⁺ |

Analog zu der oben aufgeführten Vorschrift von Beispiel 129A werden die in der folgenden Tabelle aufgeführten Beispiele 136A bis 141A aus den entsprechenden Edukten hergestellt:

| **Bsp.-Nr.** | **Struktur** | **Hergestellt aus** | **Analytische Daten** |
|---|---|---|---|
| **136A** | 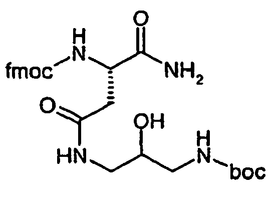 | *N*²-[(9H-Fluoren-9-ylmethoxy)-carbonyl]-*L*-alpha-asparagin und *tert*-Butyl-(3-amino-2-hydroxy-propyl)-carbamat | LC-MS (Methode 17): Rₜ = 2.09 min. MS (EI): m/z - 527 (M+H)⁺ |
| **137A** | 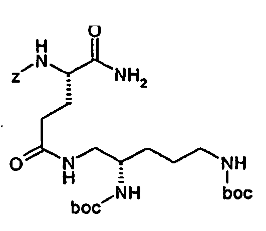 | *N*²-[(Benzyloxy)-carbonyl]*-L-*alpha-glutamin und Beispiel 86A | LC-MS (Methode 12): Rₜ = 1.93 min. MS (EI): m/z = 580 (M+H)⁺ |
| **138A** | 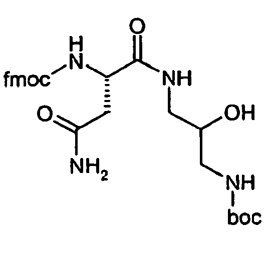 | *N*²-[(9H-Fluoren-9-ylmethoxy)-carbonyl]-*L*-asparagin und *tert*-Butyl-(3-amino-2-hydroxy-propyl)-carbamat | LC-MS (Methode 12): Rₜ = 1.88 min. MS (EI): m/z = 527 (M+H)⁺ |
| **139A** | 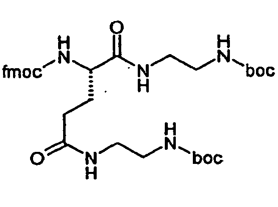 | *N-*[(9H-Fluoren-9-ylmethoxy)carbonyl]*-L-*glutaminsäure und *tert*-Butyl-(2-aminoethyl)-carbamat | LC-MS (Methode 12): Rₜ = 2.31 min. MS (EI): m/z = 654 (M+H)⁺ |
| **140A** | 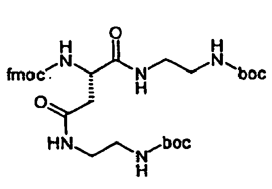 | *N-*[(9H-Fluoren-9-ylmethoxy)carbonyl]*-L-*asparaginsäure und *tert*-Butyl-(2-aminoethyl)-carbamat | LC-MS (Methode 19): Rₜ = 2.52 min. MS (EI): m/z = 640 (M+H)⁺ |
| **141A** | 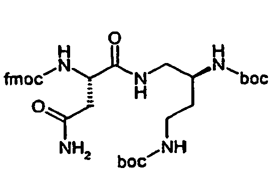 | *N²*-[(9H-Fluoren-9-ylmethoxy)-carbonyl]-*L*-asparagin und Beispiel 92A | LC-MS (Methode 17): Rₜ = 2.46 min. MS (EI): m/z = 640 (M+H)⁺ |

Analog zu der oben aufgeführten Vorschrift von Beispiel 81A werden die in der folgenden Tabelle aufgeführten Beispiele 142A bis 148A aus den entsprechenden Edukten hergestellt:

| **Bsp.- Nr.** | **Struktur** | **Hergestellt aus** | **Analytische Daten** |
|---|---|---|---|
| **142A** | 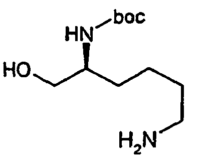 | Beispiel 116A | MS (ESI): m/z = 233 (M+H)⁺ |
| **143A** | 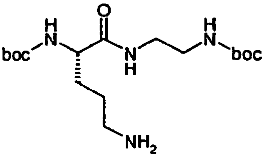 | Beispiel 131 A | MS (ESI): m/z = 375 (M+H)⁺ |
| **144A** | 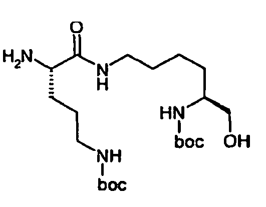 | Beispiel 133A | MS (ESI): m/z = 447 (M+H)⁺ |
| **145A** | 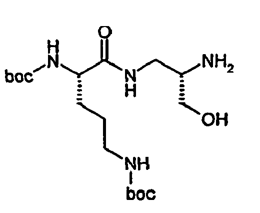 | Beispiel 132A | MS (ESI): m/z = 405 (M+H)⁺ |
| **146A** | 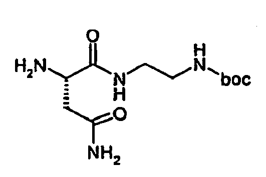 | Beispie1134A | LC-MS (Methode 12): Rₜ = 0.41 min. MS (EI): m/z = 275 (M+H)⁺ |
| **147A** | 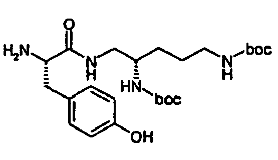 | Beispiel 135A | LC-MS (Methode 17): Rₜ = 1.67 min. MS (EI): m/z = 481 (M+H)⁺ |
| **148A** | 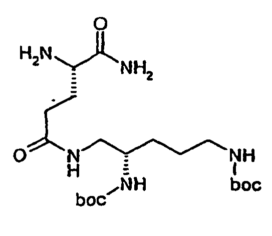 | Beispiel 137A | MS (ESI): m/z= 289 (M+H)⁺ |

Analog zu der oben aufgeführten Vorschrift von Beispiel 130A werden die in der folgenden Tabelle aufgeführten Beispiele 149A bis 153A aus den entsprechenden Edukten hergestellt:

| **Bsp.- Nr.** | **Struktur** | **Hergestellt aus** | **Analytische Daten** |
|---|---|---|---|
| **149A** | 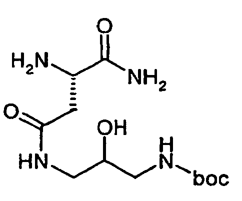 | Beispiel 136A | MS (ESI): m/z = 305 (M+H)⁺ |
| **150A** | 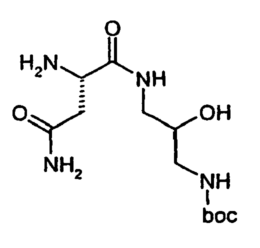 | Beispiel 138A | MS (ESI): m/z= 305 (M+H)⁺ |
| **151A** | 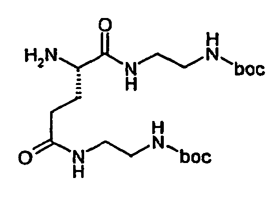 | Beispiel 139A | MS (ESI): m/z = 432 (M+H)⁺ |
| **152A** | 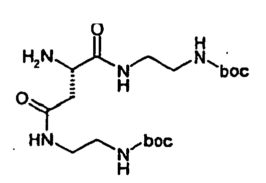 | Beispiel 140A | MS (ESI): m/z = 418 (M+H)⁺ |
| **153A** | 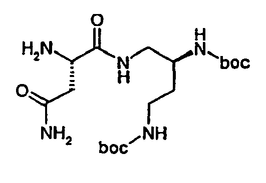 | Beispiel 141A | MS (ESI): m/z= 418 (M+H)⁺ |

### Beispiel 154A

Benzyl-[(3*S*)-3-[(*tert*-butoxycarbonyl)amino]-4-({3-[(*tert*-butoxycarbonyl)amino]-2-hydroxypropyl}amino)-4-oxobutyl]carbamat 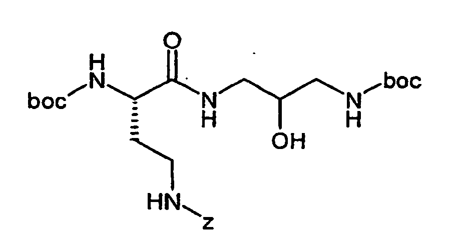 Zu einer Lösung von 133.4 mg (0.25 mmol) (2*S*)-4-{[(Benzyloxy)carbonyl]amino}-2-[(*tert-*butoxycarbonyl)amino]butansäre - *N-*Cyclohexylcyclohexanamin (1:1) in 10 ml wasserfreiem DMF werden 104.5 mg (0.275 mmol) HATU und 64.6 mg (0.500 mmol) *N,N Diiso*propylethylamin hinzugegeben. Nach 15 min Rühren bei RT werden 52.3 mg (0.275 mmol) *tert-*Butyl-(3-amino-2-hydroxypropyl)carbamat hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 25 mg (19% d. Th.)
LC-MS (Methode 17): Rₜ = 2.23 min.
MS (EI): m/z = 525 (M+H)⁺

### Beispiel 155A

*tert*-Butyl-[3-({(2*S*)-4-amino-2-[(*tert*-butoxycarbonyl)amino]butanoyl}amino)-2-hydroxypropyl]-carbamat 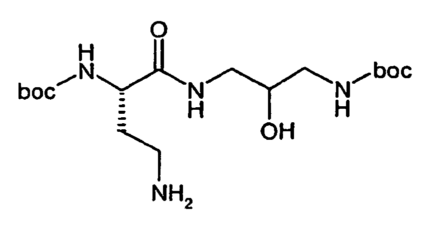 Es werden 25 mg (0.048 mmol) Benzyl-[(3*S*)-3-[(*tert*-butoxycarbonyl)amino]-4-({3-[(*tert-*butoxycarbonyl)amino]-2-hydroxypropyl}amino)-4-oxobutyl]carbamat (Beispiel 154A) in 10 ml Ethanol gelöst. Dazu gibt man 10 mg Palladium auf Aktivkohle (10%ig) und hydriert anschließend 15 h bei Normaldruck. Das Reaktionsgemisch wird über vorgewaschenem Kieselgur filtriert und das Filtrat im Vakuum einrotiert. Das Rohprodukt wird ohne weitere Reinigung umgesetzt.
Ausbeute: quant.
LC-MS (Methode 12): Rₜ = 1.10 min
. MS (EI): m/z = 391 (M+H)⁺

### Beispiel 156A

Di-*tert*-butyl-[2-({(2*S*)-4-{[(benzyloxy)carbonyl]amino}-2-[(*tert*-butoxycarbonyl)amino]-butanoyl} amino)propan-1,3-diyl]biscarbamat 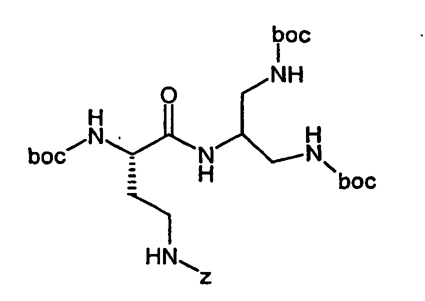 Zu einer Lösung von 142.7 mg (0.267 mmol) (2*S*)-4-{[(Benzyloxy)carbonyl]amino}-2-[(*tert-*butoxycarbonylamino]butansäure - *N-*Cyclohexylcyclohexanamin (1:1) in 10 ml wasserfreiem DMF werden 11.9 mg (0.294 mmol) HATU und 64.1 mg (0.535 mmol) *N,N-*Diisopropylethylamin hinzugegeben. Nach 15 min Rühren bei RT werden 100 mg (0.294 mmol) Di-*tert*-butyl-(2-aminopropan-1,3-diyl)biscarbamat Hydrochlorid hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 116 mg (70% d. Th.)
LC-MS (Methode 19): Rₜ = 2.71 min.
MS (EI): m/z = 624 (M+H)⁺

### Beispiel 157A

Di-*tert*-butyl-[2-({(2*S*)-4-amino-2-[(*tert*-butoxycarbonyl)amino]butanoyl}amino)propan-1,3-diyl]biscarbamat 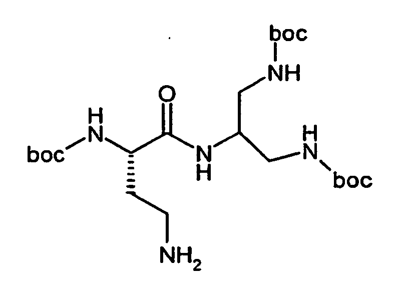 Es werden 116 mg (0.186 mmol) Di-*tert*-butyl-[2-({(2*S*)-4-{[(benzyloxy)carbonyl]amino}-2-[(*tert-*butoxycarbonyl)amino]butanoyl}amino)propan-1,3-diyl]biscarbamat (Beispiel 156A) in 20 ml Ethanol gelöst. Dazu gibt man 30 mg Palladium auf Aktivkohle (10%ig) und hydriert anschließend 15 h bei Normaldruck. Das Reaktionsgemisch wird über vorgewaschenem Kieselgur filtriert und das Filtrat im Vakuum einrotiert. Das Rohprodukt wird ohne weitere Reinigung umgeseztzt.
Ausbeute: 72 mg (80% d. Th)
LC-MS (Methode 17): Rₜ = 1.75 min
MS (EI): m/z = 490 (M+H)⁺

### Beispiel 158A

Benzyl(2-([*N-*(*tert*-butoxycarbonyl)*-L-*seryl]amino}ethyl)carbamat 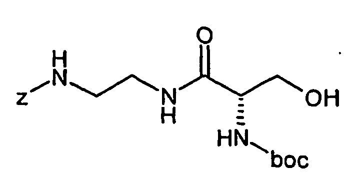 Zu einer Lösung von 307.8 mg (1.50 mmol) *N-*(*tert*-Butoxycarbonyl)*-L-*serin in 25 ml wasserfreiem DMF werden 627.4 mg (1.650 mmol) HATU und 387.7 mg (3.0 mmol) *N,N*-Diisopropylethylamin hinzugegeben. Nach 15 min Rühren bei RT werden 380.6 mg (1.650 mmol) Benzyl-(2-aminoethyl)carbamat Hydrochlorid hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute: 49 mg (7% d. Th.)
LC-MS (Methode 17): Rₜ = 1.83. min.
MS (EI): m/z= 382 (M+H)⁺

### Beispiel 159A

Benzyl-[2-(L-serylamino)ethyl]carbamat Hydrochlorid 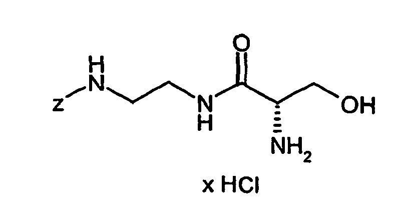 Zu einer Lösung von 49. mg (0.128 mmol) Benzyl-(2-([*N-*(*tert*-butoxycarbonyl)*-L-*seryl]amino)-ethyl)carbamat (Beispiel 158A) in 1 ml Dioxan werden 1.5 ml einer 4M Chlorwasserstoff-Dioxan-Lösung hinzugegeben. Die Mischung wird 2 h bei RT gerührt. Die Reaktionslösung wird eingeengt, mehrmals mit Dichlormethan coevaporiert und im Hochvakuum getrocknet. Das Rohprodukt wird ohne weitere Reinigung umgesetzt.
Ausbeute: 33 mg (91% d. Th.)
LC-MS (Methode 19): Rₜ = 0.89 min.
MS (EI): m/z= 282 (M-HCl+H)⁺

### Beispiel 160A

Benzyl-{(1*S*)-5-{[(benzyloxy)carbonyl]amino}-1-[2-({3-[(*tert*-butoxycarbonyl)amino]-2-hydroxypropyl}amino)-2-oxoethyl]pentyl}carbmnat 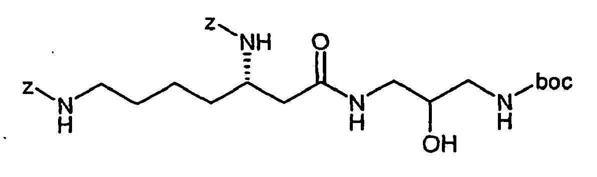 Zu einer Lösung von 428.5 mg 1 mmol) (3*S*)-3,7-Bis{[(benzyloxy)carbonyl]amino}heptansäure in 30 ml wasserfreiem DMF werden 418.2 mg (1.1 mmol) HATU" und 258.7 mg (2 mmol) *N,N*-Diisopropylethylamin hinzugegeben. Nach 15 min Rühren bei RT werden 209.2 mg (1.1 mmol) *tert*-Butyl-(3-amino-2-hydroxypropyl)carbamat hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 310 mg (47% d. Th.)
LC-MS (Methode 17): Rₜ = 2.38 min.
MS (EI): m/z = 601 (M+H)⁺

### Beispiel 161A

Benzyl-((5*S*)-7-[(3-amino-2-hydroxypropyl)amino]-5-{[(benzyloxy)carbonyl]amino}-7-oxoheptyl)carbamat Hydrochlorid 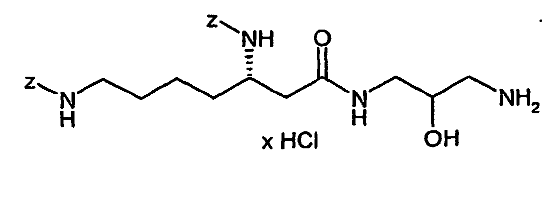 Zu einer Lösung von 160 mg (0.267 mmol) Benzyl-{(1*S*)-5-{[(benzyloxy)carbonyl]amino}-1-[2-({3-[(*tert*-butoxycarbonyl)amino]-2-hydroxypropyl}amino)-2-oxoethyl]pentyl}carbamat (Beispiel 160A) in 5.5' ml Dioxan bei 0 °C werden 11 ml einer 4M Chlorwasserstoff-Dioxan-Lösung hinzugegeben. Die Mischung wird 1h bei RT gerührt. Die Reaktionslösung wird eingeengt, mehrmals mit Dichlormethan coevaporiert und im Hochvakuum getrocknet. Das Rohprodukt wird ohne weitere Reinigung umgesetzt.
Ausbeute: quant.
LC-MS (Methode 17): Rₜ =1.77 min.
MS (EI): m/z = 501 (M-HCl+H)⁺.

### Beispiel 162A

Benzyl-[(1*S*)-2-({2-[(*tert*-butoxycarbonyl)amino]ethyl}amino)-1-(hydroxymethyl)-2-oxoethyl]-carbamat 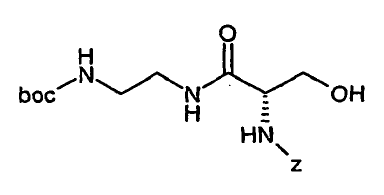 Zu einer Lösung von 600 mg (2.5 mmol) *N-*[(Benzyloxy)carbonyl]*-L-*serin in 25 ml wasserfreiem DMF werden 1.05 g (2.759 mmol) HATU und 648.3 mg (5.016 mmol) *N,N*-Diisopropylethylamin hinzugegeben. Nach 15 min Rühren bei RT werden 442.0 mg (2.76 mmol) *tert*-Butyl-(2-aminoethyl)carbamat hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 295 mg (31% d. Th.)
LC-MS (Methode 12): Rₜ =1.63 min.
MS (EI): m/z = 382 (M+H)⁺

### Beispiel 163A

Benzyl-[(1*S*)-2-[(2-aminoethyl)amino]-1-(hydroxymethyl)-2-oxoethyl]carbamat Hydrochlorid 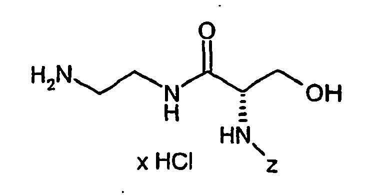 Eine Mischung von 58 mg (0.152 mmol) Benzyl-[(1S)-2-({2-[(*tert*-butoxycarbonyl)amino]-ethyl}amino)-1-(hydroxymethyl)-2-oxoethyl]carbamat (Beispiel 162A) in 25 ml Dioxan einer 4M Chlorwasserstoff-Dioxan-Lösung wird 2 h bei RT gerührt. Die Reaktionslösung wird eingeengt, mehrmals mit Dichlormethan coevaporiert und im Hochvakuum getrocknet. Das Rohprodukt wird ohne weitere Reinigung umgesetzt.
Ausbeute: 42 mg (65% d. Th.)
LC-MS (Methode 17): Rₜ = 0.59 min.
MS (EI): m/z = 282 (M-HCl+H)⁺.

### Beispiel 164A

*N⁵*-[(Benzyloxy)carbonyl]-*N²*-(*tert*-butoxycarbonyl)*-L-*ornithyl*-N-*(2-{[(benzyloxy)carbonyl]-amino} ethyl)*-L-*serinarnid 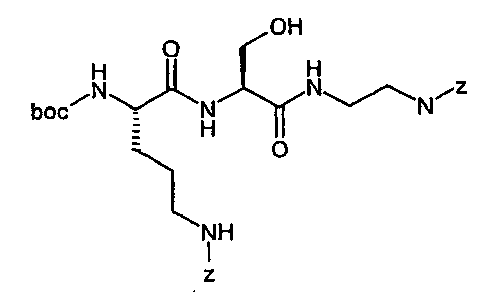 Zu einer Lösung von 2.42 g (6.61 mmol) *N⁵*-[(Benryloxy)carbonyl]-*N²*-(*tert*-butoxycarbonyl)*-L-*ornithin in 10 ml wasserfreiem DMF werden bei 0°C 2.76 g (7.27 mmol) HATU und 1.71 g (13.22 mmol) *N,N*-Diisopropylethylamin hinzugegeben. Nach 15 min Rühren bei RT werden 2.1 g (6.61 mmol) Benzyl-[2-(L-serylamino)ethyl]carbamat Hydrochlorid (Beispiel 159A) hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.
Ausbeute 122 mg (3% d. Th.)
LC-MS (Methode 17): Rₜ = 2.25 min.
MS (EI): m/z = 630 (M+H)⁺

### Beispiel 165A

*N*⁵-[(Benzyloxy)carbonyl]-*L-*ornithyl-*N*(2-{[(benzyloxy)carbonyl]amino}ethyl)-*L*-serinamid Hydrochlorid 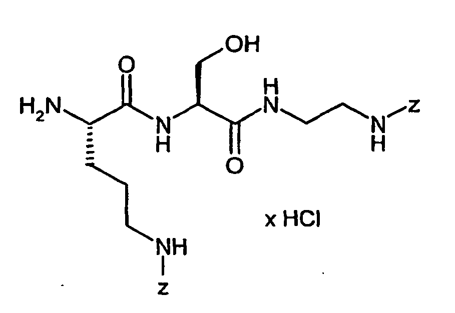 Zu einer Lösung von 120mg (0.191 mmol) *N*⁵-[(Benzyloxy)carbonyl]-*N*²-(*tert*-butoxycarbonyl)-*L-*omithyl-*N*-(2-([(benzyloxy)carbonyl]amino)ethyl)-*L*-serinamid (Beispiel 164A) in 5 ml Dioxan bei RT werden 10 ml einer 4M Chlorwasserstoff Dioxan-Lösung hinzugegeben. Die Mischung wird 1 h bei RT gerührt. Die Reaktionslösung wird eingeengt, mehrmals mit Dichlormethan coevaporiert und im Hochvakuum getrocknet. Das Rohprodukt wird ohne weitere Reinigung umgesetzt.
Ausbeute: quant.
LC-MS (Methode 19): Rₜ = 1.63 min.
MS (EI): m/z = 530 (M-HCl+H)⁺.

### Beispiel 166A

*tert*-Butyl-(3-{[(2*S*)-5-[(*tert*-butoxycarbonyl)amino]-2-({[(8*S*,11*S*,14*S*)-14-[(*tert*-butoxycarbonyl) amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)pentanoyl]amino}-2-hydroxypropyl)carbamat 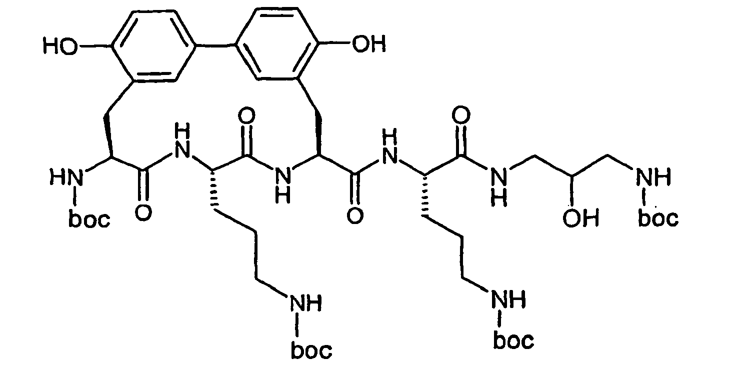 Unter Argon werden 50 mg (0.076 mmol) der Verbindung aus Beispiel 29A und 40 mg (0.10 mmol) *N*⁵-(*tert*-Butoxycarbonyl)-*N*-{3-[(*tert*-butoxycarbonyl)amino]-2-hydroxypropyl}-*L*-ornithin-amid (Beispiel 82A) in 1.7 ml Dimethylformamid gelöst. Bei 0°C (Eisbad) werden dann 19 mg (0.10 mmol) EDC und 3.1 mg (0.023 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und chromatographisch an Silicagel gereinigt (Laufmittel Dichlormethan/Isopropanol 30:1 bis 10:1).
Ausbeute: 47 mg (48% d. Th.)
LC-MS (Methode 17): Rₜ = 2.40 min.
MS (EI): m/z = 1043 (M+H)⁺

### Beispiel 167A

*tert*-Butyl-(2-{[(2*S*)-5-[(*tert*-butoxycarbonyl)amino]-2-({[(8*S*,11*S*,14*,S*)-14-[(*tert* butoxycarbonyl)-amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)pentanoyl]-amino}ethyl)carbamat 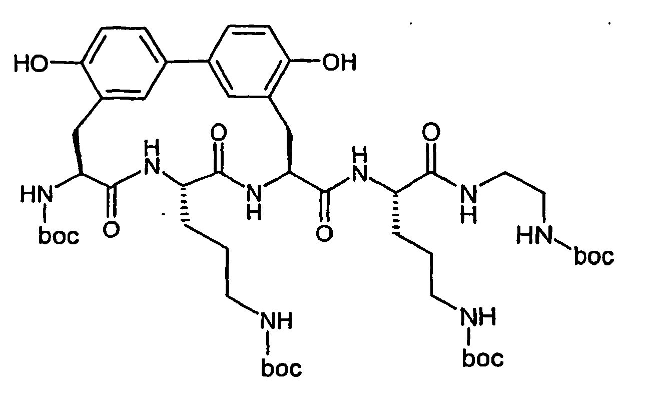 Unter Argon werden 50 mg (0.076 mmol) der Verbindung aus Beispiel 29A und 37 mg (0.10 mmol) *N*⁵-(*tert*-Butoxycarbonyl)-*N*-{2-[(*tert*-butoxycarbonyl)amino]ethyl}-*L*-ornithinamid (Beispiel 81 A) in 1.7 ml Dimethylformamid gelöst. Bei 0°C (Eisbad) werden dann 19 mg (0.10 mmol) EDC und 3.1 mg (0.023 mmol) HOBT zugegeben. Es wird langsam aufRT erwärmt und für 12 h bei RT gerührt. Die Lösung wird im Vakuum eingeengt und der Rückstand wird mit Wasser verrührt. Der verbleibende Feststoff wird abgesaugt und chromatographisch an Silicagel gereinigt (Laufmittel Dichlormethan/Isopropanol 30:1 bis 10:1).
Ausbeute: 43 mg (55% d. Th.)
LC-MS (Methode 12): Rₜ = 2.29 min.
MS (EI): m/z = 1013 (M+H)⁺

### Beispiel 168A

*tert*-Butyl-((3*S*)-3-{[(2*S*)-2-[(*tert*-butoxycarbonyl)amino]-5-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)pentanoyl]-amino}-4-hydroxybutyl)carbamat 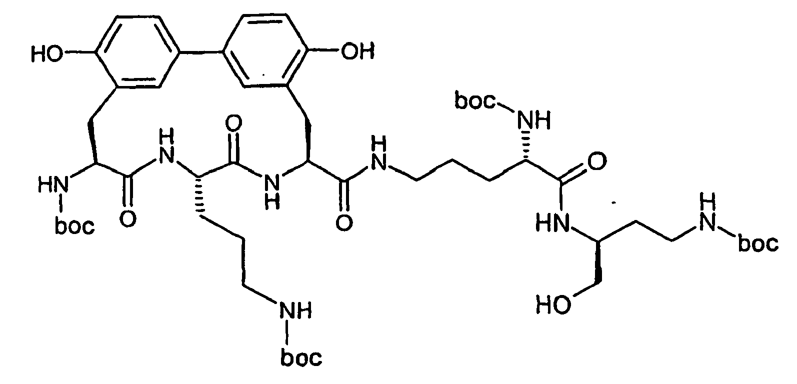 Unter Argon werden 50 mg (0.076 mmol) der Verbindung aus Beispiel 29A und 42 mg (0.10 mmol) *N*²-(*tert*-Butoxycarbonyl)-*N*-[(1*S*)-3-[(*tert*-butoxycarbonyl)amino]-1-(hydroxymethyl)-propyl]-*L*-ornithinamid (Beispiel 94A) in 1.7 ml Dimethylformamid gelöst. Bei 0°C (Eisbad) werden dann 19 mg (0.10 mmol) EDC und 3.1 mg (0.023 mmol) HOBT zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und chromatographisch an Silicagel gereinigt (Laufmittel Dichlormethan/Isopropanol 30:1 bis 10:1).
Ausbeute: 25 mg (31% d. Th.)
LC-MS (Methode 12): Rₜ = 2.18 min.
MS (EI): m/z = 1057 (M+H)⁺

### Beispiel 169A

(8*S*,11*S*,14*S*)-14-Amino-11-(3-aminopropyl)-9-ethyl-5,17-dihydroxy-10,13-dioxo-9,12-diazatricyclo[14.3.1.1^{2,6}]henicosa-1(20),2(21),3,5,16,18-hexaen-8-carbonsäure Dihydrochlorid 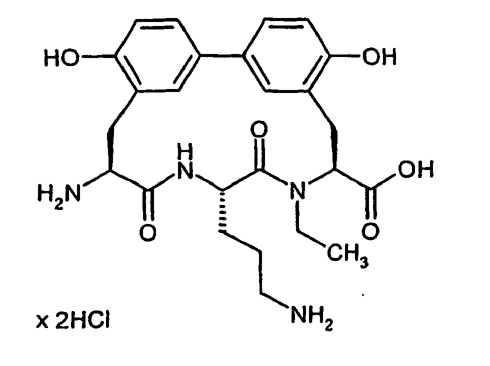 930 mg (0.91 mmol) der Verbindung aus Beispiel 78A werden in 260 ml Eisessig/Wasser/Ethanol (4/1/1) suspendiert, mit 270 mg Palladium auf Aktivkohle (10%ig) versetzt und 24 h bei Raumtemperatur unter Normaldruck hydriert. Nach Abfiltrieren des Katalysators über Kieselgur dampft man das Filtrat im Vakuum zur Trockne ein und versetzt unter Rühren mit 36.5 ml 0.1N Salzsäure. Man dampft im Vakuum zur Trockne ein und trocknet bis zur Gewichtskonstanz.
Ausbeute: 500 mg (98% d. Th.)
LC-MS (Methode 20): Rₜ = 2.45 min.
MS (ESI): m/z = 485 (M-2HCl+H)⁺

### Beispiel 170A

(8*S*,11*S*,14*S*)-14-[(*tert*-Butoxycarbonyl)amino]-11-{3-[(*tert*-butoxycarbonyl)amino]propyl}-9-ethyl-5,17-dihydroxy-10,13-dioxo-9,12-diazatricyclo[14.3.1.1^{2,6}]henicosa-1(20),2(21),3,5,16,18-hexaen-8-carbonsäure 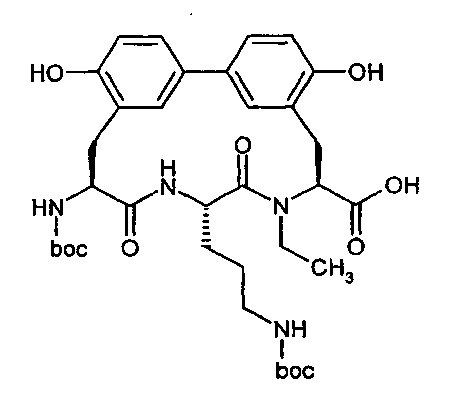 710 mg (1.27 mmol) der Verbindung aus Beispiel **169A** werden in 15 ml Wasser und 6.5 ml (6.5 mmol) 1N Natronlauge gelöst und bei Raumtemperatur unter Rühren mit 834 mg (3.82 mmol) Di-*tert*-butyl-dicarbonat, gelöst in 5.5 ml Methanol, versetzt. Nach einer Stunde ist die Reaktion beendet (Kontrolle mittels analytischer RP-HPLC, Laufmittel: Acetonitril/Wasser). Durch Zutropfen von 0.1N Salzsäure stellt man pH = 3 ein. Man extrahiert dreimal mit je 20 ml Essigsäureethylester, trocknet mit Natriumsulfat und dampft im Vakuum bis zur Gewichtskonstanz ein.
Ausbeute: 770 mg (88% d.Th.)
LC-MS (Methode 19): Rₜ = 2.16 min.
MS (ESI): m/z = 685 (M+H)⁺

### Beispiel 171A

Benzyl-(2-{[(2*S*,4*R*)-5-{[(benzyloxy)carbonyl]amino}-2-({[(8*S*,11*S*,14*S*)-14-[(*tert*-butoxycarbonyl)amino]-11-{(2*R*)-3-[(*tert*-butoxycarbonyl)amino]-2-hydroxypropyl}-5,17-dihydroxy-10,13-dioxo-9,12-diazatricyclo[14.3.1.1^{2,6}]henicosa-1(20),2(21),3,5,16,18-hexaen-8-yl]carbonyl}-amino)-4-hydroxypentanoyl]amino}ethyl)carbamat 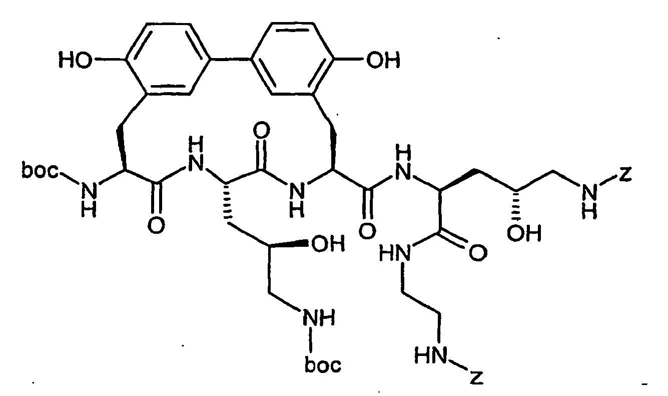 44 mg (0.07 mmol) (8*S*,11*S*,14*S*)-14-[(*tert*-Butoxycarbonyl)amino]-11-{(2*R*)-3-[(*tert*-butoxycarbonyl)amino]-2-hydroxypropyl}-5,17-dihydroxy-10,13-dioxo-9,12-diazatricyclo[14.3.1.1^{2,6}]-henicosa-1(20),2(21),3,5,16,18-hexaen-8-carbonsäure (Beispiel 65A) werden in 3 ml DMF gelöst und mit 38.9 mg (0.08 mmol) der Verbindung aus Beispiel 96A versetzt. Man kühlt auf 0°C und versetzt nacheinander mit 29.8 mg (0.08 mmol) HATU und 13 mg (0.1 mmol) Diisopropylethylamin. Nach 30 min bei 0°C läßt man auf Raumtemperatur erwärmen, gibt weitere 26 mg (0.2 mmol) Diisopropylethylamin hinzu und rührt über Nacht nach. Nach Einengen im Vakuum reinigt man per Chromatographie an Kieselgel (Dichlormethan/Methanol 9:1) und anschließend per präparativer HPLC.
Ausbeute: 14 mg (20% d. Th.).
LC-MS (Methode 19): Rₜ = 2.42 min.
MS (EI): m/z = 1114 (M+H)⁺.

### Beispiele 172A

*tert*-Butyl-{(2*R*)-3-[(8*S*,11*S*,14*S*)-8-{[((1*S*,3*R*)-4-amino-1-{[(2-aminoethyl)amino]carbonyl}-3-hydroxybutyl)amino]carbonyl}-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]-2-hydroxypropyl}-carbamat Dihydrochlorid 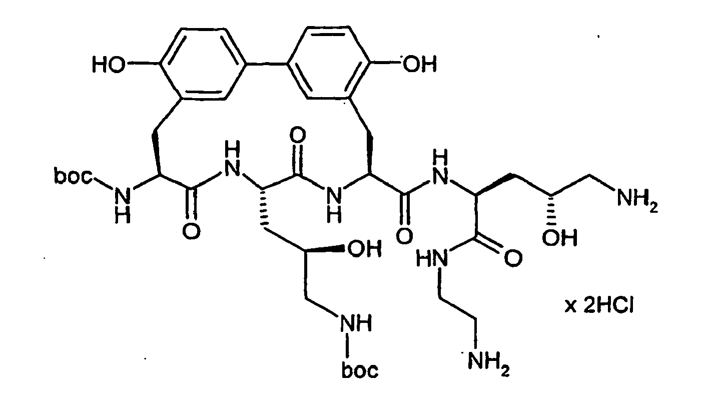 23 mg (0.02 mmol) der Verbindung aus Beispiel 171A werden in 20 ml eines Eisessig/ Methanol/Wasser-Gemisches (4/1/1) gelöst und mit 12 mg Palladium auf Aktivkohle (10%ig) versetzt. Man hydriert 4 h bei Normaldruck und filtriert dann den Katalysator ab. Die so erhaltene Mutterlauge wird im Vakuum zur Trockne eingeengt, mit 2 ml 0.1N Salzsäure verrührt und erneut bis zur Trockne eingeengt. Das so erhaltene Rohprodukt wird ohne weitere Reinigung umgesetzt.
Ausbeute: 16 mg (83% d. Th.).
LC-MS (Methode 12): Rₜ = 1.23 min.
MS (EI): m/z = 845 (M-2HCl+H)⁺.

### Beispiel 173A

*tert*-Butyl-(3-{[(2*S*)-4-[(*tert*-butoxycarbonyl)amino]-2-({[(8*S*,11*S*,14*S*)-14-[(*tert*-butoxycarbonyl)-amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)butanoyl]-amino}-2-hydroxypropyl)carbamat 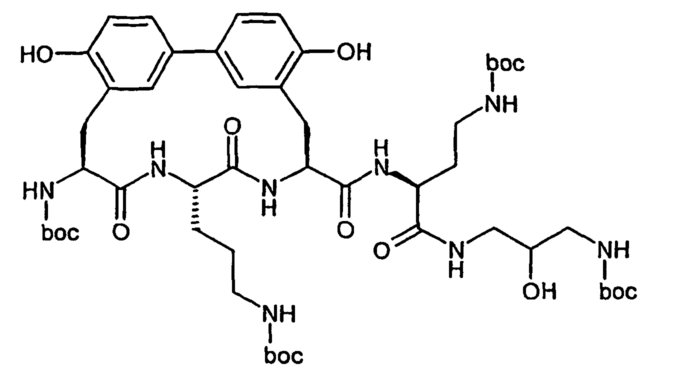 36 mg (0.06 mmol) (8*S*,11*S*,14*S*)-14-[(*tert*-Butoxycarbonyl)amino-11-[3-[(*tert*-butoxycarbonyl) amino]propyl}-5,17-dihydroxy-10,13-dioxo-9,12-diazatricyclo[14.3.1.1^{2,6}]-henicosa-1(20),2(21),3,5,16,18-hexaen-8-carbonsäure (Beispiel 29A) und 26 mg (0.07 mmol) *tert*-Butyl-[3-({(2*S*)-2-amino-4-[(*tert*-butoxycarbonyl)amino]butanoyl} amino)-2-hydroxypropyl]carbamat (Beispiel 99A) werden in 1 ml DMF gelöst, auf 0°C gekühlt, und nacheinander mit 18 mg (0.09 mmol) EDC und 2 mg (0.02 mmol) HOBt versetzt. Man läßt auf Raumtemperatur erwärmen und rührt über Nacht nach. Der Rückstand wird in Wasser ausgerührt, der dabei entstandene Niederschlag abfiltriert, getrocknet und per Chromatographie an Kieselgel (Dichlormethan/ Methanol 100:7) gereinigt.
Ausbeute: 9 mg (14% d. Th.).
LC-MS (Methode 12): Rₜ = 2.22 min.
MS (EI): m/z = 1029 (M+H)⁺.

### Beispiel 174A

Benzyl-(2-{[(2*S*,4*R*)-5-{[(benzyloxy)carbonyl]amino}-2-({[(8*S*,11*S*,14*S*)-14-[(*tert*-butoxycarbonyl)amino]-11-{3-[(*tert*-butoxycarbonyl)aminopropyl]}-5,17-dihydroxy-10,13-dioxo-9,12-diazatricyclo[14.3.1.1^{2,6}]henicosa-1(20),2(21),3,5,16,18-hexaen-8-yl]carbonyl}amino-4-hydroxypentanoyl]amino}ethyl)carbamat 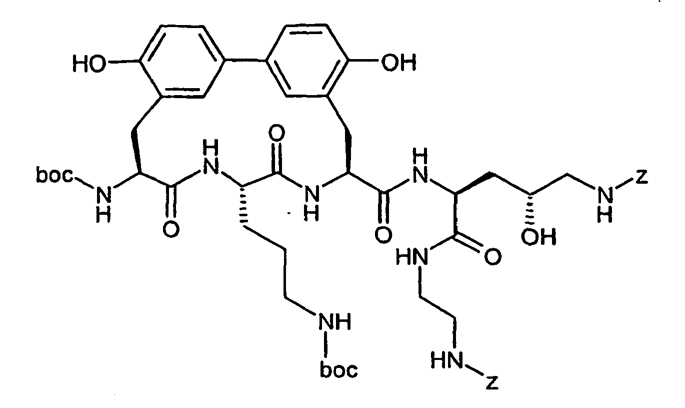 30 mg (0.05 mmol) (8*S*,11*S*,14*S*)-14-[(*tert*-Butoxycarbonyl)aminopropyl]-11-{3-[(*tert*-butoxycarbonyl)amino]}-5,17-dihydroxy-10,13-dioxo-9,12-diazatricyclo[14.3.1.1^{2,6}]henicosa-1(20),2(21),3,5,16,18-hexaen-8-carbonsäure (Beispiel 29A) werden in 1 ml DMF gelöst und mit 27 mg (0.05 mmol) der Verbindung aus Beispiel 96A versetzt. Man kühlt auf 0°C und versetzt nacheinander mit 21 mg (0.05 mmol) HATU und 10 mg (0.07 mmol) Diisopropylethylamin. Nach 30 min bei 0°C läßt man auf Raumtemperatur erwärmen, gibt weitere 0.20 mg (0.14 mmol) Düsopropylethylamin hinzu und rührt über Nacht nach. Nach Einengen im Vakuum reinigt man per Chromatographie an Kieselgel (Dichlormethan/Methanol/konzentrierte Ammoniak-Lösung 90:10:1). Nach Einengen der Fraktionen wird der Rückstand in Acetonitril ausgerührt, der Niederschlag abfiltriert und im Vakuum getrocknet.
Ausbeute: 16 mg (30% d. Th.).
LC-MS (Methode 17): Rₜ = 2.52 min.
MS (EI): m/z = 1197 (M+H)⁺.

### Beispiele 175A

*tert*-Butyl-{-3-[(8*S*,11*S*,14*S*)-8-{[((1*S*,3*R*)-4-amino-1-{[(2-aminoethyl)amino]carbonyl}-3-hydroxybutyl)amino]carbonyl}-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat 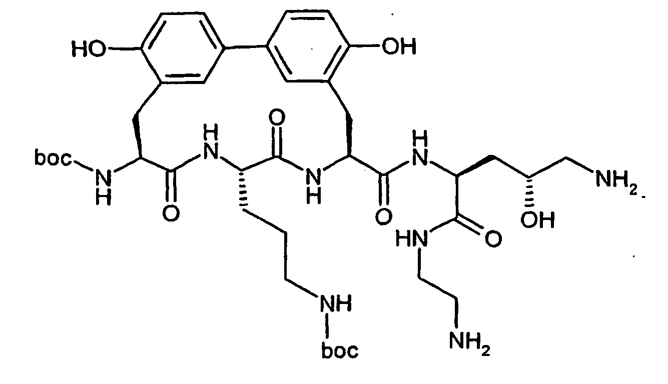 16 mg (0.01 mmol) der Verbindung aus Beispiel 174A werden in 10 ml Methanol gelöst und mit 8 mg Palladium auf Aktivkohle (10%ig) versetzt. Man hydriert 4 h bei Normaldruck und filtriert dann den Katalysator ab. Die so erhaltene Mutterlauge wird im Vakuum zur Trockne eingeengt. Das so erhaltene Rohprodukt wird ohne weitere Reinigung umgesetzt.
Ausbeute: 10 mg (74% d. Th.).
LC-MS (Methode 12): Rₜ = 1.18 min.
MS (EI): m/z = 829 (M+M)⁺.

### Beispiel 176A

Benzyl-(2-{[(3*S*)-3-{[(benzyloxy)carbonyl]amino}-6-({[(8*S*,11*S*,14*S*)-14-[(*tert*-butoxycarbonyl)-amino]-11-{3-[(*tert*-butoxycarbonyl)amino]propyl}-9-ethyl-5,17-dihydroxy-10,13-dioxo-9,12-diazatricyclo[14.3.1.1^{2,6}]henicosa-1(20),2(21),3,5,16,18-hexaen-8-yl]carbonyl}amino)hexanoyl]-amino}ethyl)carbamat 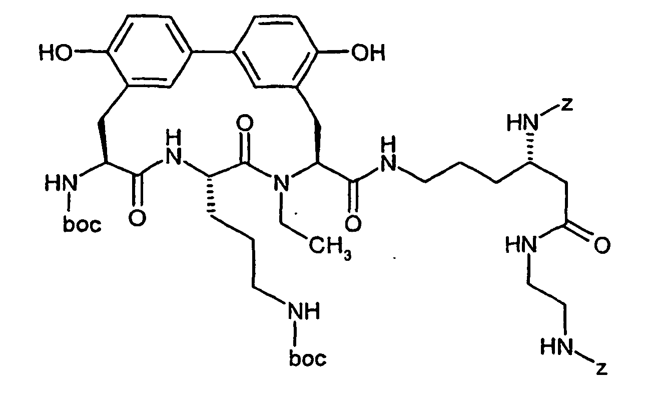 Unter Argon werden 30 mg (0.044 mmol) (8*S*,11*S*,14*S*)-14-[(*tert*-Butoxycarbonyl)amino]-11-{3-[(*tert*-butoxycarbonyl)amino]propyl}-9-ethyl-5,17-dihydroxy-10,13-dioxo-9,12-diazatricyclo-[14.3.1.1^{2,6}]henicosa-1(20),2(21),3,5,16,18-hexaen-8-carbonsäure (Beispiel 170A) und 80 mg (0.162 mmol) Benzyl-((1S)-4-amino-1-{2-[(2-{[(benzyloxy)carbonyl]amino}ethyl)amino]-2-oxo-ethyl}butyl)carbamat Hydrochlorid (Beispiel 105A) in 2 ml Dimethylformamid gelöst. Bei 0°C (Eisbad) werden dann 31.16 mg (0.162 mmol) EDC und 1.95 mg (0.014 mmol) HOBt zugegeben. Es wird langsam auf RT erwärmt und 3 Tage bei 50°C gerührt. Die Lösung wird im Vakuum eingeengt und der Rückstand durch präparative HPLC gereinigt.
Ausbeute: 14.5 mg (30% d.Th.)
LC-MS (Methode 17): Rₜ = 2.55 min.
MS (EI): m/z = 1123 (M+H)⁺.

### Beispiel 177A

*tert*-Butyl-[(4*S*)-5-({(2*S*)-2,5-bis[(*tert*-butoxycarbonyl)amino]pentyl}amino)-4-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)-5-oxopentyl]carbamat 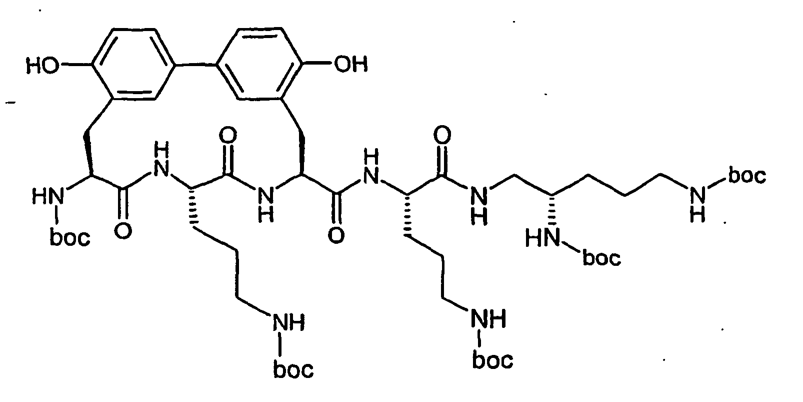 Unter Argon werden 50 mg (0.076 mmol) der Verbindung aus Beispiel **29A** und 53 mg (0.10 mmol) *tert*-Butyl-[(4*S*)-4-amino-5-({(2*S*)-2,5-bis[(*tert*-butoxycarbonyl)amino]pentyl}amino)-5-oxopentyl]carbamat (Beispiel 120A) in 1.7 ml Dimethylformamid gelöst. Bei 0°C (Eisbad) werden dann 19 mg (0.10 mmol) EDC und 3.1 mg (0.023 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und durch präparative HPLC (Kromasil, Laufmittel: Acetonitril/0.2% wässrige Trifluoressigsäure 5:95 → 95:5) gereinigt.
Ausbeute: 40 mg (45% d. Th.)
LC-MS (Methode 12): Rₜ = 2.46 min.
MS (EI): m/z = 1170 (M+H)⁺

### Beispiel 178A

*tert*-Butyl-[(1*S*)-4-[(*tert*-butoxycarbonyl)amino]-1-({[({[(8*S*,11*S,*14*S)-*14-[(*tert*-butoxycarbonyl)-amino]-11-{3-[(*tert*-butoxycarbonyl)amino)propyl]-5,17-dihydroxy-10,13-dioxo-9,12-diazatricyclo[14.3.1.1^{2,6}]henicosa-1(20),2(21),3,5,16,18-hexaen-8-yl]carbonyl}amino)acetyl]-amino}methyl)butyl]carbamat 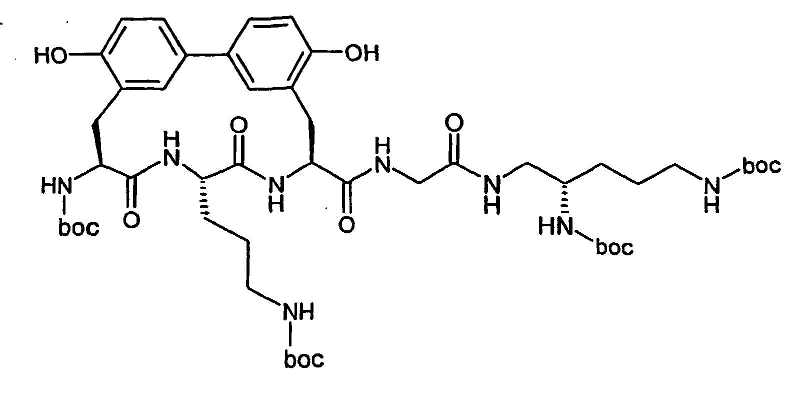 Unter Argon werden 43 mg (0.066 mmol) der Verbindung aus Beispiel 29A und 32 mg (0.085 mmol) *tert*-Butyl-{(4*S*)-5-[(aminoacetyl)amino]-4-[(*tert*-butoxycarbonyl)amino]pentyl}carbamat (Beispiel 126A) in 1.7 ml Dimethylformamid gelöst. Bei 0°C (Eisbad) werden dann 16 mg (0.085 mmol) EDC und 2.7 mg (0.02 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und durch präparative HPLC (Kromasil, Laufmittel: Acetonitril/0.2% wässrige Trifluoressigsäure 5:95 → 95:5) gereinigt.
Ausbeute: 11.5 mg (17% d. Th.)
LC-MS (Methode 19): Rₜ = 2.47 min.
MS (EI): m/z = 1013 (M+H)⁺

### Beispiel 179A

*tert-Butyl-*[(1*S*)-4-[(*tert*-butoxycarbonyl)amino]-1-({[(2*S*)-2-({[(8*S*,11*S,*14*S*)*-14-*[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)-3-hydroxy-propanoyl]amino}methyl)butyl]carbamat 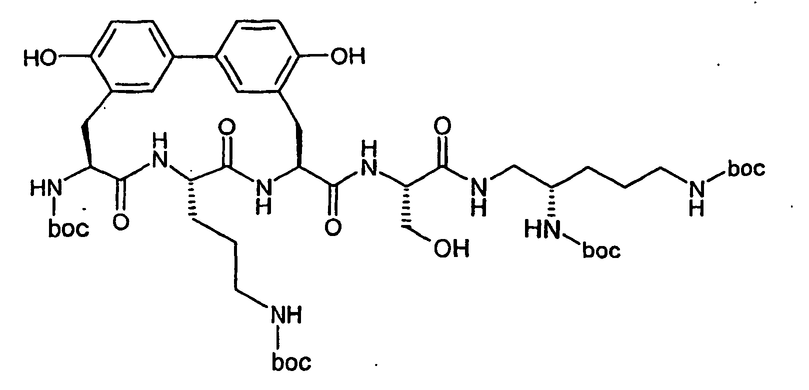 Unter Argon werden 28 mg (0.042 mmol) der Verbindung aus Beispiel 29A und 22 mg (0.055 mmol) *tert*-Butyl-{(4*S*)-5-{[(2*S*)-2-amino-3-hydroxypropanoyl]amino}-4-[(*tert* butoxycarbonyl)-amino]pentyl}carbamat (Beispiel 128A) in 1.7 ml Dimethylformamid gelöst. Bei 0°C (Eisbad) werden dann 11 mg (0.055 mmol) EDC und 1.7 mg (0.013 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und chromatographisch (Sephadex LH20, Laufmittel: Methanol / Essigsäure (0.25%)) gereinigt.
Ausbeute: 18.4 mg (42% d. Th.)
LC-MS (Methode 17): Rₜ = 2.43 min.
MS (EI): m/z = 1043 (M+H)⁺

### Beispiel 180A

*tert*-Butyl-{(1*S*)-1-({[(2*S*)-5-amino-2-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]-henicosa-1(20),2(21),3,5,16,18-hexaen-8-yl]carbonyl}amino)-5-oxopentanoyl]amino}methyl)-4-[(*tert*-butoxycarbonyl)amino]butyl}carbamat 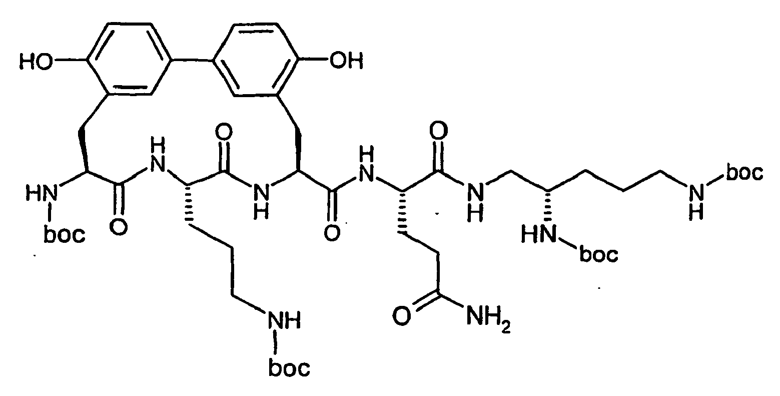 Unter Argon werden 27 mg (0.042 mmol) der Verbindung aus Beispiel 29A und 21 mg (0.047 mmol) *tert*-Butyl-[(1*S-*4-[(*tert*-butoxycarbonyl)amino]-1-({[(2*S*)-2,5-diamino-5-oxopentanoyl]-amino}methyl)butyl]carbamat (Beispiel 130A) in 2 ml Dimethylformamid gelöst. Bei 0°C (Eisbad) werden dann 10 mg (0.053 mmol) EDC und 1.7 mg (0.013 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und chromatographisch (Sephadex LH20, Laufmittel: Methanol / Essigsäure (0.25%)) gereinigt.
Ausbeute: 16 mg(36% d. Th.)
LC-MS (Methode 19): Rₜ = 2.38 min.
MS (EI): m/z = 1084 (M+H)⁺

### Beispiel 181A

*N*⁵-[(Benzyloxy)carbonyl]-*N*²-{[(8*S*,11*S*,14*S*)-14-[(*tert*-butoxycarbonyl)amino]-11-{3-[(*tert-*butoxycarbonyl)amino]propyl}-5,17-dihydroxy-10,13-dioxo-9,12-diazatricyclo[14.3.1.1^{2,6}]-henicosa-1 (20),2(21),3,5,16,18-hexaen-8-yl]carbonyl}-*L*-omithyl-*N*⁵-[(benzyloxy)carbonyl]-*N-*(2-{[(benzyloxy)carbonyl]amino}ethyl)-*L*-ornithinamid 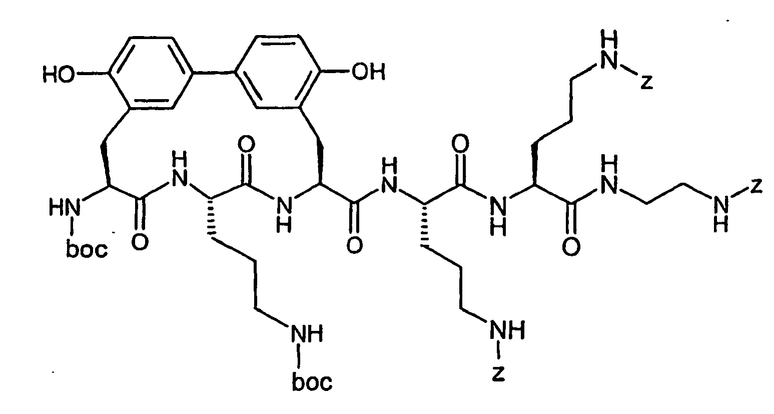 Unter Argon werden 40 mg (0.061 mmol) der Verbindung aus Beispiel 29A und 66 mg (0.091 mmol) *N*⁵-[(Benzyloxy)carbonyl]-*L*-ornithyl-*N*⁵-[(benzyloxy)carbonyl]-*N-*(2-{[(benzyloxy)-carbonyl]amino}ethyl)-*L*-ornithinamid (Beispiel 124A) in 2 ml Dimethylformamid gelöst. Bei 0°C (Eisbad) werden dann 15 mg (0.079 mmol) EDC und 2.5 mg (0.018 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und durch präparative HPLC (Laufmittel: Gradient Acetonitril / Wasser) gereinigt.
Ausbeute: 25 mg (26% d. Th.) .
LC-MS (Methode 17): Rₜ = 2.73 min.
MS (EI): m/z = 1330 (M+H)⁺

### Beispiel 182A

*tert*-Butyl-(2-{[2-({[(8*S*,11*S*,14*S*)-14-[(*tert*-butoxycarbonyl)amino]-11-{3-[(*tert*-butoxycarbonyl)-amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)ethyl]amino}-2-oxoethyl)carbamat 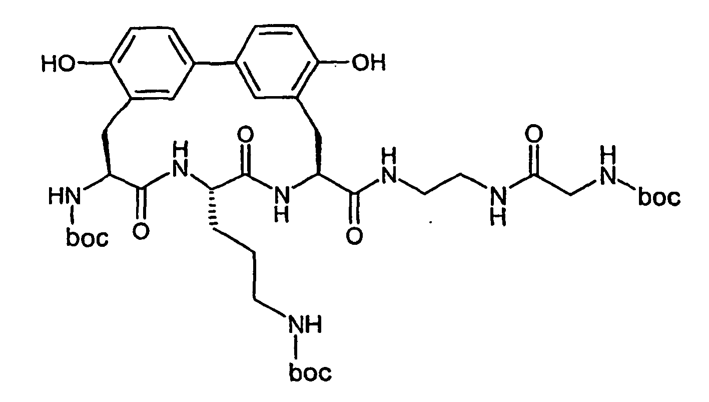 Unter Argon werden 34 mg (0.052 mmol) der Verbindung aus Beispiel 29A und 17 mg (0.078 **mmol)** *tert*-Butyl-{2-[(2-aminoethyl)amino]-2-oxoethyl}carbamat *(*Russ. J. Bioorg. Chem. 1994, 20, 397-405) in 2 ml Dimethylformamid gelöst. Bei 0°C (Eisbad) werden dann 13 mg (0.068 mmol) EDC und 2.1 mg (0.016 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und chromatographisch (Sephadex LH20, Laufmittel: Methanol/Essigsäure (0.25%)) gereinigt.
Ausbeute: 23 mg (50% d. Th.)
LC-MS (Methode 19): Rₜ = 2.16 min.
MS (EI): m/z = 856 (M+H)⁺

Analog zur Vorschrift des Beispiels 166A werden die in der folgenden Tabelle aufgeführten Beispiele **183A** bis 203A aus den entsprechenden Edukten hergestellt:

| **Beispiel- Nr.** | **Vorstufe Beispiel** | **Struktur** | **Analytische Daten** |
|---|---|---|---|
| **183A** | 29A und Methyl-glycinat | 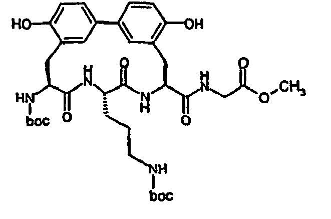 | LC-MS (Methode 12): Rₜ = 1.90 min. MS (EI): m/z = 728 (M+H)⁺ |
| **184A** | 29A und 143A | 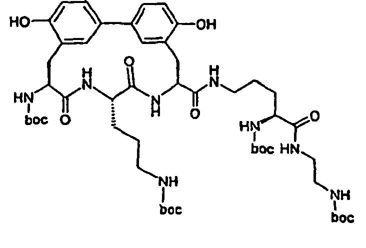 | LC-MS (Methode 19): Rₜ = 2.42 min. MS (EI): m/z = 1013 (M+H)⁺ |
| **185A** | 29A und 144A | 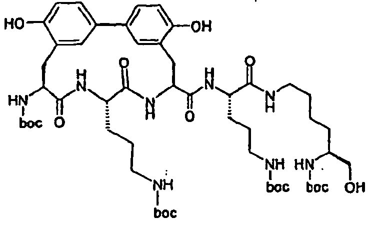 | LC-MS (Methode 17): Rₜ = 2.42 min. MS (EI): m/z = 1086 (M+H)⁺ |
| **186A** | 29A und 145A | 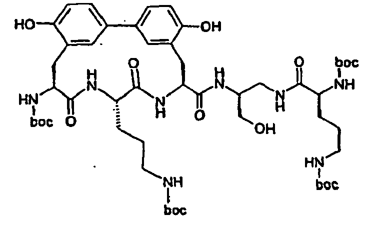 | LC-MS (Methode 17): Rₜ = 2.41 min. MS (EI): m/z = 1043 (M+H)⁺ |
| **187A** | 29A und 146A | 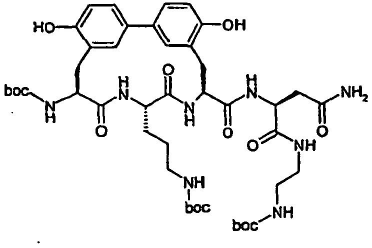 | LC-MS (Methode 12): Rₜ = 1.94 min. MS (EI): m/z = 913 (M+H)⁺ |
| **188A** | 29A und 101A | 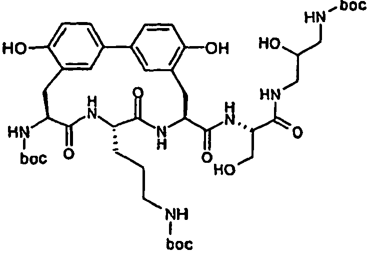 | LC-MS (Methode 12): Rₜ = 1.91 min MS (EI): m/z = 916 (M+H)⁺ |
| **189A** | 29A und 107A | 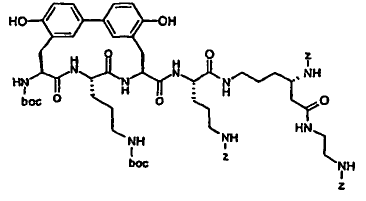 | LC-MS (Methode 17): Rₜ = 2.65 min. MS (EI): m/z =1344 (M+H)⁺ |
| **190A** | 29A und 105A | 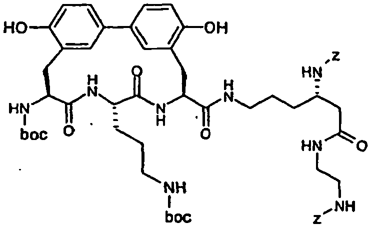 | LC-MS (Methode 12): Rₜ = 2.35 min. MS (EI): m/z = 1095 (M+H)⁺ |
| **191A** | 29A und 111A | 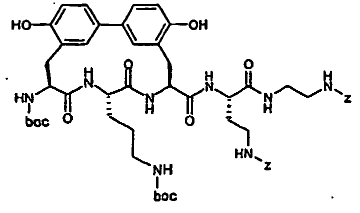 | LC-MS (Methode 19): Rₜ = 2.53 min. MS (EI): m/z = 1067 (M+H)⁺ |
| **192A** | 29A und 113A | 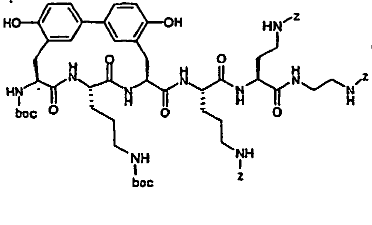 | LC-MS (Methode 19): Rₜ = 2.70 min. MS (EI): m/z = 1315 (M+H)⁺ |
| **193A** | 29A und 115A | 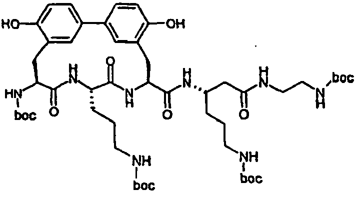 | LC-MS (Methode 19): Rₜ = 2.42 min. MS (EI): m/z = 1027 (M+H)⁺ |
| **194A** | 29A und 103A | 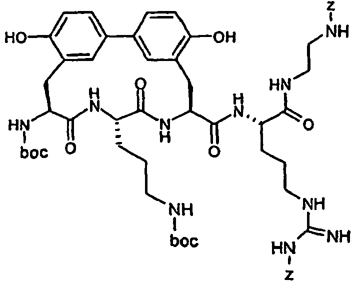 | LC-MS (Methode 19): Rₜ = 2.17 min. MS (EI): m/z = 1123 (M+H)⁺ |
| **195A** | 29A und 155A | 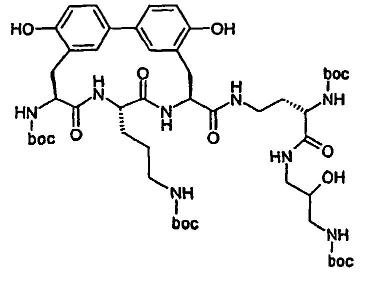 | LC-MS (Methode 12): Rₜ = 2.15 min. MS (EI): m/z = 1029 (M+H)⁺ |
| **196A** | 29A und 157A | 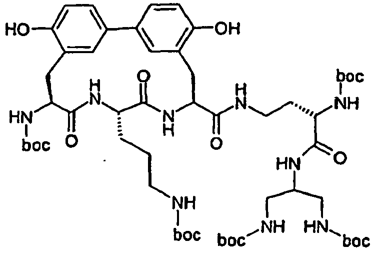 | LC-MS (Methode 19): Rₜ = 2.63 min. MS (EI): m/z = 1128 (M+H)⁺ |
| **197A** | 29A und 149A | 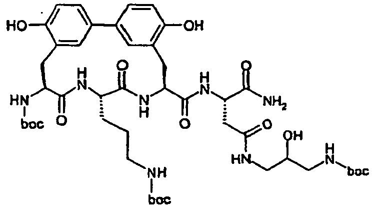 | LC-MS (Methode 19): Rₜ = 2.07 min. MS (EI): m/z = 943 (M+H)⁺ |
| **198A** | 29A und 148A | 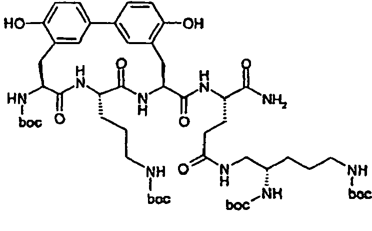 | LC-MS (Methode 12): Rₜ = 2.17 min. MS (EI): m/z = 1084 (M+H)⁺ |
| **199A** | 29A und 147A | 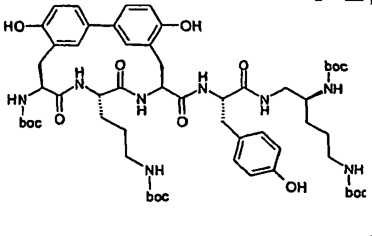 | LC-MS (Methode 19): Rₜ = 2.54 min. MS (EI): m/z = 1119 (M+H)⁺ |
| **200A** | 29A und 150A | 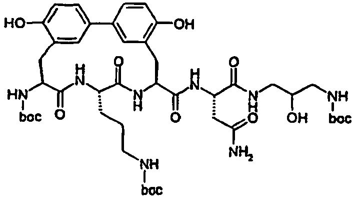 | LC-MS (Methode 17): Rₜ = 2.11 min. MS (EI): m/z = 943 (M+H)⁺ |
| **201A** | 29A und 151A | 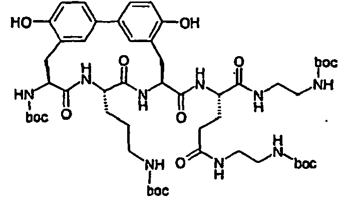 | LC-MS (Methode 17): Rₜ = 2.39 min. MS (EI): m/z = 1070 (M+H)⁺ |
| **202A** | 29A und 152A | 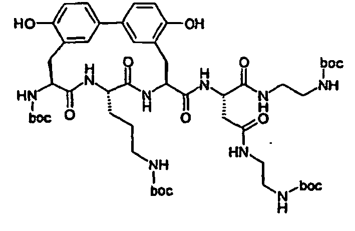 | LC-MS (Methode 17): Rₜ = 2.44 min. MS (EI): m/z = 1056 (M+H)⁺ |
| **203A** | 29A und 153A | 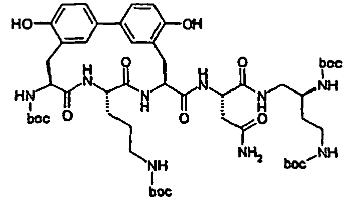 | LC-MS (Methode 17): Rₜ = 2.40 min. MS (EI): m/z = 1056 (M+H)⁺ |

### Beispiel 204A

*tert*-Butyl-{3-[(8*S*,11*S*,14*S*)-8-[({(4*S-*4-amino-6-[(2-aminoethyl)amino]-6-oxohexyl}amino) carbonyl]-14-[(*tert*-butoxycarbonyl)amino]-9-ethyl-5,17-dihydroxy-10,13-dioxo-9,12-diazatricyclo[14.3.1.1^{2,6}]henicosa-1(20),2(21),3,5,16,18-hexaen-11yl]propyl}carbamat 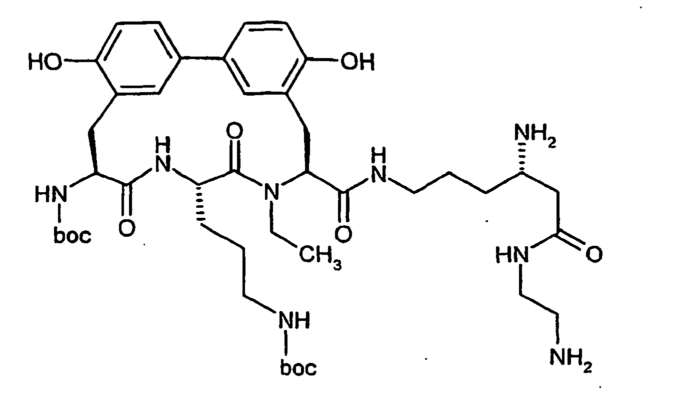 Es werden 14.5 mg (0.013 mmol) von Benzyl-(2-{[(3*S*)-3-{[(benzyloxy)carbonyl]amino}-6-({[(8*S*,11*S*,14*S*)-14-[(*tert*-butoxycarbonyl)amino]-11-{3-[(*tert*-butoxycarbonyl)amino]propyl}-9-ethyl-5,17-dihydroxy-10,13-dioxo-9,12-diazatricyclo[14.3.1.1^{2,6}]henicosa-1(20),2(21),3,5,16,18-hexaen-8-yl]carbonyl}amino)hexanoyl]amino}ethyl)carbamat (Beispiel 176A) in 3 ml Ethanol gelöst. Dazu gibt man 15 mg Palladium auf Aktivkohle (10%ig) und hydriert anschließend 15 h bei Normaldruck. Das Reaktionsgemisch wird über vorgewaschenem Kieselgur filtriert und das Filtrat im Vakuum einrotiert. Das Rohprodukt wird ohne weitere Reinigung umgesetzt.
Ausbeute: 8 mg (73% d. Th.).
LC-MS (Methode 19): Rₜ = 1.59 min.
MS (EI): m/z= 855 (M+H)⁺.

### Beispiel 205A

*tert*-Butyl-{3-[(8*S*,11*S*,14*S*)-8-[({(1*S*)-4-amino-1-[({(4*S*)-4-amino-6-[(2-aminoethyl)amino]-6-oxohexyl}amino)carbonyl]butyl}amino)carbonyl]-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Tris(hydroacetat) 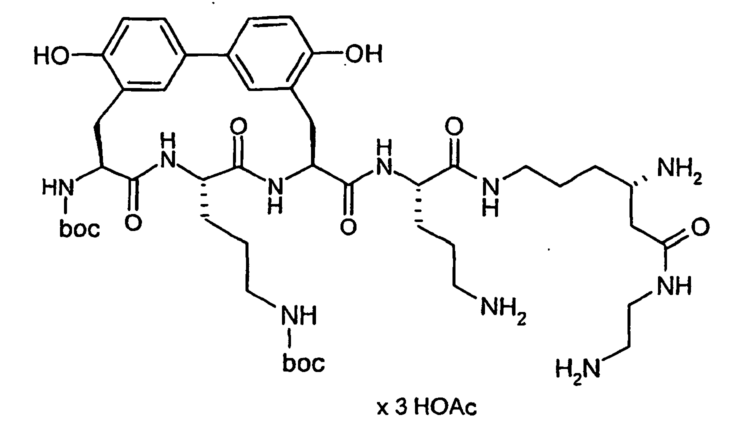 Es werden 9 mg (0.007 mmol) Benzyl-((1*S*)-4-{[(2*S*)-5-{[(benzyloxy)carbonyl]amino}-2-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)pentanoyl]amino}-1-{2-[(2-{[(benzyloxy)carbonyl]amino}ethyl)amino]-2-oxoethyl)butyl)carbamat (Beispiel 189A) in ein Gemisch aus 8 ml Essigsäure/Wasser/Ethanol 4:1:1 gegeben. Dazu gibt man 1 mg Palladium auf Aktivkohle (10%ig) und hydriert anschließend 15 h bei RT und Normaldruck. Das Reaktionsgemisch wird über vorgewaschenem Kieselgur filtriert, mit Ethanol gewaschen und das Filtrat im Vakuum einrotiert.
Ausbeute: quant.
LC-MS (Methode 17): Rₜ = 1.33 min.
MS (EI): m/z = 941 (M-3HOAc+H)⁺

### Beispiel 206A

*tert*-Butyl-{3-[(8*S*,11*S*,14*S*)-8-[({(4*S*)-4-amino-6-[(2-aminoethyl)amino]-6-oxohexyl}amino)-carbonyl]-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Bis(hydroacetat) 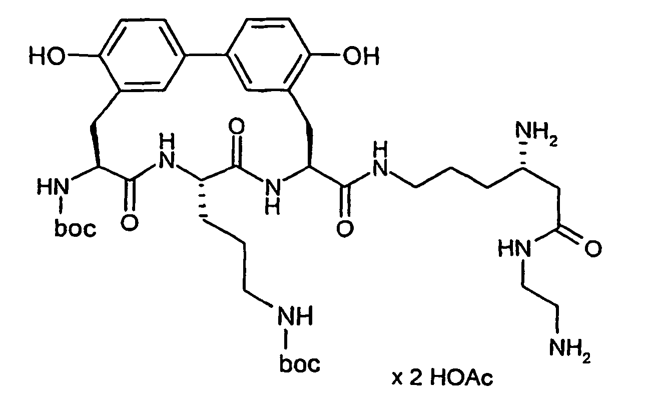 Es werden 12 mg (0.011 mmol) Benzyl-(2-{[(3*S*)-3-{[(benzyloxy)carbonyl]amino}-6-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)hexanoyl]amino)ethyl)carbamat (Beispiel 190A) in ein Gemisch aus 3 ml Essigsäure/Wasser/Ethanol 4:1:1 gegeben. Dazu gibt man 1.5 mg Palladium auf Aktivkohle (10%ig) und hydriert anschließend 15 h bei RT und Normaldruck. Das Reaktionsgemisch wird über vorgewaschenem Kieselgur filtriert, mit Ethanol gewaschen und das Filtrat im Vakuum einrotiert.
Ausbeute: quant.
LC-MS (Methode 17): Rₜ = 1.48 min.
MS (EI): m/z = 827 (M-2HOAc+H⁺

### Beispiel 207A

*tert*-Butyl-{3-[(8*S*,11*S*,14*S*)-8-([((1*S*)-3-amino-1-{[(2-aminoethyl)amino]carbonyl}propyl)amino]-carbonyl}-14-[(*tert*-butoxycarbonyl)amino]-5,17-dihydroxy-10,13-dioxo-9,12-diazatricyclo-[14.3.1.1^{2,6}]henicosa-1(20),2(21),3,5,16,18-hexaen-11-yl]propyl}carbamat Bis(hydroacetat) 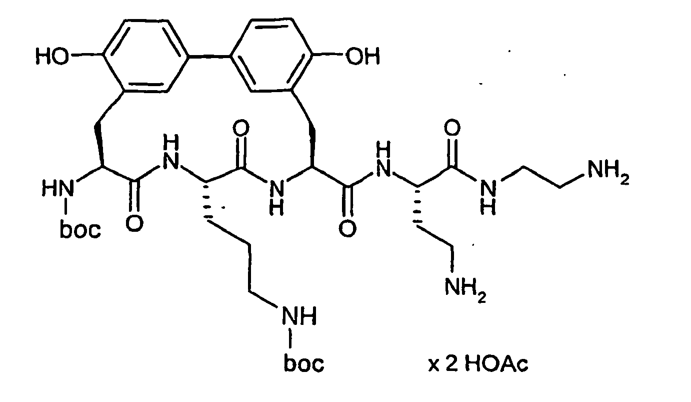 Es werden 29 mg (0.027 mmol) Benzyl-(2-{[(2*S*)-4-{[(benzyloxy)carbonyl]amino}-2-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino) butanoyl]amino}ethyl)carbamat (Beispiel 191A) in ein Gemisch aus 8 ml Essigsäure/Wasser/Ethanol 4:1:1 gegeben. Dazu gibt man 2.5 mg Palladium auf Aktivkohle (10%ig) und hydriert anschließend 15 h bei RT und Normaldruck. Das Reaktionsgemisch wird über vorgewaschenem Kieselgur filtriert, mit Ethanol gewaschen und das Filtrat im Vakuum einrotiert.
Ausbeute: 16 mg (74% d. Th.)
LC-MS (Methode 19): Rₜ = 1.48 min.
MS (EI): m/z = 799 (M-2HOAc+H)⁺

### Beispiel 208A

*tert*-Butyl-{3-[(8*S*,11*S*,14*S*)-8-{[((1*S*)-4-amino-1-{[((1*S*)-3-amino-1-{[(2-aminoethyl)amino]-carbonyl}propyl)amino]carbonyl}butyl)amino]carbonyl}-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Tris(hydroacetat) 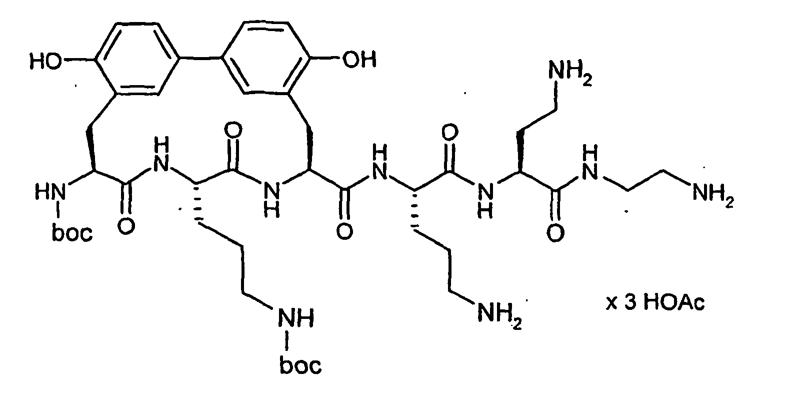 Es werden 12 mg (0.009 mmol) Benzyl-{(3*S*)-3-{[(2*S*)-5-{[(benzyloxy)carbonyl]amino}-2-({([(8*S*,11*S*,14*S-*14-[(*tert*-butoxycarbonyl)amino]-11-(3-[(*tert*-butoxycarbonyl)amino]propyl)-5,17-dihydroxy-10,13-dioxo-9,12-diazatricyclo[14.3.1.1^{2,6}]henicosa-1(20),2(21),3,5,16,18-hexaen-8-yl]carbonyl}amino)pentanoyl]amino}-4-[(2-{[(benzyloxy)carbonyl}amino}ethyl)amino]-4-oxobutyl}carbamat (Beispiel 192A) in ein Gemisch aus 8 ml Essigsäure/Wasser/Ethanol 4:1:1 gegeben. Dazu gibt man 1 mg Palladium auf Aktivkohle (10%ig) und hydriert anschließend 15 h bei RT und Normaldruck. Das Reaktionsgemisch wird über vorgewaschenem Kieselgur filtriert, mit Ethanol gewaschen und das Filtrat im Vakuum einrotiert.
Ausbeute: 7 mg (84% d. Th)
LC-MS (Methode 17): Rₜ = 1.31 min.
MS (EI): m/z = 913 (M-3HOAc+H)⁺

### Beispiel 209A

*tert*-Butyl-{3-[(8*S*,11*S*,14*S-*8-{[((1*S*)-1-{[(2-aminoethyl)amino]carbonyl}-4-{[amino(imino)-methyl]amino}butyl)amino]carbonyl}-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Di(hydrotrifluoracetat) 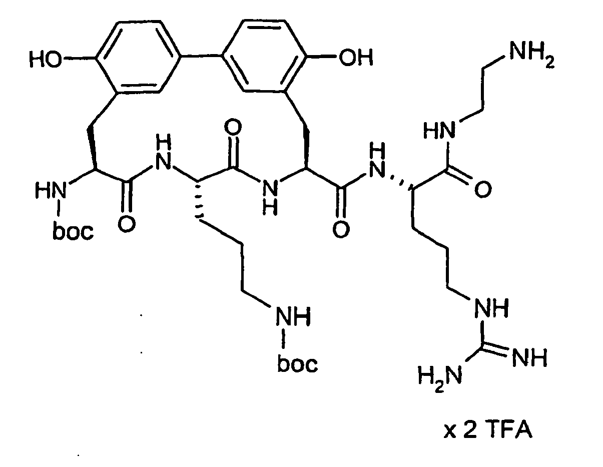 Es werden 13.4 mg (0.012 mmol) Benzyl-[(6*S*)-6-({[(8*S*,11*S*,14*S*)14-[(*tert*-butoxycarbonyl)-amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)-7,12-dioxo-14-phenyl-13-oxa-2,8,11-triazatetradecan-1-imidoyl]carbamat (Beispiel 194A) in ein Gemisch aus 5 ml Essigsäure/Wasser/Ethanol 4:1:1 gegeben. Dazu gibt man 1 mg Palladium auf Aktivkohle (10%ig) und hydriert anschließend 15 h bei RT und Normaldruck. Das Reaktionsgemisch wird über vorgewaschenem Kieselgur filtriert, mit Ethanol gewaschen und das Filtrat im Vakuum einrotiert. Das Zielprodukt wird durch präparative HPLC (Kromasil, Laufmittel Acetonitril/0.2% wässrige Trifluoressigsäure 5:95→95:5) gereinigt und in das Di(hydrotrifluoracetat) überführt.
Ausbeute: 5.3 mg (41% d. Th)
LC-MS (Methode 12): Rₜ = 1.19 min.
MS (EI): m/z = 855 (M-2TFA+H)⁺

### Beispiel 210A

*N*²-{[(8*S*,11*S*,14*S*)-14-[(*tert*-Butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}-*L*-ornithyl-*N-*(2-aminoethyl)-*L*-ornithinamidTris(hydroacetat) 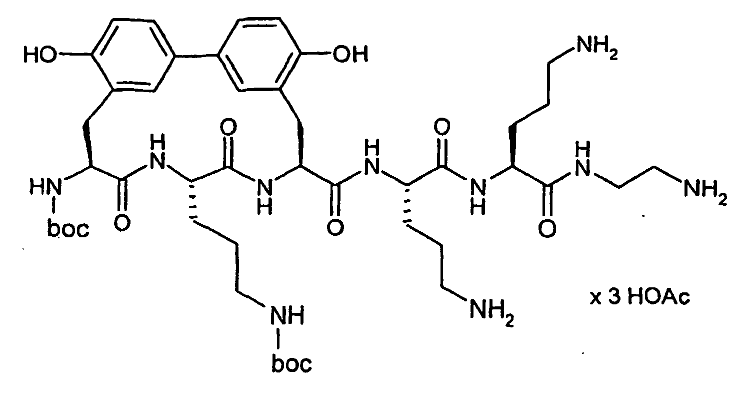 Es werden 25 mg (0.02 mmol) der Verbindung von Beispiel 181A in ein Gemisch aus 8 ml Essigsäure/Wasser/Ethanol 4:1:1 gegeben. Dazu gibt man 3 mg Palladium auf Aktivkohle (10%ig) und hydriert anschließend 48 h bei RT und Normaldruck. Das Reaktionsgemisch wird über vorgewaschenem Kieselgur filtriert, mit Ethanol gewaschen und das Filtrat im Vakuum zur Trockne eingedampft.
Ausbeute: 8.5 mg (41% d. Th)
LC-MS (Methode 20): Rₜ = 2.63 min.
MS (EI): m/z = 927 (M-3HOAc+H)⁺

Analog zur Vorschrift des Beispiels 166A werden die in der folgenden Tabelle aufgeführten Beispiele 211A bis 215A aus den entsprechenden Edukten hergestellt.

| **Beispiel- Nr.** | **Vorstufe Beispiel** | **Struktur** | **Analytische Daten** |
|---|---|---|---|
| **211A** | 29A und 159A | 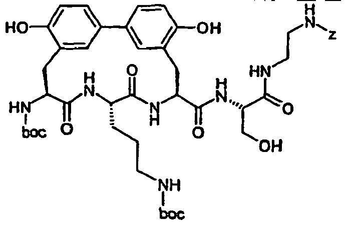 | LC-MS (Methode 17): Rₜ = 2.21 min. MS (EI): m/z = 920 (M+H)⁺ |
| **212A** | 29A und 109A | 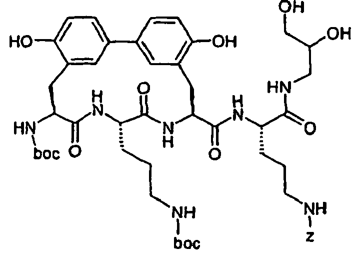 | LC-MS (Methode 17): Rₜ = 2.17 min. MS (EI): m/z = 978 (M+H)⁺ |
| **213A** | 29A und 165A | 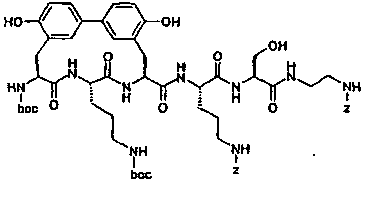 | LC-MS (Methode 19): Rₜ = 2.42 min. MS (EI): m/z =1168 (M+H)⁺ |
| **214A** | 29A und 161A | 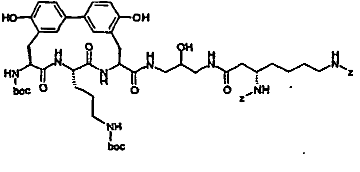 | LC-MS (Methode 19): Rₜ = 2.48 min. MS (EI): m/z = 1139 (M+H)⁺ |
| **215A** | 29A und 163A | 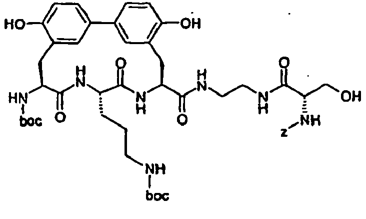 | LC-MS (Methode 12): Rₜ = 1.98 min. MS (EI): m/z = 920 (M+H)⁺ |

Analog zur Vorschrift des Beispiels 205A werden die in der folgenden Tabelle aufgeführten Beispiele 216A bis 220A aus den entsprechenden Edukten hergestellt.

| **Beispiel- Nr.** | **Vorstufe Beispiel** | **Struktur** | **Analytische Daten** |
|---|---|---|---|
| **216A** | 211A | 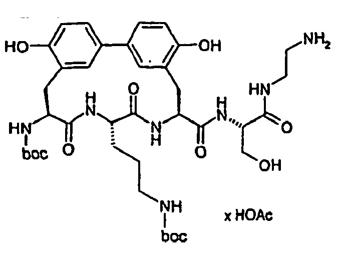 | LC-MS (Methode 19): Rₜ = 1.69 min. MS (EI): m/z = 786 (M-HOAc+H)⁺ |
| **217A** | 212A | 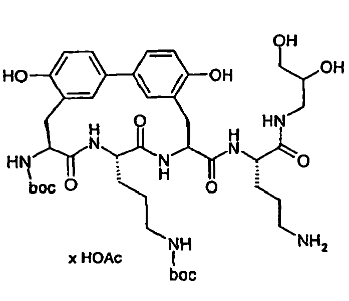 | LC-MS (Methode 17): Rₜ = 1.62 min. MS (EI): m/z = 844 (M-HOAc+H)⁺ |
| **218A** | 213A | 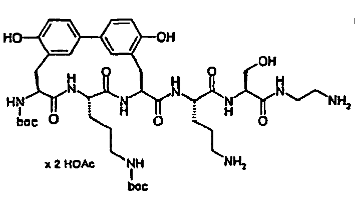 | LC-MS (Methode 19): Rₜ = 1.48 min. MS (EI): m/z = 899 (M-2HOAc+H)⁺ |
| **219A** | 214A | 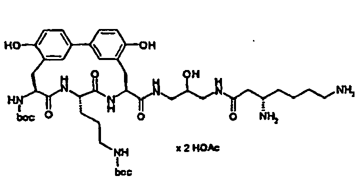 | LC-MS (Methode 19): Rₜ = 1.30 min. MS (EI): m/z = 869 (M-2HOAc-H-)⁻ |
| 220A | 215A | 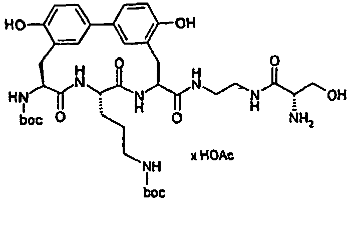 | LC-MS (Methode 17): Rₜ = 1.69 min. MS (EI): m/z = 786 (M-HOAc+H)⁺ |

### Beispiel 221A

*tert*-Butyl-{3-[(8*S*,11*S*,14*S*)-8-[({2-[(2-aminoethyl)amino]-2-oxoethyl}amino)carbonyl)-14-[(*tert-*butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Hydrotrifluoracetat 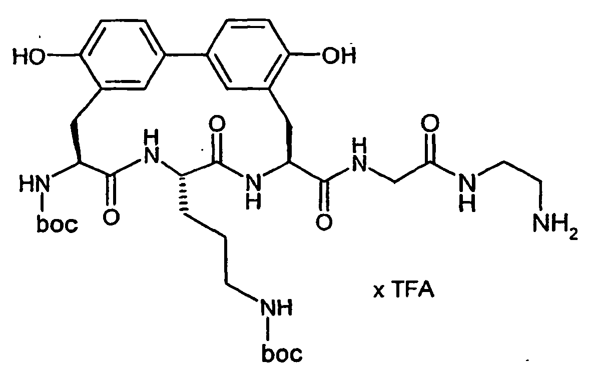 45 mg (0.057 mmol) der Verbindung aus Beispiel 183A werden in 1 ml Ethan-1,2-diamin gelöst und nach Zugabe von 0.75 mg Kaliumcyanid 12 h bei RT gerührt. Anschliessend wird mit 15 ml Wasser verdünnt und einmal mit Essigsäureethylester extrahiert. Die organische Phase wird mit Wasser gewaschen, die vereinten wässrigen Phasen werden im Vakuum zur Trochne eingedampft. Der Rückstand wird mit RP-HPLC gereinigt. (Kromasil 100C18, Laufinittel Acetonitril / Trifluoressigsäure (0.2%) 5:95 bis 95:5).
Ausbeute: 6.4 mg (15% d. Th.)
LC-MS (Methode 19): Rₜ = 1.69 min.
MS (EI): m/z = 756 (M-TFA+H)⁺

### Beispiel 222A

Benzyl-*tert*-butyl-[(2*S*)-3-({(2*S*)-2,5-bis[(*tert*-butoxycarbonyl)amino]pentyl}amino)-3-oxopropan-1,2-diyl]biscarbamat 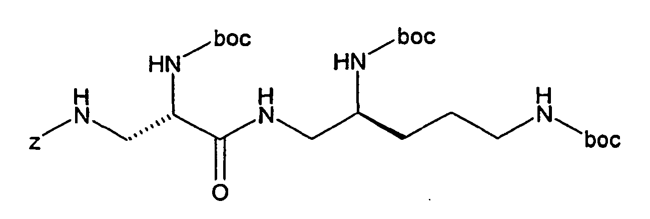 Unter Argon werden 0.208 g (0.40 mmol) 3-{[(Benzyloxy)carbonyl]amino}-*N-*(*tert*-butoxycarbonyl)-*L*-alanin - *N-*Cyclohexylcyclohexanamin (1:1), 126 mg (0.40 mmol) *tert*-Butyl-{(4*S*)-5-amino-4-[(*tert*-butoxycarbonyl)amino]pentyl}carbamat (Beispiel 86A) und 0.21 ml Triethylamin (1.48 mmol) in 5 ml Dimethylformamid gelöst. Bei 0°C (Eisbad) werden dann 130 mg (0.68 mmol) EDC und 18 mg (0.13 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Das Rohprodukt wird durch präparative HPLC (Kromasil, Laufmittel Acetonitril/ 0.25% wässrige Trifluoressigsäure 5:95 → 95:5) aufgereinigt.
Ausbeute: 88 mg (35% d. Th.)
LC-MS (Methode 19): Rₜ = 2.65 min.
MS (EI): m/z = 638 (M+H)⁺

### Beispiel 223A

*tert-Butyl-*{(4*S*)-5-({(2*S*)-3-amino-2-(*tert*-butoxycarbonyl)amino]propanoyl}amino)-4-[(*tert-*butoxycarbonyl)amino]pentyl}carbamat Hydroacetat 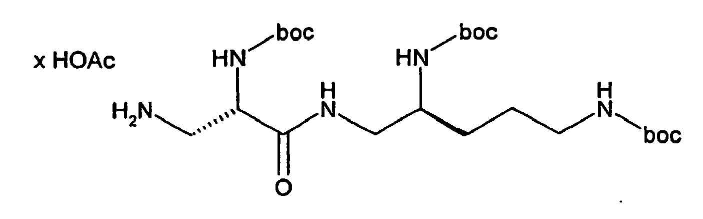 Es werden 110 mg (0.172 mmol) Benzyl-*tert*-butyl-[(2*S*)-3-({(2*S*)-2,5-bis[(*tert*-butoxycarbonyl)-amino]pentyl}amino)-3-oxopropan-1,2-diyl]biscarbamat (Beispiel 222A) in 25 ml einer Mischung-Eisessig/Wasser (4:1) gelöst. Dazu gibt man 20 mg Palladium auf Aktivkohle (10%ig) und hydriert anschließend 15 h bei Normaldruck. Das Reaktionsgemisch wird über vorgewaschenem Kieselgur filtriert und das Filtrat im Vakuum einrotiert. Das Rohprodukt wird ohne weitere Reinigung umgesetzt.
Ausbeute: 85 mg (93% d. Th.)
LC-MS (Methode 17): Rₜ = 1.67 min.
MS (EI): m/z = 503 (M-HOAc+H)⁺

### Beispiel 224A

Benzyl[(5*S*)-5-[(*tert*-butoxycarbonyl)amino]-7-({2-[(*tert*-butoxycarbonyl)amino)ethyl}amino)-7-oxoheptyl]carbamat 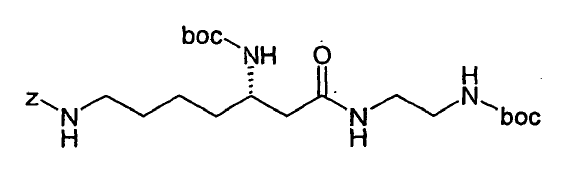 Unter Argon werden 1 g (2.54 mmol) (3*S*)-7-{[(Benzyloxy)carbonyl]amino}-3-[(*tert*-butoxycarbonyl)amino]heptansäure, 406 mg (2.54 mmol) *tert*-Butyl-(2-aminoethyl)carbamat und 0.96 ml Triethylamin (6.85 mmol) in 20 ml Dimethylformamid gelöst. Bei 0°C (Eisbad) werden dann 826 mg (4.3 mmol) EDC und 113 mg (0.84 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum getrocknet.
Ausbeute: quant.
LC-MS (Methode 12): Rₜ = 2.21 min.
MS (EI): m/z = 537 (M+H)⁺

### Beispiel 225A

*tert*-Butyl-((1*S*)-5-amino-1-{2-[(2-{[(benzyloxy)carbonyl]amino}ethyl)amino)-2-oxoethyl}pentyl)-carbamat Hydroacetat 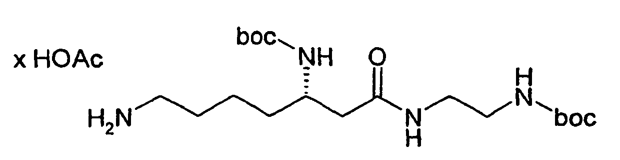 Es werden 1.3 g (2.42 mmol) Benzyl-[(5*S*)-5-[(*tert*-butoxycarbonyl)amino]-7-({2-[(*tert*-butoxycarbonyl)amino]ethyl}amino)-7-oxoheptyl]carbamat (Beispiel 224A) in 100 ml einer Mischung Eisessig/Wasser (4:1) gelöst. Dazu gibt man 70 mg Palladium auf Aktivkohle (10%ig) und hydriert anschließend 15 h bei Normaldruck. Das Reaktionsgemisch wird über vorgewaschenem Kieselgur filtriert und das Filtrat im Vakuum einrotiert. Das Rohprodukt wird ohne weitere Reinigung umgesetzt.
Ausbeute: quant.
LC-MS (Methode 19): Rₜ = 1.35 min.
MS (EI): m/z = 403 (M-HOAc+H)⁺

### Beispiel 226A

Benzyl-((1*S*,8*S*)-8-[(*tert*-butoxycarbonyl)amino]-1-{3-[(*tert*-butoxycarbonyl)amino]propyl}-17,17-dimethyl-2,10,15-trioxo-16-oxa-3,11,14-triazaoctadec-1-yl)carbamat 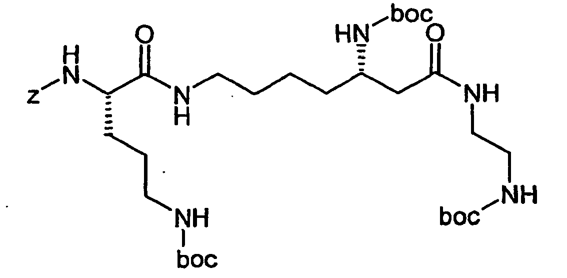 Unter Argon werden 0.397 g (1.08 mmol) *N*²-[(Benzyloxy)carbonyl]-*N*⁵-(*tert*-butoxycarbonyl)-*L-*ornithin, 0.500 g (1.08 mmol) *tert*-Butyl-((1*S*)-5-amino-1-{2-[(2-{[(benzyloxy)carbonyl]amino}-ethyl)amino]-2-oxoethyl}pentyl)carbamat (Beispiel 225A) und 0.56 ml Triethylamin (4.0 mmol) in 10 ml Dimethylformamid gelöst. Bei 0°C (Eisbad) werden dann 352 mg (1.84 mmol) EDC und 48.2 mg (0.36 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat-und Natriumchlorid-Lösung gewaschen, über Magnesiumsulfat getrocknet und im Vakuum eingedampft. Der verbleibende Feststoff wird per präparativer HPLC (Kromasil, Laufmittel Acetonitril/0.25% wässrige Trifluoressigsäure 5:95 → 95:5) gereinigt.
Ausbeute: 290 mg (36% d. Th.)
LC-MS (Methode 19): Rₜ = 2.30 min.
MS (EI): m/z = 751 (M+H)⁺

### Beispiel 227A

*tert*-Butyl-{(4*S*,11*S*)-4-amino-11-[(*tert*-butoxycarbonyl)amino]-20,20-dimethyl-5,13,18-trioxo-19-oxa-6,14,17-triazahenicos-1-yl} carbamat Hydroacetat 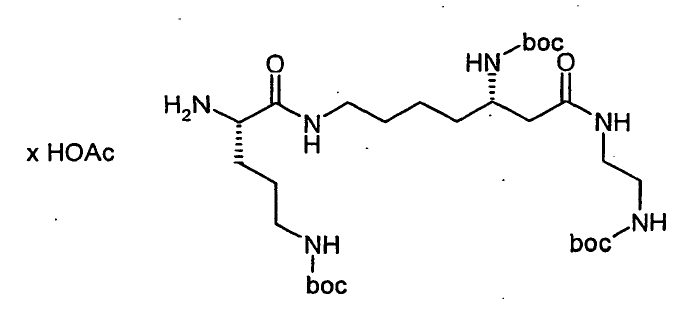 Es werden 290 mg (0.390 mmol) Benzyl-((1*S*,8*S*)-8-[(*tert*-butoxycarbonyl)amino]-1-{3-[(*tert-*butoxycarbonyl)amino]propyl}-17,17-dimethyl-2,10,15-trioxo-16-oxa-3,11,14-triazaoctadec-1-yl)-carbamat (Beispiel 226A) in 10 ml einer Mischung Eisessig/Wasser 4/1 gelöst. Dazu gibt man 75 mg Palladium auf Aktivkohle (10%ig) und hydriert anschließend 15 h bei Normaldruck. Das Reaktionsgemisch wird über einen Millipore-Filter filtriert und das Filtrat eingeengt und im Hochvakuum getrocknet. Das Rohprodukt wird ohne weitere Reinigung umgesetzt.
Ausbeute: quant.
LC-MS (Methode 17): Rₜ = 1.72 min.
MS (EI): m/z = 617 (M-HOAc+H)⁺

### Beispiel 228A

*N*⁵-[*N*²-[(Benzyloxy)carbonyl]-*N*⁵-(*tert*-butoxycarbonyl)-D-ornithyl]-*N*²-(*tert*-butoxycarbonyl)-*N-*(2-[(*tert*-butoxycarbonyl)amino]ethyl}-*L*-ornithinamid 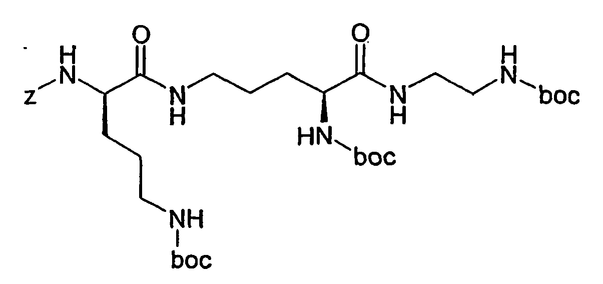 Unter Argon werden 286 mg (0.78 mmol) *N*²-[(Benzyloxy)carbonyl]-*N*⁵-{*tert*-butoxycarbonyl)-D-ornithin und 439 mg (1.17 mmol) der Verbindung aus Beispiel 143A in 16 ml Dimethylformamid gelöst. Bei 0°C (Eisbad) werden dann 255 mg (1.33 mmol) EDC und 106 mg (0.78 mmol) HOBt zugegeben. Es wird langsam auf RT erwärmt und für 48 h bei RT gerührt. Die Lösung wird im Vakuum eingeengt und der Rückstand in Dichlormethan aufgenommen und mit gesättigter wäßriger Natriumhydrogencarbonat-Lösung, 0.1 N Salzsäure und Wasser gewaschen. Die vereinigten organischen Phasen werden im Vakuum eingeengt und der so erhaltene Feststoff ohne Reinigung weiter umgesetzt.
Ausbeute: 0.58 g (quant.)
LC-MS (Methode 17): Rₜ = 2.59 min.
MS (EI): m/z = 723 (M+H)⁺

### Beispiel 229A

*N*⁵-[*N*⁵-(*tert*-Butoxycarbonyl)D-ornithyl]-*N*²-(*tert*-butoxycarbonyl)-*N-*{2-[(*tert*-butoxycarbonyl)-amino]ethyl}-*L*-ornithinamid 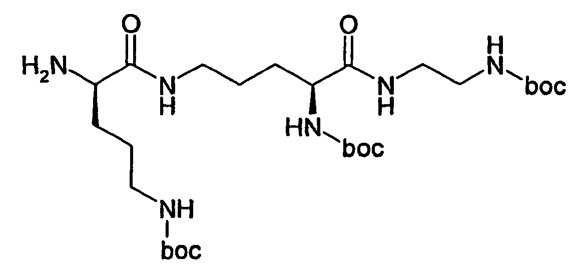 0.58 g (0.80 mmol) der Verbindung aus Beispiel 228A werden in 27 ml Ethanol gelöst und mit 0.06 g (0.06 mmol) Pd/C versetzt. Man hydriert 12 h bei Normaldruck, filtriert über Celite und engt das Filtrat im Vakuum ein. Der so erhaltene Feststoff wird ohne Reinigung weiter umgesetzt.
Ausbeute: 0.47 g (97% d. Th.)
LC-MS (Methode 19): Rₜ = 1.61 min.
MS (EI): m/z = 589 (M+H)⁺

### Beispiel 230A

Benzyl-{(4*S*)-6-({(2*S*)-2,5-bis[(*tert*-butoxycarbonyl)amino]pentyl}amino)-4-[(*tert*-butoxycarbonyl)amino]-6-oxohexyl}carbamat 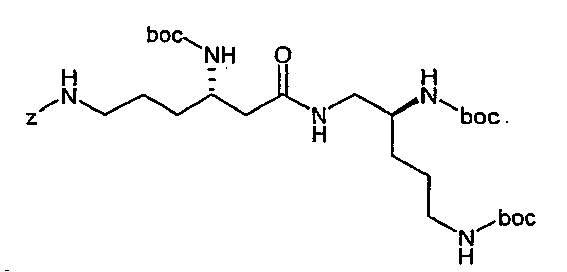 Unter Argon werden 0.1 g (0.263 mmol) (3*S*)-6-{[(Benzyloxy)carbonyl]amino}-3-[(*tert-*butoxycarbonyl)amino]hexansäure *(*Bioorg. Med. Chem. Lett. 1998, 8, 1477-1482) und 0.108 g (0.342 mmol) *tert*-Butyl-{(4*S*)-5-amino-4-[(*tert*-butoxycarbonyl)amino]pentyl}carbamat (Beispiel 86A) in 6 ml Dimethylformamid getost. Bei 0°C (Eisbad) werden dann 0.066 g (0.342 mmol) EDC und 0.011 g (0.079 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum bis zur Gewichtskonstanz getrocknet.
Ausbeute: 0.127 g (71% d. Th.)
LC-MS (Methode 19): Rₜ = 2.36 min.
MS (EI): m/z = 680 (M+H)⁺

### Beispiel 231A

*tert*-Butyl-{(1*S*)-4-amino-1-[2-({(2*S*)-2,5-bis[(*tert*-butoxycarbonyl)amino]pentyl}amino)-2-oxoethyl]butyl} carbamat 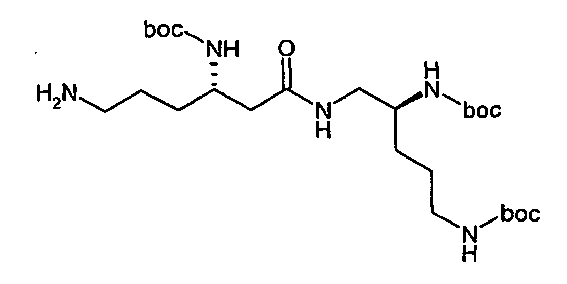 Zu einer Mischung aus 0.127 g (0.19 mmol) der Verbindung aus Beispiel 230A in 10 ml Ethanol gibt man 20 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: quant.
MS (EI): m/z = 546 (M+H)⁺

### Beispiel 232A

Benzyl-*tert*-butyl[(2*S*)-3-({(2*S*)-2,5-bis[(*tert*-butoxycarbonyl)amino]pentyl}amino)-3-oxopropan-1,2-diyl]biscarbamat 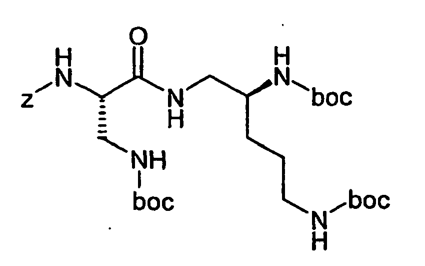 Unter Argon werden 0.127 g (0.37 mmol) *N-*[(Benzyloxy)carbonyl]-3-[(*tert*-butoxycarbonyl)-amino]-*L*-alanin und 0.193 g (0.49 mmol) *tert*-Butyl-{(4*S*)-5-amino-4-[(*tert*-butoxycarbonyl)-amino]pentyl}carbamat (Beispiel 86A) in 6 ml Dimethylformamid gelöst. Bei 0°C (Eisbad) werden dann 0.093 g (0.49 mmol) EDC und 0.015 g (0.11 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird per präparativer HPLC (Kromasil, Laufmittel Acetonitril/ 0.25% wässrige Trifluoressigsäure 5:95 → 95:5) gereinigt.
Ausbeute: 0.126 g (53% d. Th.)
LC-MS (Methode 19): Rₜ = 2.65 min.
MS (EI): m/z = 638 (M+H)⁺

### Beispiel 233A

*tert*-Butyl-[(2*S*)-2-amino-3-({(2*S*)-2,5-bis[(*tert*-butoxcarbonyl)amino]pentyl}amino)-3-oxopropyl]carbamat 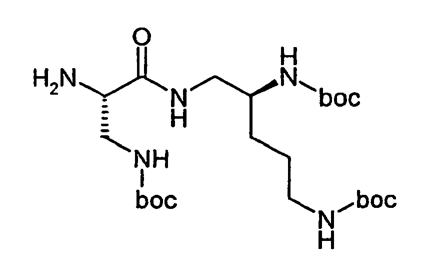 Zu einer Mischung aus 0.122 g (0.19 mmol) der Verbindung aus Beispiel 232A in 50 ml Ethanol gibt man 20 mg Palladium auf Aktivkohle (10%ig) und hydriert anschließend 4 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: quant.
MS (EI): m/z = 504 (M+H)⁺

### Beispiel 234A

Benzyl1-{(1*S*)-1-[2-({(2*S*)-2,5-bis[(*tert*-butoxycarbonyl)amino]pentyl}amino)-2-oxoethyl]-4-[(*tert-*butoxycarbonyl)amino]butyl}carbamat 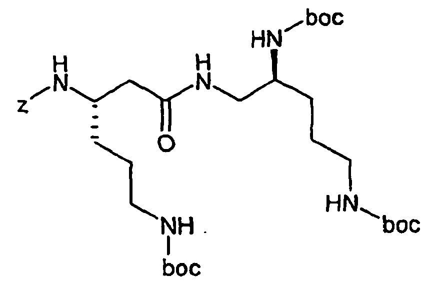 Unter Argon werden 0.1 g (0.26 mmol) (3*S*)-3-{[(Benzyloxy)carbonyl]amino}-6-[(*tert*-butoxycarbonyl)amino]hexansäure (J. Med. Chem. 2002, 45, 4246-4253) und 0.11 g (0.34 mmol) *tert-*Butyl-{(4*S*)-5-amino-4-[(*tert*-butoxycarbonyl)amino]pentyl}carbamat (Beispiel 86A) in 6 ml Dimethylformamid gelöst. Bei 0°C (Eisbad) werden dann 0.065 g (0.34 mmol) EDC und 0.011 g (0.079 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat-und Natriumchlorid-Lösung gewaschen, über Magnesiumsulfat getrocknet und im Vakuum eingedampft. Der verbleibende Feststoff wird im Hochvakuum bis zur Gewichtskonstanz getrocknet.
Ausbeute: 0.146 g (82% d. Th.)
LC-MS (Methode 12): Rₜ = 2.5 min.
MS (EI): m/z = 680 (M+H)⁺

### Beispiel 235A

*tert*-Butyl-[(4*S*)-4-amino-6-({(2*S*)-2,5-bis[(*tert*-butoxycarbonyl)amino]pentyl}amino)-6-oxohexyl]carbamat 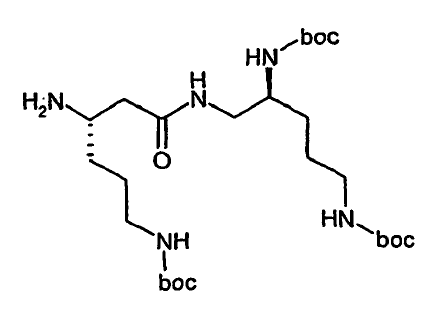 Zu einer Mischung aus 0.146 g (0.22 mmol) der Verbindung aus Beispiel 234A in 10 ml Ethanol gibt man 22 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: quant.
MS (EI): m/z = 546 (M+H)⁺

### Beispiel 236A

Benzyl-{(3*S*)-3-[(*tert* butoxycarbonyl)amino]-4-hydroxybutyl}carbamat 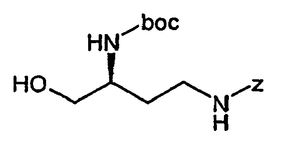 Die Herstellung erfolgt analog Beispiel 83A aus 300 mg (0.85 mmol) (2*S*)-4-{[(Benzyloxy)-carbonyl]amino}-2-[(*tert*-butoxycarbonyl)-amino]butansäure in 10 ml Tetrahydrofuran mit 86 mg (0.85 mmol) 4-Methylmorpholin, 92 mg (0.85 mmol) Chlorameisensäureethylester und 1.7 ml (1.70 mmol) einer 1M Lösung von Lithiumaluminiumhydrid in Tetrahydrofuran. Das Produkt wird ohne weitere Reinigung umgesetzt.
Ausbeute: 238 mg (82% d. Th.)
LC-MS (Methode 12): Rₜ = 1.83 min.
MS (EI): m/z = 339 (M+H)⁺

### Beispiel 237A

*tert*-Butyl[(1*S*)-3-amino-1-(hydroxymethyl)propyl]carbamat 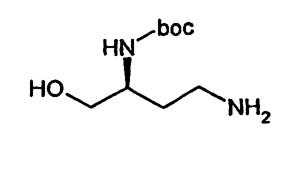 Die Herstellung erfolgt analog Beispiel 81A aus 237 mg (0.7 mmol) Benzyl-{(3*S*)-3-[(*t*ert-butoxycarbonyl)amino]-4-hydroxybutyl}carbamat (Beispiel 236A) in 50 ml Ethanol unter Zusatz von 23 mg Palladium auf Aktivkohle (10%ig).
Ausbeute: 177 mg (quant.)
MS (ESI): m/z = 205 (M+H)⁺.

### Beispiel 238A

Benzyl-{(1*S*)-4-[(*tert*-butoxycarbonyl)amino]-1-[({(3*S*)-3-[(*tert*-butoxycarbonyl)amino]-4-hydroxybutyl}amino)carbonyl]butyl}carbamat 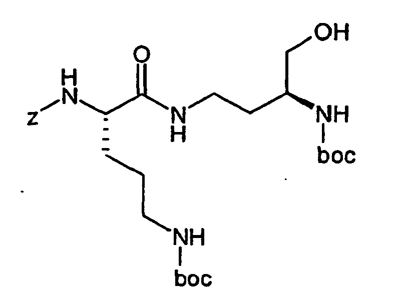 Unter Argon werden 0.082 g (0.22 mmol) *N*²-[(Benzyloxy)carbonyl]-*N*⁵-(*tert*-butoxycarbonyl)-*L-*ornithin und 0.059 g (0.29 mmol) Benzyl-{(3*S*)-3-[(*tert*-butoxycarbonyl)amino]-4-hydroxybutyl)carbamat (Beispiel 237A) in 6 ml Dimethylformamid gelöst. Bei 0°C (Eisbad) werden dann 0.056 g (0.29 mmol) EDC und 0.009 g (0.067 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum bis zur Gewichtskonstanz getrocknet.
Ausbeute: 0.088 g, (72% d. Th.)
LC-MS (Methode 12): Rₜ = 2.04 min.
MS (EI): m/z = 553 (M+H)⁺

### Beispiel 239A

*N*⁵-(*tert*-Butoxycarbonyl)-*N-*{(3*S*)-3-[(*tert*-butoxycarbonyl)amino]-4-hydroxybutyl}-*L-*ornithinamid 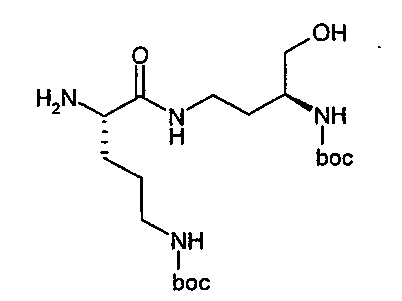 Zu einer Mischung aus 0.088 g (0.16 mmol) der Verbindung aus Beispiel 238A in 10 ml Ethanol gibt man 17 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: 0.064 g (96% d. Th.)
MS (EI): m/z = 419 (M+H)⁺

### Beispiel 240A

Benzyl-*tert*-butyl-[5-({2-[(*tert*-butoxycarbonyl)amino]ethyl}amino)-5-oxopentan-1,3-diyl]-biscarbamat 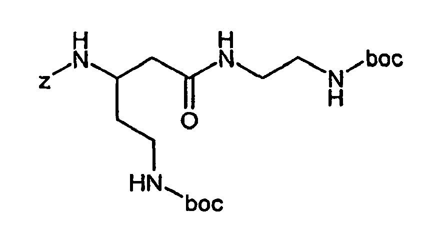 Unter Argon werden 0.20 g (0.55 mmol) 3-{[(Benzyloxy)carbonyl]amino}-5-[(*tert*-butoxycarbonyl)amino]pentansäure *(*Bioorg. Chem. Med. Lett. 2003, 13, 241-246) und 0.114 g (0.71 mmol) *tert*-Butyl-(2-aminoethyl)carbamat in 6 ml Dimethylformamid gelöst. Bei 0°C (Eisbad) werden dann 0.136 g (0.71 mmol) EDC und 0.022 g (0.164 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird per präparativer HPLC (Kromasil, Laufmittel Acetonitril/ 0.25% wässrige Trifluoressigsäure 5:95 → 95:5) gereinigt.
Ausbeute: 0.074 g (27% d. Th.)
LC-MS (Methode 17): Rₜ = 2.37 min.
MS (EI): m/z = 509 (M+H)⁺

### Beispiel 241A

*tert*-Butyl-[3-amino-5-({2-[(*tert*-butoxycarbonyl)amino]ethyl}amino)-5-oxopentyl]carbamat 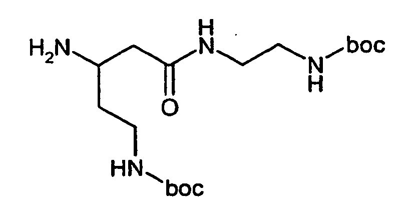 Zu einer Mischung aus 0.074 g (0.15 mmol) der Verbindung aus Beispiel 240A in 10 ml Ethanol gibt man 15 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: 0.050 g (92% d. Th.)
MS (EI): m/z = 375 (M+H)⁺

### Beispiel 242A

Benzyl-[3-({(2*S-*2,5-bis[(*tert*-butoxycarbonyl)amino]pentyl}amino)-3-oxopropyl]carbamat 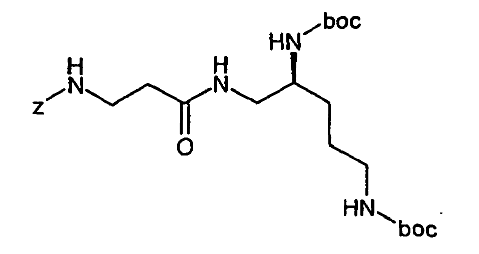 Unter Argon werden 0.10 g (0.45 mmol) *N*[(Benzyloxy)carbonyl]-beta-alanin und 0.185 g (0.58 mmol) *tert*-Butyl-{(4*S*)-5-amino-4-[(*tert*-butoxycarbonyl)amino]pentyl}carbamat (Beispiel 86A) in 6 ml Dimethylformamid gelöst. Bei 0°C (Eisbad) werden dann 0.112 g (0.58 mmol) EDC und 0.018 g (0.134 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum bis zur Gewichtskonstanz getrocknet.
Ausbeute: 0.215 g (92% d. Th.)
LC-MS (Methode 12): Rₜ = 2.19 min.
MS (EI): m/z = 523 (M+H)⁺

### Beispiel 243A

*tert*-Butyl-{(4*S*)-5-[(3-aminopropanoyl)amino]-4-[(*tert*-butoxycarbonyl)amino]pentyl)carbamat 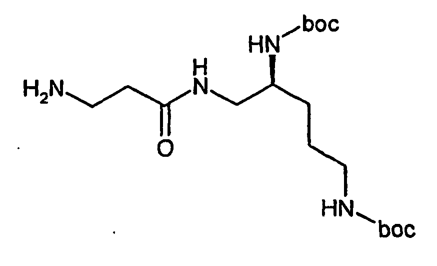 Zu einer Mischung aus 0.215 g (0.41 mmol) der Verbindung aus Beispiel 242A in 10 ml Ethanol gibt man 40 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: 0.160 g (quant.)
MS (EI): m/z = 3 89 (M+H)⁺

### Beispiel 244A

Benzyl-*tert*-butyl-[5-({(2*S*)-2,5-bis[(*tert*-butoxycarbonyl)amino]pentyl}amino)-5-oxopentan-1,3-diyl]biscarbamat 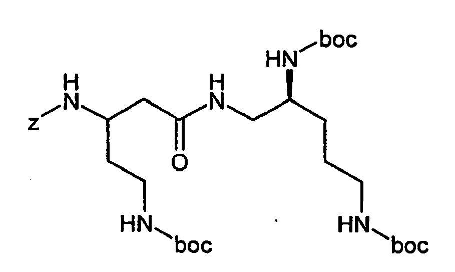 Unter Argon werden 0.146 g (0.40 mmol) 3-{[(Benzyloxy)carbonyl]amino}-5-[(*tert-*butoxycarbonyl)amino]pentansäure und 0.164 g (0.52 mmol) *tert*-Butyl-{(4*S*)-5-amino-4-[(*tert-*butoxycarbonyl)amino]pentyl)carbamat (Beispiel 86A) in 8 ml Dimethylformamid gelöst. Bei 0°C (Eisbad) werden dann 0.10 g (0.52 mmol) EDC und 0.009 g (0.12 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum bis zur Gewichtskonstanz getrocknet.
Ausbeute: 0.232 g, (87% d. Th.)
LC-MS (Methode 17): Rₜ = 2.73 min.
MS (EI): m/z = 666 (M+H)⁺

### Beispiel 245A

*tert*-Butyl-[3-amino-5-({(2*S*)-2,5-bis[(*tert*-butoxycarbonyl)amino]pentyl}amino)-5-oxopentyl]-carbamat 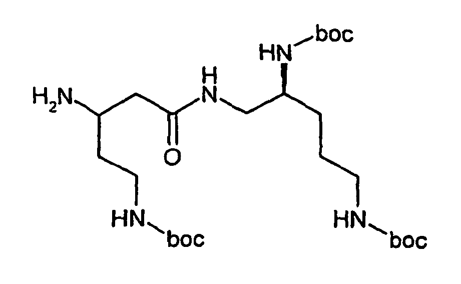 Zu einer Mischung aus 0.232 g (0.35 mmol) der Verbindung aus Beispiel 244A in 10 ml Ethanol gibt man 35 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: 0.175 g (94% d. Th.)
LC-MS (Methode 17): Rₜ = 1.8 min.
MS (EI): m/z = 532 (M+H)⁺

### Beispiel 246A

Benzyl-{(4*S*)-4-[(*tert*-butoxycarbonyl)amino]-5-hydroxypentyl}carbamat 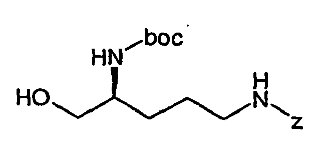 Die Herstellung erfolgt analog Beispiel 83A aus 1.0 g (2.73 mmol) *N⁵*-[(Benzyloxy)carbonyl]-*N²-*(*tert*-butoxycarbonyl)-*L*-ornithin in 35 ml Tetrahydrofuran mit 0.276 g (2.73 mmol) 4-Methylmorpholin, 0.296 g (2.73 mmol) Chlorameisensäureethylester und 5.5 ml (5.5 mmol) einer 1 M Lösung von Lithiumaluminiumhydrid in Tetrahydrofuran. Das Produkt wird per präparativer HPLC (Kromasil, Laufmittel Acetonitril/ 0.25% wässrige Trifluoressigsäure 5:95 → 95:5) gereinigt.
Ausbeute: 0.398 g (41 % d. Th.)
LC-MS (Methode 12): Rₜ =1.84 min.
MS (EI): m/z= 354 (M+H)⁺

### Beispiel 247A

*tert*-Butyl-[(1*S*)-4-amino-1-(hydroxymethyl)butyl]carbamat 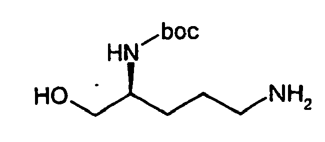 Die Herstellung erfolgt analog Beispiel 81A aus 0.232 g (0.66 mmol) Benzyl-{(4*S*)-4-[(*tert-*butoxycarbonyl)amino]-5-hydroxypentyl}carbamat (Beispiel 246A) in 50 ml Ethanol unter Zusatz von 23 mg Palladium auf Aktivkohle (10%ig).
Ausbeute: 135 mg (94% d. Th.)
MS (ESI): m/z = 219 (M+H)⁺.

### Beispiel 248A

Benzyl-{(1*S*)-4-[(*tert*-butoxycarbonyl)amino]-1-[({(4*S*)-4-[(*tert*-butoxycarbonyl)amino]-5-hydroxypentyl}amino)carbonyl]butyl}carbamat 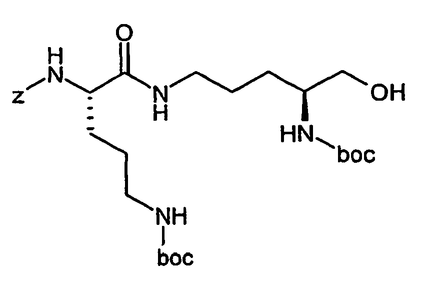 Unter Argon werden 0.155 g (0.42 mmol) *N²*-[(Benzyloxy)carbonyl]-*N⁵*-(*tert*-butoxycarbonyl)-*L-*ornithin und 0.12 g (0.55 mmol) *tert*-Butyl-[(1*S*)-4-amino-1-(hydroxymethyl)butyl]carbamat (Beispiel 247A) in 6 ml Dimethylformamid gelöst. Bei 0°C (Eisbad) werden dann 0.105 g (0.55 mmol) EDC und 0.017 g (0.13 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird per präparativer HPLC (Kromasil, Laufmittel Acetonitril/ 0.25% wässrige Trifluoressigsäure 5:95 → 95:5) gereinigt.
Ausbeute: 0.164 g (69% d. Th.)
LC-MS (Methode 12): Rₜ = 2.05 min.
MS (EI): m/z = 567 (M+H)⁺

### Beispiel 249A

*N⁵*-(*tert*-Butoxycarbonyl)-*N*-{(4*S*)-4-[(*tert*-butoxycarbonyl)amino]-5-hydroxypentyl}-*L-*omithinamid 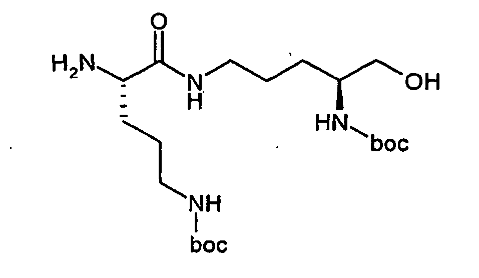 Zu einer Mischung aus 0.164 g (0.29 mmol) der Verbindung aus Beispiel 248A in 10 ml Ethanol gibt man 30 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: 0.125 g (quant.)
MS (EI): m/z = 433 (M+H)⁺

### Beispiel 250A

Benzyl-*tert*-butyl-[(2*S*)-3-hydroxypropan-1,2-diyl]biscarbamat 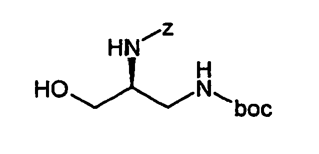 Die Herstellung erfolgt analog Beispiel 83A aus 0.40 g (1.18 mmol) *N*-[(Benzyloxy)carbonyl]-3-[(*tert*-butoxycarbonyl)amino]-*L*-alanin in 20 ml Tetrahydrofuran mit 0.12 g (1.18 mmol) 4-Methylmorpholin, 0.13 g (1.18 mmol) Chlorameisensäureethylester und 2.4 ml (2.4 mmol) einer 1M Lösung von Lithiumaluminiumhydrid in Tetrahydrofuran. Das Produkt wird per präparativer HPLC (Kromasil, Laufmittel Acetonitril/ 0.25% wässrige Trifluoressigsäure 5:95 → 95:5) gereinigt.
Ausbeute: 0.193 g (50% d. Th.)
LC-MS (Methode 12): Rₜ = 1.79 min.
MS (EI): m/z = 325 (M+M)⁺

### Beispiel 251A

*tert*-Butyl-[(2*S*)-2-amino-3-hydroxypropyl]carbamat 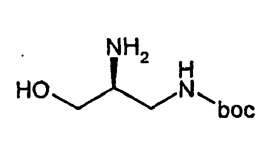 Die Herstellung erfolgt analog Beispiel 81A aus 0.193 g (0.59 mmol) Benzyl-*tert*-butyl-[(2*S*)-3-hydroxypropan-1,2-diyl]biscarbamat (Beispiel 250A) in 10 ml Ethanol unter Zusatz von 23 mg Palladium auf Aktivkohle (10%ig).
Ausbeute: 112 mg (99% d. Th.)
MS (ESI): m/z = 191 (M+H)⁺.

### Beispiel 252A

Benzyl-[(1*S*)-4-(*tert*-butoxycarbonyl)amino]-1-({[(1*S*)-2-[(*tert*-butoxycarbonyl)amino]-1-(hydroxymethyl)ethyl]amino}carbonyl)butyl]carbamat 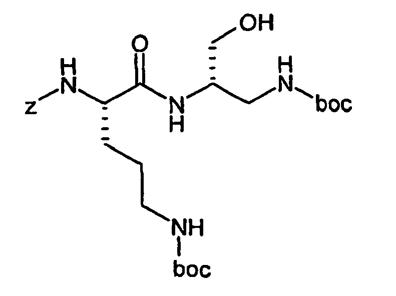 Unter Argon werden 0.18 g (0.49 mmol) *N²*-[(Benzyloxy)carbonyl]-*N⁵*-(*tert*-butoxycarbonyl)-*L-*ornithin und 0.122 g (0.64 mmol) *tert*-Butyl-[(2*S*)-2-amino-3-hydroxypropyl]carbamat (Beispiel 251A) in 6 ml Dimethylformamid gelöst. Bei 0°C (Eisbad) werden dann 0.123 g (0.64 mmol) EDC und 0.02 g (0.15 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird per präparativer HPLC (Kromasil, Laufmittel Acetonitril/ 0.25% wässrige Trifluoressigsäure 5:95 → 95:5) gereinigt.
Ausbeute: 0.216 g (81% d. Th.)
LC-MS (Methode 12): Rₜ =.2.07 min.
MS (EI): m/z = 539 (M+H)⁺

### Beispiel 253A

*N⁵*-(*tert*-Butoxycarbonyl)-*N*-[(1*S*)-2-[(*tert*-butoxycarbonyl)amino]-1-(hydroxymethyl)ethyl]-*L-*ornithinamid 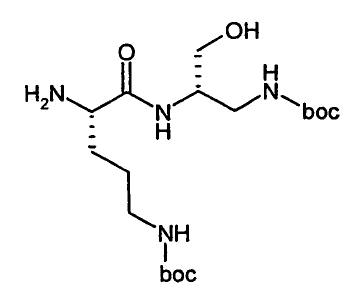 Zu einer Mischung aus 0.216 g (0.40 mmol) der Verbindung aus Beispiel 252A in 10 ml Ethanol gibt man 40 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: quant.
MS (EI): m/z = 405 (M+H)⁺

### Beispiel 254A

Benzyl-[3-({2-[(*tert*-butoxycarbonyl)amino]ethyl}amino)-3-oxopropyl]carbamat 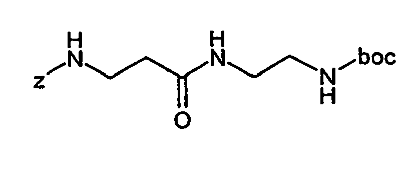 Unter Argon werden 0.20 g (0.90 mmol) *N*-[(Benzyloxy)carbonyl]-beta-alanin und 0.187 g (1.17 mmol) *tert*-Butyl-(2-aminoethyl)carbamat in 6 ml Dimethylformamid gelöst. Bei 0°C (Eisbad) werden dann 0.223 g (1.17 mmol) EDC und 0.036 g (0.27 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum bis zur Gewichtskonstanz getrocknet.
Ausbeute: 0.30 g (82% d. Th.)
LC-MS (Methode 19): Rₜ = 1.93 min.
MS (EI): m/z = 366 (M+H)⁺

### Beispiel 255A

*N*-{2-[(*tert*-Butoxycarbonyl)amino]ethyl}-beta-alaninamid 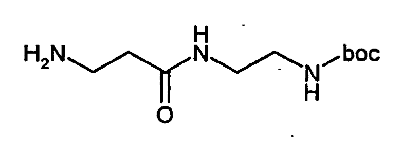 Zu einer Mischung aus 0.30 g (0.82 mmol) der Verbindung aus Beispiel 254A in 10 ml Ethanol gibt man 80 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: 0.190 g (quant.)
MS (EI): m/z = 232 (M+H)⁺

### Beispiel 256A

di-*tert*-Butyl-((6*S*)-6-{[(benzyloxy)carbonyl]amino}-10-hydroxy-15,15-dimethyl-7,13-dioxo-14-oxa-2,8,12-triazahexadecan-1-imidoyl)imidodicarbonat 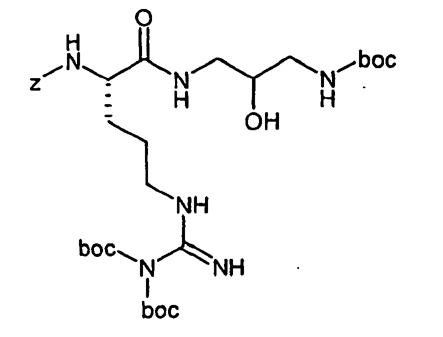 Unter Argon werden 0.30 g (0.49 mmol) *N²*-[(Benzyloxy)carbonyl]-*N⁵*-[[bis(*tert*-butoxycarbonyl) amino](imino)methyl]-*L*-omithin - Cyclohexanamin (1:1) und 0.12 g (0.64 mmol) *tert*-Butyl-(3-amino-2-hydroxypropyl)carbamat in 6 ml Dimethylformamid gelöst. Bei 0°C (Eisbad) werden dann 0.123 g (0.64 mmol) EDC und 0.02 g (0.15 mmol) HOBt zugegeben. Es wird langsam aufRT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird per präparativer HPLC (Kromasil, Laufmittel Acetonitril/ 0.25% wässrige Trifluoressigsäure 5:95 → 95:5) gereinigt.
Ausbeute: 0.183 g (54% d. Th.)
LC-MS (Methode 12): Rₜ = 2.58 min.
MS (EI): m/z = 681 (M+M)⁺

### Beispiel 257A

*N⁵*-[[bis(*tert*-Butoxycarbonyl)amino](imino)methyl]-*N*-{3-[(*tert*-butoxycarbonyl)amino]-2-hydroxypropyl}-*L*-ornithinamid 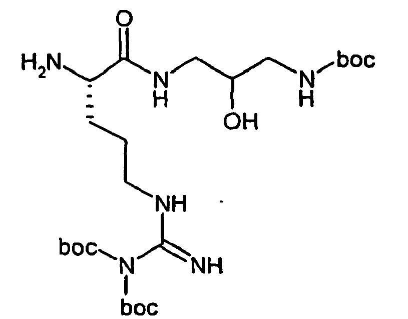 Zu einer Mischung aus 0.182 g (0.27 mmol) der Verbindung aus Beispiel 256A in 10 ml Ethanol gibt man 28 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: 0.138 g (94% d. Th.)
MS (EI): m/z = 547 (M+H)⁺

### Beispiel 258A

Benzyl-{(1*S*)-4-{[(benzyloxy)carbonyl]amino}-1-[({3-[(*tert*-butoxycarbonyl)amino]-2-hydroxypropyl}amino)carbonyl]butyl}carbamat 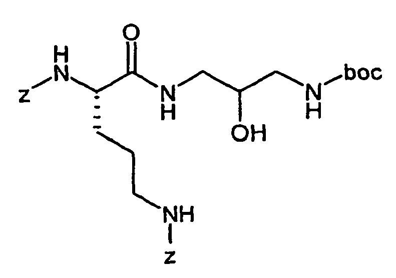 Unter Argon werden 0.20 g (0.50 mmol) *N²*,*N⁵*-Bis[(benzyloxy)carbonyl]-*L*-omithin und 0.124 g (0.65 mmol) *tert*-Butyl-(3-amino-2-hydroxypropyl)carbamat in 6 ml Dimethylformamid gelöst. Bei 0°C (Eisbad) werden dann 0.124 g (0.65 mmol) EDC und 0.02 g (0.15 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum bis zur Gewichtskonstanz getrocknet
Ausbeute: 0.245 g (86% d. Th.)
LC-MS (Methode 12): Rₜ = 2.15 min.
MS (EI): m/z = 573 (M+H)⁺

### Beispiele 259A

Benzyl-((4*S*)-5-[(3-amino-2-hydroxypropyl)amino]-4-{[(benzyloxy)carbonyl]amino}-5-oxo-pentyl)carbamat Hydrochlorid 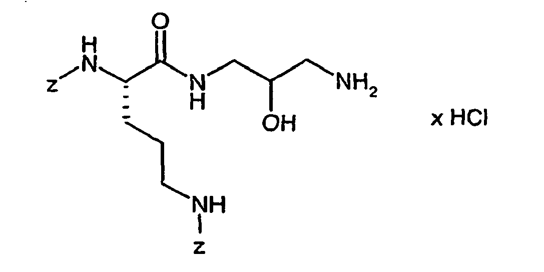 Eine Lösung von 0.263 g (0.46 mmol) der Verbindung aus Beispiel 258A in 1 mm Dioxan wird bei 0°C mit 6.8 ml einer 4N Chlorwasserstoff-Dioxan-Lösung versetzt- Nach 2 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 0.205 g (88% d. Th.)
LC-MS (Methode 12): Rₜ = 1.47 min.
MS (EI): m/z = 473 (M-HCl+H)⁺

### Beispiel 260A

Benzyl-[(2*S*)-2-[(*tert*-butoxycarbonyl)amino]-3-({2-[(*tert*-butoxycarbonyl)amino]ethyl}amino)-3-oxopropyl]carbamat 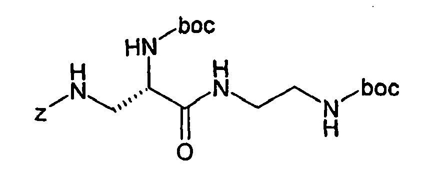 Unter Argon werden 0.50 g (0.96 mmol) 3-{[(Benzyloxy)carbonyl]amino} *N-*(*tert*-butoxycarbonyl)-*L*-alanin - *N*-Cyclohexylcyclohexanamin (1:1) und 0.154 g (0.96 mmol) *tert*-Butyl-(2-aminoethyl)carbamat in 10 ml Dimethylformamid und 0.5 ml Triethylamin gelöst. Bei 0°C (Eisbad) werden dann 0.314 g (1.64 mmol) EDC und 0.043 g (0.32 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat und Natriumchlorid-Lösung gewaschen, über Magnesiumsulfat getrocknet und im Vakuum eingedampft. Der verbleibende Feststoff wird im Hochvakuum bis zur Gewichtskonstanz getrocknet.
Ausbeute: 0.41 g (88% d. Th.)
LC-MS (Methode 12): Rₜ, = 2.17 min.
MS (EI): mlz = 481 (M+H)⁺

### Beispiel 261A

3-Amino-*N²*-(*tert*-butoxycarbonyl)-*N*-{2-[(*tert*-butoxycarbonyl)amino]ethyl}-*L*-alaninamid Hydroacetat 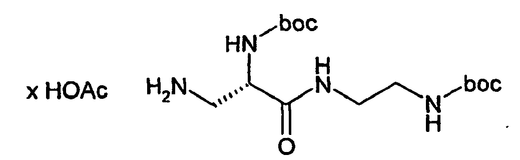 Zu einer Mischung aus 0.41 g (0.847 mmol) der Verbindung aus Beispiel 260A in 80 ml Essigsäure/Ethanol/Wasser (4:1:1) gibt man 50 mg Palladium auf Aktivkohle (10%ig) und hydriert anschließend 12 h bei Normaldruck. Das Reaktionsgemisch wird über Kieselgur filtriert, das Filtrat im Vakuum eingeengt und im Hochvakuum getrocknet. Das Rohprodukt wird ohne weitere Reinigung umgesetzt.
Ausbeute: quant.
LC-MS (Methode 12): Rₜ = 1.09 min.
MS (EI): m/z = 347 (M-HOAc+H)⁺

### Beispiel 262A

*tert*-Butyl-{3-[(8*S*,11*S*,14*S*)-8-{(6*S*,11*S*)-6,11-bis[(*tert*-butoxycarbonyl)amino]-18,18-dimethyl-8,16-dioxo-17-oxa-2,9,15-triazanonadecan-1-oyl}-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat 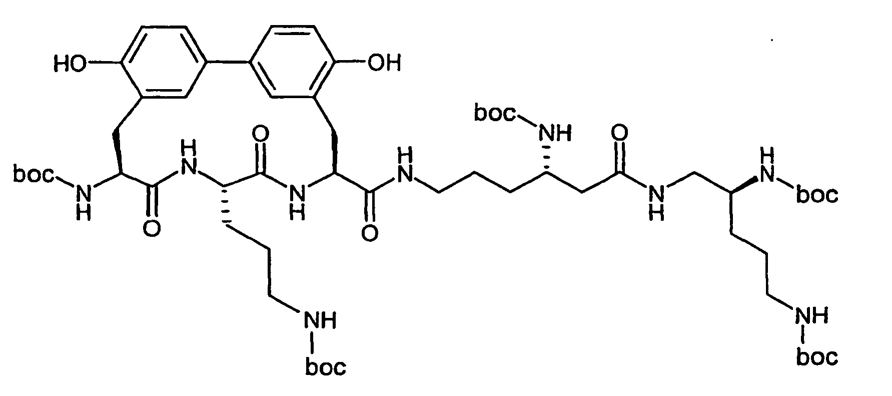 Unter Argon werden 20 mg (0.03 mmol) der Verbindung aus Beispiel 29A und 22 mg (0.04 mmol) *tert*-Butyl-{(1*S*)-4-amino-1-[2-({(2*S*)-2,5-bis[(*tert*-butoxycarbonyl)amino]pentyl}amino)-2-oxo-ethyl]butyl}carbamat (Beispiel 231A) in 1 ml Dimethylformamid gelöst. Bei 0°C (Eisbad) werden dann 7.6 mg (0.04 mmol) EDC und 1.24 mg (0.009 mmol) HOBt zugegeben. Es wird langsam auf RT erwärmt und für 12 h bei RT gerührt. Die Lösung wird im Vakuum eingeengt und der Rückstand wird mit Wasser verrührt. Der verbleibende Feststoff wird abgesaugt und chromatographisch (Sephadex LH20, Laufmittel: Methanol / Essigsäure (0.25%)) gereinigt.
Ausbeute: 25.4 mg (70% d. Th.)
LC-MS (Methode 17): Rₜ, = 2.81 min.
MS (EI): m/z = 1184 (M+H)⁺

### Beispiel 263A

*N⁵*-{*N*[(Benzyloxy)carbonyl]glycyl}-*N²*-(*tert*-butoxycarbonyl)-*N*-{2-[(*tert*-butoxycarbonyl)-amino]ethyl}-*L*-ornithinamid 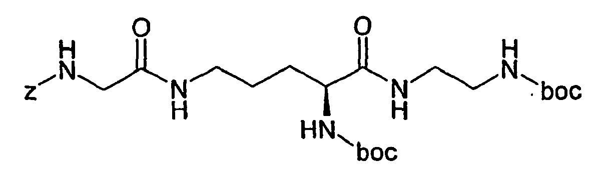 Unter Argon werden 300 mg (1.43 mmol) *N*-[(Benzyloxy)carbonyl]glycin und 830 mg (2.15 mmol) der Verbindung aus Beispiel 143A in 28 ml Dimethylformamid gelöst. Bei 0°C (Eisbad) werden dann 467 mg (2.44 mmol) EDC und 194 mg (1.43 mmol) HOBt zugegeben. Es wird langsam auf RT erwärmt und für 48 h bei RT gerührt. Die Lösung wird im Vakuum eingeengt und der Rückstand mit Dichlormethan aufgenommen und mit gesättigter Natriumhydrogencarbonat-Lösung, 0.1 N Salzsäure und Wasser gewaschen. Die vereinigten organischen Phasen werden im Vakuum eingeengt und der so erhaltene Feststoff ohne Reinigung weiter umgesetzt.
Ausbeute: quant.
LC-MS (Methode 12): Rₜ= 1.98 min.
MS (EI): m/z = 566 (M+H)⁺

### Beispiel 264A

*N⁵*-Glycyl-*N²*-(*tert*-butoxycarbonyl)-*N*-{2-[(*tert*-butoxycarbonyl)amino]ethyl}-*L*-ornithinamid 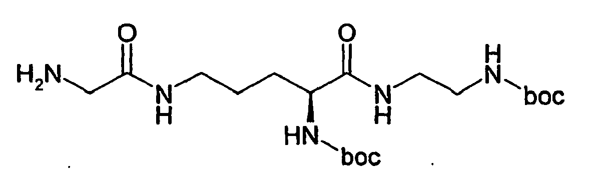 1030 mg (1.82 mmol) der Verbindung aus Beispiel 263A werden in 60 ml Ethanol gelöst und mit 100 mg (0.09 mmol) Pd/C (10%ig) versetzt. Man hydriert über Nacht bei Normaldruck, filtriert über Celite und engt das Filtrat im Vakuum ein. Der so erhaltene Feststoff wird ohne Reinigung weiter umgesetzt.
Ausbeute: 693 mg (84% d. Th.)
LC-MS (Methode 17): Rₜ = 1.41 min.
MS (EI): m/z = 432 (M+H)⁺

### Beispiel 265A

*N⁵*-[*N²*-[(Benzyloxy)carbonyl]-*N⁵*-(*tert*-butoxycarbonyl)-*L*-omithyl]-*N²*-(*tert*-butoxycarbonyl)-*N-*{2-[(*tert*-butoxycarbonyl)amino]ethyl}-*L*-ornithinamid 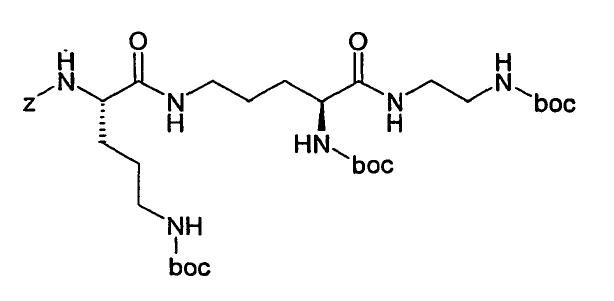 Unter Argon werden 1.95 g (5.31 mmol) *N²*-[(Benzyloxy)carbonyl]-*N⁵*-(*tert*-butoxycarbonyl)-*L-*ornithin und 3.12 g (7.97 mmol) der Verbindung aus Beispiel 143A in 100 ml Dimethylformamid gelöst. Bei 0°C (Eisbad) werden dann 1.73 g (9.03 mmol) EDC und 0.72 g (5.31 mmol) HOBt zugegeben. Es wird langsam auf RT erwärmt und für 48 h bei RT gerührt. Die Lösung wird im Vakuum eingeengt und der Rückstand mit Dichlormethan aufgenommen und mit gesättigter wäßriger Natriumhydrogencarbonat-Lösung, 0.1 N Salzsäure und Wasser gewaschen. Die vereinigten organischen Phasen werden im Vakuum eingeengt und der so erhaltene Feststoff ohne Reinigung weiter umgesetzt.
Ausbeute: 4.23 g (96% d. Th.)
LC-MS (Methode 19): Rₜ = 2.19 min.
MS (EI): m/z = 723 (M+H)⁺

### Beispiel 266A

*N⁵*-[*N⁵*-(*tert*-Butoxycarbonyl)-*L*-ornithyl]-*N²*-(*tert*-butoxycarbonyl)-*N*-{2-[(*tert*-butoxycarbonyl)-amino]ethyl}-*L*-omithinamid 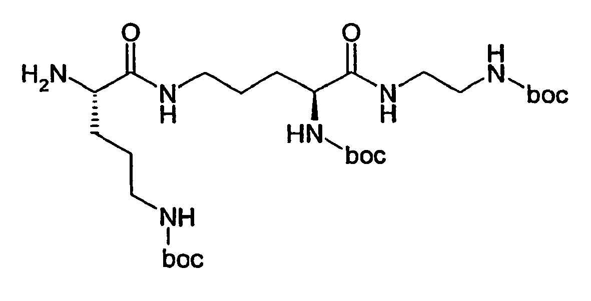 4.23 g (5.09 mmol) der Verbindung aus Beispiel 265A werden in 250 ml Ethanol gelöst und mit 0.42 g (0.39 mmol) Pd/C (10%ig) versetzt. Man hydriert 6 h bei Normaldruck, filtriert über Celite und engt das Filtrat im Vakuum ein. Der so erhaltene Feststoff wird ohne Reinigung weiter umgesetzt.
Ausbeute: 2.4 g (72% d. Th.)
LC-MS (Methode 12): Rₜ = 1.31 min.
MS (EI): m/z = 589 (M+H)⁺

### Beispiel 267A

Benzyl-((1*S*,7*S*)-7-[(*tert*-butoxycarbonyl)amino]-1-{2-[(*tert*-butoxycarbonyl)amino]ethyl}-15,15-dimethyl-2,8,13-trioxo-14-oxa-3,9,12-triazahexadec-1-yl)carbamat 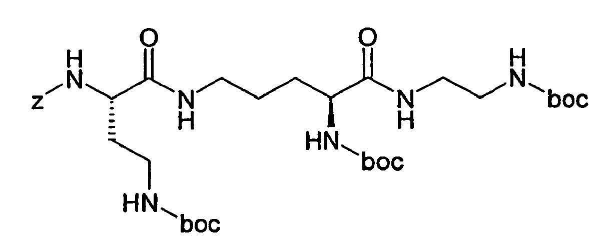 Unter Argon werden 250 mg (0.71 mmol) (2*S*)-2-{[(Benzyloxy)carbonyl]amino}-4-[(*tert-*butoxycarbonyl)amino]butansäure und 410 mg (1.06 mmol) der Verbindung aus Beispiel 143A in 14 ml Dimethylformamid gelöst. Bei 0°C (Eisbad) werden dann 231 mg (1.21 mmol) EDC und 96 mg (0.71 mmol) HOBt zugegeben. Es wird langsam auf RT erwärmt und für 48 h bei RT gerührt. Die Lösung wird im Vakuum eingeengt und der Rückstand mit Dichlormethan aufgenommen und mit gesättigter Natriumhydrogencarbonat-Lösung, 0.1 N Salzsäure und Wasser gewaschen. Die vereinigten organischen Phasen werden im Vakuum eingeengt und der so erhaltene Feststoff ohne Reinigung weiter umgesetzt.
Ausbeute: 355 mg (66% d. Th.)
LC-MS (Methode 12): Rₜ = 2.32 min.
MS (EI): m/z = 709 (M+H)⁺

### Beispiel 268A

*N⁵*-{(2*S*)-2-Amino-4-[(*tert*-butoxycarbonyl)amino]butanoyl}-*N²*-(*tert*-butoxycarbonyl)-*N*-{2-[(*tert-*butoxycarbonyl)amino]ethyl}-*L*-omithinamid 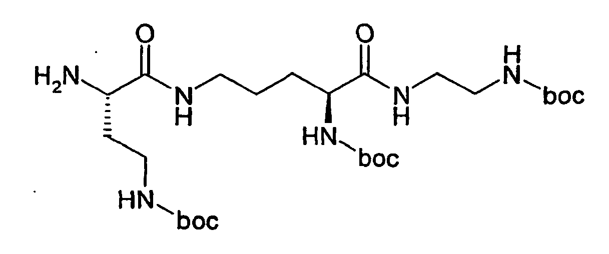 355 mg (0.5 mmol) der Verbindung aus Beispiel 267A werden in 17 ml Ethanol gelöst und mit 36 mg (0.03 mmol) Pd/C (10%ig) versetzt. Man hydriert über Nacht bei Normaldruck, filtriert über Celite und engt das Filtrat im Vakuum ein. Der so erhaltene Feststoff wird ohne Reinigung weiter umgesetzt.
Ausbeute: 304 mg (82% d. Th.)
LC-MS (Methode 17): Rₜ = 1.64 min.
MS (EI): m/z = 575 (M+H)⁺

Analog zur Vorschrift des Beispiels 262A werden die in der folgenden Tabelle aufgeführten Beispiele 269A bis 286A hergestellt.

| **Beispiel-Nr.** | **Vorstufe Beispiel** | **Struktur** | **Analytische Daten** |
|---|---|---|---|
| **269A** | 233A + 29A | 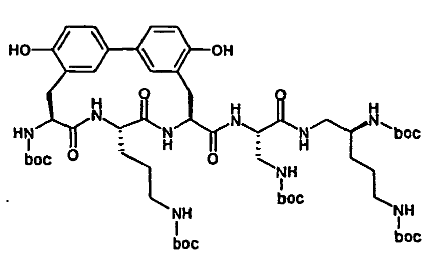 | LC-MS (Methode 19): Rₜ = 2.69 min. MS (EI): m/z = 1142 (M+H)⁺. |
| **270A** | 235A + 29A | 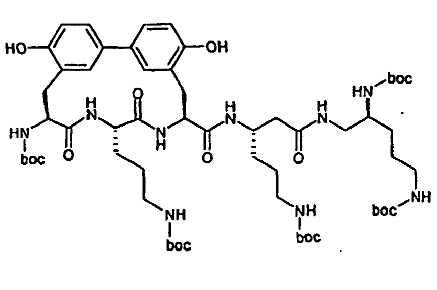 | LC- MS (Methode 12): Rₜ 2.55 min. MS (EI): m/z - 1184 (M+H)⁺. |
| **271A** | 81A + 65A | 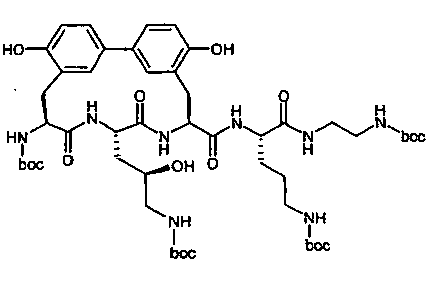 | LC-MS (Methode 17): Rₜ = 2.52 min. MS (EI): m/z = 1029 (M+H)⁺. |
| **272A** | 239A + 29A | 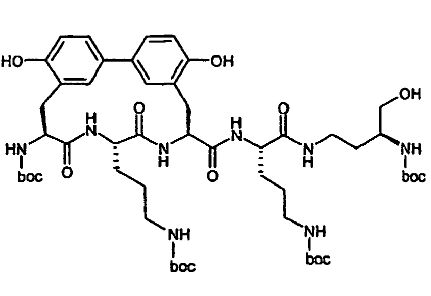 | LC-MS (Methode 19): LC-MS (Methode 19) Rₜ= 2.32 min. MS (EI): m/z = 1057 (M+H)⁺. |
| **273A** | 241A + 29A | 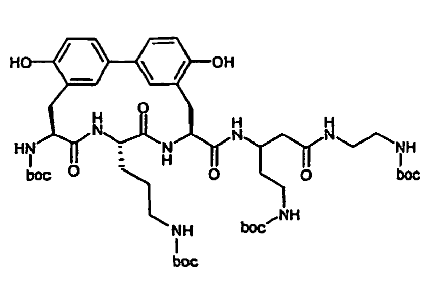 | LC-MS (Methode 12): Rₜ 2.28 min. MS (EI): m/z = 1013 (M+H)⁺. |
| **274A** | 243A + 29A | 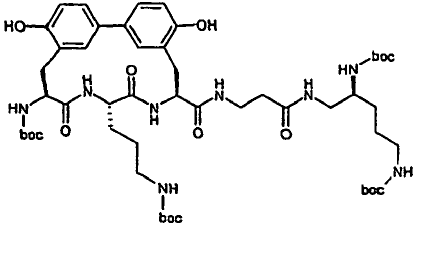 | LC-MS (Methode 12): Rₜ 2.31 min. MS (EI): m/z = 1027 (M+ii) . |
| **275A** | 245A + 29A | 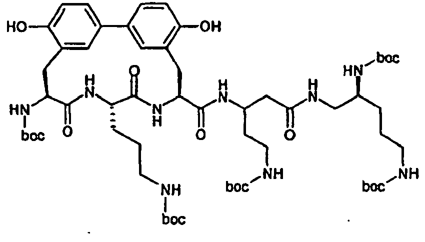 | LC-MS (Methode 19): Rₜ = 2.36 min. MS (EI): m/z = 1170 (M+H)⁺. |
| **276A** | 249A + 29A | 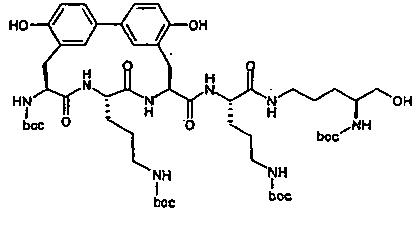 | LC-MS (Methode 19): Rₜ 2.30 min. MS (EI): m/z = 1171 (M+H)⁺. |
| **277A** | 253A + 29A | 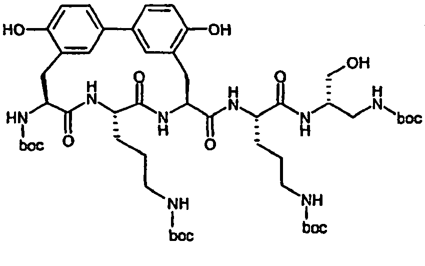 | LC-MS (Methode 19): Rₜ = 2.14 min. MS (EI): m/z = 1043 (M+H)⁺. |
| **278A** | 120A + 65A | 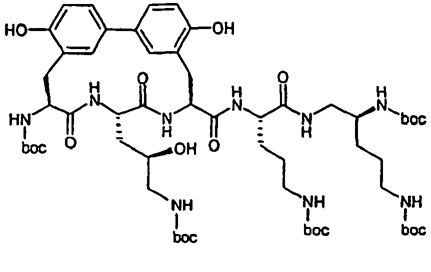 | LC-MS (Methode 12): Rₜ = 2.51 min. MS (EI): m/z = 1186 (M+H)⁺. |
| **279A** | 255A + 29A | 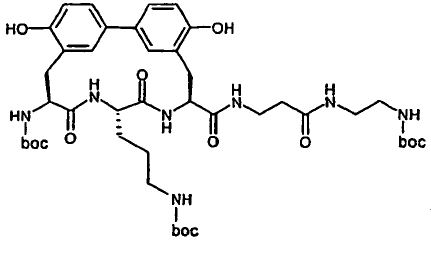 | LC-MS (Methode 12): Rₜ = 2.04 min. MS (EI): m/z = 870 (M+H)⁺. |
| **280A** | 257A + 29A | 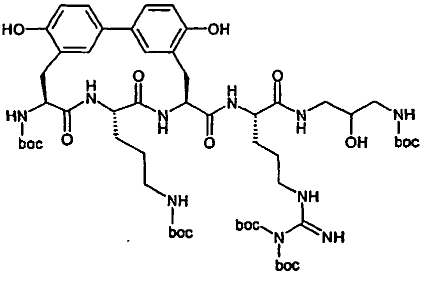 | LC-MS (Methode 12): Rₜ= 2.64 min. MS (EI): m/z = 1185 (M+H)⁺. |
| **281A** | 259A + 29A | 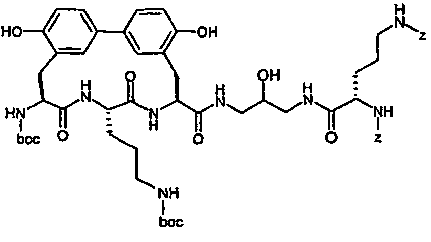 | LC-MS (Methode 17): Rₜ = 2.62 min. MS (EI): m/z = 1111 (M+H)⁺ |
| **282A** | 253A + 65A | 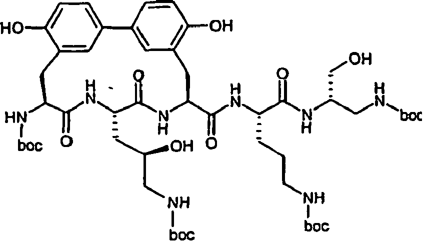 | LC-MS (Methode 19): Rₜ 2.25 min. MS (EI): m/z = 1059 (M+H)⁺. |
| **283A** | 29A + 264A | 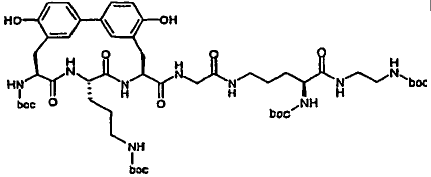 | LC-MS (Methode 17): Rₜ 2.46 min. MS (EI): m/z = 1070 (M+H)⁺ |
| **284A** | 29A + 266A | 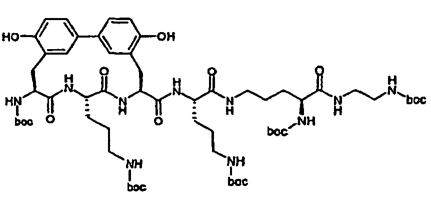 | LC-MS (Methode 17): Rₜ 2.64 min. MS (EI): m/z = 1227 (M+H)⁺. |
| **285A** | 29A + 268A | 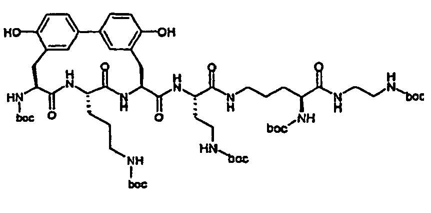 | LC-MS (Methode 17): Rₜ = 2.68 min. MS (EI): m/z = 1213 (M+H)⁺. |
| **286A** | 29A + 229A | 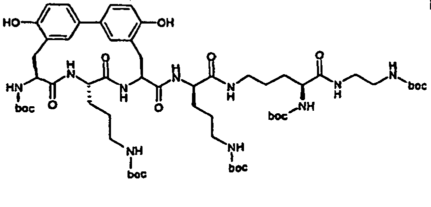 | LC-MS (Methode 17): Rₜ = 2.66 min. MS (EI): m/z = 1227 (M+H)⁺. |

Analog zur Vorschrift des Beispiels 178A werden die in der folgenden Tabelle aufgeführten Beispiele 287A bis 293A hergestellt.

| **Beispiel-Nr.** | **Vorstufe Beispiel** | **Struktur** | **Analytische Daten** |
|---|---|---|---|
| **287A** | 170A + 81A | 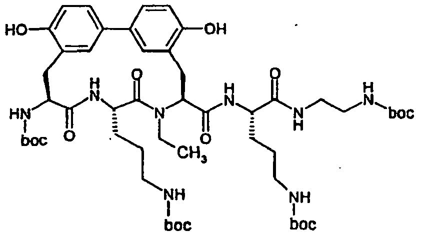 | LC-MS (Methode 17): Rₜ = 2.69 min. MS (EI): m/z = 1041 (M+H)⁺ |
| **288A** | 29A + 225A | 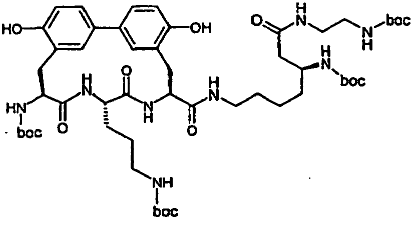 | LC-MS (Methode 17): Rₜ 2.50 min. MS (EI): m/z - 1041 (M+H)⁺. |
| **289A** | 143A + 65A | 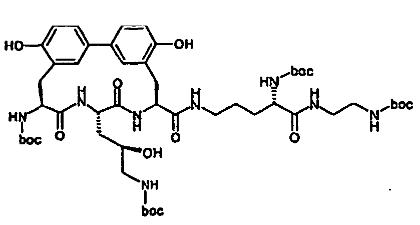 | LC-MS (Methode 17): Rₜ = 2.39 min. |
| **290A** | 52A + 223A | 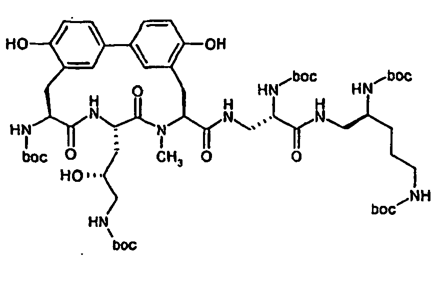 | LC-MS (Methode 17): Rₜ= 2.73min. MS (EI): m/z = 1173 (M+H)⁺. |
| **291A** | 29A + 223A | 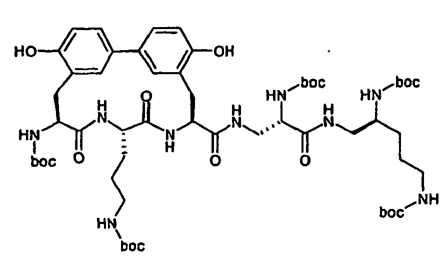 | LC-MS (Methode 17): Rₜ = 2.71 min. MS (EI): m/z = 1142 (M+H)⁺. |
| **292A** | 261A + 52A | 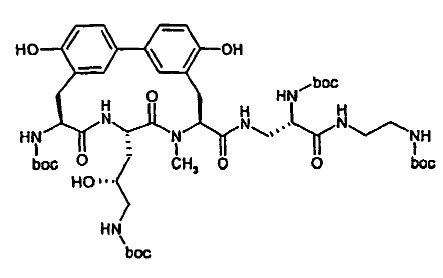 | LC-MS (Methode 19): Rₜ 2.42 min. MS (EI): m/z = 1015 (M+H)⁺. |
| **293A** | 261A + 29A | 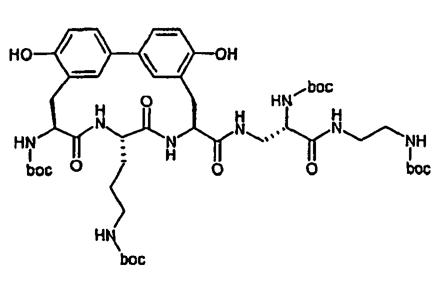 | LC-MS (Methode 19): Rₜ = 2.42 min. MS (EI): m/z - 985 (M+H)⁺. |

Analog zur Vorschrift des Beispiels 171A werden die in der folgenden Tabelle aufgeführten Beispiele 294A bis 297A hergestellt.

| **Beispiel- Nr.** | **Vorstufe Beispiel** | **Struktur** | **Analytische Daten** |
|---|---|---|---|
| **294A** | 227A + 29A | 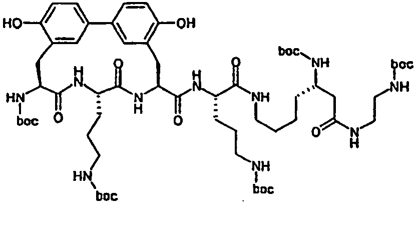 | LC-MS (Methode 12): Rₜ= 2.47 min. MS (EI): m/z = 1255 (M+H)⁺. |
| **295A** | 52A + 225A | 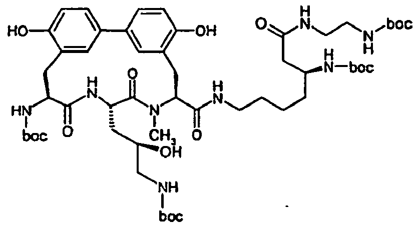 | LC-MS (Methode 12): Rₜ = 2.26 min. MS (EI): m/z - 1071 (M+H)⁺. |
| **296A** | 52A + 227A | 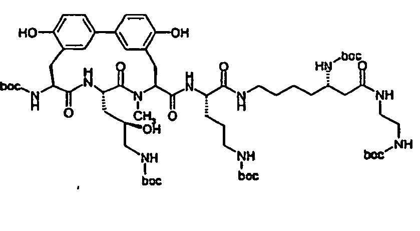 | LC-MS (Methode 12): Rₜ = 2.46 min. MS (EI): m/z = 1285 (M+H)⁺ |
| **297A** | 52A + 81A | 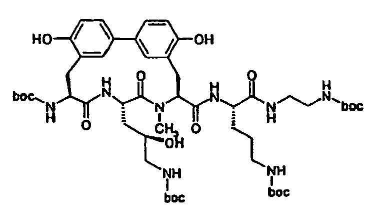 | LC-MS (Methode 12): Rₜ = 2.33 min. MS (EI): m/z = 1043 (M- H)⁺. |

Analog zur Vorschrift des Beispiels 205A werden die in der folgenden Tabelle aufgeführte Beispiel 298A hergestellt.

| **Beispiel- Nr.** | **Vorstufe Beispiel** | **Struktur** | **Analytische Daten** |
|---|---|---|---|
| **298A** | 281A | 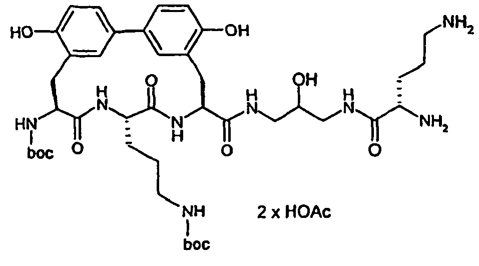 | LC-MS (Methode 12): Rₜ = 1.14 min. MS (EI): m/z = 843 (M-2HOAc+H)⁺. |

### Ausführungsbeispiele

Die Synthese von Ausführungsbeispielen kann ausgehend von partiell geschützten Biphenomycin-Derivaten (wie z.B. 29A) erfolgen.

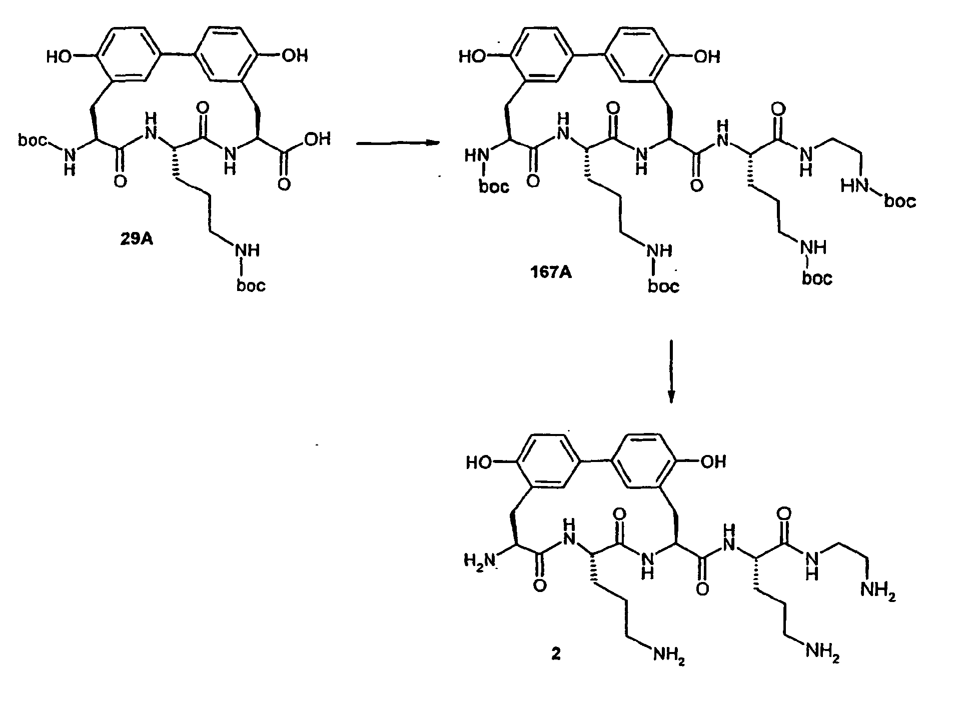

### Beispiel 1

(8*S*,11*S*,14*S*)-14-Amino-*N*-((1*S*)4-amino-1-{[(3-amino-2-hydroxypropyl)amino]carbonyl}butyl) 11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 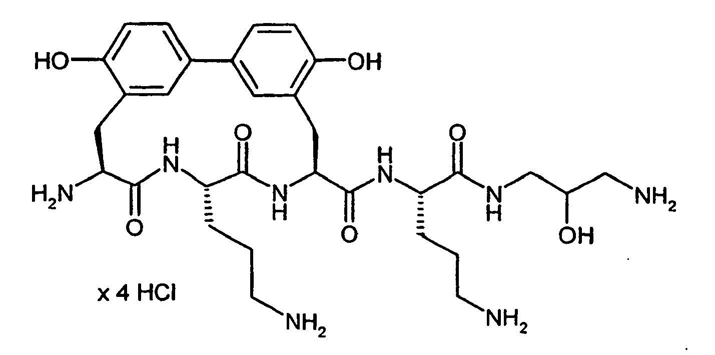 Es werden 36 mg (0.035 mmol) *tert*-Butyl-(3-{[(2*S*)-5-[(*tert*-butoxycarbonyl)amino]-2-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)pentanoyl]amino}-2-hydroxypropyl)carbamat (Beispiel 166A) in 3.0 ml 4N Chlorwasserstoff in Dioxan gelöst und 2 h. bei Raumtemperatur gerührt. Das Lösungsmittel wird im Vakuum eingedampft und der zurückgebliebene Feststoff wird im Hochvakuum bis zur Gewichtskonstanz getrocknet.
Ausbeute: 25 mg (92% d. Th.).
MS (ESI): m/z = 643 (M-4HCl+H)⁺.
¹H-NMR (400 MHz, D₂O): δ = 1.55-2.05 (m, 8H), 2.75-3.15 (m, 8H), 3.17-3.45 (m, 3H), 3.54 (m_{c}, 1H), 3.73 (mg, 1H), 3.87-4.0 (m, 2H), 4.23 (m_{c}, 1H), 4.41 (m, 1H), 4.82 (m_{c}, 1H), 6.83-6.92 (m, 2H), 6.96 (s, 1H), 7.25 (s, 1H), 7.36 (d, 1H), 7.42 (d, 1H).

Das Tetrahydrochlorid-Salz wird durch präparative HPLC (Reprosil ODS-A, Laufmittel Acetonitril / 0.2% wässrige Trifluoressigsäure 5:95 → 95:5) in das Tetra(hydrotrifluoracetat) überführt.

### Beispiel 2

(8*S*,11*S*,14*S*)-14-Amino-*N*-((1*S*)-4-amino-1-{[(2-aminoethyl)amino]carbonyl}butyl)-11-(3-aminopropyl)-5,17-dihydroxy-10, 13-dioxo-9,12-diazatricyclo[14.3.1.1^{2,6}]henicosa-1(20),2(21),3,5,16,18-hexaen-8-carboxamid Tetra(hydrotrifluoracetat) 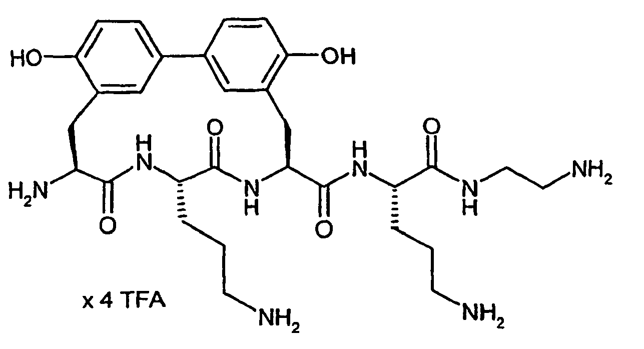 Es werden 29.7 mg (0.029 mmol) *tert*-Butyl-(2-{[(2*S*)-5-[(*tert*-butox-ycarbonyl)amino]-2-({[(8*S*,11*S*,14*S*)-14-[(*tert*-butoxycarbonyl)amino]-1,1-{3-[(3-butoxycarbonyl)amino]propyl}-5,17-dihydroxy-10,13-dioxo-9,12-diazatricyclo[14.3.1.^{2,6}]henicosa-1(20),2(21),3,5,16,18-hexaen-8-yl]carbonyl}amino)pentanoyl]amino}ethyl)carbamat (Beispiel 167A) in 3.0 ml 4N Chlorwasserstoff in Dioxan gelöst und 2 h bei Raumtemperatur gerührt. Das Lösungsmittel wird im Vakuum eingedampft und der zurückgebliebene Feststoff wird durch präparative HPLC (Reprosil ODS-A, Laufmittel Acetonitril / 0.2% wässrige Trifluoressigsäure 5:95 → 95:5) in das Tetra(hydrotrifluoracetat) überführt.
Ausbeute: 20 mg (64% d. Th.).
MS (ESI): m/z = 613 (M-4TFA+H)⁺.
¹H-NMR (400 MHz, D₂O): δ = 1.53-1.93 (m, 8H), 2.32 (m_{c}, 1H), 2.93 (m_{c}, 4H), 3.02 (mm, 1H), 3.08 (m_{c}, 2H), 3.23 (m_{c}, 1H), 3.35-3.60 (m, 3H), 4.23 (m_{c}, 2H), 4.40 (m_{c}, 1H), 4.82 (m_{c}, 1H), 6.82-6.93 (m, 2H), 6.97 (s, 1H), 7.25 (s, 1H), 7.36 (d, 1H), 7.43 (d, 1H).

### Beispiel 3

(8*S*,11*S*,14*S*)-14-Amino-*N*-((4*S*)-4-amino-5-{[(1*S*)-3-amino-1-(hydroxymethyl)propyl]amino}-5-oxopentyl)-11-(3-aminopropyl)-5,17-dihydroxy-10,13-dioxo-9,12-diazatricyclo[14.3.1.1^{2,6}]-henicosa- (20),2(21),3,5,16,18-hexaen-8-carboxamid Tetrahydrochlorid 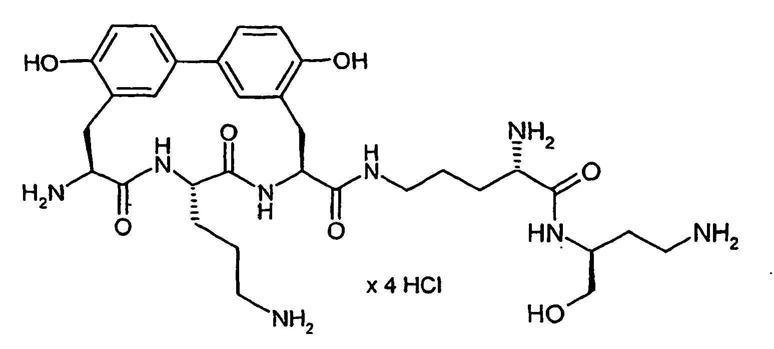 Es werden 24.9 mg (0.024 mmol) *tert*-Butyl-((3*S*)-3-{[(2*S*)-2-[(*tert*-butoxycarbonyl)amino]-5-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)pentanoyl]amino}-4-hydroxybutyl)carbamat (Beispiel 168A) in 3.0 ml 4N Chlorwasserstoff in Dioxan gelöst und 2 h bei Raumtemperatur gerührt. Das Lösungsmittel wird im Vakuum eingedampft und der zurückgebliebene Feststoff wird im Hochvakuum bis zur Gewichtskonstanz getrocknet.
Ausbeute: 17 mg (90% d. Th.).
MS (ESI: m/z = 657 (M-4HCl+H)⁺.
¹H-NMR (400 MHz, D₂O): δ = 1.5-1.95 (m, 10H), 2.75-3.05 (m, 7H), 3.08-3.18 (m, 2H), 3.3 (mm, 1H), 3.43-3.61 (m, 3H), 3.87-3.97 (m, 2H), 4.41 (m_{c}, 1H), 4.82 (m_{c}, 1H), 6.83-6.92 (m, 2H), 6.96 (s, 1H), 7.25 (s, 1H), 7.34 (d, 1H), 7.42 (d, 1H).
Das Tetrahydrochlorid-Salz wird durch präparative HPLC (Reprosil ODS-A, Laufmittel Acetonitril / 0.2% wässrige Trifluoressigsäure 5:95 → 95:5) in das Tetra(hydrotrifluoracetat) überführt.

### Beispiel 4

(8*S*,11*S*,14*S*)-14-Amino-*N*-((1*S*,3*R*)-4-amino-1-{[(2-aminoethyl)amino]carbonyl}-3-hydroxybutyl)-11-[(2*R*)-3-amino-2-hydroxypropyl]-5,17-dihydroxy-10,13-dioxo-9,12-diazatricyclo[14.3.1.1^{2,6}]-henicosa-1(20),2(21),3,5,16,18-hexaen-8-carboxamid Tetrahydrochlorid 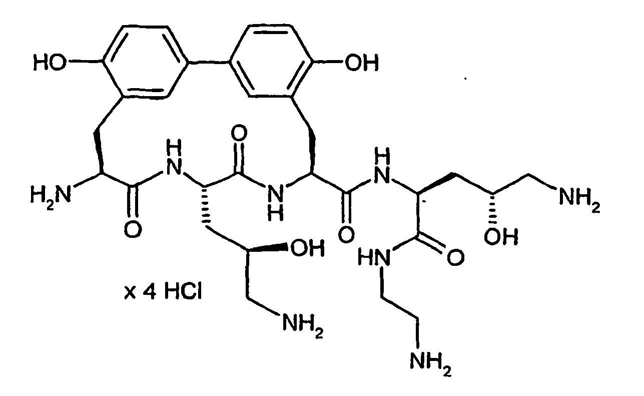 16 mg (0.02 mmol) der Verbindung aus Beispiel 172A werden in 0.5 ml Dioxan vorgelegt, auf 0°C gekühlt und mit 1 ml 4N Chlorwasserstoff in Dioxan versetzt. Man läßt auf Raumtemperatur erwärmen und rührt 1 h nach. Anschließend engt man im Vakuum ein und trocknet den Rückstand im Hochvakuum. Man rührt mit Acetonitril aus, filtriert den ausgefallenen Niederschlag ab und erhält so die Titelverbindung als Feststoff.
Ausbeute: 8 mg (42% d. Th.).
LC-MS (Methode 20): Rₜ = 1.03 min.
MS (EI): m/z = 645 (M-4HCl+H)⁺.

### Beispiel 5

(8*S*,11*S*,14*S*)-14-Amino-*N*-((1*S*)-3-amino-1-{[(3-amino-2-hydroxypropyl)amino]carbonyl}propyl)-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 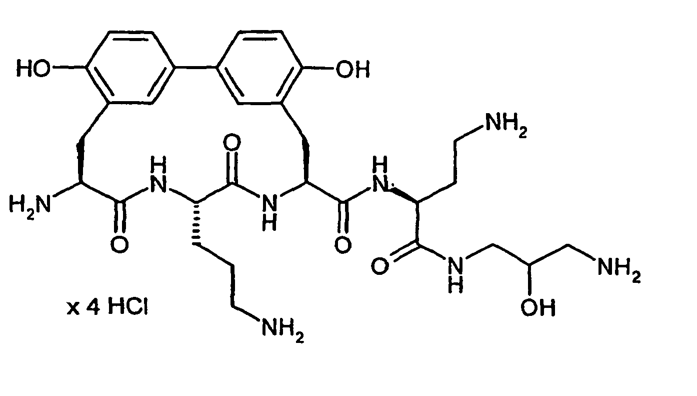 9 mg (0.01 mmol) der Verbindung aus Beispiel 173A werden auf 0°C gekühlt und mit 1 ml 4N Chlorwasserstoff in Dioxan versetzt. Nach 1 h engt man im Vakuum ein. Der Rückstand wird in Acetonitril aufgenommen und erneut eingeengt.
Ausbeute: 7 mg (98% d. Th.).
LC-MS (Methode 20): Rₜ = 0.95 min.
MS (EI): m/z = 629 (M-4HCl+H)⁺.

### Beispiel 6

(8*S*,11*S*,14*S*)-14-Amino-*N*-((1*S*,3*R*)-4-amino-1-{[(2-aminoethyl)amino]carbonyl}-3-hydroxybutyl)-11-[3-aminopropyl]-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 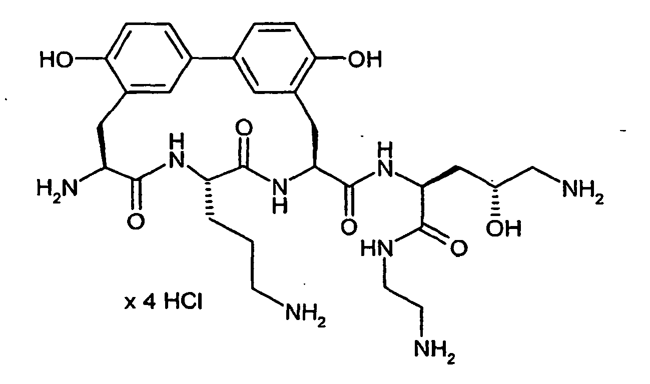 8 mg (0.01 mmol) der Verbindung aus Beispiel 175A werden bei 0°C mit 0.5 ml 4N Chlorwasserstoff in Dioxan versetzt. Man läßt auf Raumtemperatur erwärmen und rührt 1 h nach. Anschließend engt man im Vakuum ein und trocknet den Rückstand im Hochvakuum. Man rührt mit Acetonitril aus, filtriert den ausgefallenen Niederschlag ab und erhält so die Titelverbindung als Feststoff.
Ausbeute: 7 mg (59% d. Th.).
LC-MS (Methode 20): Rₜ = 0.90 min.
MS (EI): m/z = 630 (M-4HCl+H)⁺

### Beispiel 7

(8*S*,11*S*,14*S*)-14-Amino-*N*-{(4*S*)-4-amino-6-[(2-aminoethyl)amino]-6-oxoheayl}-11-(3-aminopropyl)-9-ethyl-5,17-dihydroxy-10,13-dioxo-9,12-diazatricyclo[14.3.1.1^{2,6}]henicosa-1 (20),2(21),3,5,16,18-hexaen-8-carboxamidTetrahydrochlorid 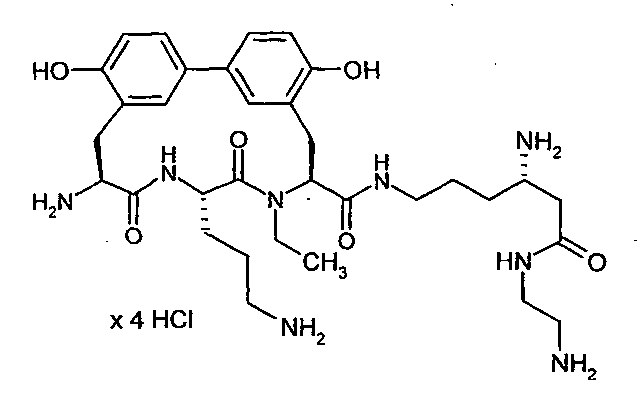 Zu einer Lösung von 7.5 mg (0.009 mmol) *tert*-Butyl-{3-[(8*S*,11*S*,14*S*)-8-[({(4*S*)-4-amino-6-[(2-aminoethyl)amino]-6-oxohexyl}amino)carbonyl]-14-[(*tert*-butoxycarbonyl)amino]-9-ethyl-5,17-dihydroxy-10,13-dioxo-9,12-diazatricyclo[14.3.1.1^{2,6}]henicosa-1(20),2(21),3,5,16,18-hexaen-11-yl]propyl}carbamat (Beispiel 204A) in 0.1 ml Dioxan werden bei 0°C 0.1 ml einer 4M Chlorwasserstoff-Dioxan-Lösung hinzugegeben. Nach 2 h bei RT wird die Reaktionslösung im Vakuum eingeengt, mehrmals mit Dichlormethan coevaporiert und im Hochvakuum getrocknet. Der Rückstand wird mit Diethylether versetzt und eingeengt.
Ausbeute: 4.9 mg (70% d. Th.)
¹H-NMR (400 MHz, D₂O): δ = 0.87 (m, 3H), 1.10-1.27 (m, 2H), 1.5-1.9 (m, 8H), 2.53-3.80 (m, 14H), 4.47 (m, 1H), 4.96 (m, 1H), 5.65 (m, 1H), 6.90 (d, 2H), 7.06 (s, 1H), 7.21 (s, 1H), 7.45 (d, 1M, 7.53 (d, 1H).

### Beispiel 8

(8*S*,11*S*,14*S*)-14-Amino-*N*-[(1*S*)-2-[(3-amino-2-hydroxypropyl)amino]-1-(hydroxymethyl)-2-oxoethyl]-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Trihydrochlorid 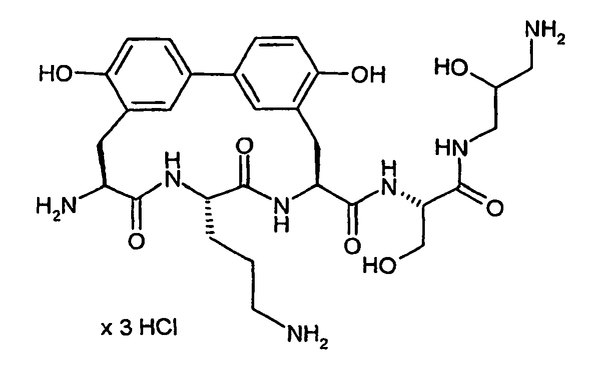 Eine Mischung von 12.7 mg (0.014 mmol) *tert*-Butyl-(3-{[(2*S*)-2-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.1^{2,6}] henicosa-1(20),2(21),3,5,16,18-hexaen-8-yl]carbonyl}amino)-3-hydroxy-propanoyl]amino}-2-hydroxypropyl)carbamat (Beispiel 188A) in 1 ml einer 4M Chlorwasserstoff-Dioxan-Lösung wird 20 min bei RT gerührt. Die Reaktionslösung wird eingeengt, mehrmals mit Dichlormethan coevaporiert und im Hochvakuum getrocknet.
Ausbeute: 9.1 mg (90% d.Th.)
LC-MS (Methode 20): Rₜ = 1.83 min.
MS (EI): m/z = 616 (M-3HCl+H)⁺
¹H-NMR (400 MHz, D₂O): δ = 1.56-1.90 (m, 4H), 2.78-3.82 (m, 14 H), 3.96 (m, 1H), 4.42 (m, 1H), 4.88 (m, 1H), 6.91 (d, 2H), 6.97 (s, 1H), 7.27 (s, 1H), 7.36 (d, 1H), 7.43 (d, 1H).

### Beispiel 9

(8*S*,11*S*,14*S*)-14-Amino-*N*-((1*S*)-4-amino-1-{2-[(2-aminoethyl)amino]-2-oxoethyl}butyl)-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 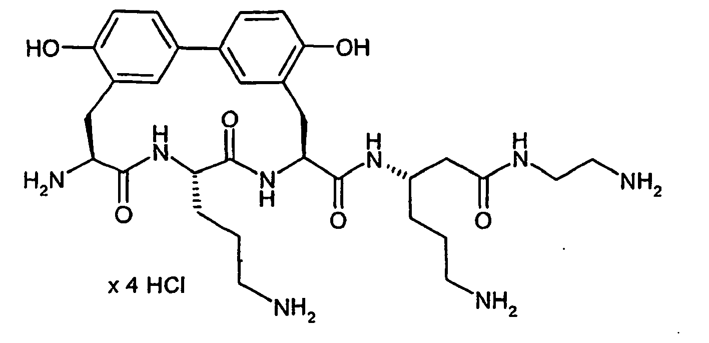 Eine Mischung von 30 mg (0.029 mmol) *tert*-Butyl-(2-{[(3*S*)-6-[(*tert*-butoxycarbonyl)amino]-3-({[(8*S*,11*S*,14*S*)-14-[(*tert*-butoxycarbonyl)amino]-11-{3-[(*tert*-butoxycarbonyl)amino]propyl}-5,17-dihydroxy-10,13-dioxo-9,12-diazatricyclo[14.3.1.^{2,6}]hecosa-1(20),2(21),3,5,16,18-hexaen-8-yl]carbonyl}amino)hexanoyl]amino}ethyl)carbamat (Beispiel 193A) in 2 ml einer 4M Chlorwasserstoff-Dioxan-Lösung wird 20 min bei RT gerührt. Die Reaktionslösung wird eingeengt, mehrmals mit Dichlormethan coevaporiert und im Hochvakuum getrocknet.
Ausbeute: quant.
¹H-NMR (400 MHz, D₂O): δ = 1.5-2.0 (m, 8H), 2.18-3.53 (m, 20 H), 4.25 (m, 1H), 4.45 (m, 1H), 6.69 (d, 2H), 6.76 (s, 1H), 7.07 (s, 1H), 7.15 (s, 1H), 7.22 (d, 1H), 7.63 (s, 1H).

### Beispiel 10

(8*S*,11*S*,14*S*)-14-Amino-*N*-{(1*S*)-4-amino-1-[({(4*R*)-4-amino-6-[(2-aminoethyl)amino]-6-oxohexyl}amino)carbonyl]butyl}-11-(3-aminopropyl)-5,17-dihydroxy-10,13-dioxo-9,12-diazatricyclo[14.3.1.12,6]henicosa (20),2(21),3,5,16,18-hexaen-8-carboxamid Pentahydrochlorid 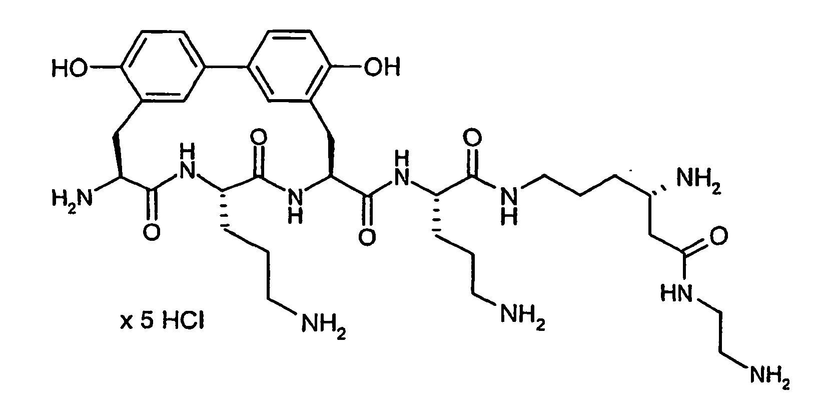 In 0.3 ml einer 4N Chlorwasserstoff Dioxan-Lösung gibt man 6 mg (0.006 mmol) *tert*-Butyl-{3-[(8*S*,11*S*,14*S*)-8-({[(1*S*)-4-amino-1-({[(4*S*)-4,8-diamino-6-oxooctyl]amino}carbonyl)butyl]amino}-carbonyl)-14-[(*tert*-butoxycarbonyl)amino]-5,17-dihydroxy-10,13-dioxo-9,12-diazatricyclo-[14.3.1.1^{2,6}]henicosa-1(20),2(21),3,5,16,18-hexaen-11-yl]propyl}carbamat Tris(hydroacetat) (Beispiel 205A) und rührt 30 min bei RT. Die Reaktionslösung wird eingeengt, mehrmals mit Dichlormethan coevaporiert und im Hochvakuum getrocknet.
Ausbeute: 5 mg (85% d. Th.)
¹H-NMR (400 MHz, D₂O): δ = 1.5-1.7 (m, 13H), 2.52-3.75 (m, 17H), 4.22 (m, 1H), 4.45 (m, 1H), 4.50 (m, 1H), 4.83 (m, 1H), 6.9 (d, 2H), 6.97 (s, 1H), 7.27 (s, 1H), 7.36 (d, 1H), 7.43 (d, 1H).

### Beispiel 11

(8*S*,11*S*,14*S*)-14-Amino-*N*-{(4*S*)-4-amino-6-[(2-aminoethyl)amino]-6-oxohexyl}-11-(3-amino-propyl)-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 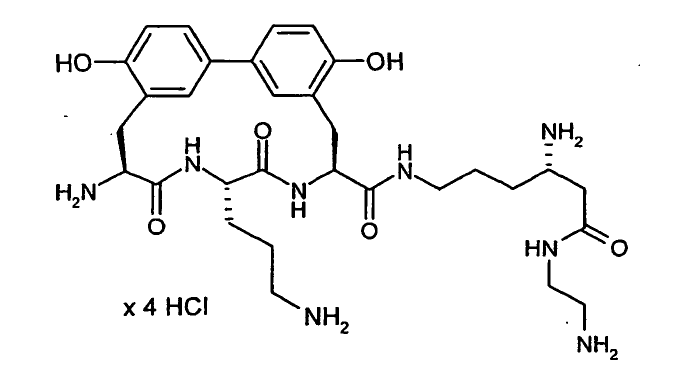 In 0.4 ml einer 4N Chlorwasserstoff-Dioxan-Lösung gibt man 8 mg (0.010 mmol) tert-Butyl-{3-[(8*S*,11*S*,14*S*)-8-[({(4*S*)-4-amino-6-[(2-aminoethyl)amino]-6-oxohexyl}amino)carbonyl]-14-[(*tert-*butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Bis(hydroacetat) (Beispiel 206A) und rührt 30 min bei RT. Die Reaktionslösung wird eingeengt, mehrmals mit Dichlormethan coevaporiert und im Hochvakuum getrocknet.
Ausbeute: quant.
LC-MS (Methode 12): Rₜ = 0.20 min.
MS (EI): m/z = 627 (M+-4HCl+H)⁺
¹H-NMR (400 MHz, D₂O): δ = 1.19 (m, 1H), 1.25 (m, 2H), 1.5-1-9 (m, 8H), 2.53-2.71 (m, 1H), 2.79-3.64 (m, 15H), 4.44 (m, 1H), 6.90 (d, 2H), 6.97 (s, 1H), 7.27 (s, 1H), 7.36 (d, 1H), 7.43 (d, 1H).

### Beispiel 12

(8*S*,11*S*,14*S*)-14-Amino-*N*-((1*S*)-3-amino-1-{[(2-aminoethyl)amino]carbonyl}propyl)-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 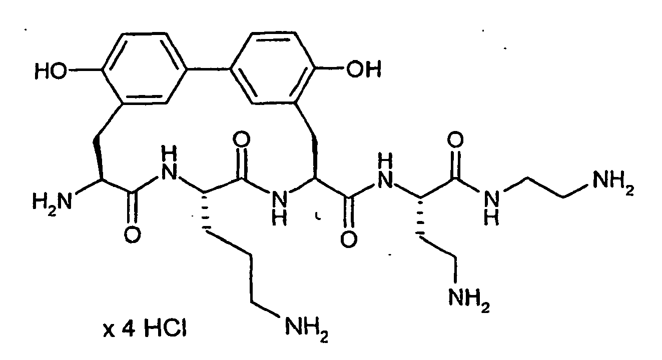 In 1 ml einer 4N Chlorwasserstoff-Dioxan-Lösung gibt man 15 mg (0.019 mmol) *tert*-Butyl-{3-[(8*S*,11*S*,14*S*)-8-{[((1*S*)-3-amino-1-{[(2-ammoethyl)amino]carbonyl}propyl)amino]carbonyl}-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Bis(hydroacetat) (Beispiel 207A) und rührt 20 min bei RT. Die Reaktionslösung wird eingeengt, mehrmals mit Dichlormethan coevaporiert und im Hochvakuum getrocknet.
Ausbeute: 12 mg (86% d. Th.)
¹H-NMR (400 MHz, D₂O): δ = 1.6-2.0 (m, 5H), 2.07-2.30 (m, 2H), 2.84-3.23 (m, 10H), 3.33 (m, 1H), 3.51-3.83 (m, 4H), 4.50 (m, 1H), 4.90 (m, 1H), 6.98 (d, 2H), 7.06 (s, 1H), 7.35 (s, 1H), 7.45 (d, 1H), 7.52 (d, 1H).

### Beispiel 13

(8*S*,11*S*,14*S*)-14-Amino-*N*-((1*S*)-4-amino-1-{[((1*S*)-3-amino-1-{[(2-aminoethyl)amino]carbonyl}-propyl)amino]carbonyl}butyl)-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Pentahydrochlorid 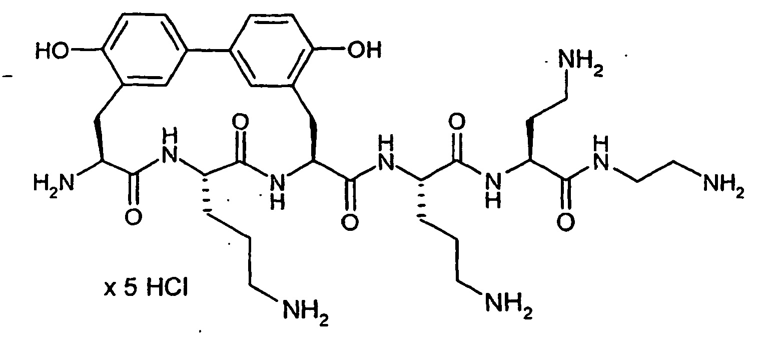 In 0.6 ml einer 4N Chlorwasserstoff-Dioxan-Lösung gibt man 7 mg (0.008 mmol) *tert*-Butyl{3-[(8*S*,11*S*,14*S*)-8-{[((1*S*)-4-amino-1-{[((1*S*)-amino-1-{[(2-aminoethyl)amino]carbonyl}propyl)-amino]carbonyl}-8-butyl)amino]carbonyl}-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Tris(hydroacetat) (Beispiel 208A) und rührt 30 min bei RT. Die Reaktionslösung wird eingeengt, mehrmals mit Dichlormethan coevaporiert und im Hochvakuum getrocknet.
Ausbeute: 6.1 mg (89% d. Th.)
¹H-NMR (400 MHz, D₂O): δ = 1.5-2.3 (m, 11H), 2.80-3.70 (m, 14H), 4.29-4.48 (m, 3H), 4.85 (m, 1H), 6.91 (d, 2H), 6.97 (s, 1H), 7.27 (s, 1H), 7.36 (d, 1H), 7.44 (d, 1H).

### Beispiel 14

(8*S*,11*S*,14*S*)-14-Amino-*N*-((1*S*)-1-{[(2-aminoethyl)amino]carbonyl}-4-{[amino(imino)methyl]-amino}-butyl)-11-(3-aminopropyl)-5,17-dihydroxy-10,13-dioxo-9,12-diazatricyclo[14.3.1.1^{2,6}]-henicosa-1(20),2(21),3,5,16,18-hexaen-8-carboxamid Pentahydrochlorid 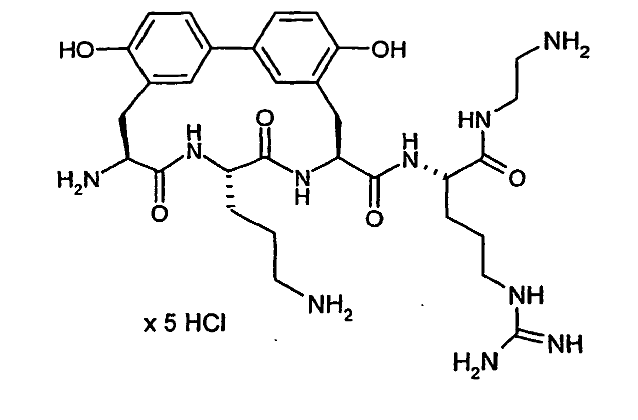 In 0.4 ml einer 4N Chlorwasserstoff-Dioxan-Lösung gibt man 5.3 mg (0.005 mmol) *tert*-Butyl-{3-[(8*S*,11*S*,14*S*)-8-{[((1*S*)-1-{[(2-aminoethyl)amino]carbonyl}-4-{[amino(imino)methyl]amino}-butyl)amino]carbonyl}-14-[(*tert*-butoxycarbonyl)amino]-5,17-dihydroxy-10,13-dioxo-9,12-diazatricyclo[14.3.1.1^{2,6}]henicosa-1(20),2(21),3,5,16,18-hexaen-11-yl]propyl}carbamat Di(hydrotrifluoracetat) (Beispiel 209A) und rührt 30 min bei RT. Die Reaktionslösung wird eingeengt, mehrmals mit Dichlormethan coevaporiert und im Hochvakuum getrocknet.
Ausbeute: quant.
LC-MS (Methode 20): Rₜ = 1.75 min.
MS (EI): m/z = 655 (M-5HCl+H)⁺
¹HER (400 MHz, D₂O): δ = 1.5-1.9 (m, 8H), 2.81-3.75 (m, 13H), 4.25 (m, 1H), 4.44 (m, 1H), 4.84 (m, 1H), 6.86 (d, 1H), 6.90 (d, 2H), 6.97 (s, 1H), 7.28 (s, 1H), 7.36 (d, 1H), 7.45 (m, 2H).

### Beispiel 15

*N²*-{[(8*S*,11*S*,14*S*)-14-Amino-11-(3-aminopropyl)-5,17-dihydroxy-10,13-dioxo-9,12-diazatricyclo[14.3.1.1^{2,6}]henicosa-1(20),2(21),3,5,16,18-hexaen-8-yl]carbonyl}-*N¹*-[(2*S*)-2,5-diaminopentyl]-*L*-glutamamid Tetrahydrochlorid 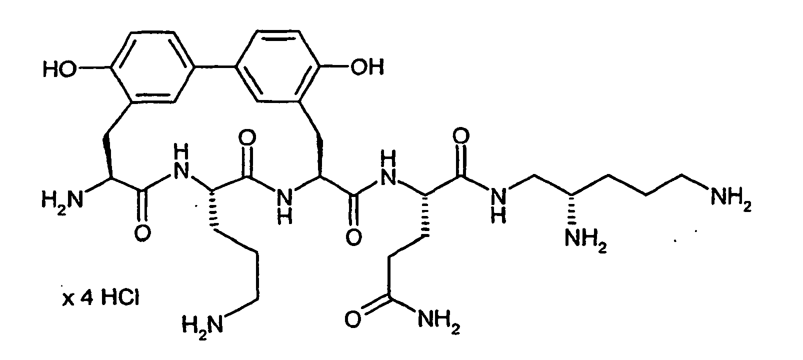 7 mg (0.01 mmol) der Verbindung aus Beispiel 180A werden mit 0.1 ml 4N Chlorwasserstoff in Dioxan versetzt. Man rührt 4 h bei RT. Anschließend engt man im Vakuum ein und trocknet den Rückstand im Hochvakuum.
Ausbeute: 4.2 mg (78% d. Th.).
M*S* (ESI): m/z = 684 (M-4HCl+H)⁺.

### Beispiel 16

(8*S*,11*S*,14*S*)-14-Amino-*N*-[(1*S*)-4-amino-1-({[(2*S*)-2,5-diaminopentyl]amino}carbonyl)butyl]-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 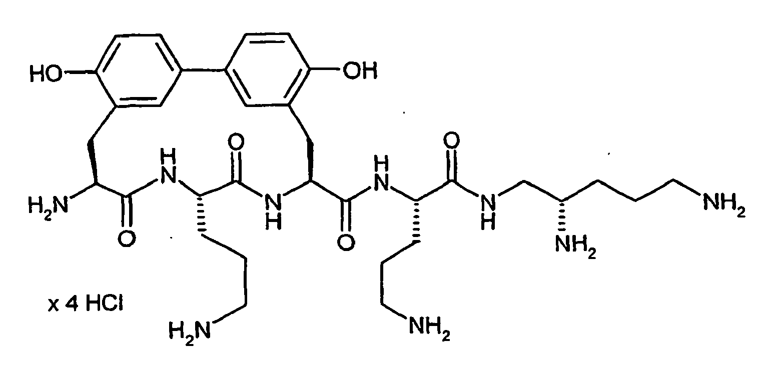 40 mg (0.034 mmol) der Verbindung aus Beispiel 177A werden bei 0°C in 1 ml Dioxan gelöst. Anschliessend werden 0.5 ml 4N Chlorwasserstofflösung in Dioxan hinzugegeben und 2 h bei RT gerührt. Der Ansatz wird im Vakuum zur Trockne eingedampft und der Rückstand im Hochvakuum bis zur Gewichtskonstanz getrocknet.
Ausbeute: 26 mg (88% d. Th.).
MS (ESI): m/z = 670 (M-4HCl+H)⁺.
¹H-NMR (400 MHz, D₂O): δ = 1.45-1.95 (m, 12H), 2.8-3.05 (m, 6H), 3.1-3.4 (m, 4H), 3.42 (m_{c}, 2H), 3.54 (m_{c}, 1H), 3.97 (m_{c}, 1H), 4.41 (m, 1H), 4.6-4.8 (m, 2H, unter D₂O), 6.83-6.9 (m, 2H), 6.95 (s, 1H), 7.24 (s, 1H), 7.32 (d, 1H), 7.4 (d, 1H).

### Beispiel 17

(8*S*,11*S*,14*S*)-14-Amino-11-(3-aminopropyl)-*N*-(2-{[(2*S*)-2,5-diaminopentyl]amino}-2-oxoethyl)-5,17-dihydroxy-10,13-dioxo-9,12-diazatricyclo[14.3.1.1^{2,6}]henicosa-1(20),2(21),3,5,16,18-hexaen-8-carboxainid Tetrahydrochlorid 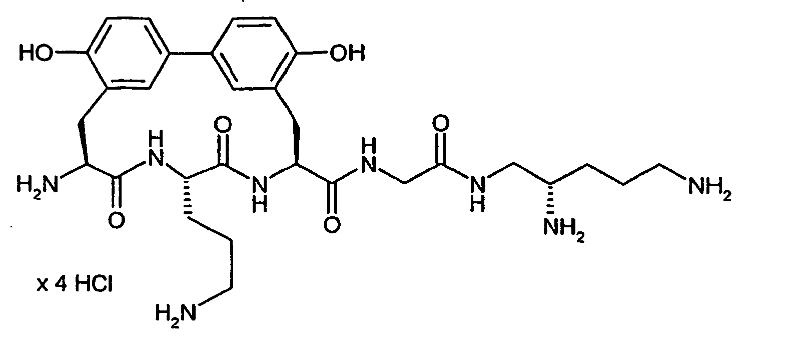 11.5 mg (0.011 mmol) der Verbindung aus Beispiel 178A werden bei 0°C in 1 ml Dioxan gelöst. Anschliessend werden 0.2 ml 4N Chlorwasserstofflösung in Dioxan hinzugegeben und 2 h bei RT gerührt. Der Ansatz wird im Vakuum zur Trockne eingedampft und der Rückstand im Hochvakuum bis zur Gewichtskonstanz getrocknet.
Ausbeute: 8.5 mg (99% d. Th.).
MS (ESI): m/z = 613 (M-4HCl+H)⁺.
¹H-NMR (400 MHz, D₂O): δ = 1.5-1.9 (m, 8H), 2.75-3.05 (m, 6H), 3.24 (mm, 1H), 3.3-3.43 (m, 2H), 3.45-3.55 (m, 2H), 3.87-3.97 (m, 2H), 4.41 (m_{c},1H), 4.7 (m, 1H, unter D₂O) 4.83 (m_{c}, 1H), 6.83-6.9 (m, 2H), 6.95 (s, 1H), 7.23 (s, 1H), 7.3 (d, 1H), 7.4 (d, 1H).

### Beispiel 18

(8*S*,11*S*,14*S*)-14-Amino-11-(3-aminopropyl)-*N*-[(1*S*)-2-{[(2*S*)-2,5-diaminopentyl]amino}-1-(hydroxymethyl)-2-oxoethyl]-5,17-dihydroxy-10,13-dioxo-9,12-diazatricyclo[14.3.1.1^{2,6}]henicosa-1(20),2(21),3,5,16,18-hexaene-8-carboxamid Tetrahydrochlorid 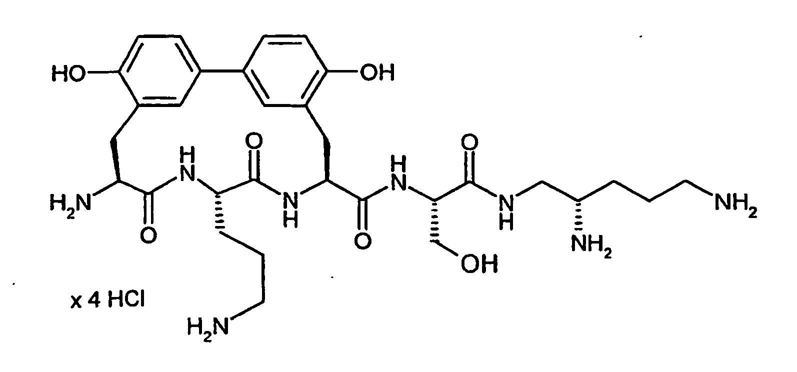 18.4 mg (0.018 mmol) der Verbindung aus Beispiel 179A werden bei 0°C in 1 ml Dioxan gelöst. Anschliessend werden 0.26 ml 4N Chlorwasserstofflösung in Dioxan hinzugegeben und 4 h bei RT gerührt. Der Ansatz wird im Vakuum zur Trockne eingedampft und der Rückstand im Hochvakuum bis zur Gewichtskonstanz getrocknet.
Ausbeute: 13 mg (93% d. Th.).
MS (ESI): m/z = 643 (M-4HCl+H)⁺.
¹H-NMR (400 MHz, D₂O): δ = 1.4-1.85 (m, 8H), 2.75-3.05 (m, 6H), 3.26 (m_{c}, 1H), 3.35-3.85 (m, 7H), 4.41 (m_{c}, 1H), 4.7 (m, 1H, unter D₂O), 4.87 (m_{c}, 1H), 6.85-6.92 (m, 2H), 6.96 (s, 1H), 7.22 (s, 1H), 7.32 (d, 1H), 7.4 (d, 1H).

### Beispiel 19

*N²*-{[(8*S*,11*S*,14*S*)-14-Amino-11-(3-aminopropyl)-5,17-dihydroxy-10,13-dioxo-9,12-diazatricyclo[14.3.1.1^{2,6}]henicosa-1(20),2(21),3,5,16,18-hexaen-8-yl]carbonyl}-*L*-ornithyl-*N*-(2-aminoethyl)-*L*-ornithinamid Pentahydrochlorid 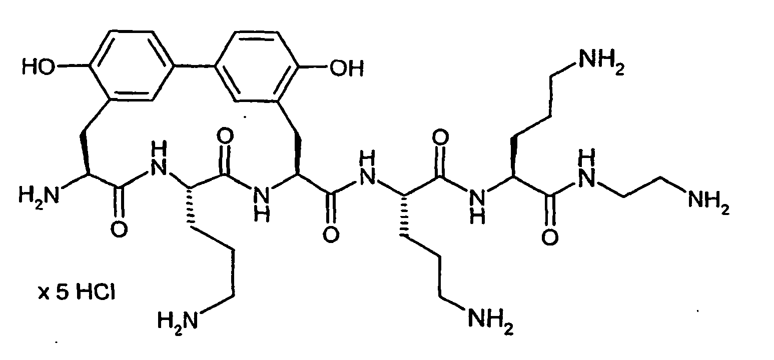 8.5 mg (0.008 mmol) der Verbindung aus Beispiel 210A werden mit 0.2 ml 4N Chlorwasserstofflösung in Dioxan versetzt und 3 h bei RT gerührt. Der Ansatz wird im Vakuum zur Trockne eingedampft und der Rückstand im Hochvakuum bis zur Gewichtskonstanz getrocknet.
Ausbeute: quant.
MS (ESI): m/z = 727 (M-5HCl+H)⁺.
¹H-NMR (400 MHz, D₂O): δ = 1.55-1.95 (m, 12H), 2.75-3.15 (m, 10H), 3.25 (m_{c}, 1H), 3.35-3.75 (m, 3H), 4.25-4.35 (m, 2H), 4.41 (m_{c}, 1H), 4.7-4.9 (m, 2H, unter D₂O), 6.91 (m_{c}, 2H), 6.98 (s, 1H), 7.28 (s, 1H), 7.38 (d, 1H), 7.45 (d, 1H).

Analog zur Vorschrift des Beispiels 1 werden die in der folgenden Tabelle aufgeführten Beispiele 20 bis 39 hergestellt, entsprechend der jeweiligen Isolierungsmethode als Hydrochlorid- oder Hydrotrifluoracetat-Salz.

| **Beispiel- Nr.** | **Vorstufe Beispiel** | **Struktur** | **Analytische Daten** |
|---|---|---|---|
| **20** | 221A | 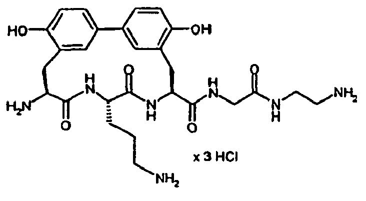 | LC-MS (Methode 20): Rₜ = 1.87 min. MS (ESI): m/z = 556 (M-3HCl+H)⁺. ¹H-NMR (400 MHz, D₂O): δ = 1.55-1.90 (m, 4H), 2.8-2.97 (m, 3H), 3.0-3.15 (m, 3H), 3.27 (m_{c}, 1H), 3.45-3.75 (m, 5H), 4.40 (mg, 1H), 4.8 (m_{c}, 1H, unter D₂O), 4.72 (m_{c}, 1H), 6.8-6.9 (m, 2H), 6.94 (s, 1H), 7.23 (s, 1H), 7.34 (d, 1H), 7.42 (d, 1H). |
| **21** | 184A | 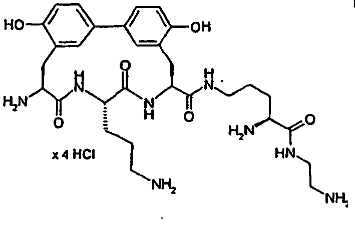 | MS (ESI): m/z= 613 (M-4HCl+H)⁺ ¹H-NMR (400 MHz, D₂O): δ = 1.45- 1.95 (m, 6H), 2.82-3.2 (m, 8H), 3.25-3.45 (m, 2H), 3.47-3.75 (m, 4H), 3.94 (m_{c}, 1H), 4.42 (m_{c}, 1H), 4.68 (m, 1H, unter D₂O), 4.77 (m_{c}, 1H), 6.83-6.92 (m, 2H), 6.97 (s, 1H); 7.25 (s, 1H), 7.36 (d, 1H), 7.43 (d, 1H). |
| **22** | 185A | 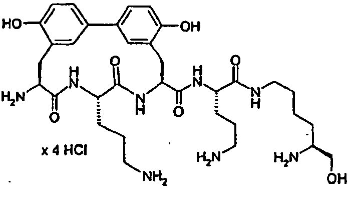 | LC-MS (Methode 20): Rₜ = 1.83 min. MS (ESI): m/z = 685 (M-4HCl+H)⁺. ¹H-NMR (400 MHz, D₂O): δ = 1.2-1.9 (m, 14H), 2.7-3.1 (m, 5H), 3.1-3.3 (m, 4H), 3.5-3.6 (m, 2H), 3.72 (m_{c}, 2H), 3.88 (m_{c}, 2H), 4.18 (m_{c}, 1H), 4.41 (m_{c}, 1H), 4.78 (m_{c}, 1H), 6.83-6.92 (m, 2H), 6.97 (s, 1H), 7.25 (s, 1H), 7.36 (d, 1H), 7.43 (d, 1H). |
| **23** | 186A | 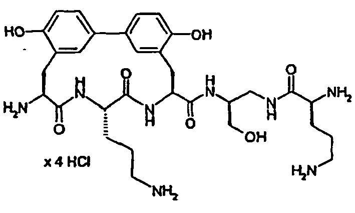 | MS (ESI): m/z = 643 (M-4HCl+H)⁺. ¹H-NMR (400 MHz, D₂O): δ = 1.5-2.0 (m, 8H), 2.7-3.1 (m, 6H), 3.22 (m, 1H), 3.3-3.8 (m, 5H), 3.9-4.05 (m, 2H), 4.4 (m_{c}, 1H), 4.6-4.8 (m, 2H unter D₂O), 6.8-6.93 (m, 2H), 6.96 (s, 1H), 7.23 (s, 1H), 7.34 (d, 1H), 7.42 (d, 1H). |
| **24** | 187A | 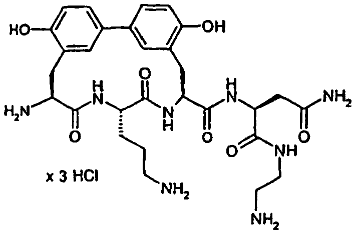 | LC-MS (Methode 12): Rₜ = 0.238 min. MS (EI): m/z = 613 (M-3HCl+H)⁺ |
| **25** | 216A | 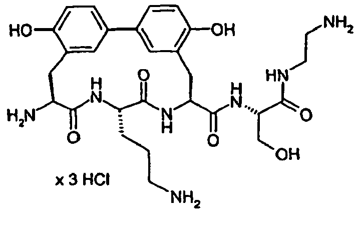 | LC-MS (Methode 20): Rₜ = 2.17 min. MS (EI): m/z = 586 (M-3HCl+H)⁺ |
| **26** | 217A | 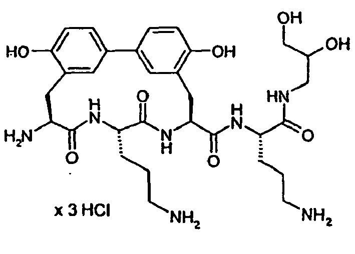 | LC-MS (Methode 20): Rₜ = 2.03 min. MS (EI): m/z= 644 (M-3HCl+H)⁺ ¹H-NMR (400 MHz, D₂O): δ = 1.6-1.7 (m, 7H), 2.80-3.78 (m, 15H), 4.24 (m, 1H), 4.43 (m, 1H), 4.80 (m, 1H), 6.90 (d, 2H), 6-97 (s, 1H), 7.28 (s, 1H), 7.36 (d, 1H), 7.43 (d, 1H). |
| **27** | 218A | 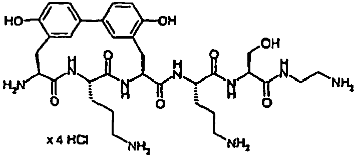 | LC-MS (Methode 17): Rₜ = 0.24 min. MS (EI): m/z = 700 (M-4HCl+H)⁺ ¹H-NMR (400 MHz, D₂O): δ = 1.5-1.9 (m, 9H), 2.81-3.83 (m, 20H), 4.32-4.39 (m, 2H), 4.44 (m, 1H), 4.84 (m, 1H), 6.90 (d, 2H), 6.97 (s, 1H), 7.28 (s, 1H), 7.36 (d, 1H), 7.44 (d, 1H). |
| **28** | 219A | 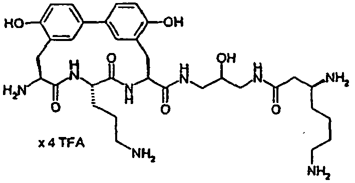 | LC-MS (Methode 20): Rₜ = 1.82 min. MS (EI): m/z = 671 (M-4TFA+H)⁺ |
| **29** | 195A | 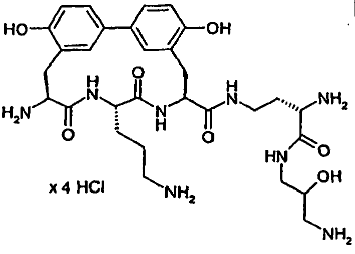 | ¹H-NMR (400 MHz, D₂O): δ = 1.58-1.89 (m, 4H), 2.02-2.16 (m, 2H), 2.80-3.75 (m, 15H), 3,97 (m, 2H), 4.44 (m, 1H), 6.90 (d, 2H), 6.98 (s, 1H), 7.26 (s, 1H), 7.37 (d, 1H), 7.44 (d, 1H). |
| **30** | 196A | 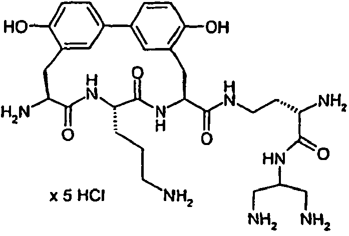 | LC-MS (Methode 20): Rₜ = 0.45 min. MS (EI): m/z = 628 (M-5HCl+H)⁺ ¹H-NMR (400 MHz, D₂O): δ = 1.57- 1.87 (m, 4H), 2.09 (m, 1H), 2.20 ( m, 1H), 2.85-3.76 (m, 15H), 4.01 (m, 1H), 4.45 (s, 1H), 4.50 (m, 1H), 6.90 (d, 2H), 6.98 (s, 1H), 7.26 (s, 1H), 7.37 (d, 1H), 7.44 (d, 1H). |
| **31** | 220A | 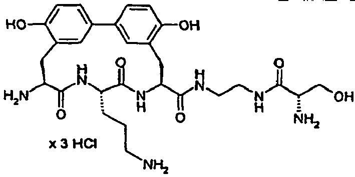 | LC-MS (Methode 20): Rₜ = 1.80 min. MS (EI): m/z = 585 (M-3HCl+H)⁺ ¹H-NMR (400 MHz, D₂O): δ = 1.53- 1.89 (m, 4H), 2.81-3.46 (m, 10H), 3.56 (m, 1H), 3.67 (s, 2H), 3.84-3.93 (m, 2H), 4.05 (m, 1H), 4.44 (m, 1H), 6.90 (d, 2H), 6.97 (s, 1H), 7.27 (s, 1H), 7.36 (d, 1H), 7.43 (d, 1H). |
| **32** | 182A | 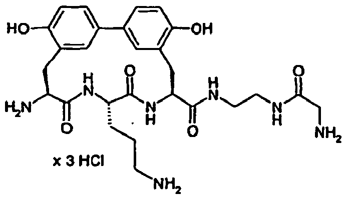 | LC-MS (Methode 20): Rₜ = 1.68 min. MS (EI): mlz= 556 (M-3HCl+H)⁺ ¹H-NMR (400 MHz, D₂O): = 1.55- 1.85 (m, 4H), 2.81-3.05 (m, 4H), 3.15-3.7 (m, 9H), 4.42 (m_{c}, 1H), 4.6-4.8 (m, 2H, unter D₂O), 6.88 (d, 2H), 6.95 (s, 1H), 7.26 (s, 1H), 7.35 (d, 1H), 7.41 (d, 1H). |
| **33** | 199A | 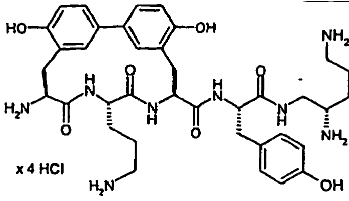 | MS (ESI): m/z = 719 (M-4HCl+H)⁺ ¹H-NMR (400 MHz, D₂O): δ = 1.45- 1.75 (m, 8H), 2.7-3.05 (m, 6H), 3.15 (mm, 1H), 3.26 (mm, 1H), 3.45 (m_{c}, 2H), 3.55 (m_{c}, 1H), 4.42 (m_{c}, 1H), 4.48 (mm, 1H), 4.6-4.8 (m, 2H, unter D₂O), 6.75 (d, 2H), 6.88 (d, 2H), 6.94 (s, 1H), 7.07 (d, 2H), 7.2 (s, 1H), 7.33 (d, 1H), 7.42 (d, 1H). |
| **34** | 197A | 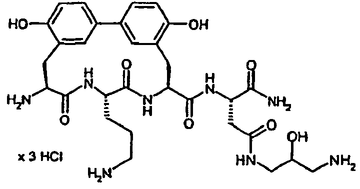 | LC-MS (Methode 20): Rₜ = 1.72 min. MS (EI): m/z = 643 (M-3HCl+H)⁺ ¹H-NMR (400 MHz, D₂O): δ = 1.55- 1.85 (m, 4H), 2.6-3.35 (m, 11 H), 3.5-3.75 (m, 2H), 3.92 (m_{c}, 1H), 4.41 (m_{c}, 1H), 4.48 (m_{c}, 1H), 4.7 (m, 1H, unter D₂O), 4.78 (d, 1H), 6.87 (d, 2H), 6.93 (s, 1H), 7.23 (s, 1H), 7.32 (d, 1H), 7.4 (d, 1H). |
| **35** | 198A | 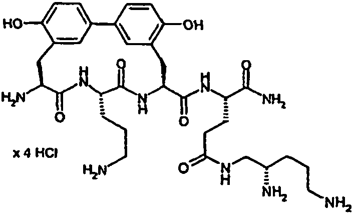 | MS (ESI): m/z = 684 (M-4HCl+H)⁺ |
| **36** | 200A | 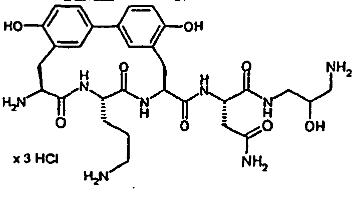 | LC-MS (Methode 20): Rₜ =1.74 min. MS (EI): m/z = 643 (M-3HCl+H)⁺ ¹H-NMR (400 MHz, D₂O): δ = 1.45-1.85 (m, 4H), 2.65-3.1 (m, 7H), 3.2-3.4 (m, 4H), 3.5-3.75 (m, 2H), 3.92 (m_{c}, 1H), 4.41 (m_{c}, 1H), 4.48 (m_{c}, 1H), 4.7 (m, 1H, unter D₂O), 4.78 (d, 1H), 6.87 (d, 2H), 6.93 (s, 1H), 7.23 (s, 1H), 7.32 (d, 1H), 7.4 (d, 1H). |
| **37** | 201A | 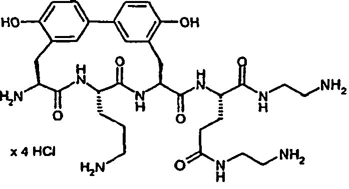 | MS (ESI): m/z = 670 (M-4HCl+H)⁺ |
| **38** | 202A | 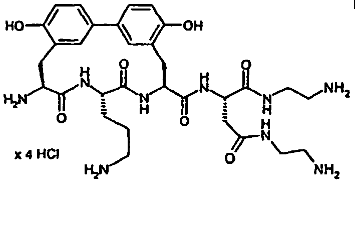 | MS (ESI): m/z = 656 (M-4HCl+H)⁺ |
| **39** | 203A | 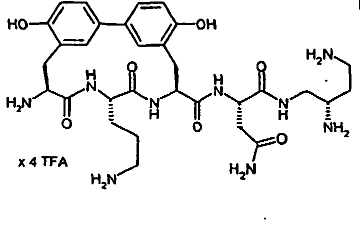 | MS (ESI): m/z = 656 (M-4TFA+H)⁺ |

### Beispiel 40

(8*S,*11*S,*14*S*)-14-Amino-*N-*((4*S*)-4-amino-6-{[(2*S*)-2,5-diaminopentyl]amino}-6-oxohexyl)-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Pentahydrochlorid 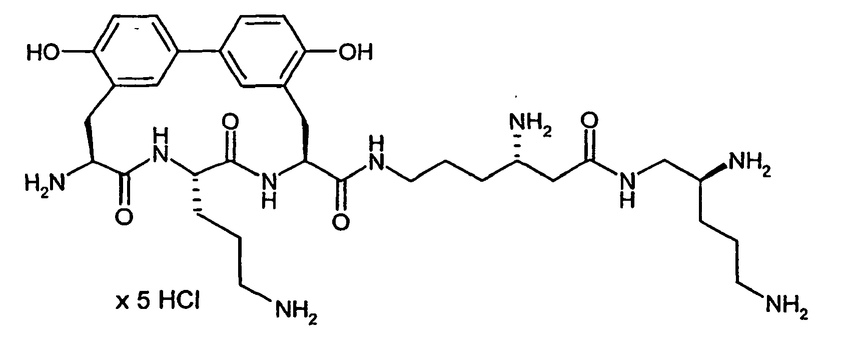 Zu einer Lösung von 25 mg (0.021 mmol) der Verbindung aus Beispiel 262A in 1 ml Dioxan werden bei 0°C 0.32 ml einer 4N Chlorwasserstoff-Dioxan-Lösung hinzugegeben. Nach 2 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 17.8 mg (96% d. Th.)
MS (ESI): m/z = 684 (M-5HCl+H)⁺.
¹H-NMR (400 MHz, D₂O): δ = 1.4-1.9 (m, 12H), 2.5-3.2 (m, 10H), 3.3-3.7 (m, 6H), 4.41 (m_{c}, 1H), 4.7-4-9 (m, 2H, unter D₂O), 6.85-6.92 (m, 2H), 6.96 (s, 1H), 7.28 (s, 1H), 7.38 (d, 1H), 7.44 (d, 1H).

### Beispiel 41

(8*S,*11*S,*14*S*)-14-Amino-*N-*((1*S*)-1-(aminomethyl)-2-{[(2*S*)-2,5-diaminopentyl]amino}-2-oxoethyl)-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Pentahydrochlorid 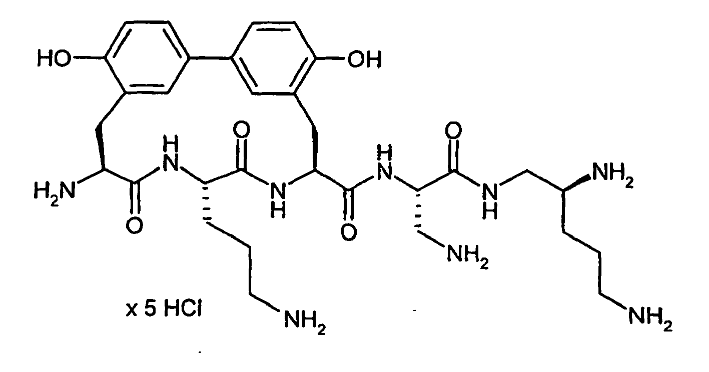 Zu einer Lösung von 47 mg (0.021 mmol) der Verbindung aus Beispiel 269A in 1 ml Dioxan werden bei 0°C 0.62 ml einer 4N Chlorwasserstoff-Dioxan-Lösung hinzugegeben. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 33 mg (98% d. Th.)
MS (ESI): m/z = 642 (M-5HCl+H)⁺.
¹H-NMR (400 MHz, D₂O): δ = 1.5-1.9 (m, 8H), 2.8-3.1 (m, 6H), 3.2-3.7 (m, 8H), 4.41 (m_{c}, 1H), 4.7-4.9 (m, 2H, unter D₂O), 6.91 (m_{c}, 2H), 7.0 (s, 1H), 7.27 (s, 1H), 7.38 (d, 1H), 7.45 (d, 1H).

### Beispiel 42

(8*S,*11*S,*14*S*)-14-Amino-*N-*[(1*S*)-4-amino-1-(2-{[(2*S*)-2,5-diaminopentyl]amino}-2-oxoethyl)-butyl]-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Pentahydrochlorid 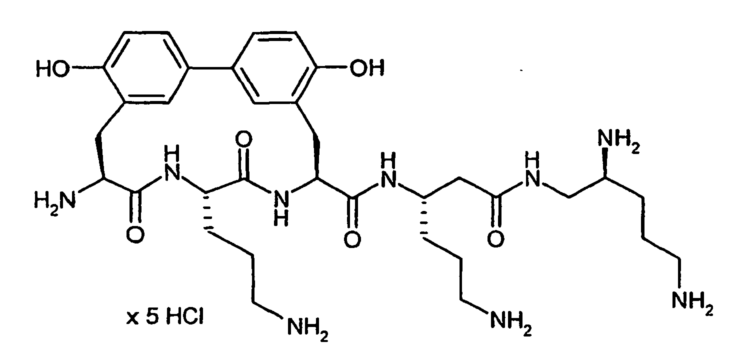 Zu einer Lösung von 17 mg (0.014 mmol) der Verbindung aus Beispiel 270A in 1 ml Dioxan werden bei 0°C 0.22 ml einer 4N Chlorwasserstoff-Dioxan-Lösung hinzugegeben. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 12 mg (99% d. Th.)
MS (ESI): m/z = 684 (M-5HCl+H)⁺.
¹H-NMR (400 MHz, D₂O): δ = 1.4-1.9 (m, 12H), 2.37 (m_{c}, 1H), 2.55 (m_{c}, 1H), 2.7-3.2 (m, 7H), 3.2-3.7 (m, 6H), 4.22 (m_{c}, 1H), 4.42 (m_{c}, 1H), 4.7-4.9 (m, 2H, unter D₂O), 6.89 (m_{c}, 2H), 6.96 (s, 1H), 7.27 (s, 1H), 7.36 (d, 1H), 7.42 (d, 1H).

### Beispiel 43

(8*S,*11*S,*14*S*)-14-Amino-*N-*((1*S*)-4-amino-1-{[(2-aminoethyl)amino]carbonyl}butyl)-11-[(2R)-3-amino-2-hydroxypropyl]-5,17-dihydroxy-10,13-dioxo-9,12-diazatricylo[14.3.1.1^{2,6}]henicosa-1(20),2(21),3,5,16,18-hexaen-8-carboxamid Tetrahydrochlorid 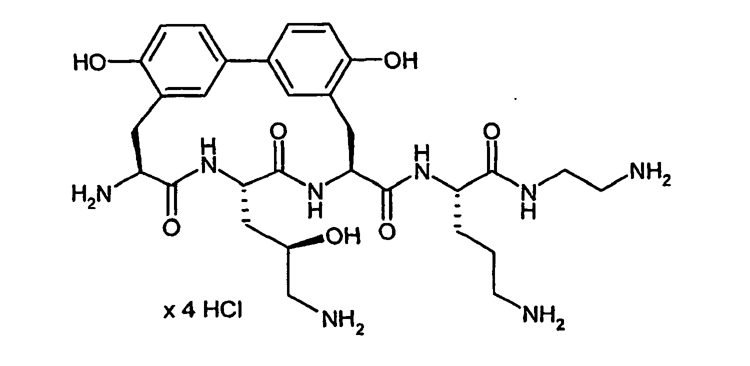 57 mg (0.014 mmol) der Verbindung aus Beispiel 271A werden bei 0°C mit 0.83 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 44 mg (99% d. Th.)
MS (ESI): m/z = 629 (M-4HCl+H)⁺.
¹H-NMR (400 MHz, D₂O): δ = 1.5-2.1 (m, 6H), 2.7-3.2 (m, 8H), 3.28 (m_{c}, 1H), 3.37-3.62 (m, 3H), 3.86 (m_{c}, 1H), 4.27 (m_{c}, 1H), 4.42 (m_{c}, 1H), 4.7-4.9 (m, 2H, unter D₂O), 6.91 (m_{c}, 2H), 6.99 (s, 1H), 7.29 (s, 1H), 7.38 (d, 1H), 7.44 (d, 1H).

### Beispiel 44

(8*S,*11*S,*14*S*)-14-Amino-*N-*[(1*S*)-4-amino-1-({[(3*S*)-3-amino-4-hydroxybutyl]amino}carbonyl)-butyl]-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 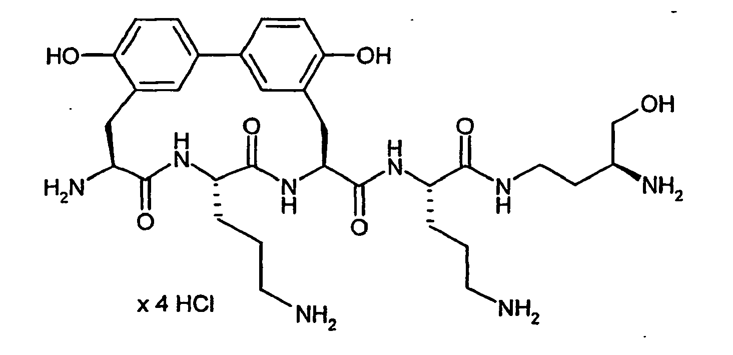 19 mg (0.018 mmol) der Verbindung aus Beispiel 272A werden bei 0°C mit 0.27 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 14 mg (97% d. Th.)
MS (ESI): m/z = 657 (M-4HCl+H)⁺.
¹H-NMR (400 MHz, D₂O): δ = 1.55-1.95 (m, 10H), 2.75-3.1 (m, 4H), 3.2-3.4 (m, 4H), 3.5-3.6 (m, 2H), 3.7-3.8 (m, 2H), 3.93 (m_{c}, 1H), 4.21 (m_{c}, 1H), 4.45 (m_{c}, 1H), 4.7-4.9 (m, 2H, unter D₂O), 6.91 (m_{c}, 2H), 6.98 (s, 1H), 7.28 (s, 1H), 7.38 (d, 1H), 7.44 (d, 1H).

### Beispiel 45

(8*S,*11*S,*14*S*)-14-Amino-*N-*{1-(2-aminoethyl)-3-[(2-aminoethyl)amino]-3-oxopropyl}-11-(3-aminopropyl)-5,17-dihydroxy-10,13-dioxo-9,12-diazatricyclo[14.3.1.1.^{2,6}]henicosa-1(20),2(21),3,5,16,18-hexaen-8-carboxamid Tetrahydrochlorid 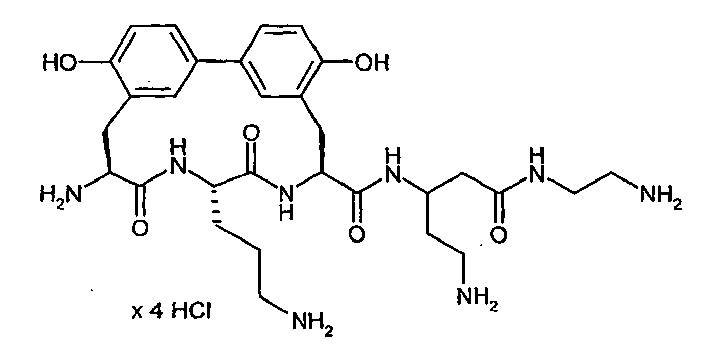 Zu einer Lösung von 28 mg (0.028 mmol) der Verbindung aus Beispiel 273A in 1 ml Dioxan werden bei 0°C mit 0.41 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 12 mg (58% d. Th.)
MS (ESI): m/z = 627 (M-4HCl+H)⁺.
¹H-NMR (400 MHz, D₂O): δ = 1.5-2.0 (m; 6H), 2.3-2.65 (m, 2H), 2.7-3.2 (m, 8H), 3.3-3.8 (m, 4H), 4.28 (m_{c}, 1H), 4.42 (m_{c}, 1H), 4.7-4.9 (m, 2H, unter D₂O), 6.90 (m_{c}, 2H), 6.96 (s, 1H), 7.27 (s, 1H), 7.36 (d, 1H), 7.43 (d, 1H).

### Beispiel 46

(8*S,*11*S,*14*S*)-14-Amino-11-(3-aminopropyl)-*N-*(3-{[(2*S*)-2,5-diaminopentyl]amino}-3-oxopropyl)-5,17-dihydroxy-10,13-dioxo-9,12-diazatricyclo[14.3.1.1^{2,6}]henicosa-1(20),2(21),3,5,16,18-hexzaen-8-carboxamid Tetrahydrochlorid 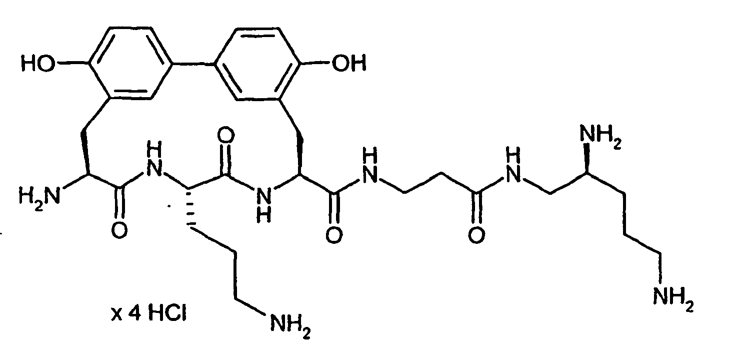 Zu einer Lösung von 20.7 mg (0.020 mmol) der Verbindung aus Beispiel 274A in 1 ml Dioxan werden bei 0°C mit 0.30 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 15 mg (98% d. Th.)
MS (ESI): m/z = 627 (M-4HCl+H)⁺.
¹H-NMR (400 MHz, D₂O): δ = 1.5-1.9 (m, 8H), 2.35-2.55 (m, 2H), 2.7-3.2 (m, 7H), 3.3-3.7 (m, 6H), 4.42 (m_{c}, 1H), 4.7-4.9 (m, 2H, unter D₂O), 6.90 (m_{c}, 2H), 6.96 (s, 1H), 7.25 (s, 1H), 7.35 (d, 1H), 7.42 (d, 1H).

### Beispiel 47

(8*S,*11*S,*14,*S*)-14-Amino-*N*-(1-(2-aminoethyl)-3-{[(2*S*)-2,5-diaminopentyl]amino}-3-oxopropyl)-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Pentahydrochlorid 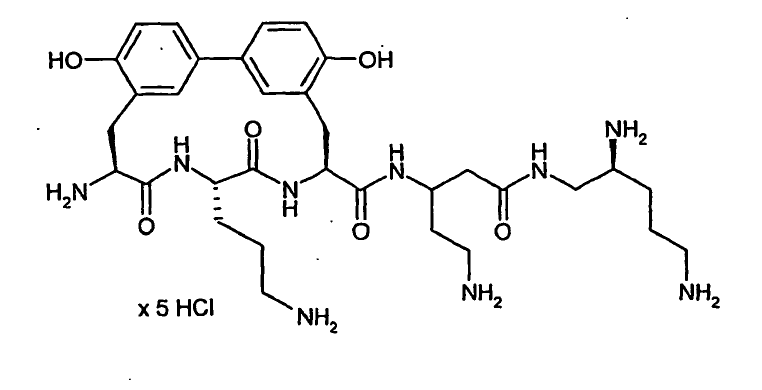 Zu einer Lösung von 27.7 mg (0.024 mmol) der Verbindung aus Beispiel 275A in 1 ml Dioxan werden bei 0°C mit 0.355 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 20 mg (99% d. Th.)
MS (ESI): m/z = 670 (M-5HCl+H)⁺.
¹H-NMR (400 MHz, D₂O): δ = 1.5-1.9 (m, 10H), 2.3-2.7 (m, 2H), 2.7-3.7 (m, 13H), 4.24 (m_{c}, 1H), 4.42 (m_{c}, 1H), 4.7-4.9 (m, 2H, unter D₂O), 6.90 (m_{c}, 2H), 6.96 (s, 1H), 7.26 (s, 1H), 7.36 (d, 1H), 7.43 (d, 1H).

### Beispiel 48

(8*S,*11*S,*14*S*)-14-Amino-*N-*[(1*S*)-4-amino-1-({[(4*S*)-4-amino-5-hydroxypentyl]amino}carbonyl)-butyl]-11-(3-aminopropyl)-5,17-dihydroxy-10,13-dioxo-9,12-diazatricyclo[14.3,1.1^{2,6}]henicosa-1(20),2(21),3,5,16,18-hexaen-8-carboxamid Tetrahydrochlorid 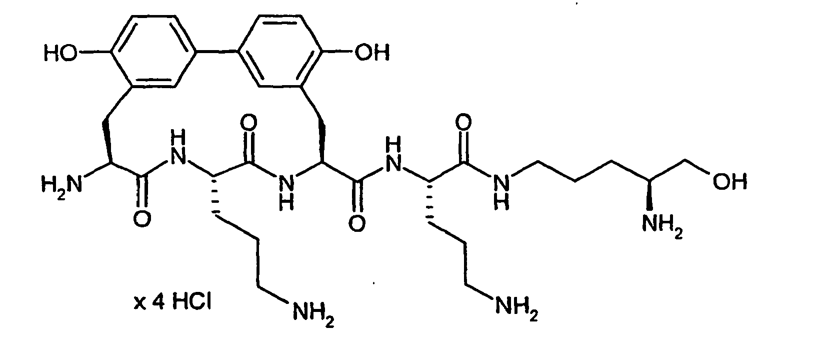 7.8 mg (0.007 mmol) der Verbindung aus Beispiel 276A werden bei 0°C mit 0.11 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 5.8 mg (99% d. Th.)
MS (ESI): m/z = 671 (M-4HCl+H)⁺.
¹H-NMR (400 MHz, D₂O): δ =1.4-1.95 (m, 12H), 2.75-3.4 (m, 10H), 3.5-3.8 (m, 3H), 4.20 (m_{c}, 1H), 4.44 (m_{c}, 1H), 4.7-4.9 (m, 2H, unter D₂O), 6.91 (m_{c}, 2H), 6.98 (s, 1H), 7.28 (s, 1H), 7.38 (d, 1H), 7.45 (d, 1H).

### Beispiel 49

(8*S,*11*S,*14*S*)-14-Amino-*N*-[(1*S*)-4-amino-1-({[(1*S*)-2-amino-1-(hydroxymethyl)ethyl]amino}-carbonyl)butyl]-11-(3-aminopropyl)-5,17-dihydroxy-10,13-dioxo-9,12-diazatricyclo-[14.3.1.1^{2,6}]henicosa-1(20),2(21),3,5,16,18-hexaen-8-carboxamid Tetrahydrochlorid 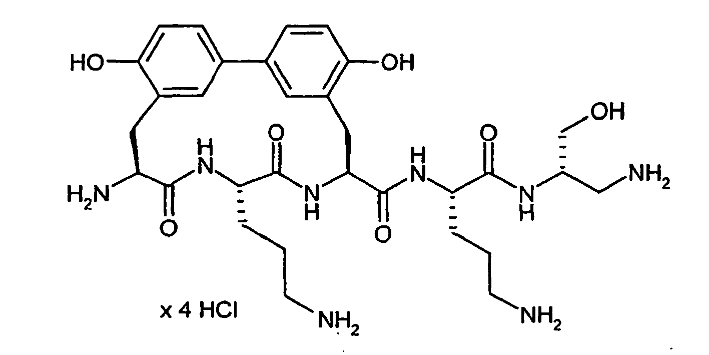 Eine Lösung von 39 mg (0.037 mmol) der Verbindung aus Beispiel 277A in 1 ml Dioxan wird bei 0°C mit 0.56 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 25 mg (85% d. Th.)
MS (ESI): m/z = 643 (M-4HCl+H)⁺.
¹H-NMR (400 MHz, D₂O): δ = 1.5-1.9 (m, 8H), 2.83 (mm, 1H), 2.9-3.1 (m, 6H), 3.15-3.3 (m, 2H), 4.19 (mm, 1H), 4.29 (m_{c}, 1H), 4.43 (m_{c}, 1H), 4.7-4.9 (m, 2H, unter D₂O), 6.89 (m_{c}, 2H), 6.96 (s, 1H), 7.26 (s, 1H), 7.36 (d, 1H), 7.43 (d, 1H).

### Beispiel 50

(8*S,*11*S,*14*S*)-14-Amino-*N-*[(1*S*)-4-amino-1-({[(2*S*)-2,5-diaminopentyl]amino}carbonyl)butyl]-11-[(2R)-3-amino-2-hydroxypropyl]-5,17-dihydroxy-10,13-dioxo-9,12-diazatricyclo[14.3.1.1^{2,6}]henicosa-1(20),2(21),3,5,16,18-hexaen-8-carboxamid Pentahydrochlorid 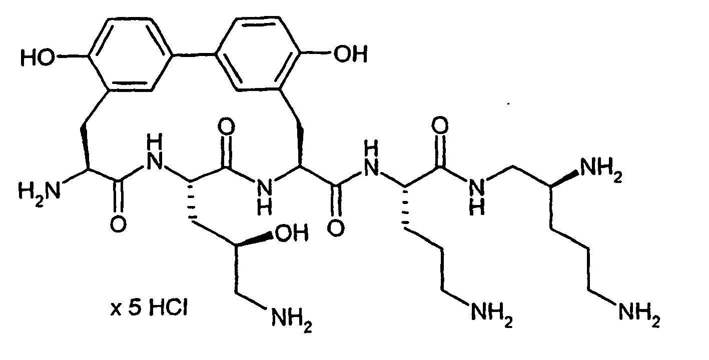 Eine Lösung von 24 mg (0.02 mmol) der Verbindung aus Beispiel 278A in 1 ml Dioxan wird bei 0°C mit 0.30 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 17 mg (97% d. Th.)
MS (ESI): m/z = 686 (M-5HCl+H)⁺.
¹H-NMR (400 MHz, D₂O): δ = 1.3-1.9 (m, 10H), 2.5-2.95 (m, 7H), 2.95-3.25 (m, 3H), 3.3-3.5 (m, 2H), 3.62 (m_{c}, 1H), 4.06 (m_{c}, 1H), 4.18 (m_{c}, 1H), 4.7-4.9 (m, 2H, unter D₂O), 6.67 (m_{c}, 2H), 6.75 (s, 1H), 7.05 (s, 1H), 7.14 (d, 1H), 7.21 (d, 1H).

### Beispiel 51

(8*S,*11*S,*14*S*)-14-Amino-*N-*{3-[(2-aminoethyl)amino]-3-oxopropyl}-11-(3-aminopropyl)-5,17-dihydroxy-10,13-dioxo-9,12-diazatricyclo[14.3.1.1^{2,6}]henicosa-1(20),2(21),3,5,16,18-hexaen-8-carboxamid Trihydrochlorid 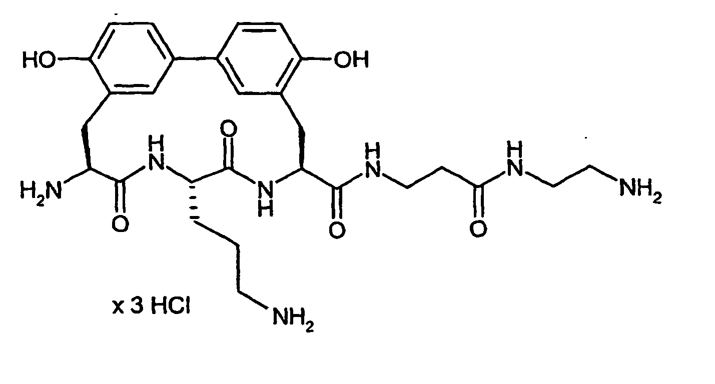 Eine Lösung von 21 mg (0.024 mmol) der Verbindung aus Beispiel 279A in 1ml Dioxan wird bei 0°C mit 0.36 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 16 mg (98% d. Th.)
LC-MS (Methode 23): Rₜ = 1.84 min.
MS (ESI): m/z = 570 (M-3HCL+H)⁺.
¹H-NMR (400 MHz, D₂O): δ = 1.5-1.9 (m, 4H), 2.3-2.6 (m, 2H), 2.7-3.2 (m, 7H), 3.3-3.7 (m, 5H), 4.42 (m_{c}, 1H), 4.7-4.9 (m, 2H, unter D₂O), 6.89 (m_{c}, 2H), 6.95 (s, 1H), 7.25 (s, 1H), 7.34 (d, 1H), 7.42 (d, 1H).

### Beispiel 52

rel-(8*S,*11*S,*14*S*)-14-Amino-*N-*((1*S*)-1-{[(3-amino-2-hydroxypropyl)amino]carbonyl}-4-{[amino(imino)methyl]amino}butyl)-11-(3-aminopropyl)-5,17-dihydroxy-10,13-dioxo-9,12-diazatricyclo[14.3.1.1^{2,6}]henicosa-1 (20),2(21),3,5,16,18-hexaen-8-carboxamid Tetrahydrochlorid 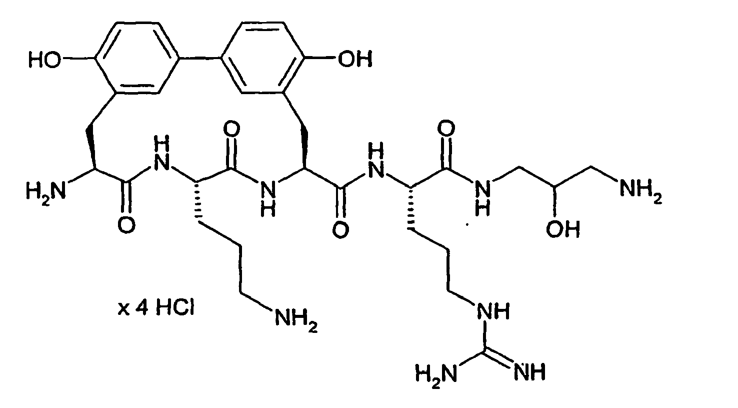 Eine Lösung von 41 mg (0.035 mmol) der Verbindung aus Beispiel 280A in 1 ml Dioxan wird bei 0°C mit 0.52 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 28.5 mg (99% d. Th.)
MS (ESI): m/z = 685 (M-4HCl+H)⁺.
¹H-NMR (400 MHz, D₂O): δ = 1.5-1.9 (m, 8H), 2.7-3.4 (m, 11H), 3.5-3.7 (m, 1H), 3.97 (m_{c}, 1H), 4.28 (m_{c}, 1H), 4.47 (m_{c}, 1H), 4.7-4.9 (m, 2H, unter D₂O), 6.94 (m_{c}, 2H), 7.01 (s, 1H), 7.31 (s, 1H), 7.40 (d, 1H), 7.48 (d, 1H).

### Beispiel 53

rel-(8*S,*11*S,*14*S*)-14-Amino-11-(3-aminopropyl)-5,17-dihydroxy-*N-*[2-hydroxy-3-(L-ornithyl-amino)propyl]-10,13-dioxo-9,12-diazatricyclo[14.3.1.1^{2,6}]henicosa-1(20),2(21),3,5,16,18-hexaen-8-carboxamid Tetrahydrotrifluoracetat 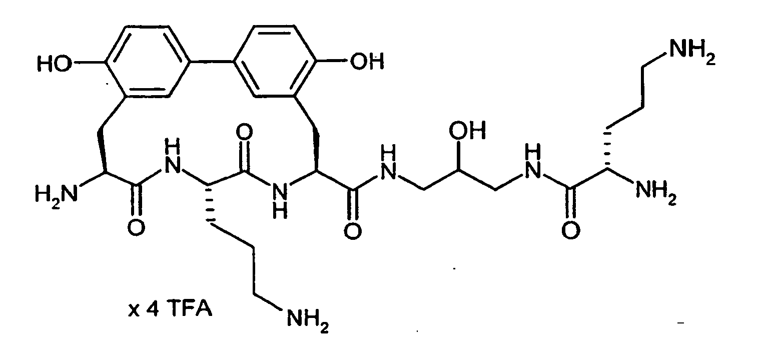 Eine Lösung von 98 mg (0.101 mmol) der Verbindung aus Beispiel 281A in 1ml Dioxan wird bei 0°C mit 1.52 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird durch präparative HPLC (Reprosil ODS-A, Laufmittel Acetonitril / 0.2% wässrige Trifluoressigsäure 5:95 → 95:5) in das Tetra(hydrotrifluoracetat) überführt.
Ausbeute: 24.6 mg (22% d. Th.)
MS (ESI): m/z = 643 (M-4TFA+H)⁺.
¹H-NMR (400 MHz, D₂O): δ = 1.55-2.0 (m, 8H), 2.8-3.5 (m, 11H), 3.56 (m_{c}, 1H), 3.86 (m_{c}, 1H), 3.97 (m_{c}, 1H), 4.43 (m_{c}, 1H), 4.7-4.9 (m, 2H, unter D₂O), 6.91 (m_{c}, 2H), 6.98 (s, 1H), 7.27 (s, 1H), 7.36 (d, 1H), 7.44 (d, 1H).

### Beispiel 54

(8*S,*11*S,*14*S*)-14-Amino-*N-*[(1*S*)-4-amino-1-({[(1*S*)-2-amino-1-(hydroxymethyl)ethyl]amino}-carbonyl)butyl]-11-[(2R)-3-amino-2-hydroxypropyl]-5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 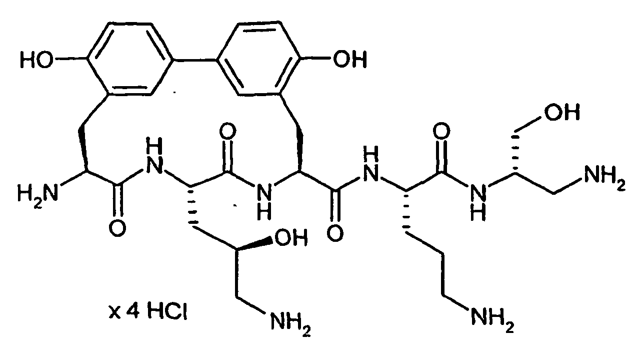 Eine Lösung von 22 mg (0.021 mmol) der Verbindung aus Beispiel 282A in 1 ml Dioxan wird bei 0°C mit 0.31 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 16 mg (98% d. Th.)
MS (ESI): m/z = 659 (M-4HCl+H)⁺.
¹H-NMR (400 MHz, D₂O): δ = 1.6-2.05 (m, 6H), 2.8-3.15 (m, 6H), 3.2-3.35 (m, 2H), 3.5-3.7 (m, 3H), 3.87 (m_{c}, 1H), 4.21 (m_{c}, 1H), 4.31 (m_{c}, 1H), 4.43 (m_{c}, 1H), 4.7-4.9 (m, 2H, unter D₂O), 6.92 (m_{c}, 2H), 6.99 (s, 1H), 7.29 (s, 1H), 7.38 (d, 1H), 7.45 (d, 1H).

### Beispiel 55

(8*S,*11*S,*14*S*)-14-Amino-*N-*{(2*S*)-2-amino-3-[(2-aminoethyl)amino]-3-oxopropyl}-11-[(2*R*)-3-amino-2-hydroxypropyl]-5,17-dihydroxy-9-methyl-10,13-dioxo-9,12-diazatricyclo[14.3.1.1^{2,6}]henicosa-1(20),2(21),3,5,16,18-hexaen-8-carboxamid Tetrahydrochlorid 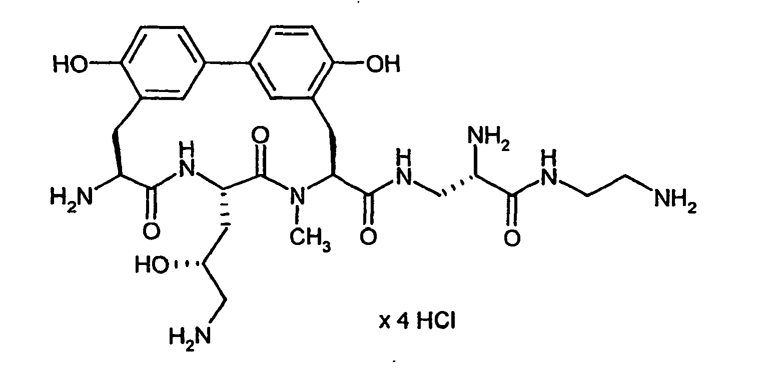 Eine Lösung von 19.4 mg (0.019 mmol) der Verbindung aus Beispiel 292A in 1 ml Dioxan wird bei 0°C mit 2 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 12 mg (81% d. Th.)
LC-MS (Methode 23): Rₜ = 0.47 min.
MS (ESI): m/z = 615 (M-4HCl+H)⁺.
¹H-NMR (400 MHz, D₂O): δ = 1.92-2.12 (m, 2H), 2.9-3.3 (m, 8H), 3.4-3.9 (m, 7H), 4.0 (m_{c}, 1H), 4.17 (m_{c}, 1H), 4.51 (m_{c}, 1H), 5.15 (mm, 1H), 5.70 (m_{c}, 1H), 6.97 (m_{c}, 2H), 7.03 (s, 1H), 7.10 (s, 1H), 7.49 (d, 1H), 7.56 (d, 1H).

### Beispiel 56

(8*S,*11*S,*14*S*)-14-Amino-*N-*{(2*S*)-2-amino-3-[(2-aminoethyl)amino]-3-oxopropyl}-11-(3-amino-propyl5,17-dihydroxy-10,13-dioxo-9,12-diazatricyclo[14.3.1.1^{2,6}]henicosa-1(20),2(21),3,5,16,18-hexaen-8-carboxamid Tetrahydrochlorid 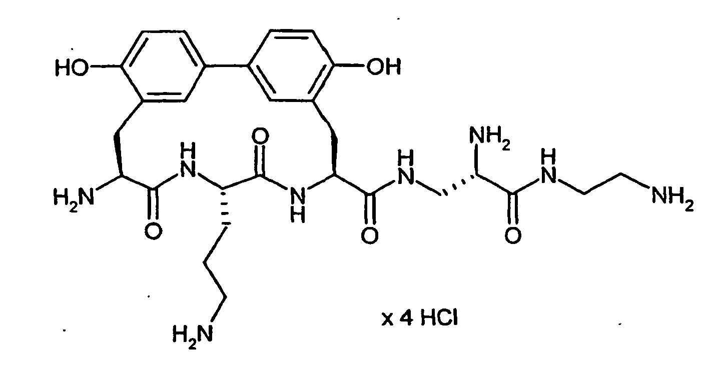 Eine Lösung von 18.2 mg (0.018 mmol) der Verbindung aus Beispiel 293A in 1 ml Dioxan wird bei 0°C mit 2 ml einer 4N Chlorwasserstoff-Dioxan-Lösung versetzt. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.
Ausbeute: 13 mg (98% d. Th.)
LC-MS (Methode 23): Rₜ = 0.43 min.
MS (ESI): m/z = 585 (M-4HCl+H)⁺.
¹H-NMR (400 MHz, D₂O): δ = 1.5-1.9 (m, 4H), 2.8-3.3 (m, 7H), 3.4-3.9 (m, 5H), 4.12 (mm, 1H), 4.45 (m_{c}, 1H), 4.7-4.9 (m, 2H, unter D₂O), 6.91 (m_{c}, 2H), 6.98 (s, 1H), 7.25 (s, 1H), 7.37 (d, 1H), 7.44 (d, 1H).

Analog zur Vorschrift des Beispiels 56 werden die in der folgenden Tabelle aufgeführten Beispiele 57 bis 69 hergestellt.

| **Beispiel-Nr.** | **Vorstufe Beispiel** | **Struktur** | **Analytische Daten** |
|---|---|---|---|
| **57** | 287A | 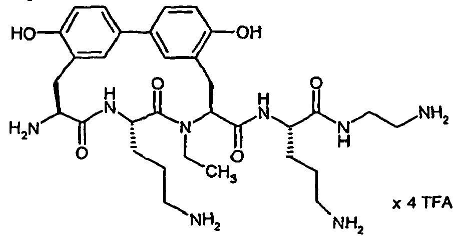 | LC-MS (Methode 23): Rₜ = 0.90 min. MS (EI): m/z = 640 (M-4TFA+H)⁺. |
| **58** | 288A | 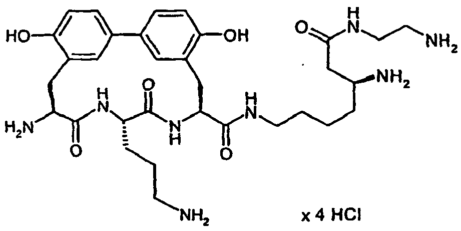 | LC-MS (Methode 17): Rₜ = 0.33 min. MS (EI): m/z = 641 (M-4HCl+H)⁺. |
| **59** | 295A | 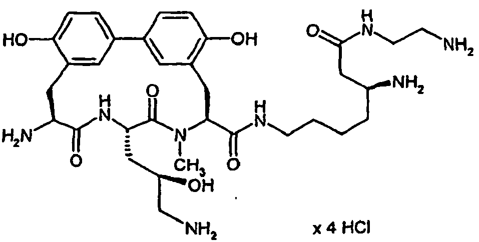 | LC-MS (Methode 23): Rₜ = 1.86 min. MS (EI): m/z = 671 (M-4HCl+H)⁺. |
| **60** | 296A | 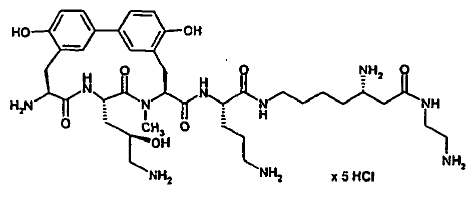 | LC-MS (Methode 23): Rₜ =1.74 min. MS (EI): m/z = 785 (M-5HCl+H)⁺. |
| **61** | 294A | 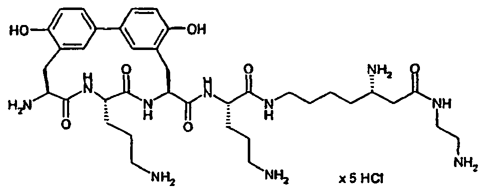 | LC-MS (Methode 23): Rₜ = 0.46 min. MS (EI): m/z = 755 (M-5HCl+H)⁺. |
| **62** | 297A | 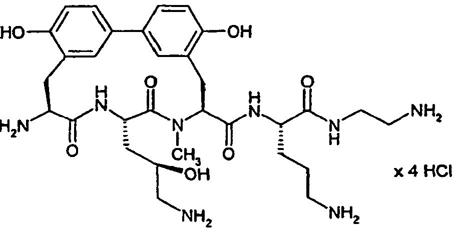 | LC-MS (Methode 23): Rₜ = 1.73 min. MS (EI): m/z = 643 (M-4HCl+H)⁺. |
| **63** | 289A | 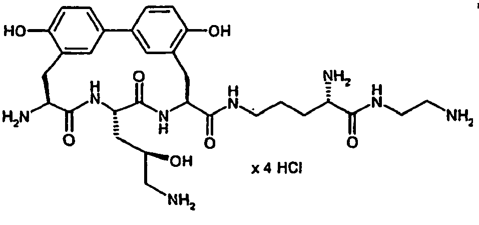 | LC-MS (Methode 19): Rₜ = 0.22 min. MS (EI): m/z = 629 (M-4HCl+H)⁺. |
| **64** | 283A | 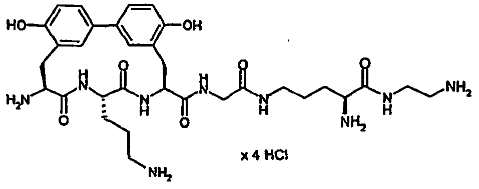 | LC-MS (Methode 23): Rₜ = 0.51 min. MS (EI): m/z = 670 (M-4HCl+H)⁺. |
| **65** | 284A | 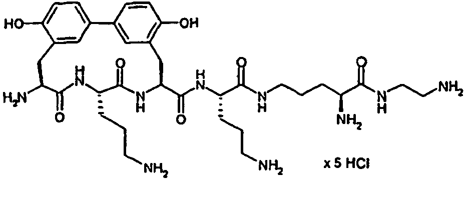 | LC-MS (Methode 23): Rₜ = 0.37 min. MS (EI): m/z = 727 (M-5HCl+H)⁺. |
| **66** | 286A | 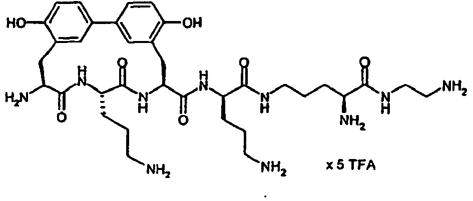 | LC-MS (Methode 23): Rₜ = 0.37 min. MS (EI): m/z = 727 (M-5TFA+H)⁺. |
| **67** | 285A | 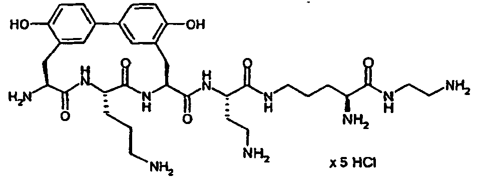 | LC-MS (Methode 23): Rₜ = 0.37 min. MS (EI): m/z = 713 (M-5HCl+H)⁺. |
| **68** | 290A | 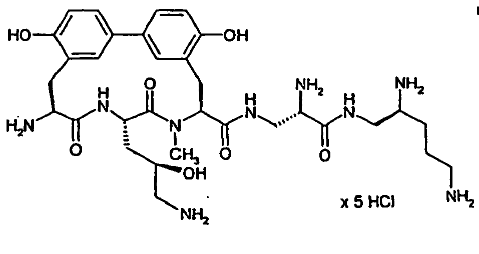 | LC-MS (Methode 23): Rₜ = 0.37 min. MS (EI): m/z = 672 (M-5HCl+H)⁺. |
| **69** | 291A | 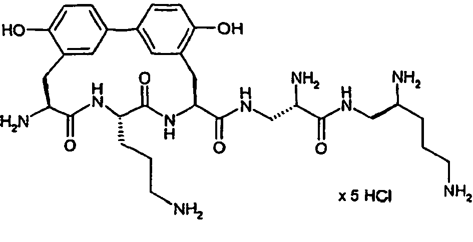 | LC-MS (Methode 23): Rₜ = 0.35 min. MS (EI): m/z = 642 (M-5HCl+H)⁺. |

### B. Bewertung der Physiologischen Wirksamkeit

### Verwendete Abkürzungen:

- AMP: Adenosinmonophosphat
- ATP: Adenosintriphosphat
- BHI Medium: Brain heart infusion medium
- CoA: Coenzym A
- DMSO: Dimethylsulfoxid
- DTT: Dithiothreitol
- EDTA: Ethylendiamintetraessigsäure
- KCl: Kaliumchlorid
- KH₂PO₄: Kaliumdihydrogenphosphat
- MgSO₄: Magnesiumsulfat
- MHK: Minimale Hemmkonzentration
- MTP: Mikrotiterplatte
- NaCl: Natriumchlorid
- Na₂HPO₄: Dinatriumhydrogenphosphat
- NH₄Cl: Ammoniumchlorid
- NTP: Nukleotidtriphosphat
- PBS: Phosphat Buffered Saline
- PCR: Polymerase Chain Reaction
- PEG: Polyethylenglykol
- PEP: Phosphoenolpyruvat
- Tris: Tris[hydroxymethyl]aminomethan

Die *in vitro*-Wirkung der erfindungsgemäßen Verbindungen kann in folgenden Assays gezeigt werden:

### In vitro Transkription-Translation mit E. coli Extrakten

Zur Herstellung eines S30-Extraktes werden logarithmisch wachsende *Escherichia coli* MRE 600 (M. Müller; University Freiburg) geerntet, gewaschen und wie beschrieben für den *in vitro* Transkriptions-Translations-Test eingesetzt (Müller, M. and Blobel, G. Proc Natl Acad Sci U S A (1984) 81, pp.7421-7425).

Dem Reaktionsmix des *in vitro* Transkriptions-Translations-Tests werden zusätzlich 1 µl cAMP (11.25 mg/ml) je 50 µl Reaktionsmix zugegeben. Der Testansatz beträgt 105 µl, wobei 5 µl der zu testenden Substanz in 5%igem DMSO vorgelegt werden. Als Transkriptionsmatrize werden 1 µg/100µl Ansatz des Plasmides pBESTLuc (Promega, Deutschland) verwendet. Nach Inkubation für 60 min bei 30°C werden 50µl Luziferinlösung (20 mM Tricine, 2.67 mM MgSO₄, 0.1 mM EDTA, 33.3 mM DTT pH 7.8, 270 µM CoA, 470 µM Luziferin, 530 µM ATP) zugegeben und die entstehende Biolumineszenz für 1 Minute in einem Luminometer gemessen. Als IC₅₀ wird die Konzentration eines Inhibitors angegeben, die zu einer 50%igen Inhibition der Translation von Firefly Luziferase führt.

### In vitro Transkription-Translation mit S. aureus Extrakten

### Konstruktion eines S. aureus Luziferase Reporterplasmids

Zur Konstruktion eines Reporterplasmids, welches in einem *in vitro* Transkriptions-Translations-Assay aus *S*. *aureus* verwendet werden kann, wird das Plasmid pBESTluc (Promega Corporation, USA) verwendet. Der in diesem Plasmid vor der Firefly Luziferase vorhandene *E*. *coli tac* Promoter wird gegen den *capA1* Promoter mit entsprechender Shine-Dalgarno Sequence aus *S*. *aureus* ausgetauscht. Dazu werden die Primer CAPFor 5'-CGGCC-AAGCTTACTCGGATCCAGAGTTTGCAAAATATACAGGGGATTATATATAATGGAAAAC AAGAAAGGAAAATAGGAGGTTTATATGGAAGACGCCA-3' und CAPRev 5'-GTCATCGTCGGGAAGACCTG-3' verwendet. Der Primer CAPFor enthält den *capA1* Promotor, die Ribosomenbindestelle und die 5'-Region des Luziferase Gens. Nach PCR unter Verwendung von pBESTluc als Template kann ein PCR-Produkt isoliert werden, welches das Firefly Luziferase Gen mit dem fusionierten *capA1* Promotor enthält. Dieses wird nach einer Restriktion mit ClaI und HindIII in den ebenfalls mit C1aI und HindIII verdauten Vektor pBESTluc ligiert. Das entstandene Plasmid pla kann in *E. coli* repliziert werden und als Template im *S*. *aureus in vitro* Transkriptions-Translations-Test verwendet werden.

### Herstellung_von S30 Extrakten aus S. aureus

Sechs Liter BHI Medium werden mit einer 250 ml Übernachtkultur eines S. aureus Stammes inokuliert und bei 37°C bis zu einer OD600nm von 2-4 wachsen gelassen. Die Zellen werden durch Zentrifugation geerntet und in 500 ml kaltem Puffer A (10 mM Tris-acetat, pH 8.0, 14 mM Magnesiumacetat, 1 mM DTT, 1 M KCl) gewaschen. Nach erneutem Abzentrifugieren werden die Zellen in 250 ml kaltem Puffer A mit 50 mM KCl gewaschen und die erhaltenen Pellets bei -20°C für 60 min eingefroren. Die Pellets werden in 30 bis 60 min auf Eis aufgetaut und bis zu einem Gesamtvolumen von 99 ml in Puffer B (10 mM Tris-acetat, pH 8.0, 20 mM Magnesiumacetat, 1 mM DTT, 50 mM KCl) aufgenommen. Je 1.5 ml Lysostaphin (0.8 mg/Ml) in Puffer B werden in 3 vorgekühlte Zentrifugenbecher vorgelegt und mit je 33 ml der Zellsuspension vermischt. Die Proben werden für 45 bis 60 min bei 37°C unter gelegentlichem Schütteln inkubiert, bevor 150 µl einer 0.5 M DTT Lösung zugesetzt werden. Die lysierten Zellen werden bei 30.000 x g 30 min bei 4°C abzentrifugiert. Das Zellpellet wird nach Aufnahme in Puffer B unter den gleichen Bedingungen nochmals zentrifugiert und die gesammelten Überstände werden vereinigt. Die Überstände werden nochmals unter gleichen Bedingungen zentrifugiert und zu den oberen 2/3 des Überstandes werden 0.25 Volumen Puffer C (670 mM Tris-acetat, pH 8.0, 20 mM Magnesiumacetat, 7 mM Na₃-Phosphoenolpyruvat, 7 mM DTT, 5.5 mM ATP, 70 µM Aminosäuren (complete von Promega), 75 µg Pyruvatkinase (Sigma, Deutschland))/ml gegeben. Die Proben werden für 30 min bei 37°C inkubiert. Die Überstände werden über Nacht bei 4°C gegen 2 1 Dialysepuffer (10 mM Tris-acetat, pH 8.0, 14 mM Magnesiumacetat, 1 mM DTT, 60 mM Kaliumacetat) mit einem Pufferwechsel in einem Dialyseschlauch mit 3500 Da Ausschluss dialysiert. Das Dialysat wird auf eine Proteinkonzentration von etwa 10 mg/ml konzentriert, indem der Dialyseschlauch mit kaltem PEG 8000 Pulver (Sigma, Deutschland) bei 4°C bedeckt wird. Die S30 Extrakte können aliquotiert bei -70°C gelagert werden.

### Bestimmung der IC₅₀ im S. aureus in vitro Transcriptions-Translations-Assay

Die Inhibition der Proteinbiosynthese der Verbindungen kann in einem *in vitro* Transkriptions-Translations-Assay gezeigt werden. Der Assay beruht auf der zellfreien Transkription und Translation von Firefly Luziferase unter Verwendung des Reporterplasmids pla als Template und aus *S. aureus* gewonnenen zellfreien S30 Extrakten. Die Aktivität der entstandenen Luziferase kann durch Lumineszenzmessung nachgewiesen werden.

Die Menge an einzusetzenden S30 Extrakt bzw. Plasmid pla muss für jede Präparation erneut ausgetestet werden, um eine optimale Konzentration im Test zu gewährleisten. 3 µl der zu testenden Substanz gelöst in 5% DMSO werden in eine MTP vorgelegt. Anschließend werden 10 µl einer geeignet konzentrierten Plasmidlösung pla zugegeben. Anschließend werden 46 µl eines Gemisches aus 23 µl Premix (500 mM Kaliumacetat, 87.5 mM Tris-acetat, pH 8.0, 67.5 mM Ammoniumacetat, 5 mM DTT, 50 µg Folsäure/ml, 87.5 mg PEG 8000/ml, 5 mM ATP, 1.25 mM je NTP, 20 µM je Aminosäure, 50 mM PEP (Na₃-Salz), 2.5 mM cAMP, 250 µg je *E. coli* tRNA/ml) und 23 µl einer geeigneten Menge *S. aureus* S30 Extrakt zugegeben und vermischt. Nach Inkubation für 60 min bei 30°C werden 50 µl Luziferinlösung (20 mM Tricine, 2.67 mM MgSO₄, 0.1 mM EDTA, 33.3 mM DTT pH 7.8, 270 µM CoA, 470 µM Luziferin, 530 µM ATP) und die entstehende Biolumineszenz für 1 min in einem Luminometer gemessen. Als IC₅₀ wird die Konzentration eines Inhibitors angegeben, die zu einer 50%igen Inhibition der Translation von Firefly Luziferase führt.

### Bestimmung der Minimalen Hemmkonzentration (MHK)

Die minimale Hemmkonzentration (MHK) ist die minimale Konzentration eines Antibiotikum, mit der ein Testkeim in seinem Wachstum über 18-24 h inhibiert wird. Die Hemmstoffkonzentration kann dabei nach mikrobiologischen Standardverfahren bestimmt werden (siehe z.B. The National Committee for Clinical Laboratory Standards. Methods for dilution antimicrobial susceptibility tests for bacteria that grow aerobically; approved standard-fifth edition. NCCLS document M7-A5 [ISBN 1-56238-394-9]. NCCLS, 940 West Valley Road, Suite 1400, Wayne, Pennsylvania 19087-1898 USA, 2000). Die MHK der erfindungsgemäßen Verbindungen wird im Flüssigdilutionstest im 96er-Mikrotiter-Platten-Maßstab bestimmt. Die Bakterienkeime werden in einem Minimalmedium (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1% Glucose, 25 µg/ml von jeder proteinogenen Aminosäure mit Ausnahme von Phenylalanin; [H.-P. Kroll; unveröffentlicht]) unter Zusatz von 0.4% BH-Bouillon kultiviert (Testmedium). Im Fall von *Enterococcus faecium* L4001 wird dem Testmedium hitzeinaktiviertes fötales Kälberserum (FCS; GibcoBRL, Deutschland) in einer Endkonzentration von 10% zugesetzt. Übernachtkulturen der Testkeime werden auf eine OD₅₇₈ von 0.001 (im Falle der Enterokokken auf 0.01) in frisches Testmedium verdünnt und 1:1 mit Verdünnungen der Testsubstanzen (Verdünnungsstufen 1:2) in Testmedium inkubiert (200 µl Endvolumen). Die Kulturen werden bei 37°C für 18-24 Stunden inkubiert; Enterokokken in Gegenwart von 5% CO₂.

Die jeweils niedrigste Substanzkonzentration, bei der kein sichtbares Bakterienwachstum mehr auftritt, wird als MHK definiert.

### Alternative Bestimmungsmethode der Minimalen Hemmkonzentration (MHK)

Die minimale Hemmkonzentration (MHK) ist die minimale Konzentration eines Antibiotikums, mit der ein Testkeim in seinem Wachstum über 18-24 h inhibiert wird. Die Hemmstoffkonzentration kann dabei nach mikrobiologischen Standardverfahren mit modifiziertem Medium im Rahmen eines Agardilutionstests bestimmt werden (siehe zB. The National Committee for Clinical Laboratory Standards. Methods for dilution antimicrobial susceptibility tests for bacteria that grow aerobically; approved standard-fifth edition. NCCLS document M7-A5 [ISBN 1-56238-394-9]. NCCLS, 940 West Valley Road, Suite 1400, Wayne, Pennsylvania 19087-1898 USA, 2000). Die Bakterienkeime werden auf 1.5%igen Agarplatten kultiviert, die 20% defibriniertes Pferdeblut enthalten. Die Testkeime, die über Nacht auf Columbia-Blutagarplatten (Becton-Dickinson) inkubiert werden, werden in PBS verdünnt, auf eine Keimzahl von ca. 5x10⁵ Keime/ml eingestellt und auf Testplatten getropft (1-3 µl). Die Testsubstanzen enthalten unterschiedliche Verdünnungen der Testsubstanzen (Verdünnungsstufen 1:2). Die Kulturen werden bei 37°C für 18-24 Stunden in Gegenwart von 5% CO₂ inkubiert.

Die jeweils niedrigste Substanzkonzentration, bei der kein sichtbares Bakterienwachstum mehr auftritt, wird als MHK definiert und in µg/ml angegeben.

**Tabelle A (mit Vergleichsbeispiel 20A (Biphenomycin B))**

| **Bsp- Nr.** | **MHK *S. aureus* 133** | ***MHK S. aureus* T17** | **MHIK** *E.* ***faecium* L4001** | **IC₅₀ *S. aureus* 133 Translation** |
|---|---|---|---|---|
| **6** | 1 | 2 | 8 | 0.1 |
| **10** | 0.5 | 0.5 | 8 | 0.07 |
| **13** | 0.5 | 0.5 | 16 | 0.06 |
| **14** | 1 | 2 | 4 | 0.07 |
| **16** | 2 | 2 | >32 | 0.1 |
| **23** | 0.5 | 1 | 4 | 0.32 |
| **20A** | <0.03 | >32 | 0.5 | 1.5 |

| | | | | |
|---|---|---|---|---|
| Konzentrationsangaben: MHK in µg/ml; **IC₅₀** in µM. | | | | |

### Systemische Infektion mit S. aureus 133

Die Eignung der erfindungsgemäßen Verbindungen zur Behandlung von bakteriellen Infektionen kann in verschiedenen Tiermodellen gezeigt werden. Dazu werden die Tiere im allgemeinen mit einem geeigneten virulenten Keim infiziert und anschließend mit der zu testenden Verbindung, die in einer an das jeweilige Therapiemodell angepassten Formulierung vorliegt, behandelt. Speziell kann die Eignung der erfindungsgemäßen Verbindungen zur Behandlung von bakteriellen Infektionen in einem Sepsismodell an Mäusen nach Infektion mit *S*. *aureus* demonstriert werden.

Dazu werden *S. aureus* 133 Zellen über Nacht in BH-Bouillon (Oxoid, Deutschland) angezüchtet. Die Übernachtkultur wurde 1:100 in frische BH-Bouillon verdünnt und für 3 Stunden hochgedreht. Die in der logarithmischen Wachstumsphase befindlichen Bakterien werden abzentrifugiert und zweimal mit gepufferter, physiologischer Kochsalzlösung gewaschen. Danach wird am Photometer (Dr. Lange LP 2W) eine Zellsuspension in Kochsalzlösung mit einer Extinktion von 50 Einheiten eingestellt. Nach einem Verdünnungsschritt (1:15) wird diese Suspension 1:1 mit einer 10%-igen Mucinsuspension gemischt. Von dieser Infektionslösung wird 0.2 ml/20 g Maus i.p. appliziert.

Dies entspricht einer Zellzahl von etwa 1-2 x 10⁶ Keimen/Maus. Die i.v.-Therapie erfolgt 30 Minuten nach der Infektion. Für den Infektionsversuch werden weibliche CFW1-Mäuse verwendet. Das Überleben der Tiere wird über 6 Tage protokolliert. Das Tiermodell ist so eingestellt, daß unbehandelte Tiere innerhalb von 24 h nach der Infektion versterben. Für die Beispielverbindung 2 konnte in diesem Modell eine therapeutische Wirkung von ED₁₀₀ = 1.25 mg/kg demonstriert werden.

### Bestimmung der Spontanresistenzfrequenzen gegen S. aureus

Die Spontanresistenzraten der erfindungsgemäßen Verbindungen werden wie folgt bestimmt: die Bakterienkeime werden in 30 ml eines Minimalmediums (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1% Glucose, 25 µg/ml von jeder proteinogenen Aminosäure unter Zusatz von 0,4% BH Bouillon) bei 37°C über Nacht kultiviert, 10 min bei 6.000xg abzentrifugiert und in 2 ml phosphat-gepufferter physiologischer NaCl-Lösung resuspendiert (ca. 2x10⁹ Keime/ml). 100 µl dieser Zellsuspension bzw. 1:10 und 1:100 Verdünnungen werden auf vorgetrockneten Agarplatten (1.5% Agar, 20% defibriniertes Pferdeblut bzw. 1.5% Agar, 20% Rinderserum in 1/10 Müller-Hinton-Medium verdünnt mit PBS), welche die zu testende erfindungsgemäße Verbindung in einer Konzentration entsprechend 5xMHK bzw. 10x.MHK enthalten, ausplattiert und 48 h bei 37°C bebrütet. Die entstehenden Kolonien (cfu) werden ausgezählt.

### Isolierung der Biphenomycin-resistenten S. aureus Stämme RN4220Bi^{R} und T17

Der *S. aureus* Stamm RN4220Bi^{R} wird *in vitro* isoliert. Dazu werden jeweils 100 µl einer S. *aureus* RN4220 Zellsuspension (ca. 1.2x10⁸ cfu/ml) auf einer antibiotikafreien Agarplatte (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1% Glucose, 25 µg/ml von jeder proteinogenen Aminosäure unter Zusatz von 0.4% BH-Bouillon und 1 % Agarose) und einer Agarplatte, die 2 µg/ml Biphenomycin B (10xMHK) enthält, ausplattiert und über Nacht bei 37°C bebrütet. Während auf der antibiotikafreien Platte ca. 1x10⁷ Zellen wachsen, wachsen auf der antibiotikahaltigen Platte ca. 100 Kolonien, entsprechend einer Resistenzfrequenz von 1x10⁻⁵. Einige der auf der antibiotikahaltigen Platte gewachsenen Kolonien werden auf MHK gegen Biphenomycin B getestet. Eine Kolonie mit einer MHK > 50 µM wird zur weiteren Verwendung ausgewählt und der Stamm mit RN4220Bi^{R} bezeichnet.

Der *S. aureus* Stamm T17 wird *in vivo* isoliert. CFW1-Mäuse werden mit 4x10⁷ *S. aureus* 133 - Zellen pro Maus intraperitoneal infiziert. 0.5 Std. nach der Infektion werden die Tiere mit 50 mg/kg Biphenomycin B intravenös behandelt. Den überlebenden Tieren werden am Tag 3 nach der Infektion die Nieren entnommen. Nach dem Homogenisieren der Organe werden die Homogenate, wie bei RN4220Bi^{R} beschrieben, auf antibiotikafreien und antibiotikahaltigen Agarplatten, ausplattiert und über Nacht bei 37°C bebrütet. Etwa die Hälfte der aus der Niere isolierten Kolonien zeigen ein Wachstum auf den antibiotikahaltigen Platten (2.2x10⁶ Kolonien), was die Anreicherung von Biphenomycin B resistenten *S. aureus* Zellen in der Niere der behandelten Tiere belegt. Ca. 20 dieser Kolonien werden auf MHK gegen Biphenomycin B getestet und eine Kolonie mit einer MHK > 50 µM wird zur Weiterkultivierung ausgewählt und der Stamm mit T17 bezeichnet.

### C. Ausführungsbeispiele für pharmazeutische Zusammensetzungen

Die erfindungsgemäßen Verbindungen können folgendermaßen in pharmazeutische Zubereitungen überführt werden:

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die erfindungsgemäße Verbindung wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 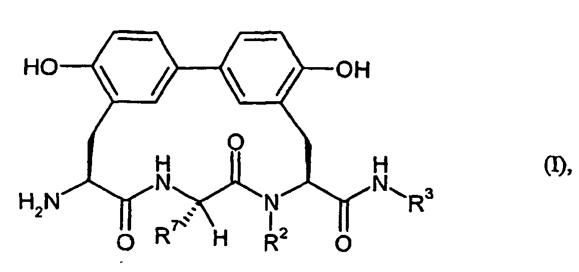 in welcher
R⁷ gleich eine Gruppe der Formel 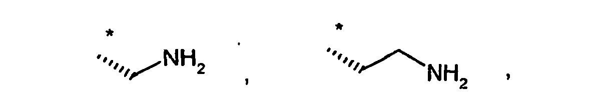 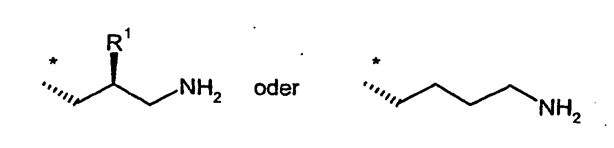
ist,
wobei
R¹ gleich Wasserstoff oder Hydroxy ist,
* die Anknüpfstelle an das Kohlenstoffatom ist,
R² gleich Wasserstoff, Methyl oder Ethyl ist,
R³ gleich eine Gruppe der Formel 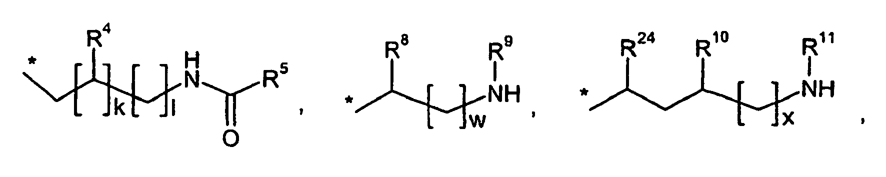 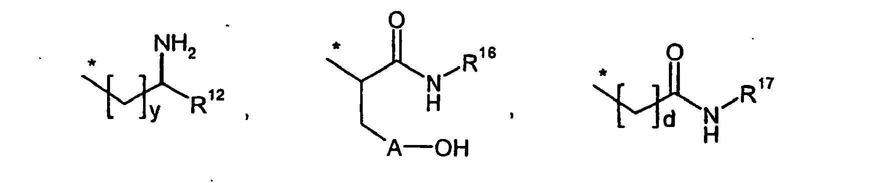
oder 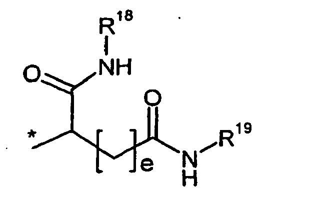 ist,
wobei
* die Anknüpfstelle an das Stickstoffatom ist,
A gleich eine Bindung oder Phenyl ist,
R⁴ gleich Wasserstoff, Amino oder Hydroxy ist,
R⁵ eine Gruppe der Formel 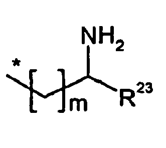
ist,
worin
* die Anknüpfstelle an das Kohlenstoffatom ist,
R²³ Wasserstoff oder eine Gruppe der Formel *-(CH₂)ₙ-OH oder *-(CH₂)ₒ-NH₂ ist,
worin
* die Anknüpfstelle an das Kohlenstoffatom ist,
n und o unabhängig voneinander eine Zahl 1, 2, 3 oder 4 sind,
m eine Zahl 0 oder 1 ist,
R⁸ und R¹² unabhängig voneinander eine Gruppe der Formel *-CONBR¹⁴ oder *-CH₂CONHR¹⁵ sind,
worin
* die Anknüpfstelle an das Kohlenstoffatom ist,
R¹⁴ und R¹⁵ unabhängig voneinander eine Gruppe der Formel 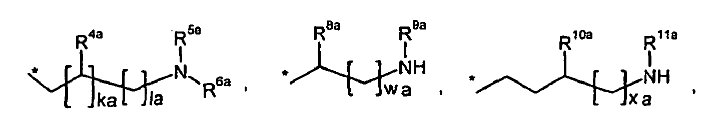 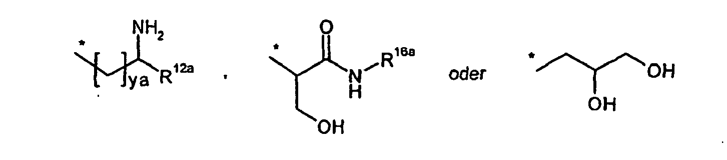
sind,
worin
* die Anknüpfstelle an das Stickstoffatom ist,
R^{4a} gleich Wasserstoff, Amino oder Hydroxy ist,
R^{5a} gleich Wasserstoff, Methyl oder Aminoethyl ist,
R^{6a} gleich Wasserstoff oder Aminoethyl ist,
oder
R^{5a} und R^{6a} bilden zusammen mit dem Stickstoffatom an das sie gebunden sind einen Piperazin-Ring,
R8^{a} und R^{12a} unabhängig voneinander *-(CH₂)_{Z1a}-OH, *-(CH₂)_{Z2a}-NHR^{13a}, *-CONHR^{14a} oder *-CH₂CONHR^{15a} sind,
worin
* die Anknüpfstelle an das Kohlenstoffatom ist,
Z1a und Z2a unabhängig voneinander eine Zahl 1, 2 oder 3 sind,
R^{13a} gleich Wasserstoff oder Methyl ist und
R^{14a} und R^{15a} unabhängig voneinander eine Gruppe der Formel 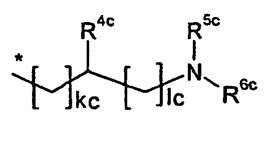
sind,
worin
* die Anknüpfstelle an das Stickstoffatom ist,
R^{4c} gleich Wasserstoff, Amino oder Hydroxy ist,
R^{5c} gleich Wasserstoff, Methyl oder Aminoethyl ist,
R^{6c}: gleich Wasserstoff oder Aminoethyl ist,
kc eine Zahl 0 oder 1 ist
und
lc eine Zahl 1, 2, 3 oder 4 ist,
R^{9a} und R^{11a} unabhängig voneinander Wasserstoff oder Methyl sind,
R^{10a} gleich Amino oder Hydroxy ist,
R^{16a} eine Gruppe der Formel 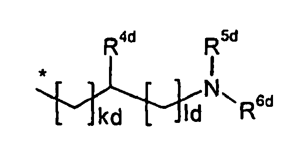
sind,
worin
* die Anknüpfstelle an das Stickstoffatom ist,
R^{4d} gleich Wasserstoff, Amino oder Hydroxy ist,
R^{5d} gleich Wasserstoff, Methyl oder Aminoethyl ist,
R^{6d} gleich Wasserstoff oder Aminoethyl ist,
kd eine Zahl 0 oder 1 ist
und
ld eine Zahl 1, 2, 3 oder 4 ist,
ka eine Zahl 0 oder 1 ist und
la, wa, xa und ya unabhängig voneinander eine Zahl 1, 2, 3 oder 4 sind,
R⁹ und R¹¹ unabhängig voneinander Wasserstoff, Methyl, *-C(NH₂)=NH oder eine Gruppe der Formel 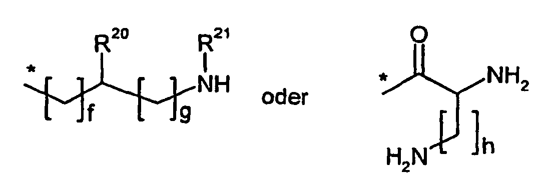
sind,
worin
* die Anknüpfstelle an das Stickstoffatom ist,
R²⁰ gleich Wasserstoff oder *(CH₂)ᵢ-NHR²² ist,
worin
R²² gleich Wasserstoff oder Methyl ist
und
i eine Zahl 1, 2 oder 3 ist,
R²¹ gleich Wasserstoff oder Methyl ist,
f eine Zahl 0, 1, 2 oder 3 ist,
g eine Zahl 1, 2 oder 3 ist
und
h eine Zahl 1, 2, 3 oder 4 ist,
oder
R⁸ gleich *-(CH₂)_{Z1}-OH ist,
worin
* die Anknüpfstelle an das Kohlenstoffatom ist,
Z1 eine Zahl 1, 2 oder 3 ist,
und
R⁹ eine Gruppe der Formel 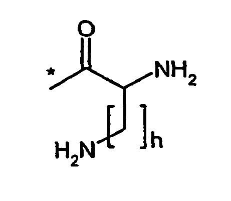
ist,
worin
* die Anknüpfstelle an das Stickstoffatom ist,
und
h eine Zahl 1, 2, 3 oder 4 ist,
R¹⁰ gleich Amino oder Hydroxy ist,
R¹⁶ und R¹⁷ unabhängig voneinander eine Gruppe der Formel 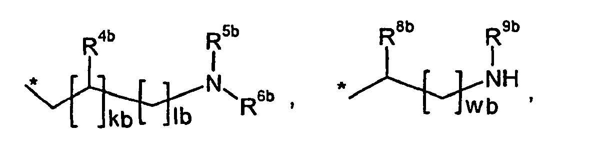 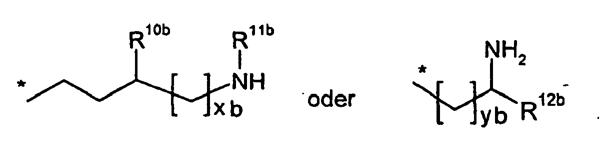
sind,
worin
* die Anknüpfstelle an das Stickstoffatom ist,
R^{4b} gleich Wasserstoff, Amino oder Hydroxy ist,
R^{5b} gleich Wasserstoff, Methyl oder Aminoethyl ist,
R^{6b} gleich Wasserstoff oder Aminoethyl ist,
oder
R^{5b} und R^{6b} bilden zusammen mit dem Stickstoffatom an das sie gebunden sind einen Piperazin-Ring,
R^{8b} und R^{12b} unabhängig voneinander ^{*}-(CH₂)_{Z1b}-OH, *-(CH₂)_{Z2b}-NHR^{13b}, *-CONHR^{14b} oder *-CH₂CONHR^{15b}sind,
worin
* die Anknüpfstelle an das Kohlenstoffatom ist,
R^{13b} gleich Wasserstoff oder Methyl ist
und
Z1b und Z2b unabhängig voneinander eine Zahl 1, 2 oder 3 sind,
und
R^{14b} und R^{15b} unabhängig voneinander eine Gruppe der Formel 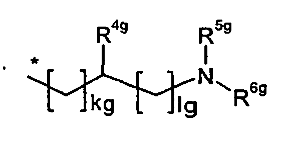
sind,
worin
* die Anknüpfstelle an das Stickstoffatom ist,
R^{4g} gleich Wasserstoff, Amino oder Hydroxy ist,
R^{5g} gleich Wasserstoff, Methyl oder Aminoethyl ist,
R^{6g} gleich Wasserstoff oder Aminoethyl ist,
kg eine Zahl 0 oder 1 ist
und
lg eine Zahl 1, 2, 3 oder 4 ist,
R^{9b} und R^{11b} unabhängig voneinander Wasserstoff oder Methyl sind,
R^{10b} gleich Amino oder Hydroxy ist,
kb eine Zahl 0 oder 1 ist,
lb, wb, xb und yb unabhängig voneinander eine Zahl 1, 2, 3 oder 4 sind,
R¹⁸ und R¹⁹ unabhängig voneinander Wasserstoff oder eine Gruppe der Formel 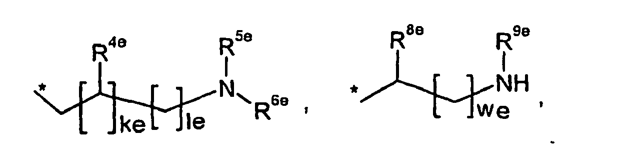 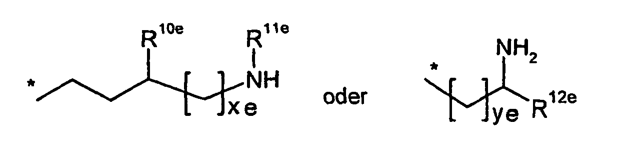
sind,
worin
* die Anknüpfstelle an das Stickstoffatom ist,
R^{4e} gleich Wasserstoff, Amino oder Hydroxy ist,
R^{5c} gleich Wasserstoff, Methyl oder Aminoethyl ist,
R^{6c} gleich Wasserstoff oder Aminoethyl ist,
oder
R^{5e} und R^{6c} bilden zusammen mit dem Stickstoffatom an das sie gebunden sind einen Piperazin-Ring,
R^{8e} und R^{12c} unabhängig voneinander *-(CH₂)_{Z1e}-OH oder *-(CH₂)_{Z2e}-NHR^{13e} sind,
worin
***** die Anknüpfstelle an das Kohlenstoffatom ist,
R^{13e} gleich Wasserstoff oder Methyl ist
und
Z1e und Z2e unabhängig voneinander eine Zahl 1, 2 oder 3 sind,
R^{9e} und R^{11e} unabhängig voneinander Wasserstoff oder Methyl sind,
R^{10e} gleich Amino oder Hydroxy ist,
ke eine Zahl 0 oder 1 ist
und
le, we, xe und ye unabhängig voneinander eine Zahl 1, 2, 3 oder 4 sind, wobei R¹⁸ und R¹⁹ nicht gleichzeitig Wasserstoff sind,
R²⁴ eine Gruppe der Formel *-CONHR²⁵ ist,
worin
* die Anknüpfstelle an das Kohlenstoffatom ist,
R²⁵ eine Gruppe der Formel 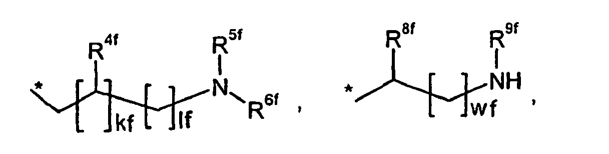 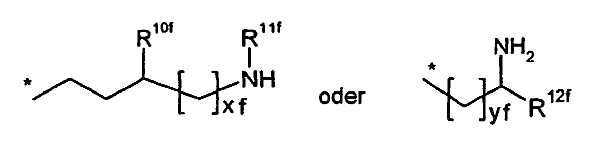
ist,
worin
* die Anknüpfstelle an das Stickstoffatom ist,
R^{4f} gleich Wasserstoff, Amino oder Hydroxy ist,
R^{5f} gleich Wasserstoff, Methyl oder Aminoethyl ist,
R^{6f} gleich Wasserstoff oder Aminoethyl ist,
oder
R^{5f} und R^{6f} bilden zusammen mit dem Stickstoffatom an das sie gebunden sind einen Piperazin-Ring,
R^{8f} und R^{12f} unabhängig voneinander *-(CH₂)_{Z1f}-OH oder *-(CH₂)_{Z2l}-NHR^{13f} sind,
worin
* die Anknüpfstelle an das Kohlenstoffatom ist,
R^{13f} gleich Wasserstoff oder Methyl ist
und
Z1f und Z2f unabhängig voneinander eine Zahl 1, 2 oder 3 sind,
R^{9f} und R^{11f} unabhängig voneinander Wasserstoff oder Methyl sind,
R^{10f} gleich Amino oder Hydroxy ist,
kf eine Zahl 0 oder 1 ist
und
lf, wf, xf und yf unabhängig voneinander eine Zahl 1, 2, 3 oder 4 sind,
d und e unabhängig voneinander eine Zahl 1, 2 oder 3 sind,
k eine Zahl 0 oder 1 ist,
l, w, x und y unabhängig voneinander eine Zahl 1, 2, 3 oder 4 sind, 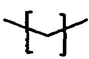
w ,x oder y
unabhängig voneinander bei w, x oder y gleich 3 eine
Hydroxy-Gruppe tragen kann,
oder eines ihrer Salze, ihrer Solvate oder der Solvate ihrer Salze.

2. Verbindung nach Anspruch 1, **dadurch gekennzeichnet, dass** sie der Formel 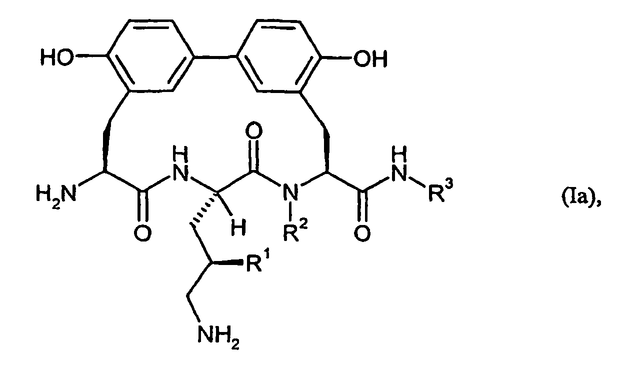 entspricht, in welcher
R¹ gleich Wasserstoff oder Hydroxy ist,
R² gleich Wasserstoff, Methyl oder Ethyl ist,
R³ wie in Anspruch 1 definiert ist, oder eines ihrer Salze, ihrer Solvate oder der Solvate ihrer Salze.

3. Verbindung nach Anspruch 1 oder 2, **dadurch gekennzeichnet, dass**
R³ gleich eine Gruppe der Formel 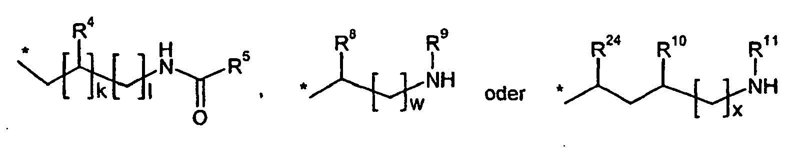
ist,
wobei
* die Anknüpfstelle an das Stickstoffatom ist,
R⁴ gleich Wasserstoff, Amino oder Hydroxy ist,
R⁵ eine Gruppe der Formel 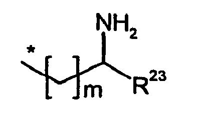
ist,
worin
* die Anknüpfstelle an das Kohlenstoffatom ist,
R²³ Wasserstoff oder eine Gruppe der Formel *-(CH₂)ₙ-OH oder *- (CH₂)ₒ-NH₂ ist,
worin
* die Anknüpfstelle an das Kohlenstoffatom ist,
n und o unabhängig voneinander eine Zahl 1, 2, 3 oder 4 sind,
m eine Zahl 0 oder 1 ist,
R⁸ eine Gruppe der Formel *-CONHR¹⁴ oder *-CH₂CONHR¹⁵ ist,
worin
* die Anknüpfstelle an das Kohlenstoffatom ist,
R¹⁴ und R¹⁵ unabhängig voneinander eine Gruppe der Formel 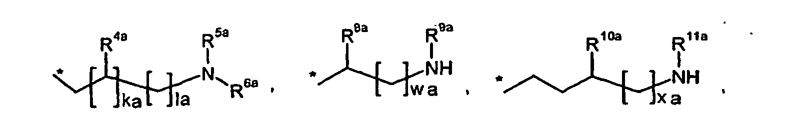 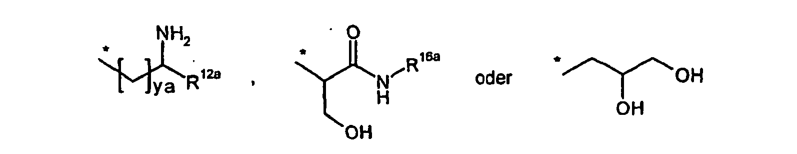
sind,
worin
* die Anknüpfstelle an das Stickstoffatom ist,
R^{4a} gleich Wasserstoff, Amino oder Hydroxy ist,
R^{5a} gleich Wasserstoff, Methyl oder Aminoethyl ist,
R^{6a} gleich Wasserstoff oder Aminoethyl ist,
oder
R^{5a} und R^{6a} bilden zusammen mit dem Stickstoffatom an das sie gebunden sind einen Piperazin-Ring,
R^{8a} und R^{12a} unabhängig voneinander ^{*}-(CH₂)_{Z1a-}OH, *-(CH₂)_{Z2a}-NHR^{13a}, *CONHR^{14a} oder *-CH₂CONHR^{15a} sind,
worin
* die Anknüpfstelle an das Kohlenstoffatom ist,
Z1a und Z2a unabhängig voneinander eine Zahl 1, 2 oder 3 sind,
R¹³⁸ gleich Wasserstoff oder Methyl ist und
R^{14a} und R^{15a} unabhängig voneinander eine Gruppe der Formel 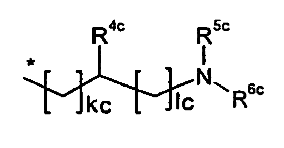
sind,
worin
* die Anknüpfstelle an das Stickstoffatom ist,
R^{4c} gleich Wasserstoff, Amino oder Hydroxy ist,
R^{5c} gleich Wasserstoff, Methyl oder Aminoethyl ist,
R^{6c} gleich Wasserstoff oder Aminoethyl ist,
kc eine Zahl 0 oder 1 ist
und
lc eine Zahl 1, 2, 3 oder 4 ist,
R⁹⁸ und R^{11a} unabhängig voneinander Wasserstoff oder Methyl sind,
R^{10a} gleich Amino oder Hydroxy ist,
R^{16a} eine Gruppe der Formel 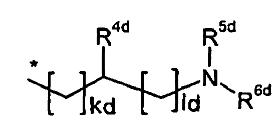
sind,
worin
* die Anknüpfstelle an das Stickstoffatom ist,
R^{4d} gleich Wasserstoff, Amino oder Hydroxy ist,
R^{5d} gleich Wasserstoff, Methyl oder Aminoethyl ist,
R^{6d} gleich Wasserstoff oder Aminoethyl ist,
kd eine Zahl 0 oder 1 ist
und
ld eine Zahl 1, 2, 3 oder 4 ist,
ka eine Zahl 0 oder 1 ist und
la, wa, xa und ya unabhängig voneinander eine Zahl 1, 2, 3 oder 4 sind,
R⁹ und R¹¹ unabhängig voneinander Wasserstoff, Methyl, *-C(NH₂)=NH oder eine Gruppe der Formel 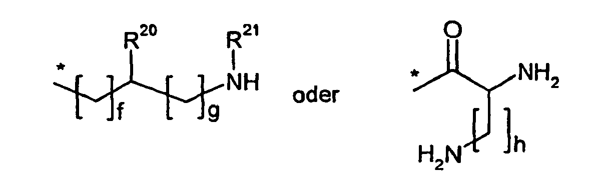
sind,
worin
* die Anknüpfstelle an das Stickstoffatom ist,
R²⁰ gleich Wasserstoff oder *-(CH₂)ᵢ-NHR²² ist,
worin
R²² gleich Wasserstoff oder Methyl ist und
i eine Zahl 1, 2 oder 3 ist,
R²¹ gleich Wasserstoff oder Methyl ist,
f eine Zahl 0, 1, 2 oder 3 ist,
g eine Zahl 1, 2 oder 3 ist
und
h eine Zahl 1, 2, 3 oder 4 ist,
oder
R⁸ gleich *-(CH₂)_{Z1}-OH ist,
worin
* die Anknüpfstelle an das Kohlenstoffatom ist,
Z1 eine Zahl 1, 2 oder 3 ist,
und
R⁹ eine Gruppe der Formel 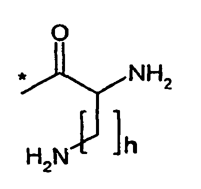
ist,
worin
* die Anknüpfstelle an das Stickstoffatom ist,
und
h eine Zahl 1, 2, 3 oder 4 ist,
R¹⁰ gleich Amino oder Hydroxy ist,
R²⁴ eine Gruppe der Formel *-CONHR²⁵ ist,
worin
* die Anknüpfstelle an das Kohlenstoffatom ist,
R²⁵ eine Gruppe der Formel 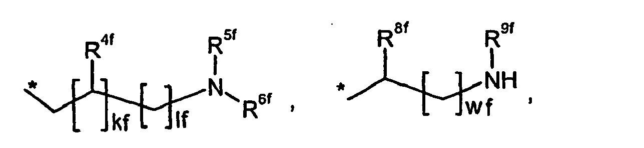 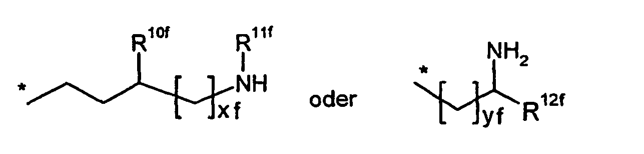
ist,
worin
* die Anknüpfstelle an das Stickstoffatom ist,
R^{4f} gleich Wasserstoff, Amino oder Hydroxy ist,
R^{5f} gleich Wasserstoff, Methyl oder Aminoethyl ist,
R^{6f} gleich Wasserstoff oder Aminoethyl ist,
oder
R^{5f} und R^{6f} bilden zusammen mit dem Stickstoffatom an das sie gebunden sind einen Piperazin-Ring,
R^{8f} und R^{12f} unabhängig voneinander ^{*}-(CH₂)_{Z1f}-OH oder
*-(CH₂)_{Z2f}-NHR^{13f} sind,
worin
* die Anknüpfstelle an das Kohlenstoffatom ist,
R^{13f} gleich Wasserstoff oder Methyl ist
und
Z1f und Z2f unabhängig voneinander eine Zahl 1, 2 oder 3 sind,
R^{9f} und R^{11f} unabhängig voneinander Wasserstoff oder Methyl sind,
R^{10f} gleich Amino oder Hydroxy ist,
kf eine Zahl 0 oder 1 ist
und
lf, wf, xf und yf unabhängig voneinander eine Zahl 1, 2, 3 oder 4 sind,
k eine Zahl 0 oder 1 ist,
1, w und x unabhängig voneinander eine Zahl 1, 2, 3 oder 4 sind, 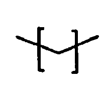
w oder x unabhängig voneinander bei w oder x gleich 3 eine Hydroxy- Gruppe tragen kann, oder eines ihrer Salze, ihrer Solvate oder der Solvate ihrer Salze.

4. Verbindung nach Anspruch 1 oder 2, **dadurch gekennzeichnet, dass**
R³ gleich eine Gruppe der Formel 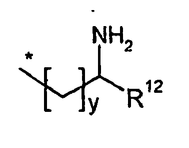
ist,
wobei
* die Anknüpfstelle an das Stickstoffatom ist,
R¹² eine Gruppe der Formel *-CONHR¹⁴ oder *-CH₂CONHR¹⁵ ist,
worin
* die Anknüpfstelle an das Kohlenstoffatom ist,
R¹⁴ und R¹⁵ unabhängig voneinander eine Gruppe der Formel 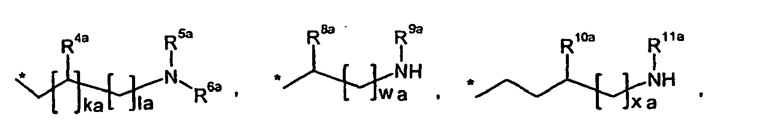 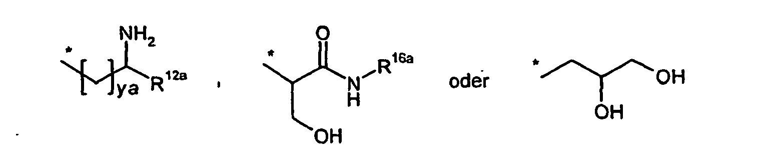
sind,
worin
* die Anknüpfstelle an das Stickstoffatom ist,
R^{4a} gleich Wasserstoff, Amino oder Hydroxy ist,
R^{5a} gleich Wasserstoff, Methyl oder Aminoethyl ist,
R^{6a} gleich Wasserstoff oder Aminoethyl ist,
oder
R^{5a} und R^{6a} bilden zusammen mit dem Stickstoffatom an das sie gebunden sind einen Piperazin-Ring,
R^{8a} und R^{12a} unabhängig voneinander *-(CH₂)_{Z1a}-OH, *-(CH₂)_{Z2a}-NHR^{13a}, *-CONHR^{14a} oder *-CH₂CONHR^{15a} sind,
worin
* die Anknüpfstelle an das Kohlenstoffatom ist,
Z1a und Z2a unabhängig voneinander eine Zahl 1, 2 oder 3 sind,
R^{13a} gleich Wasserstoff oder Methyl ist
und
R^{14a} und R^{15a} unabhängig voneinander eine Gruppe der Formel 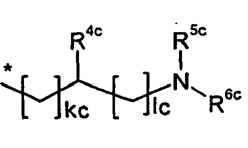
sind,
worin
* die Anknüpfstelle an das Stickstoffatom ist,
R^{4c} gleich Wasserstoff, Amino oder Hydroxy ist,
R^{5c} gleich Wasserstoff, Methyl oder Aminoethyl ist,
R^{6c} gleich Wasserstoff oder Aminoethyl ist,
kc eine Zahl 0 oder 1 ist
und
lc eine Zahl 1, 2, 3 oder 4 ist,
R^{9a} und R^{11a} unabhängig voneinander Wasserstoff oder Methyl sind,
R^{10a} gleich Amino oder Hydroxy ist,
R^{16a} eine Gruppe der Formel 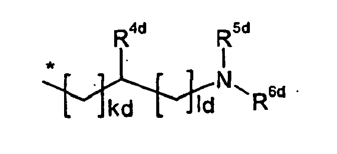
sind,
worin
* die Anknüpfstelle an das Stickstoffatom ist,
R^{4d} gleich Wasserstoff, Amino oder Hydroxy ist,
R^{5d} gleich Wasserstoff, Methyl oder Aminoethyl ist,
R^{6d} gleich Wasserstoff oder Aminoethyl ist,
kd eine Zahl 0 oder 1 ist
und
ld eine Zahl 1, 2, 3 oder 4 ist,
ka eine Zahl 0 oder 1 ist und
la, wa, xa und ya unabhängig voneinander eine Zahl 1, 2, 3 oder 4 sind,
y eine Zahl 1, 2, 3 oder 4 ist, 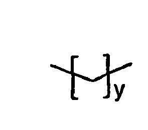
bei y gleich 3 eine Hydroxy-Gruppe tragen kann,
oder eines ihrer Salze, ihrer Solvate oder der Solvate ihrer Salze.

5. Verbindung nach Anspruch 1 oder 2, **dadurch gekennzeichnet, dass**
R³ gleich eine Gruppe der Formel 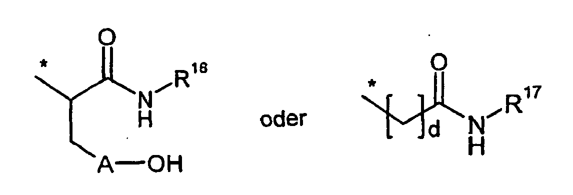
ist,
wobei
* die Anknüpfstelle an das Stickstoffatom ist,
A gleich eine Bindung oder Phenyl ist,
R¹⁶ und R¹⁷ unabhängig voneinander eine Gruppe der Formel 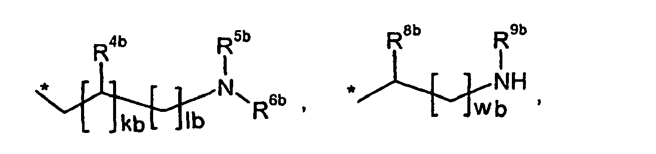 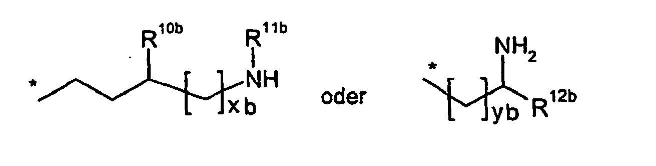
sind,
worin
* die Anknüpfstelle an das Stickstoffatom ist,
R^{4b} gleich Wasserstoff, Amino oder Hydroxy ist,
R^{5b} gleich Wasserstoff, Methyl oder Aminoethyl ist,
R^{6b} gleich Wasserstoff oder Aminoethyl ist,
oder
R^{5b} und R^{6b} bilden zusammen mit dem Stickstoffatom an das sie gebunden sind einen Piperazin-Ring,
R^{8b} und R^{12b} unabhängig voneinander *-(CH₂)_{Z1b}-OH oder *-(CH₂)_{Z2b}-NHR^{13b} sind,
worin
* die Anknüpfstelle an das Kohlenstoffatom ist,
R^{13b} gleich Wasserstoff oder Methyl ist
und Z1b und Z2b unabhängig voneinander eine Zahl 1, 2 oder 3 sind,
R^{9b} und R^{11b} unabhängig voneinander Wasserstoff oder Methyl sind,
R^{10b} gleich Amino oder Hydroxy ist,
kb eine Zahl 0 oder 1 ist,
lb, wb, xb und yb unabhängig voneinander eine Zahl 1, 2, 3 oder 4 sind,
d eine Zahl 1, 2 oder 3 ist,
oder eines ihrer Salze, ihrer Solvate oder der Solvate ihrer Salze.

6. Verbindung nach Anspruch 1 oder 2, **dadurch gekennzeichnet, dass**
R³ gleich eine Gruppe der Formel 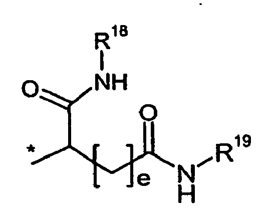
ist,
wobei
* die Anknüpfstelle an das Stickstoffatom ist,
R¹⁸ und R¹⁹ unabhängig voneinander Wasserstoff oder eine Gruppe der Formel 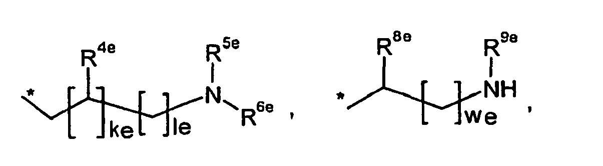 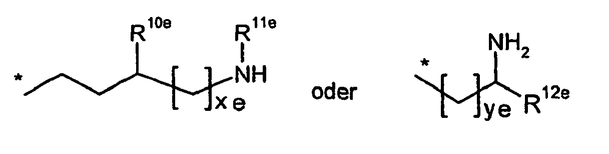
sind,
worin
* die Anknüpfstelle an das Stickstoffatom ist,
R^{4e} gleich Wasserstoff, Amino oder Hydroxy ist,
R^{5e} gleich Wasserstoff, Methyl oder Aminoethyl ist,
R^{6e} gleich Wasserstoff oder Aminoethyl ist, oder
R^{5e} und R^{6e} bilden zusammen mit dem Stickstoffatom an das sie gebunden sind einen Piperazin-Ring,
R^{8e} und R^{12e} unabhängig voneinander *-(CH₂)_{Z1e}-OH oder * -(CH₂)_{Z2c}-NHR^{13e} sind,
worin
* die Anknüpfstelle an das Kohlenstoffatom ist,
R^{13e} gleich Wasserstoff oder Methyl ist
und
Z1e und Z2e unabhängig voneinander eine Zahl 1, 2 oder 3 sind,
R^{9c} und R^{11e} unabhängig voneinander Wasserstoff oder Methyl sind,
R^{10c} gleich Amino oder Hydroxy ist,
ke eine Zahl 0 oder 1 ist
und
le, we, xe und ye unabhängig voneinander eine Zahl 1, 2, 3 oder 4 sind,
wobei R¹⁸ und R¹⁹ nicht gleichzeitig Wasserstoff sind,
e eine Zahl 1, 2 oder 3 ist,
oder eines ihrer Salze, ihrer Solvate oder der Solvate ihrer Salze.

7. Verfahren zur Herstellung einer Verbindung der Formel (I) nach Anspruch 1 oder eines ihrer Salze, Solvate oder der Solvate ihrer Salze, **dadurch gekennzeichnet, dass**
[A] eine Verbindung der Formel 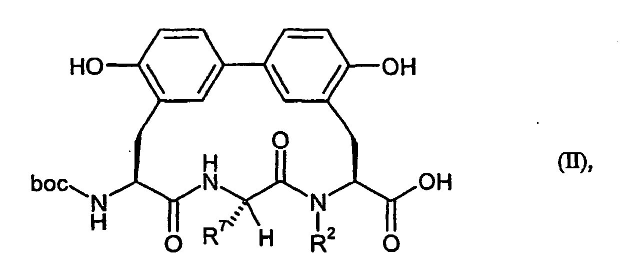 worin R² und R⁷ die in Anspruch 1 angegebene Bedeutung haben und boc gleich *tert-*Butoxycarbonyl ist,
in einem zweistufigen Verfahren zunächst in Gegenwart von einem oder mehreren Dehydratisierungsreagenzien mit einer Verbindung der Formel
H₂NR³ (ITI),
worin R³ die in Anspruch 1 angegebene Bedeutung hat,
und anschließend mit einer Säure und/oder durch Hydrogenolyse umgesetzt wird,
oder
[B] eine Verbindung der Formel 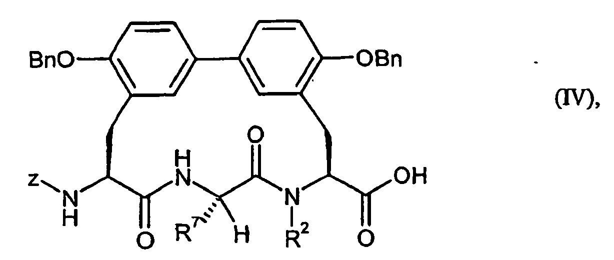 worin R² und R⁷ die in Anspruch 1 angegebene Bedeutung haben und Z gleich Benzyloxycarbonyl ist,
in einem zweistufigen Verfahren zunächst in Gegenwart von einem oder mehreren Dehydratisierungsreagenzien mit einer Verbindung der Formel
H₂NR₃ (III),
worin R³ die in Anspruch 1 angegebene Bedeutung hat,
und anschließend mit einer Säure oder durch Hydrogenolyse umgesetzt wird.

8. Verfahren zur Herstellung einer Verbindung der Formel (I) nach Anspruch 1 oder eines ihrer Solvate, **dadurch gekennzeichnet, dass** ein Salz der Verbindung oder ein Solvat eines Salzes der Verbindung durch Chromatographie unter Zusatz einer Base in die Verbindung überführt wird.

9. Verbindung nach einem der Ansprüche 1 bis 6 zur Behandlung und/oder Prophylaxe von Krankheiten.

10. Verwendung einer Verbindung nach einem der Ansprüche 1 bis 6 zur Herstellung eines Arzneimittels zur Behandlung und/oder Prophylaxe von Krankheiten.

11. Verwendung einer Verbindung nach einem der Ansprüche 1 bis 6 zur Herstellung eines Arzneimittels zur Behandlung und/oder Prophylaxe von bakteriellen Erkrankungen.

12. Arzneimittel enthaltend mindestens eine Verbindung nach einem der Ansprüche 1 bis 6 in Kombination mit mindestens einem inerten, nichttoxischen, pharmazeutisch geeigneten Hilfsstoff.

13. Arzneimittel nach Anspruch 12 zur Behandlung und/oder Prophylaxe von bakteriellen Infektionen.

14. Verwendung einer antibakteriell wirksamen Menge mindestens einer Verbindung nach einem der Ansprüche 1 bis 6 oder eines Arzneimittels nach Anspruch 12 oder 13 zur Herstellung eines Arzneimittels zur Bekämpfung von bakteriellen Infektionen in Menschen und Tieren.

## Claims

1. Compound of formula 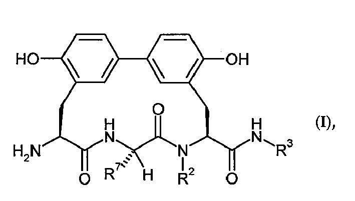 in which
R⁷ represents a group of formula 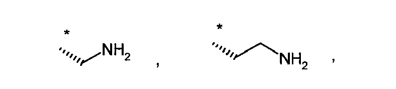 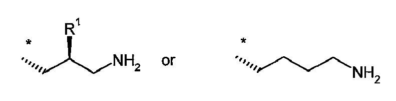
whereby
R¹ represents hydrogen or hydroxy,
* is the linkage site to the carbon atom,
R² represents hydrogen, methyl or ethyl,
R³ represents a group of formula 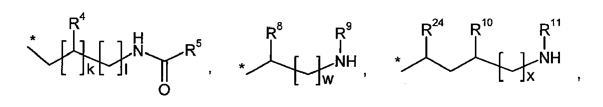 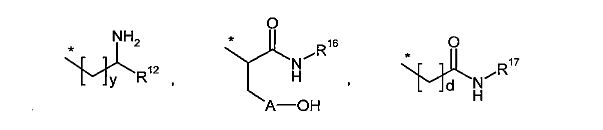
or 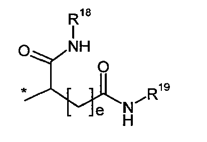 whereby
* is the linkage site to the nitrogen atom,
A represents a bond or phenyl,
R⁴ represents hydrogen, amino or hydroxy,
R⁵ represents a group of formula 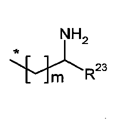
wherein
* is the linkage site to the carbon atom,
R²³ represents hydrogen or a group of formula *-(CH₂)ₙ-OH or *-(CH₂)ₒ-NH₂,
wherein
* is the linkage site to the carbon atom,
n and o independently of one another are a num- ber 1, 2, 3 or 4,
m is a number 0 or 1,
R⁸ and R¹² independently of one another represent a group of formula *-CONHR¹⁴ or *-CH₂CONHR¹⁵, wherein
* is the linkage site to the carbon atom,
R¹⁴ and R¹⁵ independently of one another represent a group of formula 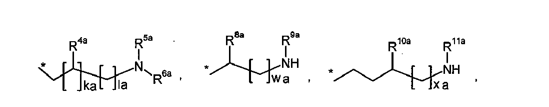 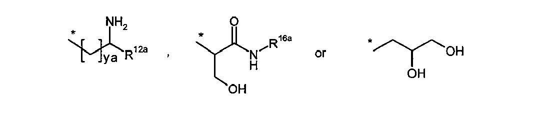
wherein
* is the linkage site to the nitrogen atom,
R^{4a} represents hydrogen, amino or hydroxy,
R^{5a} represents hydrogen, methyl or aminoethyl,
R^{6a} represents hydrogen or aminoethyl,
or
R^{5a} and R^{6a} together with the nitrogen atom to which they are bonded form a piperazine ring,
R^{8a} and R^{12a} independently of one another represent *-(CH₂)_{Z1a}-OH, *-(CH₂)_{Z2a}-NHR¹³, *-CONHR^{14a} or *-CH₂CONHR^{15a},
wherein
* is the linkage site to the carbon atom,
Z1a and Z2a independently of one another are a number 1, 2 or 3,
R^{13a} represents hydrogen or methyl,
and
R^{14a} and R^{15a} independently of one another repre- sent a group of formula 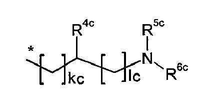
in which
* is the linkage site to the nitrogen atom,
R^{4c} represents hydrogen, amino or hydroxy,
R^{5c} represents hydrogen, methyl or aminoethyl,
R^{6c} represents hydrogen or aminoethyl,
kc is a number 0 or 1, and
lc is a number 1, 2, 3 or 4,
R^{9a} and R^{11a} independently of one another represent hydrogen or methyl,
R^{10a} represents amino or hydroxy,
R^{16a} represents a group of formula 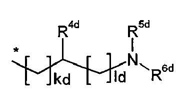
wherein
* is the linkage site to the nitrogen atom,
R^{4d} represents hydrogen, amino or hydroxy,
R^{5d} represents hydrogen, methyl or aminoethyl,
R^{6d} represents hydrogen or aminoethyl,
kd is a number 0 or 1,
and
ld is a number 1, 2, 3 or 4,
ka is a number 0 or 1,
and independently of one another are a number 1, 2, 3 or 4,
R⁹ and R¹¹ independently of one another represent hydrogen, methyl, *-C(NH₂)=NH or a group of formula 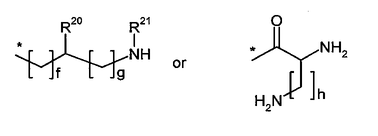
wherein
* is the linkage site to the nitrogen atom,
R²⁰ represents hydrogen or *-(CH₂)ᵢ-NHR²², wherein
R²² represents hydrogen or methyl,
and
i is a number 1, 2 or 3,
R²¹ represents hydrogen or methyl,
f is a number 0, 1, 2 or 3,
g is a number 1, 2 or 3,
and
h is a number 1, 2, 3 or 4,
or
R⁸ represents *-(CH₂)_{Z1}-OH,
wherein
* is the linkage site to the carbon atom,
Z1 is a number 1, 2 or 3,
and
R⁹ represents a group of formula 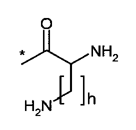
wherein
* is the linkage site to the nitrogen atom,
and
h is a number 1, 2, 3 or 4,
R¹⁰ represents amino or hydroxy,
R¹⁶ and R¹⁷ independently of one another represent a group of formula 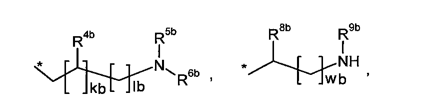 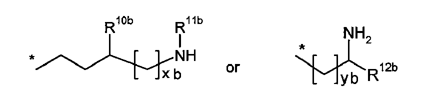
wherein
* is the linkage site to the nitrogen atom,
R^{4b} represents hydrogen, amino or hydroxy,
R^{5b} represents hydrogen, methyl or aminoethyl,
R^{6b} represents hydrogen or aminoethyl,
or
R^{5b} and R^{6b} together with the nitrogen atom to which they are bonded form a piperazine ring,
R^{8b} and R^{12b} independently of one another represent *-(CH₂)_{Z1b}-OH, *-(CH₂)_{Z2b}-NHR^{13b}, *-CONHR^{14b} or *-CH₂CONHR¹⁵b,
wherein
* is the linkage site to the carbon atom,
R^{13b} represents hydrogen or methyl,
and
Z1b and Z2b independently of one another are a number 1, 2 or 3, and
R^{14b} and R^{15b} independently of one another repre- sent a group of formula 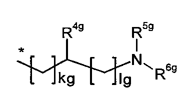
wherein
* is the linkage site to the nitrogen atom,
R^{4g} represents hydrogen, amino or hy- droxy,
R^{5g} represents hydrogen, methyl or ami- noethyl,
R^{6g} represents hydrogen or aminoethyl,
kg is a number 0 or 1,
and
lg is a number 1, 2, 3 or 4,
R^{9b} and R^{11b} independently of one another represent hydrogen or methyl,
R^{10b} represents amino or hydroxy,
kb is a number 0 or 1,
lb, wb, xb and yb independently of one another are a number 1, 2, 3 or 4,
R¹⁸ and R¹⁹ independently of one another represent hydrogen or a group of formula 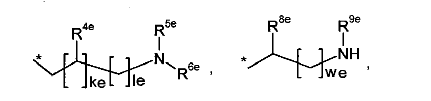 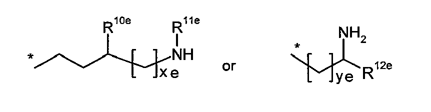
wherein
* is the linkage site to the nitrogen atom,
R^{4e} represents hydrogen, amino or hydroxy,
R^{5e} represents hydrogen, methyl or aminoethyl,
R^{6e} represents hydrogen or aminoethyl,
or
R^{5e} and R^{6e} together with the nitrogen atom to which they are bonded form a piperazine ring,
R^{8e} and R^{12e} independently of one another represent *-(CH₂)_{z1e}-OH or *-(CH_{z})_{z2e}-NHR^{13e},
wherein
* is the linkage site to the carbon atom,
R^{13e} represents hydrogen or methyl,
and
Z1e and Z2e independently of one another are a number 1, 2 or 3,
R^{9e} and R^{11e} independently of one another represent hydrogen or methyl,
R^{10e} represents amino or hydroxy,
ke is a number 0 or 1,
and
le, we, xe and ye independently of one another are a number 1, 2, 3 or 4,
whereby R¹⁸ and R¹⁹ are not simultaneously hydrogen,
R²⁴ represents a group of formula *-CONHR²⁵,
wherein
* is the linkage site to the carbon atom,
R²⁵ represents a group of formula 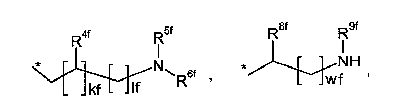 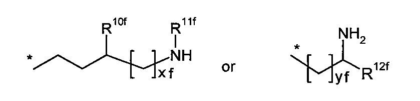
wherein
* is the linkage site to the nitrogen atom,
R^{4f} represents hydrogen, amino or hydroxy,
R^{5f} represents hydrogen, methyl or aminoethyl,
R^{6f} represents hydrogen or aminoethyl,
or
R^{5f} and R^{6f} together with the nitrogen atom to which they are bonded form a piperazine ring,
R^{8t} and R^{12f} independently of one another represent *-(CH₂)_{z1f}-OH or *-(CH₂)_{z2f}-NHR^{13f},
wherein
* is the linkage site to the carbon atom,
R^{13f} represents hydrogen or methyl,
and
Z1f and Z2f independently of one another are a number 1, 2 or 3,
R^{9f} and R^{11f} independently of one another represent hydrogen or methyl,
R^{10f} represents amino or hydroxy,
kf is a number 0 or 1,
If, wf, xf and yf independently of one another are a num- ber 1, 2, 3 or 4,
d and e independently of one another are a number 1, 2 or 3,
k is a number 0 or 1,
l, w, x and y independently of one another are a number 1, 2, 3 or 4, 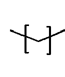
w, x or y independently of one another may when w, x or y equals 3 carry a hydroxy group, or one of its salts, its solvates or the solvates of its salts.

2. Compound according to claim 1, **characterized in that** it corresponds to formula 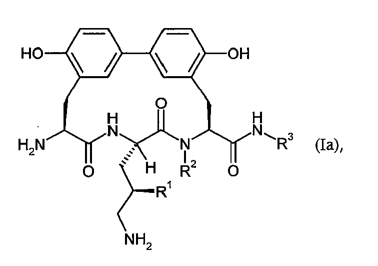 in which
R¹ represents hydrogen or hydroxy,
R² represents hydrogen, methyl or ethyl,
R³ is as defined in claim 1,
or one of its salts, its solvates or the solvates of its salts.

3. Compound according to claim 1 or 2, **characterized in that**
R³ represents a group of formula 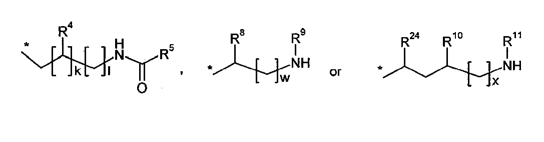
whereby
* is the linkage site to the nitrogen atom,
R⁴ represents hydrogen, amino or hydroxy,
R⁵ represents a group of formula 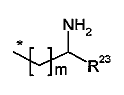
wherein
* is the linkage site to the carbon atom,
R²³ represents hydrogen or a group of formula *-(CH₂)ₙ-OH
or *-(CH₂)ₒ-NH₂,
wherein
* is the linkage site to the carbon atom,
n and o independently of one another are a number 1, 2, 3 or 4,
m is a number 0 or 1,
R⁸ represents a group of formula *-CONHR¹⁴ or *-CH₂CONHR¹⁵,
wherein
* is the linkage site to the carbon atom,
R¹⁴ and R¹⁵ independently of one another represent a group of formula 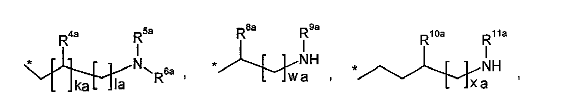 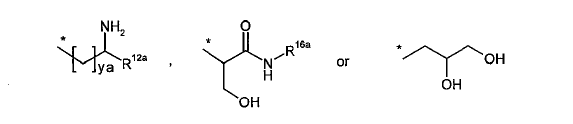
wherein
* is the linkage site to the nitrogen atom,
R^{4a} represents hydrogen, amino or hydroxy,
R^{5a} represents hydrogen, methyl or aminoethyl,
R^{6a} represents hydrogen or aminoethyl,
or
R^{5a} and R^{6a} together with the nitrogen atom to which they are bonded form a piperazine ring,
R^{8a} and R^{12a} independently of one another represent *-(CH₂)_{z1a-}OH, *-(CH2)_{Z2a}-NHR^{13a}, *-CONHR^{14a} or *-CH₂CONHR^{15a},
wherein
* is the linkage site to the carbon atom,
Z1a and Z2a independently of one another are a number 1, 2 or 3,
R^{13a} represents hydrogen or methyl,
and
R^{14a} and R^{15a} independently of one another repre- sent a group of formula 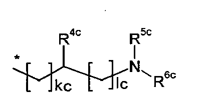
wherein
* is the linkage site to the nitrogen atom,
R^{4c} represents hydrogen, amino or hydroxy,
R^{5c} represents hydrogen, methyl or aminoethyl,
R^{6c} represents hydrogen or aminoethyl,
kc is a number 0 or 1,
and
lc is a number 1, 2, 3 or 4,
R^{9a} and R^{11a} independently of one another represent hydrogen or methyl,
R^{10a} represents amino or hydroxy,
R^{16a} represents a group of formula 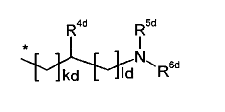
wherein
* is the linkage site to the nitrogen atom,
R^{4d} represents hydrogen, amino or hydroxy,
R^{5d} represents hydrogen, methyl or aminoethyl,
R^{6d} represents hydrogen or aminoethyl,
kd is a number 0 or 1,
and
ld is a number 1, 2, 3 or 4,
ka is a number 0 or 1,
and
la, wa, xa and ya independently of one another are a number 1, 2, 3 or 4,
R⁹ and R¹¹ independently or one another represent hydrogen, methyl, *-C(NH₂)=NH or a group of formula 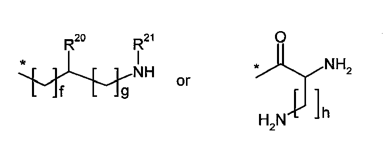
wherein
* is the linkage site to the nitrogen atom,
R²⁰ represents hydrogen or *-(CH₂)ᵢ-NHR²²,
wherein
R²² represents hydrogen or methyl,
and
i is a number 1, 2 or 3,
R²¹ represents hydrogen or methyl,
f is a number 0, 1, 2 or 3,
g is a number 1, 2 or 3,
and
h is a number 1, 2, 3 or 4,
or
R⁸ represents*-(CH₂)_{z1}-OH
wherein
* is the linkage site to the carbon atom,
Z1 is a number 1, 2 or 3,
and
R⁹ represents a group of formula 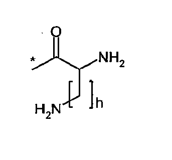
wherein
* is the linkage site to the nitrogen atom,
and
h is a number 1, 2, 3 or 4,
R¹⁰ represents amino or hydroxy,
R²⁴ represents a group of formula *-CONHR²⁵,
wherein
* is the linkage site to the carbon atom,
R²⁵ is a group of formula 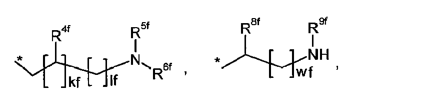 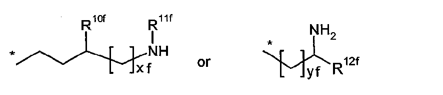
wherein
* is the linkage site to the nitrogen atom,
R^{4f} represents hydrogen, amino or hydroxy,
R^{5f} represents hydrogen, methyl or aminoethyl,
R^{6f} represents hydrogen or aminoethyl,
or,
R^{5f} and R^{6f} together with the nitrogen atom to which they are bonded form a piperazine ring,
R^{8f} and R^{12f} independently of one another represent *-(CH₂)_{z1f}-OH or *-(CH₂)_{z2f-}NHR^{13f},
wherein
* is the linkage site to the carbon atom,
R^{13f} represents hydrogen or methyl,
and Z1f and Z2f independently of one another are a number 1, 2 or 3,
R^{9f} and R^{11f} independently of one another represent hydrogen or methyl,
R^{10f} represents amino or hydroxy,
kf is a number 0 or 1,
and
If, wf, xf and yf independently of one another are a num- ber 1, 2, 3 or 4,
k is a number 0 or 1,
l, w and x independently of one another are a number 1, 2, 3 or 4, 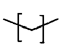
w or x independently of one another may when w or x equals 3 carry a hydroxy group,
or one of its salts, its solvates or the solvates of its salts.

4. Compound according to claim 1 or 2, **characterized in that**
R³ represents a group of formula 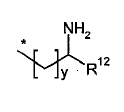
whereby
* is the linkage site to the nitrogen atom,
R¹² represents a group of formula *-CONHR¹⁴ or *-CH₂CONHR¹⁵,
wherein
* is the linkage site to the carbon atom,
R¹⁴ and R¹⁵ independently of one another represent a group of formula 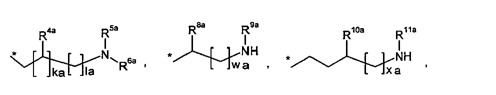 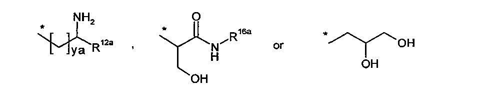
wherein
* is the linkage site to the nitrogen atom,
R^{4a} represents hydrogen, amino or hydroxy,
R^{5a} represents hydrogen, methyl or aminoethyl,
R^{6a} represents hydrogen or aminoethyl,
or
R^{5a} and R^{6a} together with the nitrogen atom to which they are bonded form a piperazine ring,
R^{8a} and R^{12a} independently of one another represent *-(CH₂)_{Z1a}-OH, *-(CH₂)_{z2a}-NHR^{13a}, *-CONHR^{14a} or *-CH₂CONHR^{15a},
wherein
* is the linkage site to the carbon atom,
Z1a and Z2a independently of one another are a number 1, 2 or 3,
R^{13a} represents hydrogen or methyl,
and
R^{14a} and R^{15a} independently of one another repre- sent a group of formula 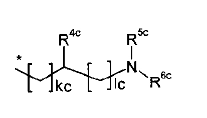
wherein
* is the linkage site to the nitrogen atom,
R^{4c} represents hydrogen, amino or hydroxy,
R^{5c} represents hydrogen, methyl or aminoethyl,
R^{6c} represents hydrogen or aminoethyl,
kc is a number 0 or 1,
and
lc is a number 1, 2, 3 or 4,
R^{9a} and R^{11a} independently of one another represent hydrogen or methyl,
R^{10a} represents amino or hydroxy,
R^{16a} represents a group of formula 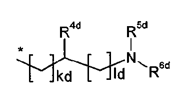
wherein
* is the linkage site to the nitrogen atom,
R^{4d} represents hydrogen, amino or hydroxy,
R^{5d} represents hydrogen, methyl or aminoethyl,
R^{6d} represents hydrogen or aminoethyl,
kd is a number 0 or 1,
and
ld is a number 1, 2, 3 or 4,
ka is a number 0 or 1,
and
la, wa, xa and ya independently of one another are a number 1, 2, 3 or 4,
y is a number 1, 2, 3 or 4, 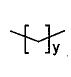
may when y equals 3 carry a hydroxy group,
or one of its salts, its solvates or the solvates of its salts.

5. Compound according to claim 1 or 2, **characterized in that**
R³ represents a group of formula 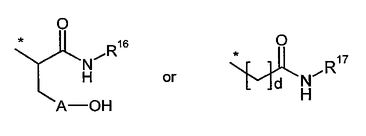
whereby
* is the linkage site to the nitrogen atom,
A represents a bond or phenyl,
R¹⁶ and R¹⁷ independently of one another represent a group of formula 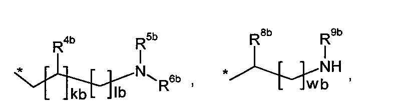 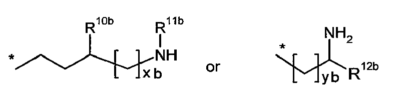
wherein
* is the linkage site to the nitrogen atom,
R^{4b} represents hydrogen, amino or hydroxy,
R^{5b} represents hydrogen, methyl or aminoethyl,
R^{6b} represents hydrogen or aminoethyl,
or
R^{5b} and R^{6b} together with the nitrogen atom to which they are bonded form a piperazine ring,
R^{8b} and R^{12b} independently of one another represent *-(CH₂)_{z1b}-OH or *-(CH₂)_{z2b}-NHR^{13b},
wherein
* is the linkage site to the carbon atom,
R^{13b} represents hydrogen or methyl,
and
Z1b and Z2b independently of one another are a number 1, 2 or 3,
R^{9b} and R^{11b} independently of one another represent hydrogen or methyl,
R^{10b} represents amino or hydroxy,
kb is a number 0 or 1,
lb, wb, xb and yb independently of one another are a number 1, 2, 3 or 4,
d is a number 1, 2 or 3,
or one of its salts, its solvates or the solvates of its salts.

6. Compound according to claim 1 or 2, **characterized in that**
R³ represents a group of formula 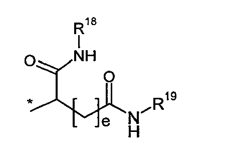
whereby
* is the linkage site to the nitrogen atom,
R¹⁸ and R¹⁹ independently of one another represent hydrogen or a group of formula 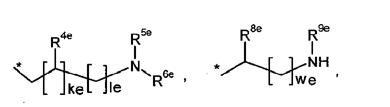 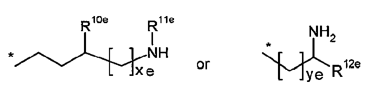
wherein
* is the linkage site to the nitrogen atom,
R^{4e} represents hydrogen, amino or hydroxy,
R^{5e} represents hydrogen, methyl or aminoethyl,
R^{6e} represents hydrogen or aminoethyl, or
R^{5e} and R^{6e} together with the nitrogen atom to which they are bonded form a piperazine ring,
R^{8e} and R^{12e} independently of one another represent *-(CH₂)_{z1e}-OH or *-(CH₂)_{z2e}-NHR^{13e},
wherein
* is the linkage site to the carbon atom,
R^{13e} represents hydrogen or methyl,
and
Z1e and Z2e independently of one another are a number 1, 2 or 3,
R^{9e} and R^{11e} independently of one another represent hydrogen or methyl,
R^{10e} represents amino or hydroxy,
ke is a number 0 or 1,
and
le, we, xe and ye independently of one another are a number 1, 2, 3 or 4,
whereby R¹⁸ and R¹⁹are not simultaneously hydrogen,
e is a number 1, 2 or 3,
or one of its salts, its solvates or the solvates of its salts.

7. Method for preparing a compound of formula (I) according to claim 1 or one of its salts, its solvates or the solvates of its salts, **characterized in that**
[A] a compound of formula 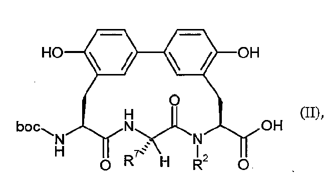 wherein R² and R⁷ have the meaning indicated in claim 1, and boc represents *tert*-butoxycarbonyl,
is reacted in a two-stage process firstly in the presence of one or more dehydrating reagents with a compound of formula
H₂NR³ (III),
wherein R³ has the meaning indicated in claim 1,
and subsequently with an acid and/or by hydrogenolysis,
or
[B] a compound of formula 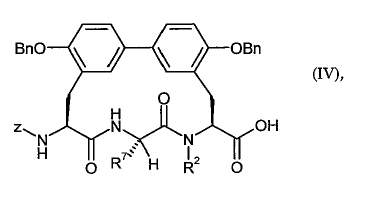 wherein R² and R⁷ have the meaning indicated in claim 1, and Z represents benzyloxycarbonyl,
is reacted in a two-stage process firstly in the presence of one or more dehydrating reagents with a compound of formula
H₂NR³ (III),
wherein R³ has the meaning indicated in claim 1,
and subsequently with an acid or by hydrogenolysis.

8. Method for preparing a compound of formula (I) according to claim 1 or one of its solvates, **characterized in that** a salt of the compound or a solvate of a salt of the compound is converted into the compound by chromatography with the addition of a base.

9. Compound according to any one of claims 1 to 6 for the treatment and/or prophylaxis of diseases.

10. Use of a compound according to any one of claims 1 to 6 for the manufacture of a medicament for the treatment and/or prophylaxis of diseases.

11. Use of a compound according to any one of claims 1 to 6 for the manufacture of a medicament for the treatment and/or prophylaxis of bacterial diseases.

12. Medicament comprising at least one compound according to any one of claims 1 to 6 in combination with at least one inert, non-toxic, pharmaceutically acceptable excipient.

13. Medicament according to claim 12 for the treatment and/or prophylaxis of bacterial infections.

14. Use of an antibacterially effective amount of at least one compound according to any one of claims 1 to 6 or of a medicament according to claim 12 or 13 for the manufacture of a medicament for controlling bacterial infections in humans and animals.

## Revendications

1. Composé de la formule 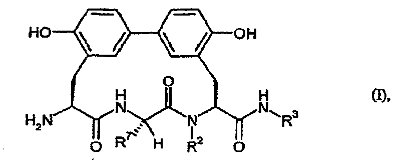 dans laquelle
R⁷ signifie un groupe de la formule 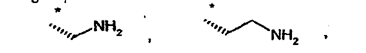 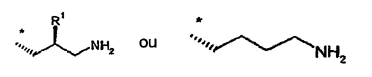
dans laquelle
R¹ signifie un hydrogène ou un hydroxyle,
* est l'emplacement de la liaison à l'atome de carbone,
R² signifie un hydrogène, un méthyle ou un éthyle,
R³ signifie un groupe de la formule 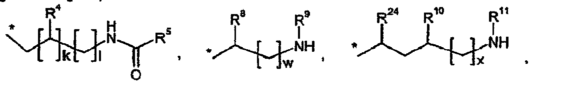 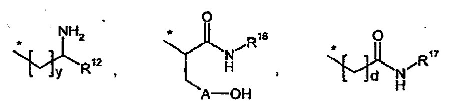
ou 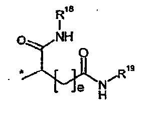 dans laquelle
* est l'emplacement de la liaison à l'atome d'azote,
A signifie une liaison ou un phényle,
R⁴ signifie un hydrogène, un amino ou un hydroxyle,
R⁵ est un groupe de la formule 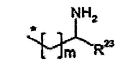
dans laquelle
* est l'emplacement de la liaison à l'atome de carbone,
R²³ est un hydrogène ou un groupe de la formule *-(CH₂)ₙ-OH ou *- (CH₂)ₒ-NH₂, sachant que
* est l'emplacement de la liaison à l'atome de carbone, n et o signifient indépendamment l'un de l'autre un nombre 1, 2, 3 ou 4,
m est un nombre 0 ou 1,
R⁸ et R¹² signifient indépendamment l'un de l'autre un groupe de la formule *-- CONHR¹⁴ ou *-CH₂CONHR¹⁵,
sachant que
* est l'emplacement de la liaison à l'atome de carbone,
R¹⁴ et R¹⁵ signifient indépendamment l'un de l'autre un groupe de la formule 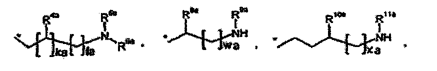 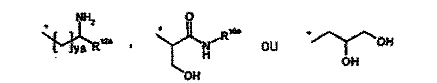
dans laquelle
* est l'emplacement de la liaison à l'atome d'azote,
R^{4a} signifie un hydrogène ; un amino ou un hydroxyle,
R^{5a} signifie un hydrogène, un méthyle ou un aminoéthyle,
R^{6a} signifie un hydrogène ou un aminoéthyle,
ou
R^{5a} et R^{6a} forment conjointement avec l'atome d'azote auquel ils sont liés un cycle pipérazine,
R^{8a} et R^{12a} signifient indépendamment l'un de l'autre *—(CH₂)_{z1a}- OH, *-(CH₂)_{Z2a}-NHR^{13a}, *-CONHR^{14a} ou *-CH₂CONHR^{15a},
sachant que
* est l'emplacement de la liaison à l'atome de carbone,
Z1a et Z2a signifient indépendamment l'un de l'autre un nombre
R^{13a} signifie 1,2 ou 3, un hydrogène ou un méthyl
et
R^{14a} et R^{15a} signifient indépendamment l'un de l'autre un groupe de la formule 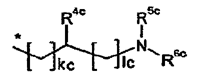
dans laquelle
* est l'emplacement de la liaison à l'atome d'azote,
R^{4c} signifie un hydrogène, un amino ou un hydroxyle,
R^{5c} signifie un hydrogène, un méthyle ou un aminoéthyle,
R^{6c} signifie un hydrogène ou un aminoéthyle,
kc est un nombre 0 ou 1
et
lc est un nombre 1, 2, 3 ou 4,
R^{9a} et R^{11a} signifient indépendamment l'un de l'autre un hydrogène ou un méthyl,
R^{10a} est un amino ou un hydroxyle,
R^{16a} est un groupe de la formule 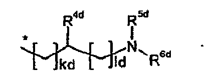
dans laquelle
* est l'emplacement de la liaison à l'atome d'azote,
R^{4d} signifie un hydrogène, un amino ou un hydroxyle,
R^{5d} signifie un hydrogène, un méthyle ou un aminoéthyle,
R^{6d} signifie un hydrogène ou un aminoéthyle,
kd est un nombre 0 ou 1
et
ld est un nombre 1, 2, 3 ou 4,
ka est un nombre 0 ou 1 et
la, wa, xa et ya, sont indépendamment l'un de l'autre un nombre 1, 2, 3 ou 4,
R⁹ et R¹¹ signifient indépendamment l'un de l'autre un hydrogène, un méthyl, un *-C(NH₂)=NH ou un groupe de la formule 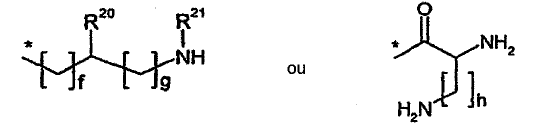
dans laquelle
* est l'emplacement de la liaison à l'atome d'azote,
R²⁰ signifie un hydrogène ou *-(CH₂)ᵢ-NHR²²,
sachant que
R²² signifie un hydrogène ou un méthyl
et
i est un nombre 1, 2 ou 3,
R²¹ signifie un hydrogène ou un méthyl,
f est un nombre 0, 1, 2 ou 3,
g est un nombre 1, 2 ou 3
et
h est un nombre 1, 2, 3 ou 4,
ou
R⁸ signifie *-(CH₂)_{z1}-OH,
sachant que
* est l'emplacement de la liaison à l'atome de carbone,
Z1 est un nombre 1, 2 ou 3,
et
R⁹ est un groupe de la formule 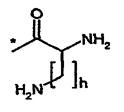
dans laquelle
* est l'emplacement de la liaison à l'atome d'azote,
et
h est un nombre 1, 2, 3 ou 4,
R¹⁰ signifie un amino ou un hydroxyle,
R¹⁶ et R¹⁷ signifient indépendamment l'un de l'autre un groupe de la formule 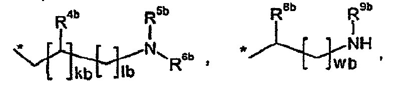 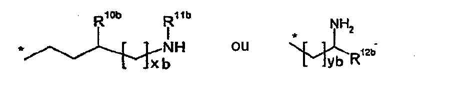
dans laquelle
* est l'emplacement de la liaison à l'atome d'azote,
R^{4b} signifie un hydrogène, un amino ou un hydroxyle,
R^{5b} signifie un hydrogène, un méthyle ou un aminoéthyle,
R^{6b} signifie un hydrogène ou un aminoéthyle,
ou
R^{5b} et R^{6b} forment conjointement avec l'atome d'azote auquel ils sont liés un cycle pipérazine,
R^{8b} et R^{12b} signifient indépendamment l'un de l'autre *-(CH₂)_{z1b}-OH, *-(CH₂)_{Z2b}-NHR^{13b}, *-CONHR^{14b} ou *-CH₂CONHR^{15b},
sachant que
* est l'emplacement de la liaison à l'atome de carbone,
R^{13b} signifie un hydrogène ou un méthyl et
Z1b etZ2b signifient indépendamment l'un de l'autre un nombre 1,2ou3,
et
R¹⁴b et R^{15b} signifient indépendamment l'un de l'autre un groupe de la formule 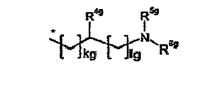
dans laquelle
* est l'emplacement de la liaison à l'atome d'azote,
R^{4g} signifie un hydrogène, un amino ou un hydroxyle,
R^{5g} signifie un hydrogène, un méthyle ou un aminoéthyle,
R^{6g} signifie un hydrogène ou un aminoéthyle,
kg est un nombre 0 ou 1
et
lg est un nombre 1, 2, 3 ou 4,
R^{9b} et R^{11b}, signifient indépendamment l'un de l'autre un hydrogène ou un méthyle,
R^{10b} signifie un amino ou un hydroxyle,
kb est un nombre 0 ou 1,
lb, wb, xb et yb signifient indépendamment l'un de l'autre, un nombre 1, 2, 3 ou 4,
R¹⁸ et R¹⁹ sont, indépendamment l'un de l'autre, un hydrogène ou un groupe de la formule 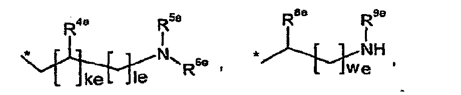 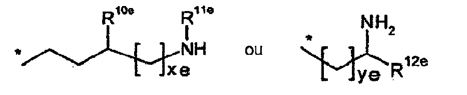
dans laquelle
* est l'emplacement de la liaison à l'atome d'azote,
R^{4e} signifie un hydrogène, un amino ou un hydroxyle,
R^{5e} signifie un hydrogène, un méthyle ou un aminoéthyle,
R^{6e} signifie un hydrogène ou un aminoéthyle,
ou
R^{5e} et R^{8e} forment conjointement avec l'atome d'azote auquel ils sont liés un cycle pipérazine
R^{8e}et R^{12e} signifient indépendamment l'un de l'autre *-(CH₂)_{Z1e}-OH ou
*-(CH₂)_{z2e}NHR^{13e}, sachant que
* est l'emplacement de la liaison à l'atome de carbone,
R^{13e} signifie un hydrogène ou un méthyle
et
Z1e et Z2e signifient indépendamment l'un de l'autre un nombre 1,2ou3,
R^{9e} et R^{11e} signifient indépendamment l'un de l'autre un hydrogène ou un méthyle,
R^{10e} signifie un amino ou un hydroxyle,
ke est un nombre 0 ou 1
et
le, we, xe et ye sont indépendamment l'un de l'autre un nombre 1, 2, 3 ou 4,
sachant que R¹⁸ et R¹⁹ ne sont pas en même temps des hydrogènes,
R²⁴ est un groupe de la formule *-CONHR²⁵,
dans laquelle
* est remplacement de la liaison à l'atome de carbone,
R²⁵ est un groupe de la formule 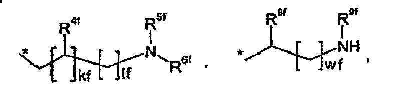 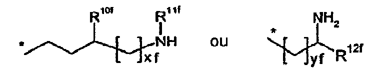
dans laquelle
* est l'emplacement de la liaison à l'atome d'azote,
R^{4f} signifie un hydrogène, un amino ou un hydroxyle,
R^{5f} signifie un hydrogène, un méthyle ou un aminoéthyle,
R^{6f} signifie un hydrogène ou un aminoéthyle,
ou
R^{5f} et R^{6f} forment conjointement avec l'atome d'azote auquel ils sont liés un cycle pipérazine,
R^{8f} et R^{12f} signifient indépendamment l'un de l'autre *-(CH₂)_{Z1f}-OH ou
*-(CH₂)_{z2f}NHR^{13f}, sachant que
* est l'emplacement de la liaison à l'atome de carbone, R^{13f}signifie un hydrogène ou un méthyle
et
Z1f et Z2f signifient indépendamment l'un de l'autre un nombre 1, 2 ou 3,
R^{9f} et R^{11f} signifient indépendamment l'un de l'autre un hydrogène ou un méthyle,
R^{10f} signifie un amino ou un hydroxyle,
kf est un nombre 0 ou 1
et
If, wf, xf et yf signifient indépendamment l'un de l'autre un nombre 1, 2, 3 ou 4,
d et e signifient indépendamment l'un de l'autre un nombre 1, 2 ou 3,
k est un nombre 0 ou 1,
1, w, x et y signifient indépendamment l'un de l'autre un nombre 1, 2, 3 ou 4, 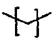
w, x ou y pouvent porter indépendamment l'un de l'autre un groupe hydroxyle quand w, x ou y sont égaux à 3,
ou un de ses sels, de ses solvates ou des solvates de ses sels.

2. Composé selon la revendication 1, **caractérisé en ce qu'**elle correspond à la formule 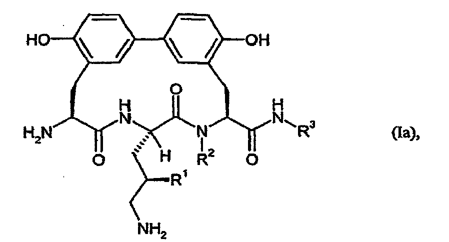 dans laquelle
R¹ signifie un hydrogène ou un hydroxyle,
R² signifie un hydrogène, un méthyle ou un éthyle,
R³ est défini comme dans la revendication 1, ou un de ses sels, de ses solvates ou des solvates de ses sels.

3. Composé selon la revendication 1 ou 2, **caractérisé en ce que**
R³ signifie un groupe de la formule 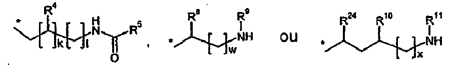
dans laquelle
* est l'emplacement de la liaison à l'atome d'azote,
R⁴ signifie un hydrogène, un amino ou un hydroxyle,
R⁵ est un groupe de la formule 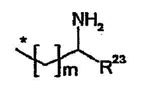
dans laquelle
* est remplacement de la liaison à l'atome de carbone,
R²³ est un hydrogène ou un groupe de la formule *-(CH₂)ₙ-OH ou
* -(CH₂)ₒ-NH₂, sachant que
* est l'emplacement de la liaison à l'atome de carbone,
n et o sont indépendamment l'un de l'autre un nombre 1, 2, 3 ou 4,
m est un nombre 0 ou 1,
R⁸ est un groupe de la formule *-CONHR¹⁴ ou *-CH₂CONHR¹⁵, sachant que * est l'emplacement de la liaison à l'atome de carbone, R¹⁴ et R¹⁵ signifient, indépendamment l'un de l'autre, un groupe de la formule 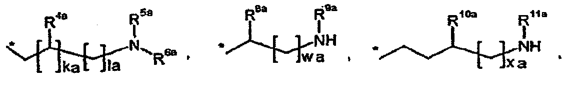 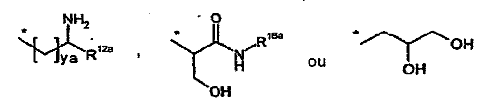
dans laquelle
* est l'emplacement de la liaison à l'atome d'azote,
R^{4a} signifie un hydrogène, un amino ou un hydroxyle,
R^{5a} signifie un hydrogène, un méthyle ou un aminoéthyle,
R^{6a} signifie un hydrogène ou un aminoéthyle,
ou
R^{5a} et R^{6a} forment conjointement avec l'atome d'azote auquel ils sont liés un cycle pipérazine,
R^{6a} et R^{12a} signifient indépendamment l'un de l'autre *-(CH₂)_{z1a}-OH, *-(CH₂)_{z2a}-NHR^{13a}, *-CONHR^{14a} ou *-CH₂CONHR^{15a},
sachant que
* est l'emplacement de la liaison à l'atome de carbone,
Z1a et Z2a signifient indépendamment l'un de l'autre un nombre 1,2ou3,
R^{13a} signifie un hydrogène ou un méthyle et
R¹⁴a et R^{15a} sont indépendamment l'un de l'autre un groupe de la formule 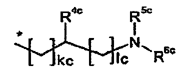
dans laquelle
* est l'emplacement de la liaison à l'atome d'azote,
R^{4c} signifie un hydrogène, un amino ou un hydroxyle,
R^{5c} signifie un hydrogène, un méthyle ou un aminoéthyle,
R^{6c} signifie un hydrogène ou un aminoéthyle,
kc est un nombre 0 ou 1
et
lc est un nombre 1, 2, 3 ou 4,
R^{9a} et R^{11a} signifient indépendamment l'un de l'autre un hydrogène ou un méthyle,
R^{10a} signifie un amino ou un hydroxyle,
R^{16a} est un groupe de la formule 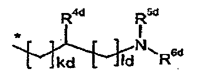
dans laquelle
* est l'emplacement de la liaison à l'atome d'azote,
R^{4d} signifie un hydrogène, un amino ou un hydroxyle,
R^{5d} signifie un hydrogène, un méthyle ou un aminoéthyle,
R^{6d} signifie un hydrogène ou un aminoéthyle,
kd est un nombre 0 ou 1
et
ld est un nombre 1, 2, 3 ou 4,
ka est un nombre 0 ou 1
et
la, wa, xa et ya sont indépendamment l'un de l'autre un nombre 1, 2, 3 ou 4,
R⁹ et R¹¹ sont indépendamment l'un de l'autre un hydrogène, un méthyle, un
* —C(NH₂)=NH ou un groupe de la formule 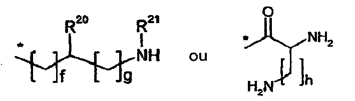
dans laquelle
* est l'emplacement de la liaison à l'atome d'azote,
R²⁰ signifie un hydrogène ou un *-(CH₂)ᵢ-NHR²²,
sachant que
R²² signifie un hydrogène ou un méthyle
et
i est un nombre 1, 2 ou 3,
R²¹ signifie un hydrogène ou un méthyle,
f est un nombre 0, 1, 2 ou 3,
g est un nombre 1, 2 ou 3
et
h est un nombre 1, 2, 3 ou 4,
ou
R⁸ signifie *-(CH₂)_{Z1}-OH,
sachant que
* est l'emplacement de la liaison à l'atome de carbone,
Z1 est un nombre 1, 2 ou 3,
et
R⁹ est un groupe de la formule 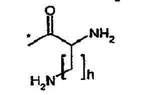
dans laquelle
* est l'emplacement de la liaison à l'atome d'azote,
et
h est un nombre 1, 2, 3 ou 4,
R¹⁰ signifie un amino ou un hydroxyle,
R²⁴ est un groupe de la formule *-CONHR²⁵,
dans laquelle
* est l'emplacement de la liaison à l'atome de carbone,
R²⁵ est un groupe de la formule 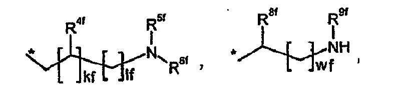 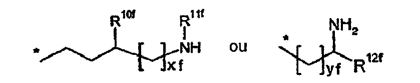
dans laquelle
* est l'emplacement de la liaison à l'atome d'azote,
R^{4f} signifie un hydrogène, un amino ou un hydroxyle,
R^{5f} signifie un hydrogène, un méthyle ou un aminoéthyle,
R^{6f} signifie un hydrogène ou un aminoéthyle,
ou
R^{5f} et R^{6f} forment conjointement avec l'atome d'azote auquel ils sont liés un cycle pipérazine,
R^{8f} et R^{12f} signifient indépendamment l'un de l'autre un
* ou un *-(CH₂)_{z2f}-NHR^{13f}, sachant que
* est l'emplacement de la liaison à l'atome de carbone,
R^{13f} signifie un hydrogène ou un méthyle et
Z1f et Z2f signifient indépendamment l'un de l'autre un nombre 1, 2 ou 3,
R^{9f} et R^{11f} signifient indépendamment l'un de l'autre un hydrogène
o u un méthyle,
R^{10f} signifie un amino ou un hydroxyle,
kf est un nombre 0 ou 1
et
If, wf, xf et yf sont indépendamment l'un de l'autre un nombre 1, 2, 3 ou 4,
k est un nombre 0 ou 1,
l, w et x sont, indépendamment l'un de l'autre, un nombre 1, 2, 3 ou 4, 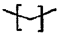
w ou x peuvent porter indépendamment l'un de l'autre un groupe hydroxyle quand w ou x sont égaux à 3 ou un de ses sels, de ses solvates ou des solvates de ses sels.

4. Composé selon la revendication 1 ou 2, **caractérisé en ce que**
R³ signifie un groupe de la formule 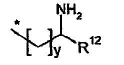
dans laquelle
* est l'emplacement de la liaison à l'atome d'azote,
R¹² est un groupe de la formule *—CONHR¹⁴ ou *-CH₂CONHR¹⁵, sachant que * est l'emplacement de la liaison à l'atome de carbone, R¹⁴ et R¹⁵ signifient indépendamment l'un de l'autre un groupe des formules 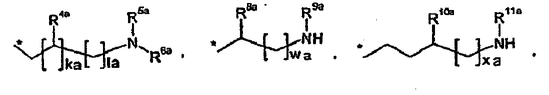 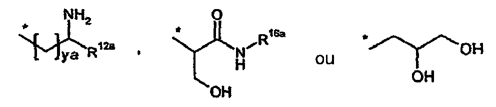
dans lesquelles
* est l'emplacement de la liaison à l'atome d'azote,
R^{4a} signifie un hydrogène, un amino ou un hydroxyle,
R^{5a} signifie un hydrogène, un méthyle ou un aminoéthyle,
R^{6a} signifie un hydrogène ou un aminoéthyle, ou
R^{5a} et R^{6a} forment conjointement avec l'atome d'azote auquel ils sont liés un cycle pipérazine,
R^{8a} et R^{12a} signifient indépendamment l'un de l'autre *-(CH₂)_{z1a}OH, *-(CH₂)_{z2a}-NHR^{13a}, *-CONHR^{14a} ou *-CH₂CONHR^{15a},
sachant que
* est remplacement de la liaison à l'atome de carbone,
Z1a et Z2a sont indépendamment l'un de l'autre un nombre 1, 2 ou 3,
R^{13a} signifie un hydrogène ou un méthyle
et
R^{14a} et R^{15a} signifient indépendamment l'un de l'autre un groupe de la formule 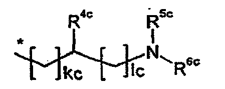
dans laquelle
* est l'emplacement de la liaison à l'atome d'azote,
R^{4c} signifie un hydrogène, un amino ou un hydroxyle,
R^{5c} signifie un hydrogène, un méthyle ou un aminoéthyle,
R^{6c} signifie un hydrogène ou un aminoéthyle,
kc est un nombre 0 ou 1
et
lc est un nombre 1, 2, 3 ou 4,
R^{9a} et R^{11a} signifient indépendamment l'un de l'autre un hydrogène ou un méthyle,
R^{10a} signifie un amino ou un hydroxyle,
R^{16a} est un groupe de la formule 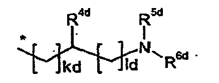
dans laquelle
* est l'emplacement de la liaison à l'atome d'azote,
R^{4d} signifie un hydrogène, un amino ou un hydroxyle,
R^{5d} signifie un hydrogène, un méthyle ou un aminoéthyle,
R^{6d} signifie un hydrogène ou un aminoéthyle,
kd est un nombre 0 ou 1
et
ld est un nombre 1, 2, 3 ou 4, ka est un nombre 0 ou 1 et la, wa, xa et ya signifient indépendamment l'un de l'autre un nombre 1, 2, 3 ou 4,
y est un nombre 1, 2, 3 ou 4,
y peut porter un groupe hydroxyle quand y est égal à 3, ou un de ses sels, de ses solvates ou des solvates de ses sels.

5. Composé selon la revendication 1 ou 2, **caractérisé en ce que**
R³ signifie un groupe de la formule 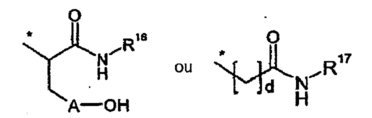
dans laquelle
* est l'emplacement de la liaison à l'atome d'azote,
A signifie une liaison ou un phényle,
R¹⁶ et R¹⁷ signifient indépendamment l'un de l'autre un groupe de la formule 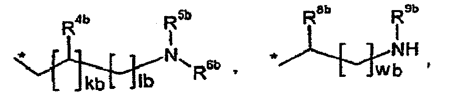 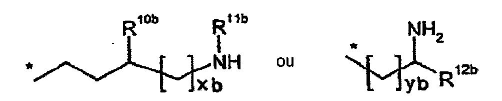
dans laquelle
* est l'emplacement de la liaison à l'atome d'azote,
R^{4b} signifie un hydrogène, un amino ou un hydroxyle,
R ^{5b} signifie un hydrogène, un méthyle ou un aminoéthyle,
R^{6b} signifie un hydrogène ou un aminoéthyle,
ou
R^{5b} et R^{6b} forment conjointement avec l'atome d'azote auquel ils sont liés un cycle pipérazine,
R^{8b} et R^{12b} signifient indépendamment l'un de l'autre *-(CH₂)_{z1b}-OH ou *-(CH₂)_{Z2b}-NHR^{13b},
sachant que
* est l'emplacement de la liaison à l'atome de carbone,
R^{13b} signifie un hydrogène ou un méthyle
et
Z1b et Z2b signifient indépendamment l'un de l'autre un nombre 1, 2 ou 3,
R^{9b} et R^{11b} signifient indépendamment l'un de l'autre un hydrogène ou un méthyle, R^{10b} signifie un amino ou un hydroxyle, kb est un nombre 0 ou 1, lb, wb, xb et yb signifient indépendamment l'un de l'autre un nombre 1, 2, 3 ou 4,
d est un nombre 1, 2 ou 3,
ou un de ses sels, de ses solvates ou des solvates de ses sels.

6. Composé selon la revendication 1 ou 2, **caractérisé en ce que**
R³ signifie un groupe de la formule 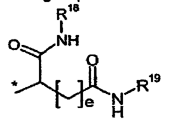
dans laquelle
* est l'emplacement de la liaison à l'atome d'azote,
R¹⁸ et R¹⁹ signifient indépendamment l'un de l'autre un hydrogène ou un groupe de la formule 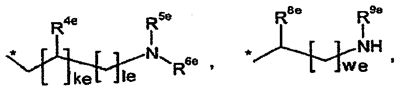 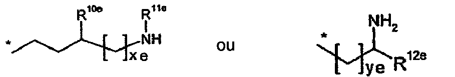
dans laquelle
* est l'emplacement de la liaison à l'atome d'azote,
R^{4e} signifie un hydrogène, un amino ou un hydroxyle,
R^{5e} signifie un hydrogène, un méthyle ou un aminoéthyle,
R^{6e} signifie un hydrogène ou un aminoéthyle,
ou
R^{5e} et R^{6e} forment conjointement avec l'atome d'azote auquel ils sont liés un cycle pipérazine,
R^{8e} et R^{12e} signifient indépendamment l'un de l'autre *-(CH₂)_{z1e}-OH ou *-(CH₂)_{z2e}-NHR^{13e}, sachant que * est l'emplacement de la liaison à l'atome de carbone, R^{13e} signifie un hydrogène ou un méthyle et Z1e et Z2e signifient indépendamment l'un de l'autre un nombre 1, 2 ou 3,
R^{9e} et R^{11e} signifient indépendamment l'un de l'autre un hydrogène ou un méthyle, R^{10e} signifie un amino ou un hydroxyle, ke est un nombre 0 ou 1 et le, we, xe et ye signifient indépendamment l'un de l'autre un nombre 1, 2, 3 ou 4, sachant que R¹⁸ et R¹⁹ ne sont pas en même temps un hydrogène,
e est un nombre 1, 2 ou 3,
ou un de ses sels, de ses solvates ou des solvates de ses sels.

7. Procédé pour la préparation d'un composé de la formule (I) selon la revendication 1, ou d'un de ses sels, de ses solvates ou des solvates de ses sels, **caractérisé en ce que**
[A] on fait réagir un composé de la formule 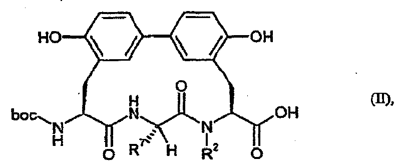 dans laquelle R² et R⁷ ont la signification spécifiée dans la revendication 1 et boc signifie un tert-butoxycarbonyle,
dans un procédé biétagé, d'abord en présence d'un ou de plusieurs réactifs déshydratants, avec un composé de la formule
H₂NR³ (III),
dans laquelle R³ a la signification spécifiée dans la revendication 1,
et ensuite avec un acide et/ou par hydrogénolyse,
ou
[B] on fait réagir un composé de la formule 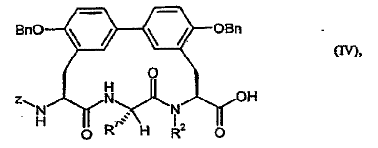 dans laquelle R² et R⁷ont la signification spécifiée dans la revendication 1 et Z signifie un benzyloxycarbonyle,
dans un procédé biétagé, d'abord en présence d'un ou de plusieurs réactifs déshydratants, avec un composé de la formule
H₂NR³ (III),
dans laquelle R³ a la signification spécifiée dans la revendication 1,
et ensuite avec un acide et/ou par hydrogénolyse.

8. Procédé pour la préparation d'un composé de la formule (I) selon la revendication 1 ou d'un de ses solvates, **caractérisé en ce qu'**un sel du composé ou un solvate d'un sel du composé est transformé en ledit composé par chromatographie moyennant l'addition d'une base.

9. Composé selon l'une quelconque des revendications 1 à 6 pour le traitement et/ou la prophylaxie de maladies.

10. Utilisation d'un composé selon l'une quelconque des revendications 1 à 6 pour la préparation d'un produit pharmaceutique pour le traitement et/ou la prophylaxie de maladies.

11. Utilisation d'un composé selon l'une quelconque des revendications 1 bis 6 pour la préparation d'un produit pharmaceutique pour le traitement et/ou la prophylaxie de maladies bactériennes.

12. ₋ Produit pharmaceutique contenant au moins un composé selon l'une quelque des revendications 1 à 6 en combinaison avec au moins un produit pharmaceutique auxiliaire approprié, inerte et non toxique.

13. Produit pharmaceutique selon la revendication 12 pour le traitement et/ou la prophylaxie d'infections bactériennes.

14. Utilisation d'une quantité antibactérienne efficace d'au moins un composé selon l'une quelque des revendications 1 à 6 ou d'un produit pharmaceutique selon la revendication 12 ou 13 pour la préparation d'un produit pharmaceutique pour la lutte contre les infections bactériennes chez l'homme et chez l'animal.
